# EUROPEAN PATENT APPLICATION

(11) **EP 4 317 126 A1**
(43) Date of publication of application: **07.02.2024**
(21) Application number: 22781029.8
(22) Date of filing: 30.03.2022
(51) Int. Cl.: C07C 211/61, C07D 333/76, C07D 307/91, H01L 51/50

(54) **COMPOUND, MATERIAL FOR ORGANIC ELECTROLUMINESCENT ELEMENTS, ORGANIC ELECTROLUMINESCENT ELEMENT, AND ELECTRONIC DEVICE**

(30) Priority: 31.03.2021 JP 2021062123
(71) Applicant: Idemitsu Kosan Co., Ltd, Tokyo 100-8321 (JP)
(72) Inventor: HAKETA, Tasuku, Tokyo 100-8321 (JP); TANAKA, Shota, Tokyo 100-8321 (JP); TAKAHASHI, Yusuke, Tokyo 100-8321 (JP); FUKAMI, Takuto, Tokyo 100-8321 (JP); SAWATO, Tsukasa, Tokyo 100-8321 (JP)
(74) Representative: Vossius & Partner Patentanwälte Rechtsanwälte mbB
(86) International application number: PCT/JP2022/015767
(87) International publication number: WO 2022/210818

(57) **Abstract**

A compound represented by the following formula (1) or (2) in which the symbols are as defined in the description, an organic electroluminescent device containing the compound, and an electronic device including the organic electroluminescent device.

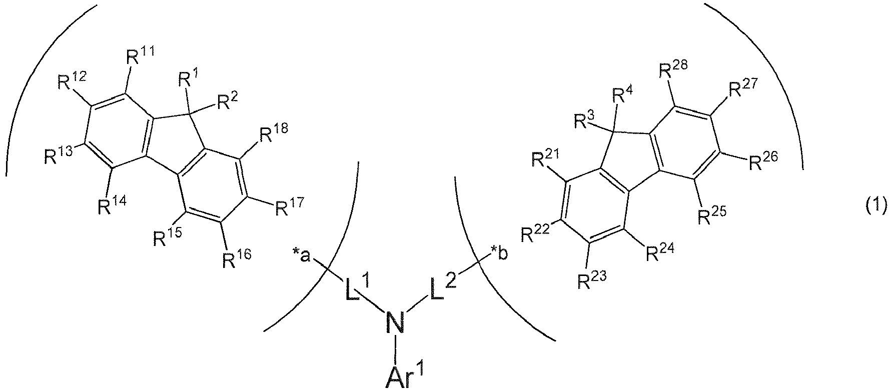

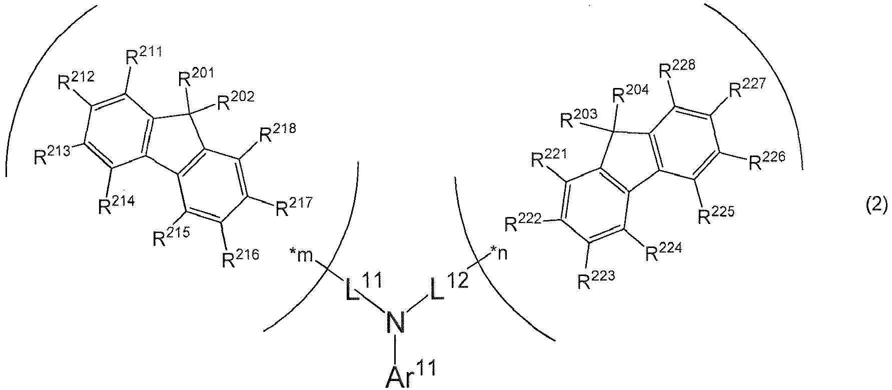

## Description

### Technical Field

The present invention relates to a compound, a material for an organic electroluminescent device, an organic electroluminescent device, and an electronic device including the organic electroluminescent device.

### Background Art

In general, an organic electroluminescent device (hereinafter, also referred to as an "organic EL device") includes an anode, a cathode, and an organic layer interposed between the anode and the cathode. When a voltage is applied between both electrodes, electrons and holes are injected into a light-emitting region, from the cathode side and the anode side, respectively. The injected electrons and holes recombine in the light-emitting region to form an excited state, and then emit light when the excited state is returned to a ground state. Therefore, development of a material that efficiently transports electrons or holes to the light-emitting region and facilitates recombination of the electrons and the holes is important in obtaining a high-performance organic EL device.

PTLs 1 to 4 disclose compounds used as materials for organic electroluminescent devices.

### Citation List

### Patent Literature

PTL 1: US 10840455 B
PTL 2: US 2019/0148650 A
PTL 3: WO 2019/139419
PTL 4: WO 2014/034795

### Summary of Invention

### Technical Problem

Although, many compounds for organic EL devices have been conventionally reported, there is still a need for compounds that further improve the performance of organic EL devices.

The present invention has been made to solve the above problems, and an object thereof is to provide a compound that further improves the performance of an organic EL device, an organic EL device having a further improved device performance, and an electronic device including such an organic EL device.

### Solution to Problem

The present inventors have repeatedly conducted intensive studies on the performance of organic EL devices containing compounds described in PTLs 1 to 4. As a result, they have found that an organic EL device containing a compound represented by the following formula (1), and an organic EL device containing a compound represented by the following formula (2) are more improved in performance.

In one embodiment, the present invention provides a compound represented by the following formula (1): 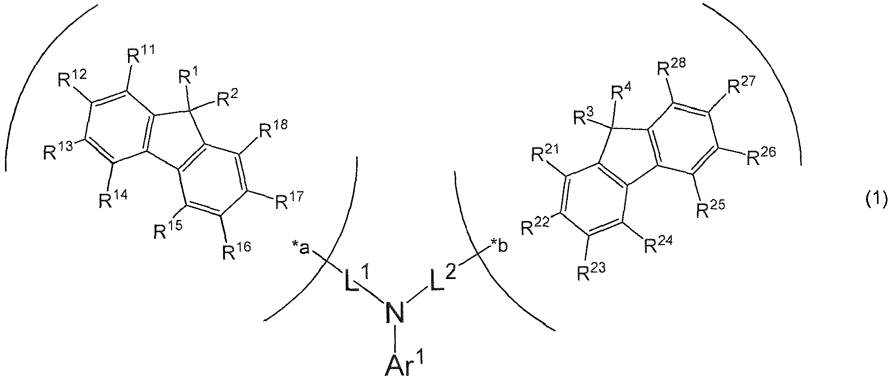
wherein in the formula (1),
one of R¹ and R² is a methyl group, and the other is an unsubstituted phenyl group, and
R¹ and R² are not bonded to each other and therefore do not form a ring structure.
one of R³ and R⁴ is a methyl group, and the other is an unsubstituted phenyl group, and
R³ and R⁴ are not bonded to each other and therefore do not form a ring structure.
R¹¹ to R¹⁸, and R²¹ to R²⁸ are hydrogen atoms.
provided that,
one selected from R¹¹ to R¹⁸ is a single bond bonded to *a, and
one selected from R²¹ to R²⁸ is a single bond bonded to *b.
L¹ and L² are each independently a single bond, or a substituted or unsubstituted phenylene group.
Ar¹ is a group represented by any one of formulas (1-1) to (1-6): 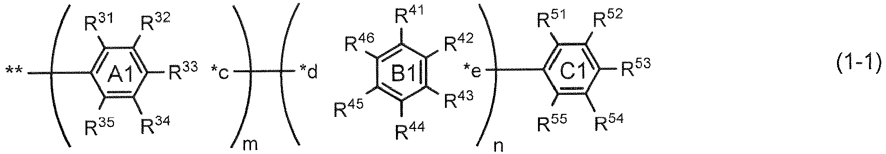 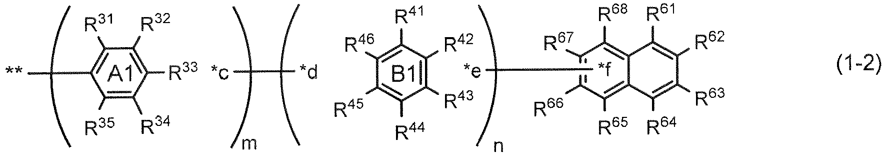 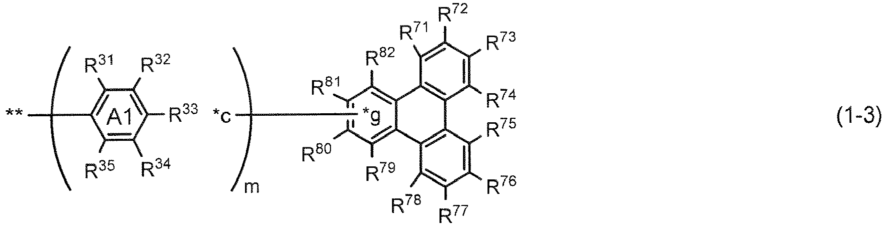 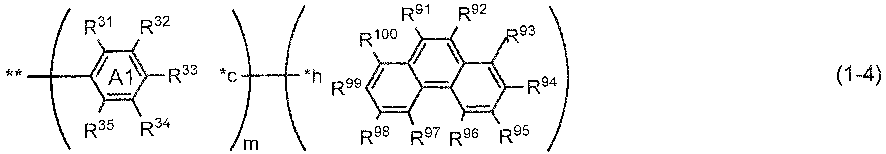 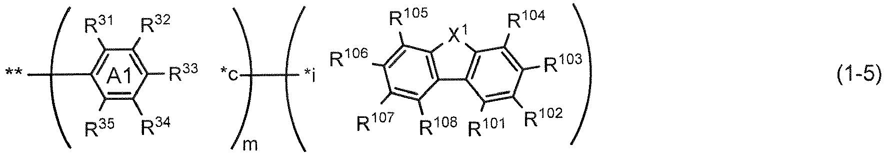 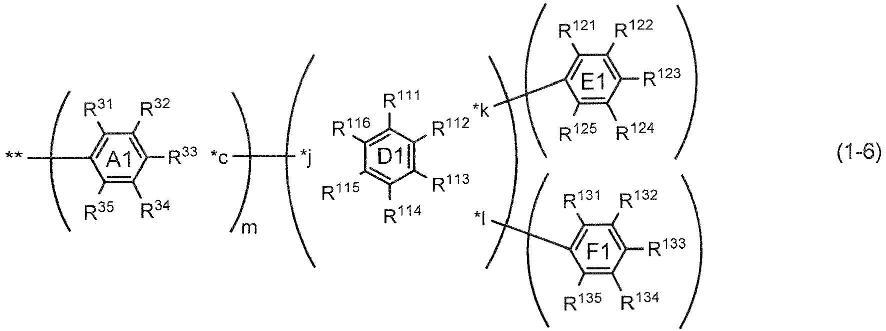
wherein
R³¹ to R³⁵, R⁴¹ to R⁴⁶, R⁵¹ to R⁵⁵, R⁶¹ to R⁶⁸, R⁷¹ to R⁸², R⁹¹ to R¹⁰⁰, R¹⁰¹ to R¹⁰⁸, R¹¹¹ to R¹¹⁶, R¹²¹ to R¹²⁵, and R¹³¹ to R¹³⁵ are each independently a hydrogen atom, a substituted or unsubstituted alkyl group having 1 to 50 carbon atoms, a substituted or unsubstituted cycloalkyl group having 3 to 50 ring carbon atoms, a halogen atom, a cyano group, a nitro group, a substituted or unsubstituted aryl group having 6 to 50 ring carbon atoms, or a substituted or unsubstituted heterocyclic group having 5 to 50 ring atoms;
X¹ is an oxygen atom, a sulfur atom, or CR^{a}R^{b};
R^{a} and R^{b} are each independently a hydrogen atom, a methyl group, or a substituted or unsubstituted phenyl group, and R^{a} and R^{b} may be bonded to each other to form a substituted or unsubstituted ring.
provided that,
one selected from R³¹ to R³⁵ is a single bond bonded to *c,
one selected from R⁴¹ to R⁴⁶ is a single bond bonded to *d, and the other one selected from R⁴¹ to R⁴⁶ is a single bond bonded to *e,
one selected from R⁶⁵ to R⁶⁸ is a single bond bonded to *f,
one selected from R⁷⁹ to R⁸² is a single bond bonded to *g,
one selected from R⁹¹, and R⁹⁷ to R¹⁰⁰ is a single bond bonded to *h,
one selected from R¹⁰⁵ to R¹⁰⁸ is a single bond bonded to *i,
one selected from R¹¹¹ to R¹¹⁶ is a single bond bonded to *j, the other one selected from R¹¹¹ to R¹¹⁶ is a single bond bonded to *k, and the other one selected from R¹¹¹ to R¹¹⁶ is a single bond bonded to *l,
adjacent two selected from R³¹ to R³⁵ that are not the single bond, adjacent two selected from R⁴¹ to R⁴⁶ that are not the single bond, adjacent two selected from R⁵¹ to R⁵⁵, adjacent two selected from R⁶¹ to R⁶⁸ that are not the single bond, adjacent two selected from R⁷¹ to R⁸² that are not the single bond, adjacent two selected from R⁹¹ to R¹⁰⁰ that are not the single bond, adjacent two selected from R¹⁰¹ to R¹⁰⁸ that are not the single bond, adjacent two selected from R¹¹¹ to R¹¹⁶ that are not the single bond, adjacent two selected from R¹²¹ to R¹²⁵, and adjacent two selected from R¹³¹ to R¹³⁵ are not bonded to each other and therefore do not form ring structures,
the benzene ring A1 and the benzene ring B1, the benzene ring A1 and the benzene ring C1, the benzene ring A1 and the benzene ring D1, the benzene ring A1 and the benzene ring E1, the benzene ring A1 and the benzene ring F1, the benzene ring B1 and the benzene ring C1, the benzene ring D1 and the benzene ring E1, and the benzene ring D1 and the benzene ring F1 are not crosslinked,
** represents a bonding position to the central nitrogen atom N,
m is 0 or 1, and n is 0 or 1,
in the formulas (1-1) and (1-2), when m is 0 and n is 0, *e is bonded to the central nitrogen atom N, when m is 0 and n is 1, *c is bonded to the central nitrogen atom N, and when m is 1 and n is 0, *e is bonded to one selected from R³¹ to R³⁵,
in the formulas (1-3) to (1-6), when m is 0, *c is bonded to the central nitrogen atom N, and
when R¹² or R¹⁷ is a single bond bonded to *a, and R²² or R²⁷ is a single bond bonded to *b, the formula (1-1) excludes a p-biphenyl group.

In another embodiment, the present invention provides a compound represented by the following formula (2): 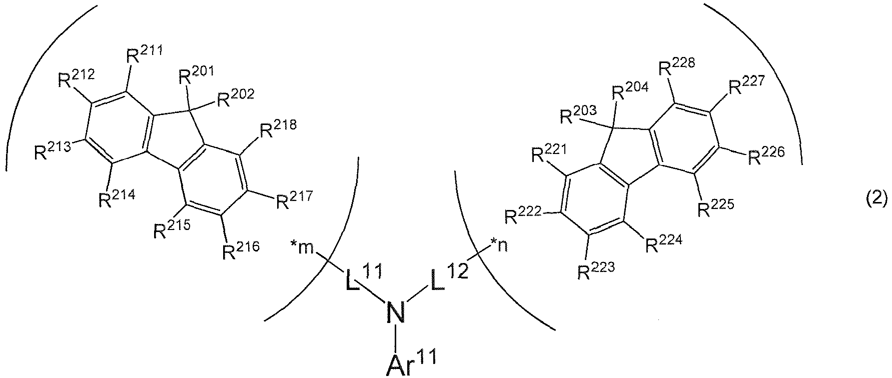 wherein
one selected from R²⁰¹ to R²⁰⁴ is a methyl group, and the other three selected from R²⁰¹ to R²⁰⁴ are unsubstituted phenyl groups,
R²⁰¹ and R²⁰² are not bonded to each other and therefore do not form a ring structure, and
R²⁰³ and R²⁰⁴ are not bonded to each other and therefore do not form a ring structure.
R²¹¹ to R²¹⁸, and R²²¹ to R²²⁸ are hydrogen atoms.
provided that,
one selected from R²¹¹ to R²¹⁸ is a single bond bonded to *m, and
one selected from R²²¹ to R²²⁸ is a single bond bonded to *n.
L¹¹ and L¹² are each independently a single bond, or a substituted or unsubstituted phenylene group.
Ar¹¹ is a group represented by any one of formulas (2-1) to (2-6): 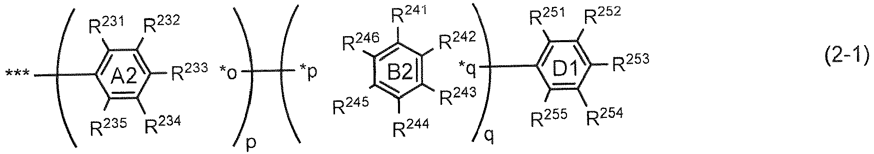 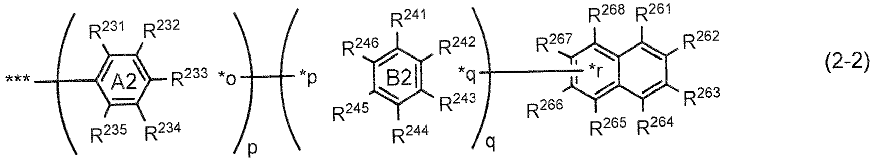 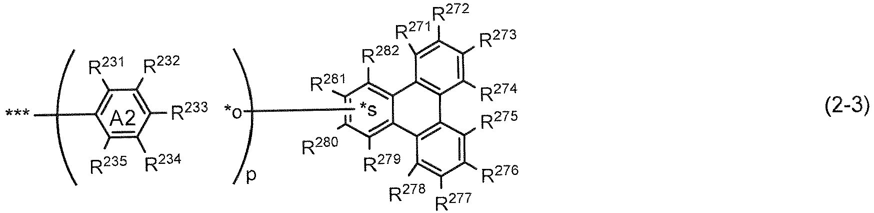 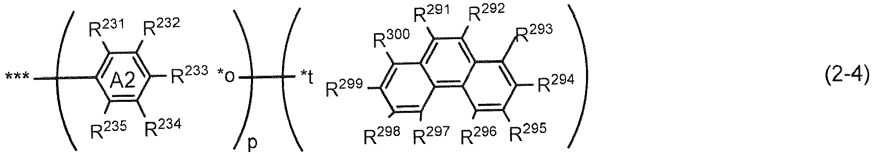 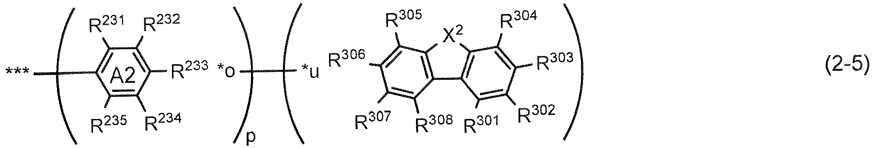 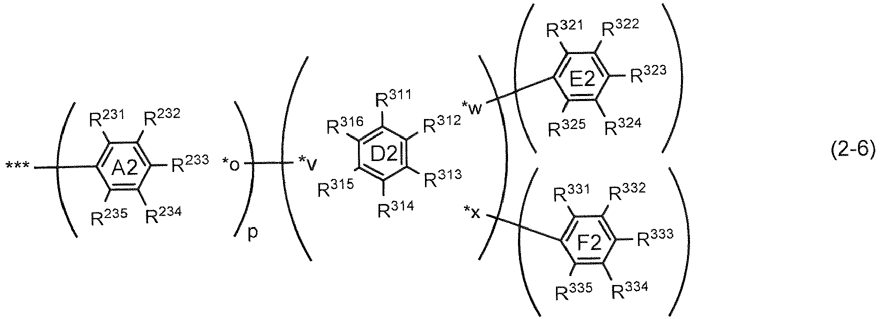
wherein
R²³¹ to R²³⁵, R²⁴¹ to R²⁴⁶, R²⁵¹ to R²⁵⁵, R²⁶¹ to R²⁶⁸, R²⁷¹ to R²⁸², R²⁹¹ to R³⁰⁰, R³⁰¹ to R³⁰⁸, R³¹¹ to R³¹⁶, R³²¹ to R³²⁵, and R³³¹ to R³³⁵ are each independently a hydrogen atom, a substituted or unsubstituted alkyl group having 1 to 50 carbon atoms, a substituted or unsubstituted cycloalkyl group having 3 to 50 ring carbon atoms, a halogen atom, a cyano group, a nitro group, a substituted or unsubstituted aryl group having 6 to 50 ring carbon atoms, or a substituted or unsubstituted heterocyclic group having 5 to 50 ring atoms;
X² is an oxygen atom, a sulfur atom, or CR^{c}R^{d}; and
R^{c} and R^{d} are each independently a hydrogen atom, or a methyl group, and R^{c} and R^{d} are not bonded to each other and therefore do not form a ring structure.
provided that,
one selected from R²³¹ to R²³⁵ is a single bond bonded to *o,
one selected from R²⁴¹ to R²⁴⁶ is a single bond bonded to *p, and the other one selected from R²⁴¹ to R²⁴⁶ is a single bond bonded to *q,
one selected from R²⁶⁵ to R²⁶⁸ is a single bond bonded to *r,
one selected from R²⁷⁹ to R²⁸² is a single bond bonded to *s,
one selected from R²⁹¹, and R²⁹⁷ to R³⁰⁰ is a single bond bonded to *t,
one selected from R³⁰⁵ to R³⁰⁸ is a single bond bonded to *u,
one selected from R³¹¹ to R³¹⁶ is a single bond bonded to *v, the other one selected from R³¹¹ to R³¹⁶ is a single bond bonded to *w, and the other one selected from R³¹¹ to R³¹⁶ is a single bond bonded to *x,
adjacent two selected from R²³¹ to R²³⁵ that are not the single bond, adjacent two selected from R²⁴¹ to R²⁴⁶ that are not the single bond, adjacent two selected from R²⁵¹ to R²⁵⁵, adjacent two selected from R²⁶¹ to R²⁶⁸ that are not the single bond, adjacent two selected from R²⁷¹ to R²⁸² that are not the single bond, adjacent two selected from R²⁹¹ to R³⁰⁰ that are not the single bond, adjacent two selected from R³⁰¹ to R³⁰⁸ that are not the single bond, adjacent two selected from R³¹¹ to R³¹⁶ that are not the single bond, adjacent two selected from R³²¹ to R³²⁵, and adjacent two selected from R³³¹ to R³³⁵ are not bonded to each other and therefore do not form ring structures,
the benzene ring A2 and the benzene ring B2, the benzene ring A2 and the benzene ring C2, the benzene ring A2 and the benzene ring D2, the benzene ring A2 and the benzene ring E2, the benzene ring A2 and the benzene ring F2, the benzene ring B2 and the benzene ring C2, the benzene ring D2 and the benzene ring E2, and the benzene ring D2 and the benzene ring F2 are not crosslinked,
*** represents a bonding position to the central nitrogen atom N,
p is 0 or 1, and q is 0 or 1;
in the formulas (2-1) and (2-2), when p is 0 and q is 0, *q is bonded to the central nitrogen atom N, when p is 0 and q is 1, *o is bonded to the central nitrogen atom N, and when p is 1 and q is 0, *q is bonded to one selected from R²³¹ to R²³⁵,
in the formulas (2-3) to (2-6), when p is 0, *o is bonded to the central nitrogen atom N,
in the formula (2-5), when X² is CR^{c}R^{d} and p is 0, at least one selected from L¹¹ and L¹² is a substituted or unsubstituted phenylene group,
in the formula (2-5), when X² is an oxygen atom and p is 1, one selected from R³⁰⁶ to R³⁰⁸ is a single bond bonded to *u, and
when R²¹² or R²¹⁷ is a single bond bonded to *m, and R²²² or R²²⁷ is a single bond bonded to *n, the formula (2-1) excludes a p-biphenyl group.

In still another embodiment, the present invention provides a material for an organic electroluminescent device, which includes at least one selected from the compound represented by the formula (1), and the compound represented by the formula (2).

In still another embodiment, the present invention provides an organic electroluminescent device including a cathode, an anode, and organic layers between the cathode and the anode. In the organic electroluminescent device, the organic layers include a light emitting layer, and at least one layer of the organic layers contains at least one selected from the compound represented by the formula (1) and the compound represented by the formula (2).

In still another embodiment, the present invention provides an electronic device including the organic electroluminescent device.

### Advantageous Effects of Invention

The organic EL device containing the compound represented by the formula (1) exhibits improved device performance. Further, the organic EL device containing the compound represented by the formula (2) exhibits improved device performance.

### Brief Description of Drawings

FIG. 1 is a schematic view illustrating an example of a layer configuration of an organic EL device according to one embodiment of the present invention.
FIG. 2 is a schematic view illustrating another example of the layer configuration of the organic EL device according to one embodiment of the present invention.
FIG. 3 is a schematic view illustrating a still further example of the layer configuration of the organic EL device according to one embodiment of the present invention.

### Description of Embodiments

### [Definitions]

In the description herein, the hydrogen atom encompasses isotopes thereof having different numbers of neutrons, i.e., a light hydrogen atom (protium), a heavy hydrogen atom (deuterium), and tritium.

In the description herein, the bonding site where the symbol, such as "R", or "D" representing a deuterium atom is not shown is assumed to have a hydrogen atom, i.e., a protium atom, a deuterium atom, or a tritium atom, bonded thereto.

In the description herein, the number of ring carbon atoms shows the number of carbon atoms among the atoms constituting the ring itself of a compound having a structure including atoms bonded to form a ring (such as a monocyclic compound, a condensed ring compound, a bridged compound, a carbocyclic compound, and a heterocyclic compound). In the case where the ring is substituted by a substituent, the carbon atom contained in the substituent is not included in the number of ring carbon atoms. The same definition is applied to the "number of ring carbon atoms" described hereinafter unless otherwise indicated. For example, a benzene ring has 6 ring carbon atoms, a naphthalene ring has 10 ring carbon atoms, a pyridine ring has 5 ring carbon atoms, and a furan ring has 4 ring carbon atoms. For example, 9,9-diphenylfluorenyl group has 13 ring carbon atoms, and 9,9'-spirobifluorenyl group has 25 ring carbon atoms.

In the case where a benzene ring has, for example, an alkyl group substituted thereon as a substituent, the number of carbon atoms of the alkyl group is not included in the number of ring carbon atoms of the benzene ring. Accordingly, a benzene ring having an alkyl group substituted thereon has 6 ring carbon atoms. In the case where a naphthalene ring has, for example, an alkyl group substituted thereon as a substituent, the number of carbon atoms of the alkyl group is not included in the number of ring carbon atoms of the naphthalene ring. Accordingly, a naphthalene ring having an alkyl group substituted thereon has 10 ring carbon atoms.

In the description herein, the number of ring atoms shows the number of atoms constituting the ring itself of a compound having a structure including atoms bonded to form a ring (such as a monocyclic ring, a condensed ring, and a set of rings) (such as a monocyclic compound, a condensed ring compound, a bridged compound, a carbocyclic compound, and a heterocyclic compound). The atom that does not constitute the ring (such as a hydrogen atom terminating the bond of the atom constituting the ring) and, in the case where the ring is substituted by a substituent, the atom contained in the substituent are not included in the number of ring atoms. The same definition is applied to the "number of ring atoms" described hereinafter unless otherwise indicated. For example, a pyridine ring has 6 ring atoms, a quinazoline ring has 10 ring atoms, and a furan ring has 5 ring atoms. For example, the number of hydrogen atoms bonded to a pyridine ring or atoms constituting a substituent is not included in the number of ring atoms of the pyridine ring. Accordingly, a pyridine ring having a hydrogen atom or a substituent bonded thereto has 6 ring atoms. For example, the number of hydrogen atoms bonded to carbon atoms of a quinazoline ring or atoms constituting a substituent is not included in the number of ring atoms of the quinazoline ring. Accordingly, a quinazoline ring having a hydrogen atom or a substituent bonded thereto has 10 ring atoms.

In the description herein, the expression "having XX to YY carbon atoms" in the expression "substituted or unsubstituted ZZ group having XX to YY carbon atoms" means the number of carbon atoms of the unsubstituted ZZ group, and, in the case where the ZZ group is substituted, the number of carbon atoms of the substituent is not included. Herein, "YY" is larger than "XX", "XX" represents an integer of 1 or more, and "YY" represents an integer of 2 or more.

In the description herein, the expression "having XX to YY atoms" in the expression "substituted or unsubstituted ZZ group having XX to YY atoms" means the number of atoms of the unsubstituted ZZ group, and, in the case where the ZZ group is substituted, the number of atoms of the substituent is not included. Herein, "YY" is larger than "XX", "XX" represents an integer of 1 or more, and "YY" represents an integer of 2 or more.

In the description herein, an unsubstituted ZZ group means the case where the "substituted or unsubstituted ZZ group" is an "unsubstituted ZZ group", and a substituted ZZ group means the case where the "substituted or unsubstituted ZZ group" is a "substituted ZZ group".

In the description herein, the expression "unsubstituted" in the expression "substituted or unsubstituted ZZ group" means that hydrogen atoms in the ZZ group are not substituted by a substituent. The hydrogen atoms in the "unsubstituted ZZ group" each are a protium atom, a deuterium atom, or a tritium atom.

In the description herein, the expression "substituted" in the expression "substituted or unsubstituted ZZ group" means that one or more hydrogen atom in the ZZ group is substituted by a substituent. The expression "substituted" in the expression "BB group substituted by an AA group" similarly means that one or more hydrogen atom in the BB group is substituted by the AA group.

### Substituents in Description

The substituents described in the description herein will be explained. Unless otherwise stated, each substituent described in this specification is defined as follows.

In the description herein, the number of ring carbon atoms of the "unsubstituted aryl group" is 6 to 50, preferably 6 to 30, and more preferably 6 to 18, unless otherwise indicated in the description.

In the description herein, the number of ring atoms of the "unsubstituted heterocyclic group" is 5 to 50, preferably 5 to 30, and more preferably 5 to 18, unless otherwise indicated in the description.

In the description herein, the number of carbon atoms of the "unsubstituted alkyl group" is 1 to 50, preferably 1 to 20, and more preferably 1 to 6, unless otherwise indicated in the description.

In the description herein, the number of carbon atoms of the "unsubstituted alkenyl group" is 2 to 50, preferably 2 to 20, and more preferably 2 to 6, unless otherwise indicated in the description.

In the description herein, the number of carbon atoms of the "unsubstituted alkynyl group" is 2 to 50, preferably 2 to 20, and more preferably 2 to 6, unless otherwise indicated in the description.

In the description herein, the number of ring carbon atoms of the "unsubstituted cycloalkyl group" is 3 to 50, preferably 3 to 20, and more preferably 3 to 6, unless otherwise indicated in the description.

In the description herein, the number of ring carbon atoms of the "unsubstituted arylene group" is 6 to 50, preferably 6 to 30, and more preferably 6 to 18, unless otherwise indicated in the description.

In the description herein, the number of ring atoms of the "unsubstituted divalent heterocyclic group" is 5 to 50, preferably 5 to 30, and more preferably 5 to 18, unless otherwise indicated in the description.

In the description herein, the number of carbon atoms of the "unsubstituted alkylene group" is 1 to 50, preferably 1 to 20, and more preferably 1 to 6, unless otherwise indicated in the description.

### Substituted or Unsubstituted Aryl Group

In the description herein, specific examples (set of specific examples G1) of the "substituted or unsubstituted aryl group" include the unsubstituted aryl groups (set of specific examples G1A) and the substituted aryl groups (set of specific examples G1B) shown below. (Herein, the unsubstituted aryl group means the case where the "substituted or unsubstituted aryl group" is an "unsubstituted aryl group", and the substituted aryl group means the case where the "substituted or unsubstituted aryl group" is a "substituted aryl group".) In the description herein, the simple expression "aryl group" encompasses both the "unsubstituted aryl group" and the "substituted aryl group".

The "substituted aryl group" means a group formed by substituting one or more hydrogen atom of the "unsubstituted aryl group" by a substituent. Examples of the "substituted aryl group" include groups formed by one or more hydrogen atom of each of the "unsubstituted aryl groups" in the set of specific examples G1A by a substituent, and the examples of the substituted aryl groups in the set of specific examples G1B. The examples of the "unsubstituted aryl group" and the examples of the "substituted aryl group" enumerated herein are mere examples, and the "substituted aryl group" in the description herein encompasses groups formed by substituting a hydrogen atom bonded to the carbon atom of the aryl group itself of each of the "substituted aryl groups" in the set of specific examples G1B by a substituent, and groups formed by substituting a hydrogen atom of the substituent of each of the "substituted aryl groups" in the set of specific examples G1B by a substituent.

Unsubstituted Aryl Group (Set of Specific Examples G1A):
   a phenyl group,
   a p-biphenyl group,
   a m-biphenyl group,
   an o-biphenyl group,
   a p-terphenyl-4-yl group,
   a p-terphenyl-3-yl group,
   a p-terphenyl-2-yl group,
   a m-terphenyl-4-yl group,
   a m-terphenyl-3-yl group,
   a m-terphenyl-2-yl group,
   an o-terphenyl-4-yl group,
   an o-terphenyl-3-yl group,
   an o-terphenyl-2-yl group,
   a 1-naphthyl group,
   a 2-naphthyl group,
   an anthryl group,
   a benzanthryl group,
   a phenanthryl group,
   a benzophenanthryl group,
   a phenarenyl group,
   a pyrenyl group,
   a chrysenyl group,
   a benzochrysenyl group,
   a triphenylenyl group,
   a benzotriphenylenyl group,
   a tetracenyl group,
   a pentacenyl group,
   a fluorenyl group,
   a 9,9'-spirobifluorenyl group,
   a benzofluorenyl group,
   a dibenzofluorenyl group,
   a fluoranthenyl group,
   a benzofluoranthenyl group,
   a perylenyl group, and
   monovalent aryl groups derived by removing one hydrogen atom from each of the ring structures represented by the following general formulae (TEMP-1) to (TEMP-15): 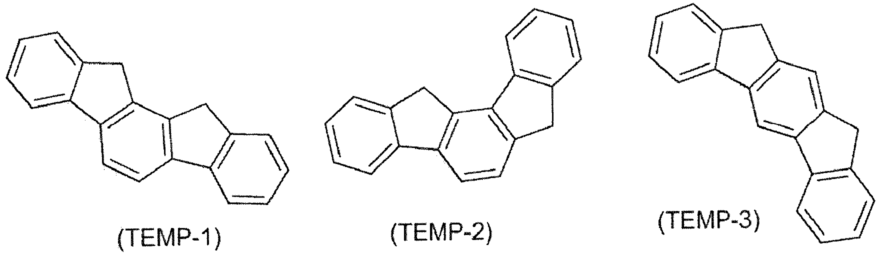 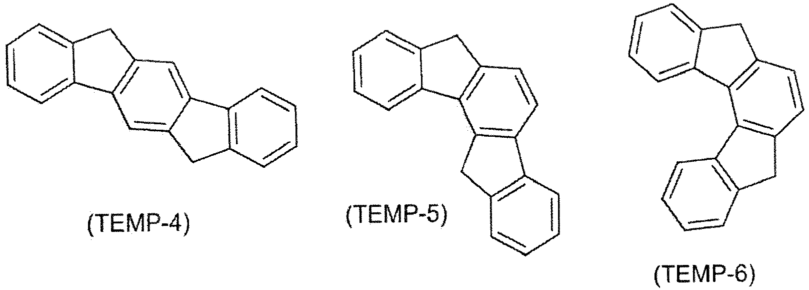 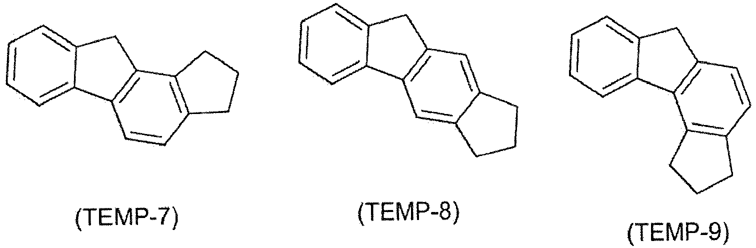 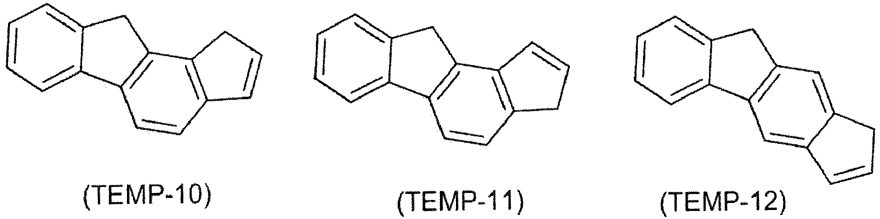 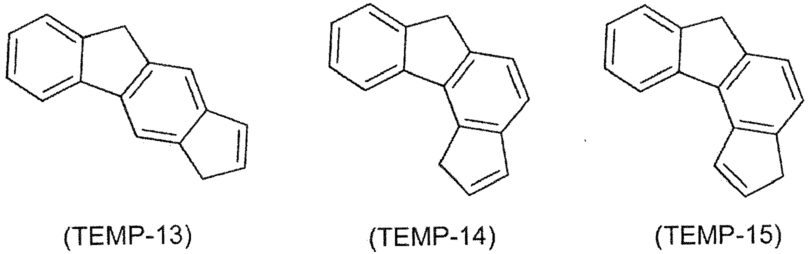
Substituted Aryl Group (Set of Specific Examples G1B):
   an o-tolyl group,
   a m-tolyl group,
   a p-tolyl group,
   a p-xylyl group,
   a m-xylyl group,
   an o-xylyl group,
   a p-isopropylphenyl group,
   a m-isopropylphenyl group,
   an o-isopropylphenyl group,
   a p-t-butylphenyl group,
   a m-t-butylphenyl group,
   a o-t-butylphenyl group,
   a 3,4,5-trimethylphenyl group,
   a 9,9-dimethylfluorenyl group,
   a 9,9-diphenylfluorenyl group,
   a 9,9-bis(4-methylphenyl)fluorenyl group,
   a 9,9-bis(4-isopropylphenyl)fluorenyl group,
   a 9,9-bis(4-t-butylphenyl)fluorenyl group,
   a cyanophenyl group,
   a triphenylsilylphenyl group,
   a trimethylsilylphenyl group,
   a phenylnaphthyl group,
   a naphthylphenyl group, and
   groups formed by substituting one or more hydrogen atom of each of monovalent aryl groups derived from the ring structures represented by the general formulae (TEMP-1) to (TEMP-15) by a substituent.

### Substituted or Unsubstituted Heterocyclic Group

In the description herein, the "heterocyclic group" means a cyclic group containing at least one hetero atom in the ring atoms. Specific examples of the hetero atom include a nitrogen atom, an oxygen atom, a sulfur atom, a silicon atom, a phosphorus atom, and a boron atom. In the description herein, the "heterocyclic group" is a monocyclic group or a condensed ring group.

In the description herein, the "heterocyclic group" is an aromatic heterocyclic group or a non-aromatic heterocyclic group.

In the description herein, specific examples (set of specific examples G2) of the "substituted or unsubstituted heterocyclic group" include the unsubstituted heterocyclic groups (set of specific examples G2A) and the substituted heterocyclic groups (set of specific examples G2B) shown below. (Herein, the unsubstituted heterocyclic group means the case where the "substituted or unsubstituted heterocyclic group" is an "unsubstituted heterocyclic group", and the substituted heterocyclic group means the case where the "substituted or unsubstituted heterocyclic group" is a "substituted heterocyclic group".) In the description herein, the simple expression "heterocyclic group" encompasses both the "unsubstituted heterocyclic group" and the "substituted heterocyclic group".

The "substituted heterocyclic group" means a group formed by substituting one or more hydrogen atom of the "unsubstituted heterocyclic group" by a substituent. Specific examples of the "substituted heterocyclic group" include groups formed by substituting a hydrogen atom of each of the "unsubstituted heterocyclic groups" in the set of specific examples G2A by a substituent, and the examples of the substituted heterocyclic groups in the set of specific examples G2B. The examples of the "unsubstituted heterocyclic group" and the examples of the "substituted heterocyclic group" enumerated herein are mere examples, and the "substituted heterocyclic group" in the description herein encompasses groups formed by substituting a hydrogen atom bonded to the ring atom of the heterocyclic group itself of each of the "substituted heterocyclic groups" in the set of specific examples G2B by a substituent, and groups formed by substituting a hydrogen atom of the substituent of each of the "substituted heterocyclic groups" in the set of specific examples G2B by a substituent.

The set of specific examples G2A includes, for example, the unsubstituted heterocyclic group containing a nitrogen atom (set of specific examples G2A1), the unsubstituted heterocyclic group containing an oxygen atom (set of specific examples G2A2), the unsubstituted heterocyclic group containing a sulfur atom (set of specific examples G2A3), and monovalent heterocyclic groups derived by removing one hydrogen atom from each of the ring structures represented by the following general formulae (TEMP-16) to (TEMP-33) (set of specific examples G2A4).

The set of specific examples G2B includes, for example, the substituted heterocyclic groups containing a nitrogen atom (set of specific examples G2B1), the substituted heterocyclic groups containing an oxygen atom (set of specific examples G2B2), the substituted heterocyclic groups containing a sulfur atom (set of specific examples G2B3), and groups formed by substituting one or more hydrogen atom of each of monovalent heterocyclic groups derived from the ring structures represented by the following general formulae (TEMP-16) to (TEMP-33) by a substituent (set of specific examples G2B4).

Unsubstituted Heterocyclic Group containing Nitrogen Atom (Set of Specific Examples G2A1):
   a pyrrolyl group,
   an imidazolyl group,
   a pyrazolyl group,
   a triazolyl group,
   a tetrazolyl group,
   an oxazolyl group,
   an isoxazolyl group,
   an oxadiazolyl group,
   a thiazolyl group,
   an isothiazolyl group,
   a thiadiazolyl group,
   a pyridyl group,
   a pyridazinyl group,
   a pyrimidinyl group,
   a pyrazinyl group,
   a triazinyl group,
   an indolyl group,
   an isoindolyl group,
   an indolizinyl group,
   a quinolizinyl group,
   a quinolyl group,
   an isoquinolyl group,
   a cinnolinyl group,
   a phthalazinyl group,
   a quinazolinyl group,
   a quinoxalinyl group,
   a benzimidazolyl group,
   an indazolyl group,
   a phenanthrolinyl group,
   a phenanthridinyl group,
   an acridinyl group,
   a phenazinyl group,
   a carbazolyl group,
   a benzocarbazolyl group,
   a morpholino group,
   a phenoxazinyl group,
   a phenothiazinyl group,
   an azacarbazolyl group, and
   a diazacarbazolyl group.
Unsubstituted Heterocyclic Group containing Oxygen Atom (Set of Specific Examples G2A2):
   a furyl group,
   an oxazolyl group,
   an isoxazolyl group,
   an oxadiazolyl group,
   a xanthenyl group,
   a benzofuranyl group,
   an isobenzofuranyl group,
   a dibenzofuranyl group,
   a naphthobenzofuranyl group,
   a benzoxazolyl group,
   a benzisoxazolyl group,
   a phenoxazinyl group,
   a morpholino group,
   a dinaphthofuranyl group,
   an azadibenzofuranyl group,
   a diazadibenzofuranyl group,
   an azanaphthobenzofuranyl group, and
   a diazanaphthobenzofuranyl group.
Unsubstituted Heterocyclic Group containing Sulfur Atom (Set of Specific Examples G2A3):
   a thienyl group,
   a thiazolyl group,
   an isothiazolyl group,
   a thiadiazolyl group,
   a benzothiophenyl group (benzothienyl group),
   an isobenzothiophenyl group (isobenzothienyl group),
   a dibenzothiophenyl group (dibenzothienyl group),
   a naphthobenzothiophenyl group (naphthobenzothienyl group),
   a benzothiazolyl group,
   a benzisothiazolyl group,
   a phenothiazinyl group,
   a dinaphthothiophenyl group (dinaphthothienyl group),
   an azadibenzothiophenyl group (azadibenzothienyl group),
   a diazadibenzothiophenyl group (diazadibenzothienyl group),
   an azanaphthobenzothiophenyl group (azanaphthobenzothienyl group), and
   a diazanaphthobenzothiophenyl group (diazanaphthobenzothienyl group).
Monovalent Heterocyclic Group derived by removing One Hydrogen Atom from Ring Structures represented by General Formulae (TEMP-16) to (TEMP-33) (Set of Specific Examples G2A4)

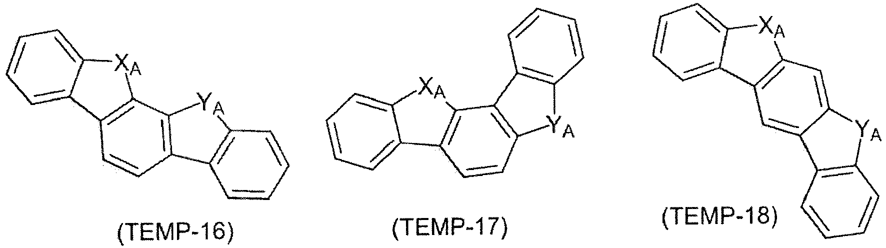

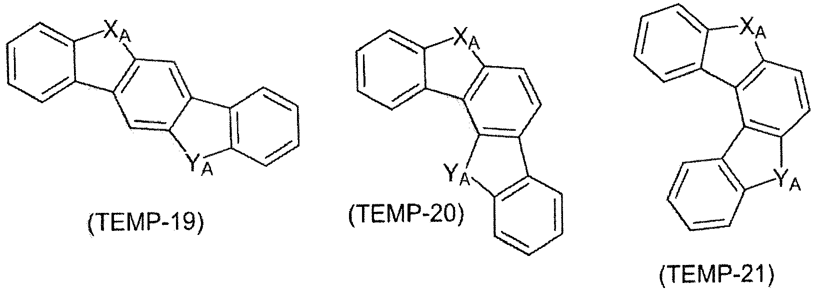

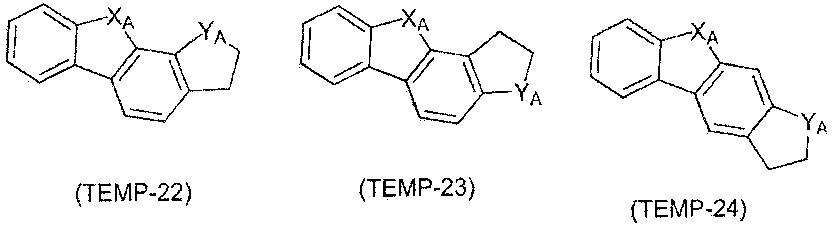

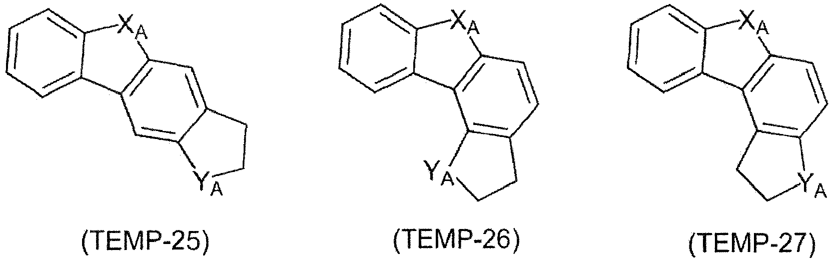

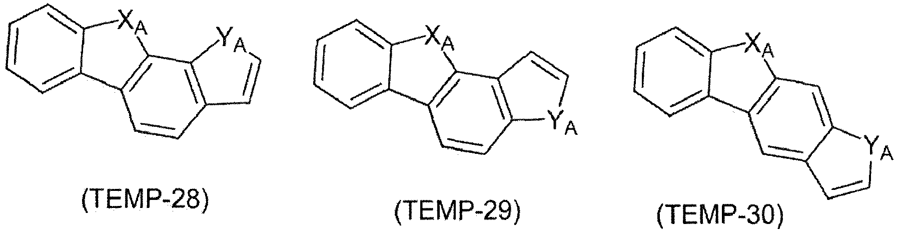

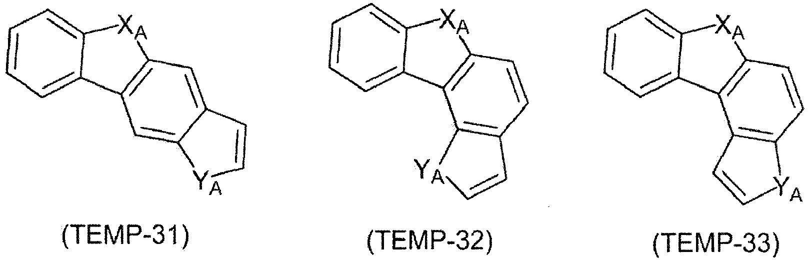

In the general formulae (TEMP-16) to (TEMP-33), X_{A} and Y_{A} each independently represent an oxygen atom, a sulfur atom, NH, or CH₂, provided that at least one of X_{A} and Y_{A} represents an oxygen atom, a sulfur atom, or NH.

In the general formulae (TEMP-16) to (TEMP-33), in the case where at least one of X_{A} and Y_{A} represents NH or CH₂, the monovalent heterocyclic groups derived from the ring structures represented by the general formulae (TEMP-16) to (TEMP-33) include monovalent groups formed by removing one hydrogen atom from the NH or CH₂.

Substituted Heterocyclic Group containing Nitrogen Atom (Set of Specific Examples G2B1):
   a (9-phenyl)carbazolyl group,
   a (9-biphenylyl)carbazolyl group,
   a (9-phenyl)phenylcarbazolyl group,
   a (9-naphthyl)carbazolyl group,
   a diphenylcarbazol-9-yl group,
   a phenylcarbazol-9-yl group,
   a methylbenzimidazolyl group,
   an ethylbenzimidazolyl group,
   a phenyltriazinyl group,
   a biphenyltriazinyl group,
   a diphenyltriazinyl group,
   a phenylquinazolinyl group, and
   a biphenylquinazolinyl group.
Substituted Heterocyclic Group containing Oxygen Atom (Set of Specific Examples G2B2):
   a phenyldibenzofuranyl group,
   a methyldibenzofuranyl group,
   a t-butyldibenzofuranyl group, and
   a monovalent residual group of spiro[9H-xanthene-9,9'-[9H]fluorene].
Substituted Heterocyclic Group containing Sulfur Atom (Set of Specific Examples G2B3):
   a phenyldibenzothiophenyl group,
   a methyldibenzothiophenyl group,
   a t-butyldibenzothiophenyl group, and
   a monovalent residual group of spiro[9H-thioxanthene-9,9'-[9H]fluorene].
Group formed by substituting one or more Hydrogen Atom of Monovalent Heterocyclic Group derived from Ring Structures represented by General Formulae (TEMP-16) to (TEMP-33) by Substituent (Set of Specific Examples G2B4)

The "one or more hydrogen atom of the monovalent heterocyclic group" means one or more hydrogen atom selected from the hydrogen atom bonded to the ring carbon atom of the monovalent heterocyclic group, the hydrogen atom bonded to the nitrogen atom in the case where at least one of X_{A} and Y_{A} represents NH, and the hydrogen atom of the methylene group in the case where one of X_{A} and Y_{A} represents CH₂.

### Substituted or Unsubstituted Alkyl Group

In the description herein, specific examples (set of specific examples G3) of the "substituted or unsubstituted alkyl group" include the unsubstituted alkyl groups (set of specific examples G3A) and the substituted alkyl groups (set of specific examples G3B) shown below. (Herein, the unsubstituted alkyl group means the case where the "substituted or unsubstituted alkyl group" is an "unsubstituted alkyl group", and the substituted alkyl group means the case where the "substituted or unsubstituted alkyl group" is a "substituted alkyl group".) In the description herein, the simple expression "alkyl group" encompasses both the "unsubstituted alkyl group" and the "substituted alkyl group".

The "substituted alkyl group" means a group formed by substituting one or more hydrogen atom of the "unsubstituted alkyl group" by a substituent. Specific examples of the "substituted alkyl group" include groups formed by substituting one or more hydrogen atom of each of the "unsubstituted alkyl groups" (set of specific examples G3A) by a substituent, and the examples of the substituted alkyl groups (set of specific examples G3B). In the description herein, the alkyl group in the "unsubstituted alkyl group" means a chain-like alkyl group. Accordingly, the "unsubstituted alkyl group" encompasses an "unsubstituted linear alkyl group" and an "unsubstituted branched alkyl group". The examples of the "unsubstituted alkyl group" and the examples of the "substituted alkyl group" enumerated herein are mere examples, and the "substituted alkyl group" in the description herein encompasses groups formed by substituting a hydrogen atom of the alkyl group itself of each of the "substituted alkyl groups" in the set of specific examples G3B by a substituent, and groups formed by substituting a hydrogen atom of the substituent of each of the "substituted alkyl groups" in the set of specific examples G3B by a substituent.

Unsubstituted Alkyl Group (Set of Specific Examples G3A):
   a methyl group,
   an ethyl group,
   a n-propyl group,
   an isopropyl group,
   a n-butyl group,
   an isobutyl group,
   a s-butyl group, and
   a t-butyl group.
Substituted Alkyl Group (Set of Specific Examples G3B):
   a heptafluoropropyl group (including isomers),
   a pentafluoroethyl group,
   a 2,2,2-trifluoroethyl group, and
   a trifluoromethyl group.

### Substituted or Unsubstituted Alkenyl Group

In the description herein, specific examples (set of specific examples G4) of the "substituted or unsubstituted alkenyl group" include the unsubstituted alkenyl groups (set of specific examples G4A) and the substituted alkenyl groups (set of specific examples G4B) shown below. (Herein, the unsubstituted alkenyl group means the case where the "substituted or unsubstituted alkenyl group" is an "unsubstituted alkenyl group", and the substituted alkenyl group means the case where the "substituted or unsubstituted alkenyl group" is a "substituted alkenyl group".) In the description herein, the simple expression "alkenyl group" encompasses both the "unsubstituted alkenyl group" and the "substituted alkenyl group".

The "substituted alkenyl group" means a group formed by substituting one or more hydrogen atom of the "unsubstituted alkenyl group" by a substituent. Specific examples of the "substituted alkenyl group" include the "unsubstituted alkenyl groups" (set of specific examples G4A) that each have a substituent, and the examples of the substituted alkenyl groups (set of specific examples G4B). The examples of the "unsubstituted alkenyl group" and the examples of the "substituted alkenyl group" enumerated herein are mere examples, and the "substituted alkenyl group" in the description herein encompasses groups formed by substituting a hydrogen atom of the alkenyl group itself of each of the "substituted alkenyl groups" in the set of specific examples G4B by a substituent, and groups formed by substituting a hydrogen atom of the substituent of each of the "substituted alkenyl groups" in the set of specific examples G4B by a substituent.

Unsubstituted Alkenyl Group (Set of Specific Examples G4A):
   a vinyl group,
   an allyl group,
   a 1-butenyl group,
   a 2-butenyl group, and
   a 3-butenyl group.
Substituted Alkenyl Group (Set of Specific Examples G4B):
   a 1,3-butanedienyl group,
   a 1-methylvinyl group,
   a 1-methylallyl group,
   a 1,1-dimethylallyl group,
   a 2-methylallyl group, and
   a 1,2-dimethylallyl group.

### Substituted or Unsubstituted Alkynyl Group

In the description herein, specific examples (set of specific examples G5) of the "substituted or unsubstituted alkynyl group" include the unsubstituted alkynyl group (set of specific examples G5A) shown below. (Herein, the unsubstituted alkynyl group means the case where the "substituted or unsubstituted alkynyl group" is an "unsubstituted alkynyl group".) In the description herein, the simple expression "alkynyl group" encompasses both the "unsubstituted alkynyl group" and the "substituted alkynyl group".

The "substituted alkynyl group" means a group formed by substituting one or more hydrogen atom of the "unsubstituted alkynyl group" by a substituent. Specific examples of the "substituted alkenyl group" include groups formed by substituting one or more hydrogen atom of the "unsubstituted alkynyl group" (set of specific examples G5A) by a substituent.

Unsubstituted Alkynyl Group (Set of Specific Examples G5A):
   an ethynyl group.
Substituted or Unsubstituted Cycloalkyl Group

In the description herein, specific examples (set of specific examples G6) of the "substituted or unsubstituted cycloalkyl group" include the unsubstituted cycloalkyl groups (set of specific examples G6A) and the substituted cycloalkyl group (set of specific examples G6B) shown below. (Herein, the unsubstituted cycloalkyl group means the case where the "substituted or unsubstituted cycloalkyl group" is an "unsubstituted cycloalkyl group", and the substituted cycloalkyl group means the case where the "substituted or unsubstituted cycloalkyl group" is a "substituted cycloalkyl group".) In the description herein, the simple expression "cycloalkyl group" encompasses both the "unsubstituted cycloalkyl group" and the "substituted cycloalkyl group".

The "substituted cycloalkyl group" means a group formed by substituting one or more hydrogen atom of the "unsubstituted cycloalkyl group" by a substituent. Specific examples of the "substituted cycloalkyl group" include groups formed by substituting one or more hydrogen atom of each of the "unsubstituted cycloalkyl groups" (set of specific examples G6A) by a substituent, and the example of the substituted cycloalkyl group (set of specific examples G6B). The examples of the "unsubstituted cycloalkyl group" and the examples of the "substituted cycloalkyl group" enumerated herein are mere examples, and the "substituted cycloalkyl group" in the description herein encompasses groups formed by substituting one or more hydrogen atom bonded to the carbon atoms of the cycloalkyl group itself of the "substituted cycloalkyl group" in the set of specific examples G6B by a substituent, and groups formed by substituting a hydrogen atom of the substituent of the "substituted cycloalkyl group" in the set of specific examples G6B by a substituent.

Unsubstituted Cycloalkyl Group (Set of Specific Examples G6A):
   a cyclopropyl group,
   a cyclobutyl group,
   a cyclopentyl group,
   a cyclohexyl group,
   a 1-adamantyl group,
   a 2-adamantyl group,
   a 1-norbornyl group, and
   a 2-norbornyl group.
Substituted Cycloalkyl Group (Set of Specific Examples G6B):
   a 4-methylcyclohexyl group.
Group represented by -Si(R₉₀₁)(R₉₀₂)(R₉₀₃)

In the description herein, specific examples (set of specific examples G7) of the group represented by -Si(R₉₀₁)(R₉₀₂)(R₉₀₃) include:
- Si(G1)(G1)(G1),
- Si(G1)(G2)(G2),
- Si(G1)(G1)(G2),
- Si(G2)(G2)(G2),
- Si(G3)(G3)(G3), and
- Si(G6)(G6)(G6).

Herein,
G1 represents the "substituted or unsubstituted aryl group" described in the set of specific examples G1,
G2 represents the "substituted or unsubstituted heterocyclic group" described in the set of specific examples G2,
G3 represents the "substituted or unsubstituted alkyl group" described in the set of specific examples G3, and
G6 represents the "substituted or unsubstituted cycloalkyl group" described in the set of specific examples G6.

Plural groups represented by G1 in -Si(G1)(G1)(G1) are the same as or different from each other.

Plural groups represented by G2 in -Si(G1)(G2)(G2) are the same as or different from each other.

Plural groups represented by G1 in -Si(G1)(G1)(G2) are the same as or different from each other.

Plural groups represented by G2 in -Si(G2)(G2)(G2) are the same as or different from each other.

Plural groups represented by G3 in -Si(G3)(G3)(G3) are the same as or different from each other.

Plural groups represented by G6 in -Si(G6)(G6)(G6) are the same as or different from each other.

### Group represented by -O-(R₉₀₄)

In the description herein, specific examples (set of specific examples G8) of the group represented by -O-(R₉₀₄) include:
- O(G1),
- O(G2),
- O(G3), and
- O(G6).

Herein,
G1 represents the "substituted or unsubstituted aryl group" described in the set of specific examples G1,
G2 represents the "substituted or unsubstituted heterocyclic group" described in the set of specific examples G2,
G3 represents the "substituted or unsubstituted alkyl group" described in the set of specific examples G3, and
G6 represents the "substituted or unsubstituted cycloalkyl group" described in the set of specific examples G6.

### Group represented by -S-(R₉₀₅)

In the description herein, specific examples (set of specific examples G9) of the group represented by -S-(R₉₀₅) include:
- S(G1),
- S(G2),
- S(G3), and
- S(G6).

Herein,
G1 represents the "substituted or unsubstituted aryl group" described in the set of specific examples G1,
G2 represents the "substituted or unsubstituted heterocyclic group" described in the set of specific examples G2,
G3 represents the "substituted or unsubstituted alkyl group" described in the set of specific examples G3, and
G6 represents the "substituted or unsubstituted cycloalkyl group" described in the set of specific examples G6.

### Group represented by -N(R₉₀₆)(R₉₀₇)

In the description herein, specific examples (set of specific examples G10) of the group represented by -N(R₉₀₆)(R₉₀₇) include:
- N(G1)(G1),
- N(G2)(G2),
- N(G1)(G2),
- N(G3)(G3), and
- N(G6)(G6).

G1 represents the "substituted or unsubstituted aryl group" described in the set of specific examples G1,
G2 represents the "substituted or unsubstituted heterocyclic group" described in the set of specific examples G2,
G3 represents the "substituted or unsubstituted alkyl group" described in the set of specific examples G3, and
G6 represents the "substituted or unsubstituted cycloalkyl group" described in the set of specific examples G6.

Plural groups represented by G1 in -N(G1)(G1) are the same as or different from each other.

Plural groups represented by G2 in -N(G2)(G2) are the same as or different from each other.

Plural groups represented by G3 in -N(G3)(G3) are the same as or different from each other.

Plural groups represented by G6 in -N(G6)(G6) are the same as or different from each other.

### Halogen Atom

In the description herein, specific examples (set of specific examples G11) of the "halogen atom" include a fluorine atom, a chlorine atom, a bromine atom, and an iodine atom.

### Substituted or Unsubstituted Fluoroalkyl Group

In the description herein, the "substituted or unsubstituted fluoroalkyl group" means a group formed by substituting at least one hydrogen atom bonded to the carbon atom constituting the alkyl group in the "substituted or unsubstituted alkyl group" by a fluorine atom, and encompasses a group formed by substituting all the hydrogen atoms bonded to the carbon atoms constituting the alkyl group in the "substituted or unsubstituted alkyl group" by fluorine atoms (i.e., a perfluoroalkyl group). The number of carbon atoms of the "unsubstituted fluoroalkyl group" is 1 to 50, preferably 1 to 30, and more preferably 1 to 18, unless otherwise indicated in the description. The "substituted fluoroalkyl group" means a group formed by substituting one or more hydrogen atom of the "fluoroalkyl group" by a substituent. In the description herein, the "substituted fluoroalkyl group" encompasses a group formed by substituting one or more hydrogen atom bonded to the carbon atom of the alkyl chain in the "substituted fluoroalkyl group" by a substituent, and a group formed by substituting one or more hydrogen atom of the substituent in the "substituted fluoroalkyl group" by a substituent. Specific examples of the "unsubstituted fluoroalkyl group" include examples of groups formed by substituting one or more hydrogen atom in each of the "alkyl group" (set of specific examples G3) by a fluorine atom.

### Substituted or Unsubstituted Haloalkyl Group

In the description herein, the "substituted or unsubstituted haloalkyl group" means a group formed by substituting at least one hydrogen atom bonded to the carbon atom constituting the alkyl group in the "substituted or unsubstituted alkyl group" by a halogen atom, and encompasses a group formed by substituting all the hydrogen atoms bonded to the carbon atoms constituting the alkyl group in the "substituted or unsubstituted alkyl group" by halogen atoms. The number of carbon atoms of the "unsubstituted haloalkyl group" is 1 to 50, preferably 1 to 30, and more preferably 1 to 18, unless otherwise indicated in the description. The "substituted haloalkyl group" means a group formed by substituting one or more hydrogen atom of the "haloalkyl group" by a substituent. In the description herein, the "substituted haloalkyl group" encompasses a group formed by substituting one or more hydrogen atom bonded to the carbon atom of the alkyl chain in the "substituted haloalkyl group" by a substituent, and a group formed by substituting one or more hydrogen atom of the substituent in the "substituted haloalkyl group" by a substituent. Specific examples of the "unsubstituted haloalkyl group" include examples of groups formed by substituting one or more hydrogen atom in each of the "alkyl group" (set of specific examples G3) by a halogen atom. A haloalkyl group may be referred to as a halogenated alkyl group in some cases.

### Substituted or Unsubstituted Alkoxy Group

In the description herein, specific examples of the "substituted or unsubstituted alkoxy group" include a group represented by -O(G3), wherein G3 represents the "substituted or unsubstituted alkyl group" described in the set of specific examples G3. The number of carbon atoms of the "unsubstituted alkoxy group" is 1 to 50, preferably 1 to 30, and more preferably 1 to 18, unless otherwise indicated in the description.

### Substituted or Unsubstituted Alkylthio Group

In the description herein, specific examples of the "substituted or unsubstituted alkylthio group" include a group represented by -S(G3), wherein G3 represents the "substituted or unsubstituted alkyl group" described in the set of specific examples G3. The number of carbon atoms of the "unsubstituted alkylthio group" is 1 to 50, preferably 1 to 30, and more preferably 1 to 18, unless otherwise indicated in the description.

### Substituted or Unsubstituted Aryloxy Group

In the description herein, specific examples of the "substituted or unsubstituted aryloxy group" include a group represented by -O(G1), wherein G1 represents the "substituted or unsubstituted aryl group" described in the set of specific examples G1. The number of ring carbon atoms of the "unsubstituted aryloxy group" is 6 to 50, preferably 6 to 30, and more preferably 6 to 18, unless otherwise indicated in the description.

### Substituted or Unsubstituted Arylthio Group

In the description herein, specific examples of the "substituted or unsubstituted arylthio group" include a group represented by -S(G1), wherein G1 represents the "substituted or unsubstituted aryl group" described in the set of specific examples G1. The number of ring carbon atoms of the "unsubstituted arylthio group" is 6 to 50, preferably 6 to 30, and more preferably 6 to 18, unless otherwise indicated in the description.

### Substituted or Unsubstituted Trialkylsilyl Group

In the description herein, specific examples of the "trialkylsilyl group" include a group represented by -Si(G3)(G3)(G3), wherein G3 represents the "substituted or unsubstituted alkyl group" described in the set of specific examples G3. Plural groups represented by G3 in -Si(G3)(G3)(G3) are the same as or different from each other. The number of carbon atoms of each of alkyl groups of the "substituted or unsubstituted trialkylsilyl group" is 1 to 50, preferably 1 to 20, and more preferably 1 to 6, unless otherwise indicated in the description.

### Substituted or Unsubstituted Aralkyl Group

In the description herein, specific examples of the "substituted or unsubstituted aralkyl group" include a group represented by -(G3)-(G1), wherein G3 represents the "substituted or unsubstituted alkyl group" described in the set of specific examples G3, and G1 represents the "substituted or unsubstituted aryl group" described in the set of specific examples G1. Accordingly, the "aralkyl group" is a group formed by substituting a hydrogen atom of an "alkyl group" by an "aryl group" as a substituent, and is one embodiment of the "substituted alkyl group". The "unsubstituted aralkyl group" is an "unsubstituted alkyl group" that is substituted by an "unsubstituted aryl group", and the number of carbon atoms of the "unsubstituted aralkyl group" is 7 to 50, preferably 7 to 30, and more preferably 7 to 18, unless otherwise indicated in the description.

Specific examples of the "substituted or unsubstituted aralkyl group" include a benzyl group, a 1-phenylethyl group, a 2-phenylethyl group, a 1-phenylisopropyl group, a 2-phenylisopropyl group, a phenyl-t-butyl group, an α-naphthylmethyl group, a 1-α-naphthylethyl group, a 2-α-naphthylethyl group, a 1-α-naphthylisopropyl group, a 2-α-naphthylisopropyl group, a β-naphthylmethyl group, a 1-β-naphthylethyl group, a 2-6-naphthylethyl group, a 1-β-naphthylisopropyl group, and a 2-β-naphthylisopropyl group.

In the description herein, the substituted or unsubstituted aryl group is preferably a phenyl group, a p-biphenyl group, a m-biphenyl group, an o-biphenyl group, a p-terphenyl-4-yl group, a p-terphenyl-3-yl group, a p-terphenyl-2-yl group, a m-terphenyl-4-yl group, a m-terphenyl-3-yl group, a m-terphenyl-2-yl group, an o-terphenyl-4-yl group, an o-terphenyl-3-yl group, an o-terphenyl-2-yl group, a 1-naphthyl group, a 2-naphthyl group, an anthryl group, a phenanthryl group, a pyrenyl group, a chrysenyl group, a triphenylenyl group, a fluorenyl group, a 9,9'-spirobifluorenyl group, a 9,9-dimethylfluorenyl group, a 9,9-diphenylfluorenyl group, and the like, unless otherwise indicated in the description.

In the description herein, the substituted or unsubstituted heterocyclic group is preferably a pyridyl group, a pyrimidinyl group, a triazinyl group, a quinolyl group, an isoquinolyl group, a quinazolinyl group, a benzimidazolyl group, a phenanthrolinyl group, a carbazolyl group (e.g., a 1-carbazolyl, group, a 2-carbazolyl, group, a 3-carbazolyl, group, a 4-carbazolyl, group, or a 9-carbazolyl, group), a benzocarbazolyl group, an azacarbazolyl group, a diazacarbazolyl group, a dibenzofuranyl group, a naphthobenzofuranly group, an azadibenzofuranyl group, a diazadibenzofuranyl group, a dibenzothiophenyl group, a naphthobenzothiophenyl group, an azadibenzothiophenyl group, a diazadibenzothiophenyl group, a (9-phenyl)carbazolyl group (e.g., a (9-phenyl)carbazol-1-yl group, a (9-phenyl)carbazol-2-yl group, a (9-phenyl)carbazol-3-yl group, or a (9-phenyl)carbazol-4-yl group), a (9-biphenylyl)carbazolyl group, a (9-phenyl)phenylcarbazolyl group, a diphenylcarbazol-9-yl group, a phenylcarbazol-9-yl group, a phenyltriazinyl group, a biphenylyltriazinyl group, a diphenyltriazinyl group, a phenyldibenzofuranyl group, a phenyldibenzothiophenyl group, and the like, unless otherwise indicated in the description.

In the description herein, the carbazolyl group is specifically any one of the following groups unless otherwise indicated in the description.

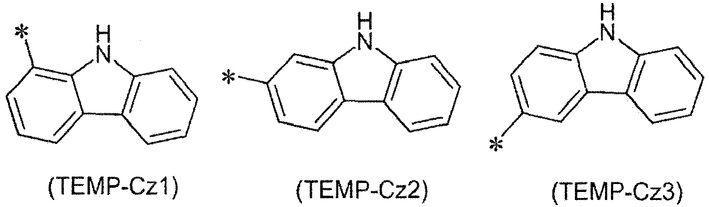

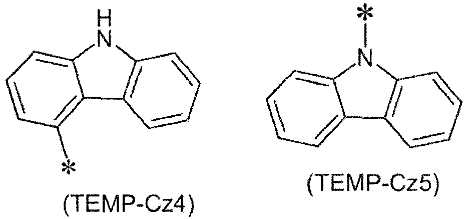

In the description herein, the (9-phenyl)carbazolyl group is specifically any one of the following groups unless otherwise indicated in the description.

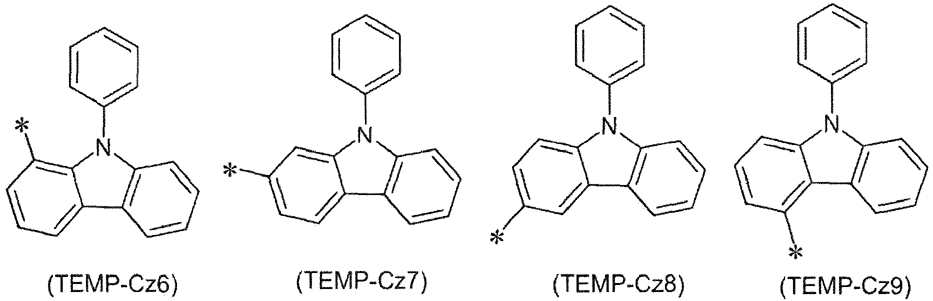

In the general formulae (TEMP-Cz1) to (TEMP-Cz9), * represents a bonding site.

In the description herein, the dibenzofuranyl group and the dibenzothiophenyl group are specifically any one of the following groups unless otherwise indicated in the description.

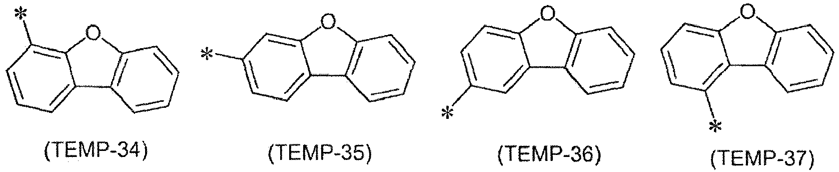

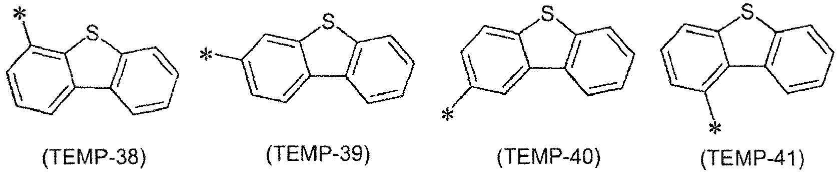

In the general formulae (TEMP-34) to (TEMP-41), * represents a bonding site.

In the description herein, the substituted or unsubstituted alkyl group is preferably a methyl group, an ethyl group, a propyl group, an isopropyl group, a n-butyl group, an isobutyl group, a t-butyl group, or the like unless otherwise indicated in the description.

### Substituted or Unsubstituted Arylene Group

In the description herein, the "substituted or unsubstituted arylene group" is a divalent group derived by removing one hydrogen atom on the aryl ring from the "substituted or unsubstituted aryl group" described above unless otherwise indicated in the description. Specific examples (set of specific examples G12) of the "substituted or unsubstituted arylene group" include divalent groups derived by removing one hydrogen atom on the aryl ring from the "substituted or unsubstituted aryl groups" described in the set of specific examples G1.

### Substituted or Unsubstituted Divalent Heterocyclic Group

In the description herein, the "substituted or unsubstituted divalent heterocyclic group" is a divalent group derived by removing one hydrogen atom on the heterocyclic ring from the "substituted or unsubstituted heterocyclic group" described above unless otherwise indicated in the description. Specific examples (set of specific examples G13) of the "substituted or unsubstituted divalent heterocyclic group" include divalent groups derived by removing one hydrogen atom on the heterocyclic ring from the "substituted or unsubstituted heterocyclic groups" described in the set of specific examples G2.

### Substituted or Unsubstituted Alkylene Group

In the description herein, the "substituted or unsubstituted alkylene group" is a divalent group derived by removing one hydrogen atom on the alkyl chain from the "substituted or unsubstituted alkyl group" described above unless otherwise indicated in the description. Specific examples (set of specific examples G14) of the "substituted or unsubstituted alkylene group" include divalent groups derived by removing one hydrogen atom on the alkyl chain from the "substituted or unsubstituted alkyl groups" described in the set of specific examples G3.

In the description herein, the substituted or unsubstituted arylene group is preferably any one of the groups represented by the following general formulae (TEMP-42) to (TEMP-68) unless otherwise indicated in the description.

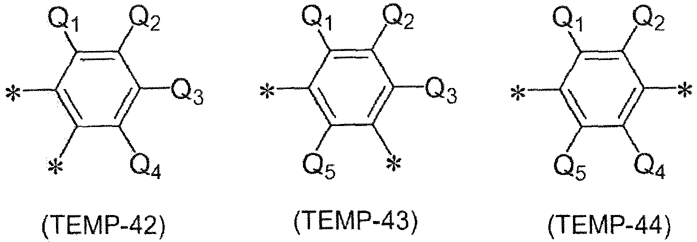

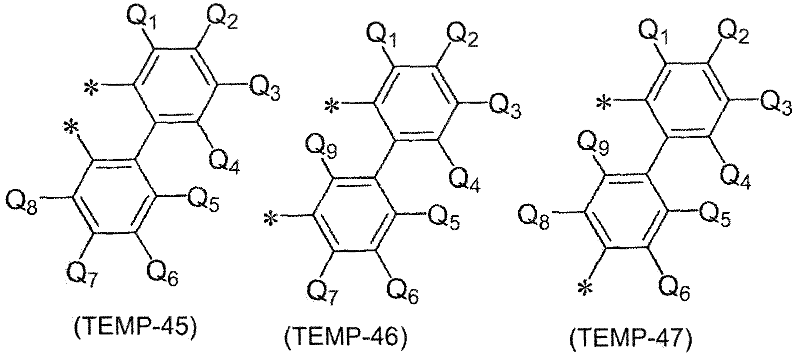

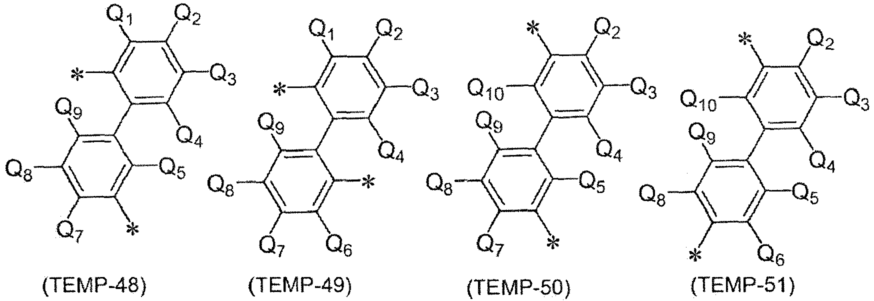

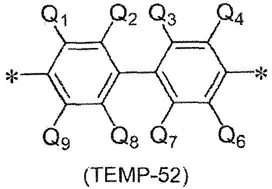

In the general formulae (TEMP-42) to (TEMP-52), Q₁ to Q₁₀ each independently represent a hydrogen atom or a substituent.

In the general formulae (TEMP-42) to (TEMP-52), * represents a bonding site.

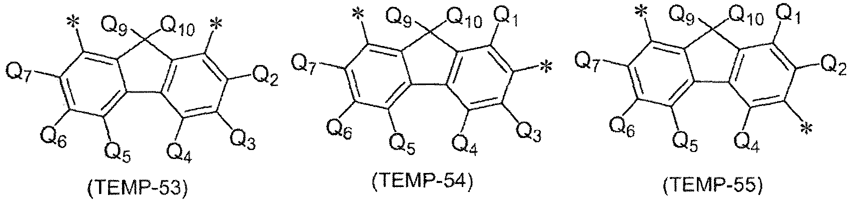

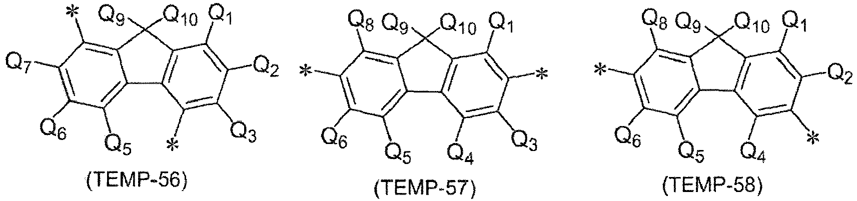

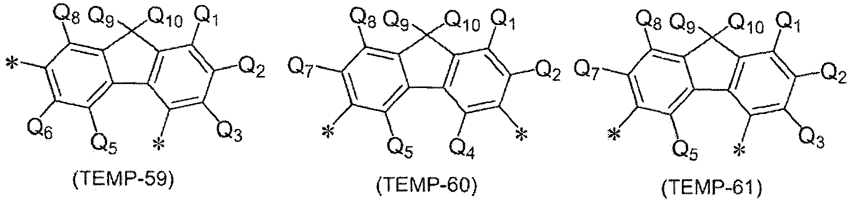

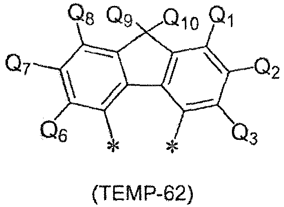

In the general formulae (TEMP-53) to (TEMP-62), Q₁ to Q₁₉ each independently represent a hydrogen atom or a substituent.

The formulae Q₉ and Q₁₀ may be bonded to each other to form a ring via a single bond.

In the general formulae (TEMP-53) to (TEMP-62), * represents a bonding site.

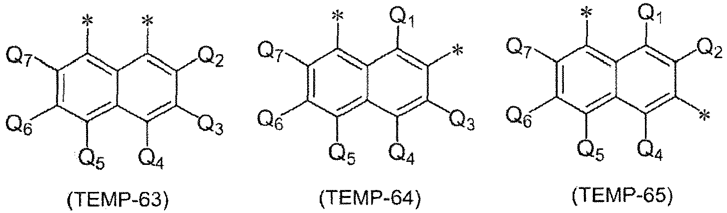

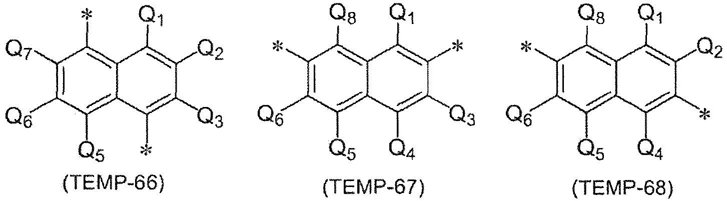

In the general formulae (TEMP-63) to (TEMP-68), Q₁ to Q₈ each independently represent a hydrogen atom or a substituent.

In the general formulae (TEMP-63) to (TEMP-68), * represents a bonding site.

In the description herein, the substituted or unsubstituted divalent heterocyclic group is preferably the groups represented by the following general formulae (TEMP-69) to (TEMP-102) unless otherwise indicated in the description.

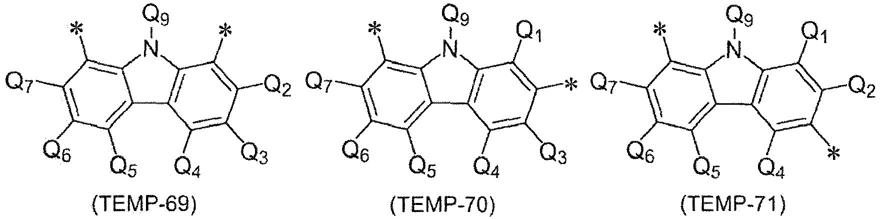

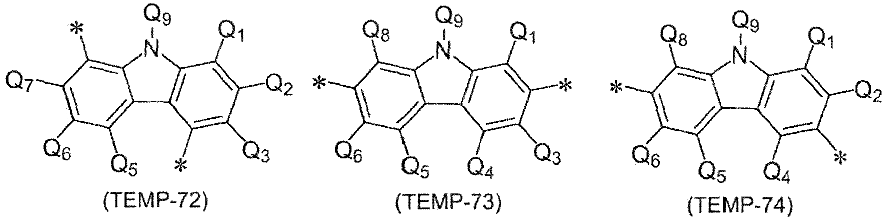

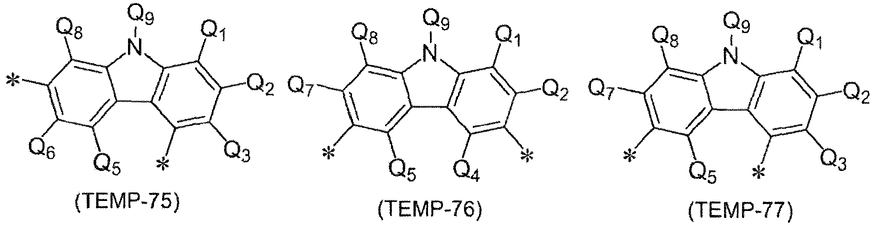

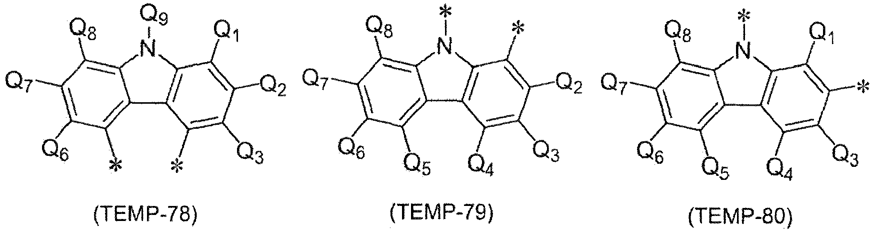

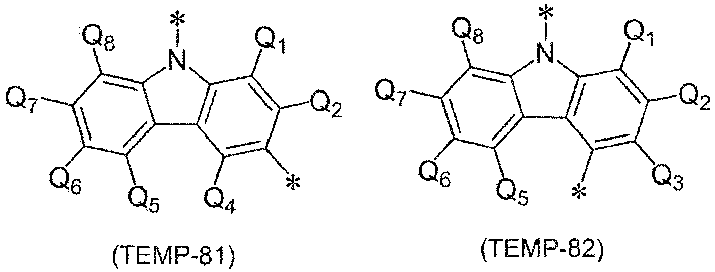

In the general formulae (TEMP-69) to (TEMP-82), Q₁ to Q₉ each independently represent a hydrogen atom or a substituent.

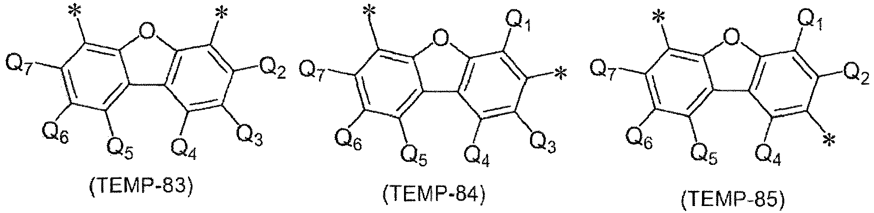

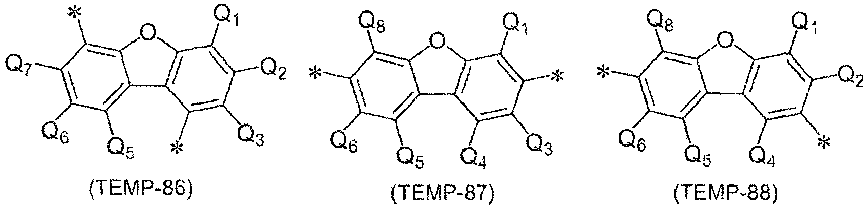

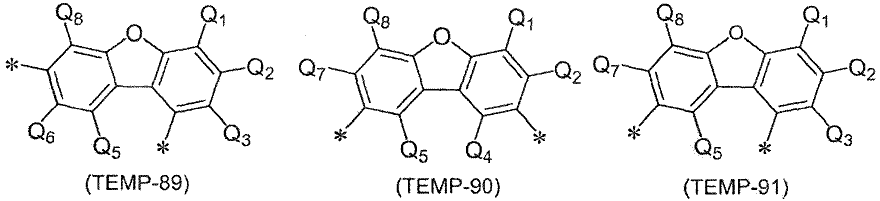

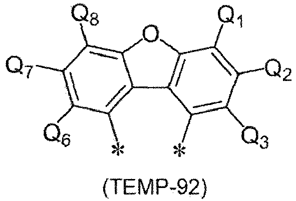

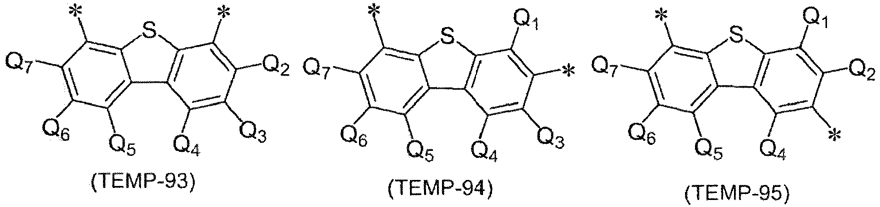

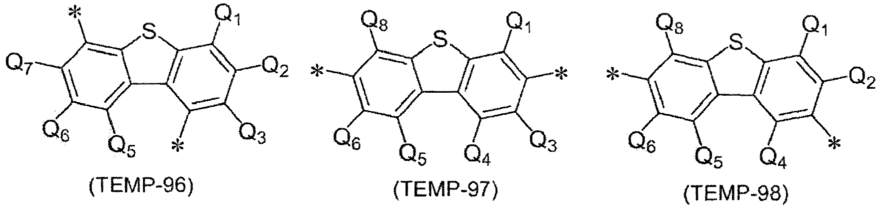

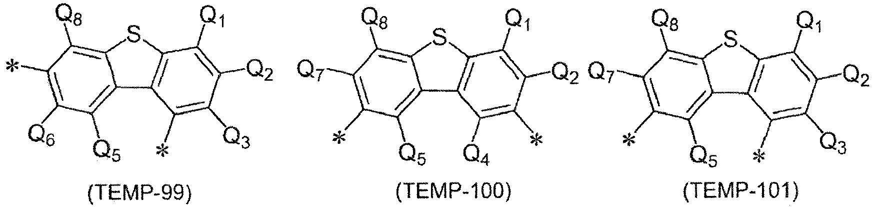

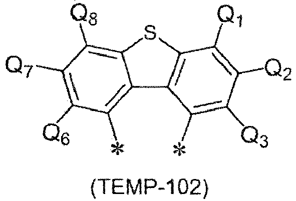

In the general formulae (TEMP-83) to (TEMP-102), Q₁ to Q₈ each independently represent a hydrogen atom or a substituent.

The above are the explanation of the "substituents in the description herein".

### Case forming Ring by bonding

In the description herein, the case where "one or more combinations of combinations each including adjacent two or more each are bonded to each other to form a substituted or unsubstituted monocyclic ring, or each are bonded to each other to form a substituted or unsubstituted condensed ring, or each are not bonded to each other" means a case where "one or more combinations of combinations each including adjacent two or more each are bonded to each other to form a substituted or unsubstituted monocyclic ring", a case where "one or more combinations of combinations each including adjacent two or more each are bonded to each other to form a substituted or unsubstituted condensed ring", and a case where "one or more combinations of combinations each including adjacent two or more each are not bonded to each other".

In the description herein, the case where "one or more combinations of combinations each including adjacent two or more each are bonded to each other to form a substituted or unsubstituted monocyclic ring" and the case where "one or more combinations of combinations each including adjacent two or more each are bonded to each other to form a substituted or unsubstituted condensed ring" (which may be hereinafter collectively referred to as a "case forming a ring by bonding") will be explained below. The cases will be explained for the anthracene compound represented by the following general formula (TEMP-103) having an anthracene core skeleton as an example.

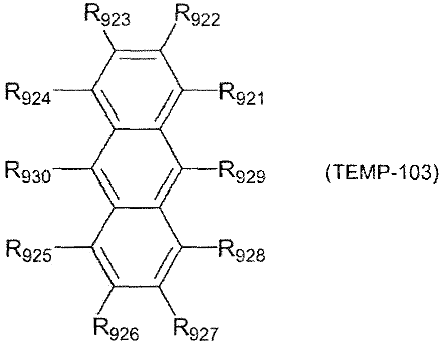

For example, in the case where "one or more combinations of combinations each including adjacent two or more each are bonded to each other to form a ring" among R₉₂₁ to R₉₃₀, the combinations each including adjacent two as one combination include a combination of R₉₂₁ and R₉₂₂, a combination of R₉₂₂ and R₉₂₃, a combination of R₉₂₃ and R₉₂₄, a combination of R₉₂₄ and R₉₃₀, a combination of R₉₃₀ and R₉₂₅, a combination of R₉₂₅ and R₉₂₆, a combination of R₉₂₆ and R₉₂₇, a combination of R₉₂₇ and R₉₂₈, a combination of R₉₂₈ and R₉₂₉, and a combination of R₉₂₉ and R₉₂₁.

The "one or more combinations" mean that two or more combinations each including adjacent two or more may form rings simultaneously. For example, in the case where R₉₂₁ and R₉₂₂ are bonded to each other to form a ring Q_{A}, and simultaneously R₉₂₅ and R₉₂₆ are bonded to each other to form a ring Q_{B}, the anthracene compound represented by the general formula (TEMP-103) is represented by the following general formula (TEMP-104).

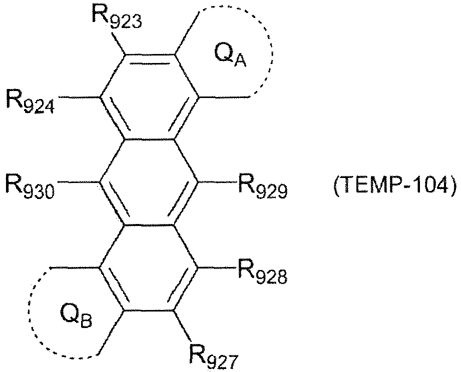

The case where the "combination including adjacent two or more forms rings" encompasses not only the case where adjacent two included in the combination are bonded as in the aforementioned example, but also the case where adjacent three or more included in the combination are bonded. For example, this case means that R₉₂₁ and R₉₂₂ are bonded to each other to form a ring Q_{A}, R₉₂₂ and R₉₂₃ are bonded to each other to form a ring Qc, and adjacent three (R₉₂₁, R₉₂₂, and R₉₂₃) included in the combination are bonded to each other to form rings, which are condensed to the anthracene core skeleton, and in this case, the anthracene compound represented by the general formula (TEMP-103) is represented by the following general formula (TEMP-105). In the following general formula (TEMP-105), the ring Q_{A} and the ring Qc share R₉₂₂.

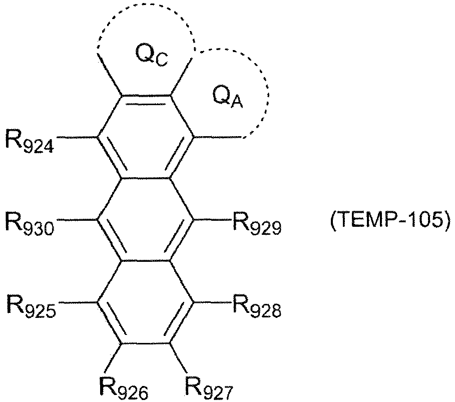

The formed "monocyclic ring" or "condensed ring" may be a saturated ring or an unsaturated ring in terms of structure of the formed ring itself. In the case where the "one combination including adjacent two" forms a "monocyclic ring" or a "condensed ring", the "monocyclic ring" or the "condensed ring" may form a saturated ring or an unsaturated ring. For example, the ring Q_{A} and the ring Q_{B} formed in the general formula (TEMP-104) each are a "monocyclic ring" or a "condensed ring". The ring Q_{A} and the ring Qc formed in the general formula (TEMP-105) each are a "condensed ring". The ring Q_{A} and the ring Qc in the general formula (TEMP-105) form a condensed ring through condensation of the ring Q_{A} and the ring Qc. In the case where the ring Q_{A} in the general formula (TMEP-104) is a benzene ring, the ring Q_{A} is a monocyclic ring. In the case where the ring Q_{A} in the general formula (TMEP-104) is a naphthalene ring, the ring Q_{A} is a condensed ring.

The "unsaturated ring" means an aromatic hydrocarbon ring or an aromatic heterocyclic ring. The "saturated ring" means an aliphatic hydrocarbon ring or a non-aromatic heterocyclic ring.

Specific examples of the aromatic hydrocarbon ring include the structures formed by terminating the groups exemplified as the specific examples in the set of specific examples G1 with a hydrogen atom.

Specific examples of the aromatic heterocyclic ring include the structures formed by terminating the aromatic heterocyclic groups exemplified as the specific examples in the set of specific examples G2 with a hydrogen atom.

Specific examples of the aliphatic hydrocarbon ring include the structures formed by terminating the groups exemplified as the specific examples in the set of specific examples G6 with a hydrogen atom.

The expression "to form a ring" means that the ring is formed only with the plural atoms of the core structure or with the plural atoms of the core structure and one or more arbitrary element. For example, the ring Q_{A} formed by bonding R₉₂₁ and R₉₂₂ each other shown in the general formula (TEMP-104) means a ring formed with the carbon atom of the anthracene skeleton bonded to R₉₂₁, the carbon atom of the anthracene skeleton bonded to R₉₂₂, and one or more arbitrary element. As a specific example, in the case where the ring Q_{A} is formed with R₉₂₁ and R₉₂₂, and in the case where a monocyclic unsaturated ring is formed with the carbon atom of the anthracene skeleton bonded to R₉₂₁, the carbon atom of the anthracene skeleton bonded to R₉₂₂, and four carbon atoms, the ring formed with R₉₂₁ and R₉₂₂ is a benzene ring.

Herein, the "arbitrary element" is preferably at least one kind of an element selected from the group consisting of a carbon element, a nitrogen element, an oxygen element, and a sulfur element, unless otherwise indicated in the description. For the arbitrary element (for example, for a carbon element or a nitrogen element), a bond that does not form a ring may be terminated with a hydrogen atom or the like, and may be substituted by an "arbitrary substituent" described later. In the case where an arbitrary element other than a carbon element is contained, the formed ring is a heterocyclic ring.

The number of the "one or more arbitrary element" constituting the monocyclic ring or the condensed ring is preferably 2 or more and 15 or less, more preferably 3 or more and 12 or less, and further preferably 3 or more and 5 or less, unless otherwise indicated in the description.

What is preferred between the "monocyclic ring" and the "condensed ring" is the "monocyclic ring" unless otherwise indicated in the description.

What is preferred between the "saturated ring" and the "unsaturated ring" is the "unsaturated ring" unless otherwise indicated in the description.

The "monocyclic ring" is preferably a benzene ring unless otherwise indicated in the description.

The "unsaturated ring" is preferably a benzene ring unless otherwise indicated in the description.

In the case where the "one or more combinations of combinations each including adjacent two or more" each are "bonded to each other to form a substituted or unsubstituted monocyclic ring", or each are "bonded to each other to form a substituted or unsubstituted condensed ring", it is preferred that the one or more combinations of combinations each including adjacent two or more each are bonded to each other to form a substituted or unsubstituted "unsaturated ring" containing the plural atoms of the core skeleton and 1 or more and 15 or less at least one kind of an element selected from the group consisting of a carbon element, a nitrogen element, an oxygen element, and a sulfur element, unless otherwise indicated in the description.

In the case where the "monocyclic ring" or the "condensed ring" has a substituent, the substituent is, for example, an "arbitrary substituent" described later. In the case where the "monocyclic ring" or the "condensed ring" has a substituent, specific examples of the substituent include the substituents explained in the section "Substituents in Description" described above.

In the case where the "saturated ring" or the "unsaturated ring" has a substituent, the substituent is, for example, an "arbitrary substituent" described later. In the case where the "monocyclic ring" or the "condensed ring" has a substituent, specific examples of the substituent include the substituents explained in the section "Substituents in Description" described above.

The above are the explanation of the case where "one or more combinations of combinations each including adjacent two or more" each are "bonded to each other to form a substituted or unsubstituted monocyclic ring", and the case where "one or more combinations of combinations each including adjacent two or more" each are "bonded to each other to form a substituted or unsubstituted condensed ring" (i.e., the "case forming a ring by bonding").

### Substituent for "Substituted or Unsubstituted"

In one embodiment in the description herein, the substituent for the case of "substituted or unsubstituted" (which may be hereinafter referred to as an "arbitrary substituent") is, for example, a group selected from the group consisting of
an unsubstituted alkyl group having 1 to 50 carbon atoms,
an unsubstituted alkenyl group having 2 to 50 carbon atoms,
an unsubstituted alkynyl group having 2 to 50 carbon atoms,
an unsubstituted cycloalkyl group having 3 to 50 ring carbon atoms,
   - Si(R₉₀₁)(R₉₀₂)(R₉₀₃),
   - O-(R₉₀₄),
   - S-(R₉₀₅),
   - N(R₉₀₆)(R₉₀₇),
a halogen atom, a cyano group, a nitro group,
an unsubstituted aryl group having 6 to 50 ring carbon atoms, and
an unsubstituted heterocyclic group having 5 to 50 ring atoms,
wherein R₉₀₁ to R₉₀₇ each independently represent
a hydrogen atom,
a substituted or unsubstituted alkyl group having 1 to 50 carbon atoms
a substituted or unsubstituted cycloalkyl group having 3 to 50 ring carbon atoms,
a substituted or unsubstituted aryl group having 6 to 50 ring carbon atoms, or
a substituted or unsubstituted heterocyclic group having 5 to 50 ring atoms. In the case where two or more groups each represented by R₉₀₁ exist, the two or more groups each represented by R₉₀₁ are the same as or different from each other,
in the case where two or more groups each represented by R₉₀₂ exist, the two or more groups each represented by R₉₀₂ are the same as or different from each other,
in the case where two or more groups each represented by R₉₀₃ exist, the two or more groups each represented by R₉₀₃ are the same as or different from each other,
in the case where two or more groups each represented by R₉₀₄ exist, the two or more groups each represented by R₉₀₄ are the same as or different from each other,
in the case where two or more groups each represented by R₉₀₅ exist, the two or more groups each represented by R₉₀₅ are the same as or different from each other,
in the case where two or more groups each represented by R₉₀₆ exist, the two or more groups each represented by R₉₀₆ are the same as or different from each other, and
in the case where two or more groups each represented by R₉₀₇ exist, the two or more groups each represented by R₉₀₇ are the same as or different from each other.

In one embodiment, the substituent for the case of "substituted or unsubstituted" may be a group selected from the group consisting of
an alkyl group having 1 to 50 carbon atoms,
an aryl group having 6 to 50 ring carbon atoms, and
a heterocyclic group having 5 to 50 ring atoms.

In one embodiment, the substituent for the case of "substituted or unsubstituted" may be a group selected from the group consisting of
an alkyl group having 1 to 18 carbon atoms,
an aryl group having 6 to 18 ring carbon atoms, and
a heterocyclic group having 5 to 18 ring atoms.

The specific examples of the groups for the arbitrary substituent described above are the specific examples of the substituent described in the section "Substituents in Description" described above.

In the description herein, the arbitrary adjacent substituents may form a "saturated ring" or an "unsaturated ring", preferably form a substituted or unsubstituted saturated 5-membered ring, a substituted or unsubstituted saturated 6-membered ring, a substituted or unsubstituted unsaturated 5-membered ring, or a substituted or unsubstituted unsaturated 6-membered ring, and more preferably form a benzene ring, unless otherwise indicated.

In the description herein, the arbitrary substituent may further have a substituent unless otherwise indicated in the description. The definition of the substituent that the arbitrary substituent further has may be the same as the arbitrary substituent.

In the description herein, a numerical range shown by "AAto BB" means a range including the numerical value AA as the former of "AA to BB" as the lower limit value and the numerical value BB as the latter of "AA to BB" as the upper limit value.

Hereinafter, a compound of the present invention will be described.

A compound according to one embodiment of the present invention is represented by the following formula (1).

Meanwhile, hereinafter, the compound of the present invention, which is represented by the formula (1) and formulas that are described below and are included in the formula (1), may be simply referred to as an "inventive compound (1)". Further, a compound according to another embodiment of the present invention is represented by the formula (2) to be described below. Hereinafter, the compound of the present invention, which is represented by the formula (2) to be described below and formulas that are described below and are included in the formula (2), may be simply referred to as an "inventive compound (2)". Furthermore, in the case of the "inventive compound (1)" and the "inventive compound (2)", these may be simply referred to as an "inventive compound" meaning both.

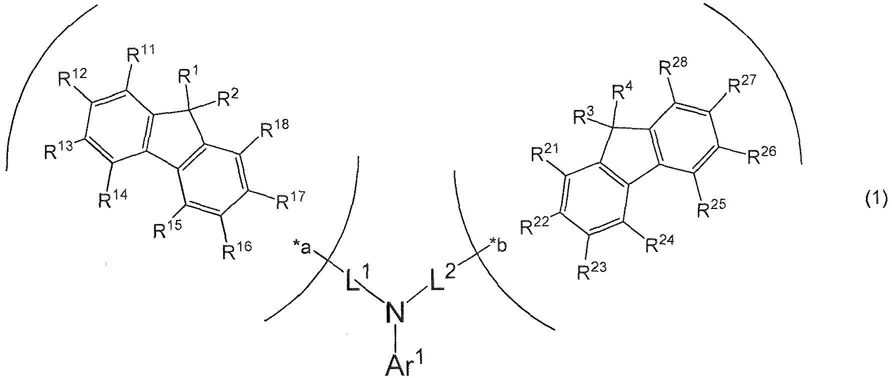

Hereinafter, descriptions will be made on symbols in the formula (1) and the formulas that are described below and are included in the formula (1). The same symbols have the same meanings.

In the formula (1),
one of R¹ and R² is a methyl group, and the other is an unsubstituted phenyl group, and
R¹ and R² are not bonded to each other and therefore do not form a ring structure.

One of R³ and R⁴ is a methyl group, and the other is an unsubstituted phenyl group, and
R³ and R⁴ are not bonded to each other and therefore do not form a ring structure.

R¹¹ to R¹⁸, and R²¹ to R²⁸ are hydrogen atoms.
provided that,
one selected from R¹¹ to R¹⁸ is a single bond bonded to *a, and
one selected from R²¹ to R²⁸ is a single bond bonded to *b.

It is preferable that one selected from R¹², R¹³, R¹⁴, R¹⁵, R¹⁶, and R¹⁷ is a single bond bonded to *a, and it is more preferable that one selected from R¹² and R¹⁷ is a single bond bonded to *a.

It is preferable that one selected from R²², R²³, R²⁴, R²⁵, R²⁶, and R²⁷ is a single bond bonded to *b, and it is more preferable that one selected from R²² and R²⁷ is a single bond bonded to *b.

L¹ and L² are each independently a single bond, or a substituted or unsubstituted phenylene group.

L¹ and L² are preferably single bonds.

Ar¹ is a group represented by any one of formulas (1-1) to (1-6).

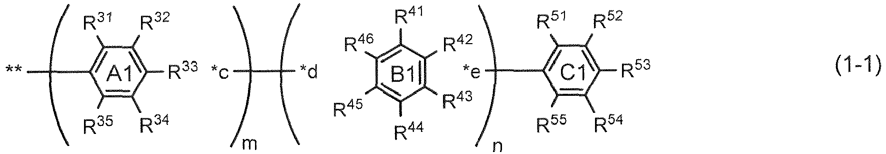

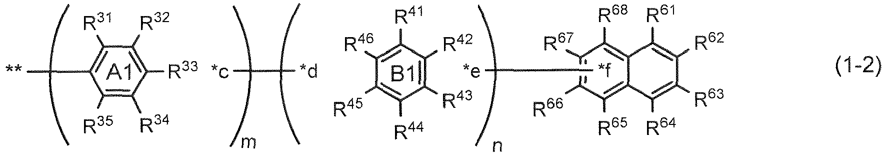

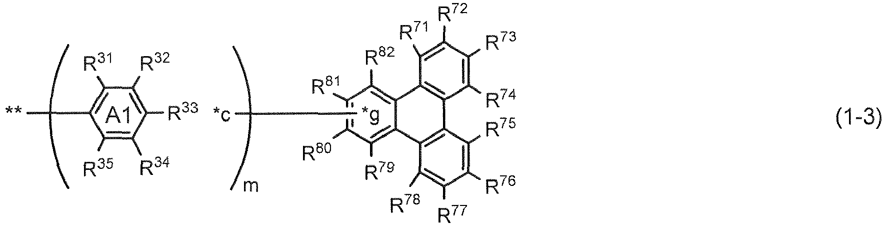

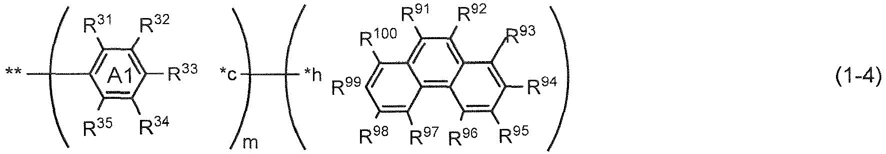

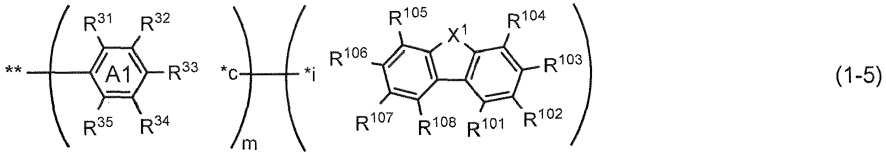

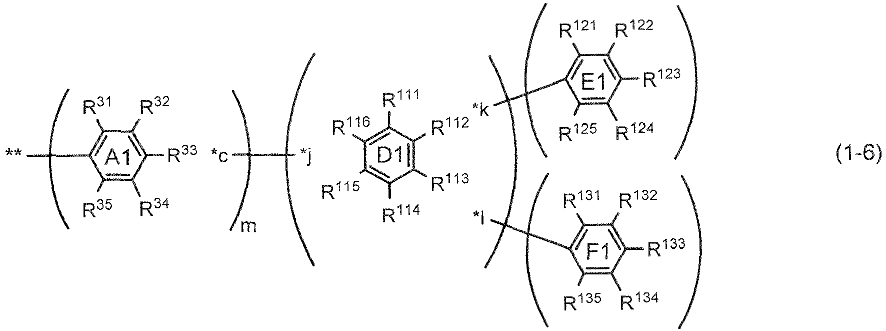

In the formulas (1-1) to (1-6),
R³¹ to R³⁵, R⁴¹ to R⁴⁶, R⁵¹ to R⁵⁵, R⁶¹ to R⁶⁸, R⁷¹ to R⁸², R⁹¹ to R¹⁰⁰, R¹⁰¹ to R¹⁰⁸, R¹¹¹ to R¹¹⁶, R¹²¹ to R¹²⁵, and R¹³¹ to R¹³⁵ are each independently a hydrogen atom, a substituted or unsubstituted alkyl group having 1 to 50 carbon atoms, a substituted or unsubstituted cycloalkyl group having 3 to 50 ring carbon atoms, a halogen atom, a cyano group, a nitro group, a substituted or unsubstituted aryl group having 6 to 50 ring carbon atoms, or a substituted or unsubstituted heterocyclic group having 5 to 50 ring atoms.

R³¹ to R³⁵, R⁴¹ to R⁴⁶, R⁵¹ to R⁵⁵, R⁶¹ to R⁶⁸, R⁷¹ to R⁸², R⁹¹ to R¹⁰⁰, R¹⁰¹ to R¹⁰⁸, R¹¹¹ to R¹¹⁶, R¹²¹ to R¹²⁵, and R¹³¹ to R¹³⁵ are each independently preferably a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms, more preferably a hydrogen atom, a substituted or unsubstituted alkyl group having 1 to 50 carbon atoms, a substituted or unsubstituted aryl group having 6 to 50 ring carbon atoms, or a substituted or unsubstituted heterocyclic group having 5 to 50 ring atoms, further preferably, a hydrogen atom.

Regarding the substituted or unsubstituted alkyl group having 1 to 50 carbon atoms, which is represented by R³¹ to R³⁵, R⁴¹ to R⁴⁶, R⁵¹, to R⁵⁵, R⁶¹ to R⁶⁸, R⁷¹ to R⁸², R⁹¹ to R¹⁰⁰, R¹⁰¹ to R¹⁰⁸, R¹¹¹ to R¹¹⁶, R¹²¹ to R¹²⁵, and R¹³¹ to R¹³⁵, the details are as described above in the section of "Substituents in Description".

The unsubstituted alkyl group represented by R³¹ to R³⁵, R⁴¹ to R⁴⁶, R⁵¹ to R⁵⁵, R⁶¹ to R⁶⁸, R⁷¹ to R⁸², R⁹¹ to R¹⁰⁰, R¹⁰¹ to R¹⁰⁸, R¹¹¹ to R¹¹⁶, R¹²¹ to R¹²⁵, and R¹³¹ to R¹³⁵ is preferably a methyl group, an ethyl group, an n-propyl group, an isopropyl group, an n-butyl group, an isobutyl group, a s-butyl group, or a t-butyl group, more preferably a methyl group, an ethyl group, an isopropyl group, or a t-butyl group, further preferably a methyl group or a t-butyl group.

Regarding the substituted or unsubstituted cycloalkyl group having 3 to 50 ring carbon atoms, which is represented by R³¹ to R³⁵, R⁴¹ to R⁴⁶, R⁵¹ to R⁵⁵, R⁶¹ to R⁶⁸, R⁷¹ to R⁸², R⁹¹ to R¹⁰⁰, R¹⁰¹ to R¹⁰⁸, R¹¹¹ to R¹¹⁶, R¹²¹ to R¹²⁵, and R¹³¹ to R¹³⁵, the details are as described above in the section of "Substituents in Description".

The unsubstituted cycloalkyl group represented by R³¹ to R³⁵, R⁴¹ to R⁴⁶, R⁵¹ to R⁵⁵, R⁶¹ to R⁶⁸, R⁷¹ to R⁸², R⁹¹ to R¹⁰⁰, R¹⁰¹ to R¹⁰⁸, R¹¹¹ to R¹¹⁶, R¹²¹ to R¹²⁵, and R¹³¹ to R¹³⁵ is preferably a cyclopropyl group, a cyclobutyl group, a cyclopentyl group, a cyclohexyl group, a 1-adamantyl group, a 2-adamantyl group, a 1-norbornyl group, or a 2-norbornyl group, more preferably a cyclopropyl group, a cyclobutyl group, a cyclopentyl group, or a cyclohexyl group, further preferably a cyclopentyl group or a cyclohexyl group.

Details of the halogen atom represented by R³¹ to R³⁵, R⁴¹ to R⁴⁶, R⁵¹ to R⁵⁵, R⁶¹ to R⁶⁸, R⁷¹ to R⁸², R⁹¹ to R¹⁰⁰, R¹⁰¹ to R¹⁰⁸, R¹¹¹ to R¹¹⁶, R¹²¹ to R¹²⁵, and R¹³¹ to R¹³⁵ are as described above in the section of "Substituents in Description", and a fluorine atom is preferred.

Regarding the substituted or unsubstituted aryl group having 6 to 50 ring carbon atoms, which is represented by R³¹ to R³⁵, R⁴¹ to R⁴⁶, R⁵¹ to R⁵⁵, R⁶¹ to R⁶⁸, R⁷¹ to R⁸², R⁹¹ to R¹⁰⁰, R¹⁰¹ to R¹⁰⁸, R¹¹¹ to R¹¹⁶, R¹²¹ to R¹²⁵, and R¹³¹ to R¹³⁵, the details are as described in "Substituents in Description".

The unsubstituted aryl group represented by R³¹ to R³⁵, R⁴¹ to R⁴⁶, R⁵¹ to R⁵⁵, R⁶¹ to R⁶⁸, R⁷¹ to R⁸², R⁹¹ to R¹⁰⁰, R¹⁰¹ to R¹⁰⁸, R¹¹¹ to R¹¹⁶, R¹²¹ to R¹²⁵, and R¹³¹ to R¹³⁵ is preferably a phenyl group, a biphenyl group, a naphthyl group, or a phenanthryl group, more preferably a phenyl group, a biphenyl group, or a naphthyl group, further preferably a phenyl group.

Regarding the substituted or unsubstituted heterocyclic group having 5 to 50 ring atoms, which is represented by R³¹ to R³⁵, R⁴¹ to R⁴⁶, R⁵¹ to R⁵⁵, R⁶¹ to R⁶⁸, R⁷¹ to R⁸², R⁹¹ to R¹⁰⁰, R¹⁰¹ to R¹⁰⁸, R¹¹¹ to R¹¹⁶, R¹²¹ to R¹²⁵, and R¹³¹ to R¹³⁵, the details are as described in "Substituents in Description".

The unsubstituted heterocyclic group is preferably a dibenzofuranyl group, or a dibenzothiophenyl group.

X¹ is an oxygen atom, a sulfur atom, or CR^{a}R^{b}, and
R^{a} and R^{b} are each independently a hydrogen atom, a methyl group, or a substituted or unsubstituted phenyl group, and R^{a} and R^{b} may be bonded to each other to form a substituted or unsubstituted ring.

In one embodiment of the present invention, X¹ is preferably an oxygen atom. In another embodiment, X¹ is preferably a sulfur atom. In still another embodiment, X¹ is preferably CR^{a}R^{b}, and R^{a} and R^{b} are preferably methyl groups, or substituted or unsubstituted phenyl groups.

Details of the optional substituted or unsubstituted ring formed through bonding between R^{a} and R^{b} are as described above in "Substituents in Description", and it is selected from a substituted or unsubstituted aromatic hydrocarbon ring, a substituted or unsubstituted aliphatic hydrocarbon ring, a substituted or unsubstituted aromatic heterocycle, and a substituted or unsubstituted non-aromatic heterocycle.

The aromatic hydrocarbon ring is, for example, a benzene ring, a biphenylene ring, a naphthalene ring, or a fluorene ring, preferably a naphthalene ring, or a fluorene ring.

The aliphatic hydrocarbon ring is, for example, a cyclopentene ring, a cyclopentadiene ring, a cyclohexene ring, a cyclohexadiene ring, or a hydrocarbon ring obtained by partially hydrogenating the aromatic hydrocarbon ring.

The aromatic heterocycle is, for example, a pyrrole ring, a furan ring, a thiophene ring, a pyridine ring, an imidazole ring, a pyrazole ring, an indole ring, an isoindole ring, a benzofuran ring, an isobenzofuran ring, a benzothiophene ring, a benzoimidazole ring, an indazole ring, a dibenzofuran ring, a naphthobenzofuran ring, a dibenzothiophene ring, a naphthobenzothiophene ring, a carbazole ring, or a benzocarbazole ring, preferably a dibenzofuran ring, or a dibenzothiophene ring.

The non-aromatic heterocycle is, for example, a heterocycle obtained by partially hydrogenating the aromatic heterocycle.

In one embodiment of the present invention, R^{a} and R^{b} may not be bonded to each other and thus do not form a substituted or unsubstituted ring.

One selected from R³¹ to R³⁵ is a single bond bonded to *c,
one selected from R⁴¹ to R⁴⁶ is a single bond bonded to *d, and the other one selected from R⁴¹ to R⁴⁶ is a single bond bonded to *e,
one selected from R⁶⁵ to R⁶⁸ is a single bond bonded to *f,
one selected from R⁷⁹ to R⁸² is a single bond bonded to *g,
one selected from R⁹¹, and R⁹⁷ to R¹⁰⁰ is a single bond bonded to *h,
one selected from R¹⁰⁵ to R¹⁰⁸ is a single bond bonded to *i, and
one selected from R¹¹¹ to R¹¹⁶ is a single bond bonded to *j, the other one selected from R¹¹¹ to R¹¹⁶ is a single bond bonded to *k, and the other one selected from R¹¹¹ to R¹¹⁶ is a single bond bonded to *l.

Adjacent two selected from R³¹ to R³⁵ that are not the single bond, adjacent two selected from R⁴¹ to R⁴⁶ that are not the single bond, adjacent two selected from R⁵¹ to R⁵⁵, adjacent two selected from R⁶¹ to R⁶⁸ that are not the single bond, adjacent two selected from R⁷¹ to R⁸² that are not the single bond, adjacent two selected from R⁹¹ to R¹⁰⁰ that are not the single bond, adjacent two selected from R¹⁰¹ to R¹⁰⁸ that are not the single bond, adjacent two selected from R¹¹¹ to R¹¹⁶ that are not the single bond, adjacent two selected from R¹²¹ to R¹²⁵, and adjacent two selected from R¹³¹ to R¹³⁵ are not bonded to each other and therefore do not form ring structures.

The benzene ring A1 and the benzene ring B1, the benzene ring A1 and the benzene ring C1, the benzene ring A1 and the benzene ring D1, the benzene ring A1 and the benzene ring E1, the benzene ring A1 and the benzene ring F1, the benzene ring B1 and the benzene ring C1, the benzene ring D1 and the benzene ring E1, and the benzene ring D1 and the benzene ring F1 are not crosslinked.

** represents a bonding position to the central nitrogen atom N,
m is 0 or 1, and n is 0 or 1,
in the formulas (1-1) and (1-2), when m is 0 and n is 0, *e is bonded to the central nitrogen atom N, when m is 0 and n is 1, *c is bonded to the central nitrogen atom N, and when m is 1 and n is 0, *e is bonded to one selected from R³¹ to R³⁵,
in the formulas (1-3) to (1-6), when m is 0, *c is bonded to the central nitrogen atom N, and
when R¹² or R¹⁷ is a single bond bonded to *a, and R²² or R²⁷ is a single bond bonded to *b, the formula (1-1) excludes the p-biphenyl group.

That is, when R¹² or R¹⁷ is a single bond bonded to *a, R²² or R²⁷ is a single bond bonded to *b, m is 1 and n is 0, in the formula (1-1), one selected from R³¹, R³², R³⁴, and R³⁵ is a single bond bonded to *c, and preferably one selected from R³¹ and R³⁵ is a single bond bonded to *c.

Further, when R¹² or R¹⁷ is a single bond bonded to *a, R²² or R²⁷ is a single bond bonded to *b, m is 0 and n is 1, in the formula (1-1), R⁴⁶ is a single bond bonded to *d, and one selected from R⁴¹, R⁴², R⁴⁴, and R⁴⁵ is a single bond bonded to *e, preferably one selected from R⁴¹ and R⁴⁵ is a single bond bonded to *e.

The p-biphenyl group is the following formula.

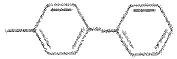

In the formulas (1-1) and (1-2), in one embodiment of the present invention, it is preferable that m is 0 and n is 0, in another embodiment, it is preferable that m is 0 and n is 1, in still another embodiment, it is preferable that m is 1 and n is 0, and in still another embodiment, it is preferable that m is 1 and n is 1.

In the formula (1-1), it is preferable that m or n is 0.

In the formulas (1-3) to (1-6), in one embodiment of the present invention, m is preferably 0, and in another embodiment, m is preferably 1.

The group represented by the formula (1-1) is more preferably a substituted or unsubstituted group selected from the following formulas.

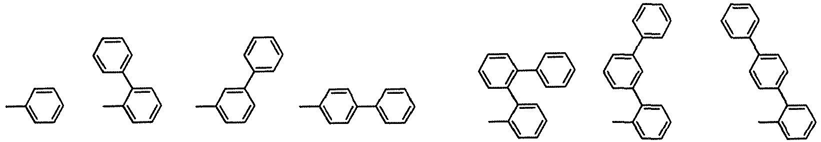

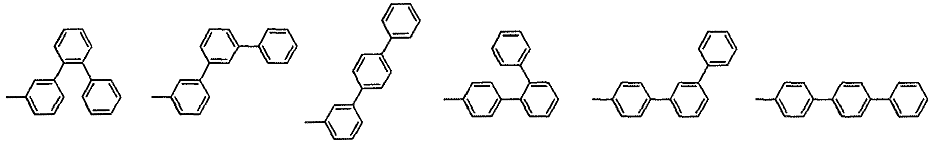

In the formulas, optional substituents are omitted.

The group represented by the formula (1-2) is preferably a substituted or unsubstituted group selected from the following formulas.

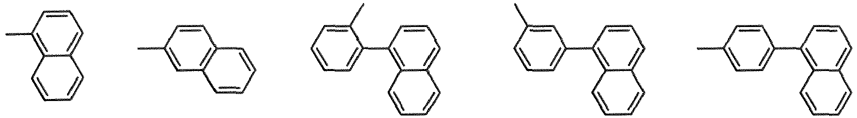

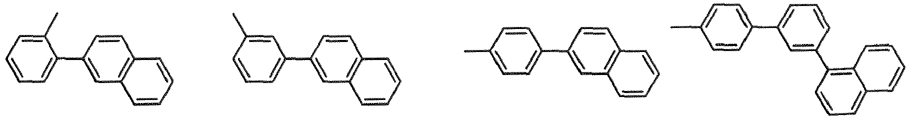

In the formulas, optional substituents are omitted.

The group represented by the formula (1-3) is preferably a substituted or unsubstituted group selected from the following formulas.

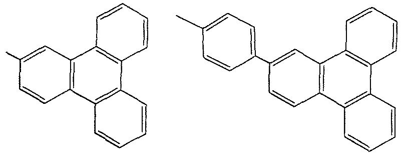

In the formulas, optional substituents are omitted.

The group represented by the formula (1-4) is preferably an unsubstituted group selected from the following formulas.

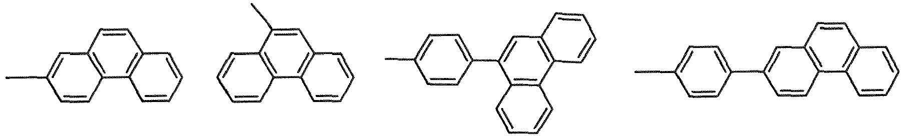

In the formulas, optional substituents are omitted.

The group represented by the formula (1-5) is preferably a substituted or unsubstituted group selected from the following formulas.

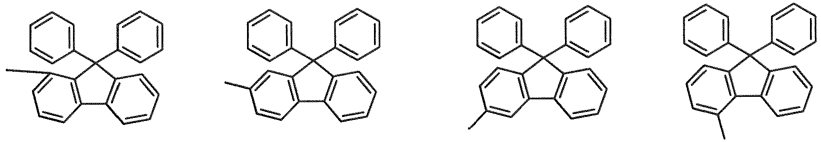

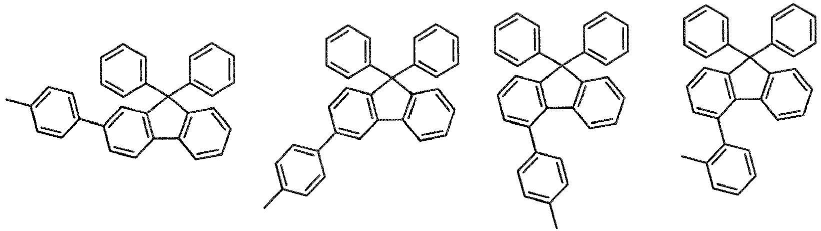

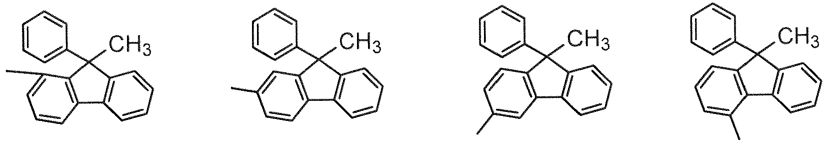

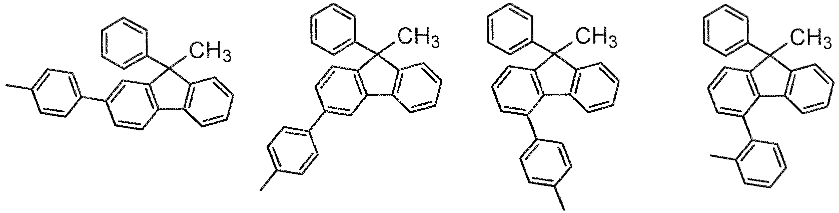

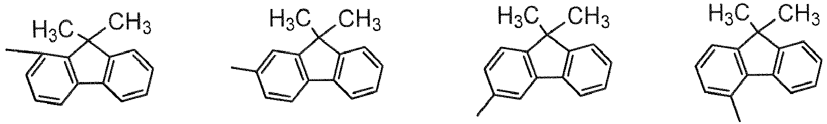

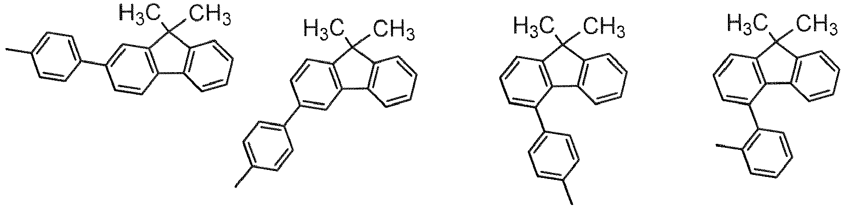

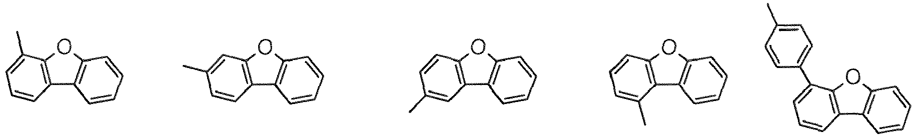

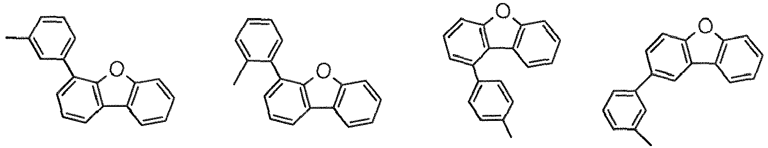

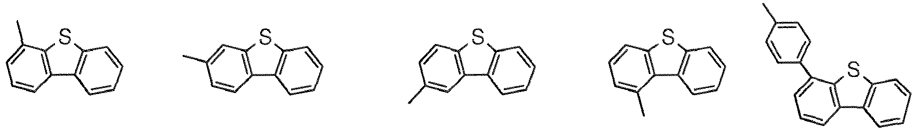

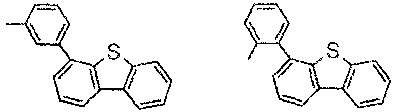

In the formulas, optional substituents are omitted.

The group represented by the formula (1-6) is preferably a substituted or unsubstituted group selected from the following formulas.

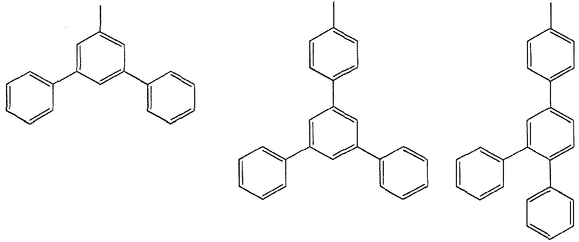

In the formulas, optional substituents are omitted.

Ar¹ is preferably a group represented by any one of the formulas (1-1), (1-2), (1-5), and (1-6), more preferably a group represented by the formula (1-1).

In one embodiment of the present invention,
(1-1) R³¹ to R³⁵ that are not the single bond bonded to *c may be all hydrogens,
(1-2) R⁴¹ to R⁴⁶ that are not the single bond bonded to *d, and are not the single bond bonded to *e may be all hydrogen atoms,
(1-3) R⁵¹ to R⁵⁵ may be all hydrogen atoms,
(1-4) R⁶¹ to R⁶⁴, and R⁶⁵ to R⁶⁸ that are not the single bond bonded to *f may be all hydrogen atoms,
(1-5) R⁷¹ to R⁷⁸, and R⁷⁹ to R⁸² that are not the single bond bonded to *g may be all hydrogen atoms,
(1-6) R⁹² to R⁹⁶, and R⁹¹, and R⁹⁷ to R¹⁰⁰ that are not the single bond bonded to *h may be all hydrogen atoms,
(1-7) R¹⁰¹ to R¹⁰⁴, and R¹⁰⁵ to R¹⁰⁸ that are not the single bond bonded to *i may be all hydrogen atoms, and
(1-8) R¹¹¹ to R¹¹⁶ that are not the single bond bonded to *j, are not the single bond bonded to *k, and are not the single bond bonded to *l, and R¹²¹ to R¹²⁵, and R¹³¹ to R¹³⁵ may be all hydrogen atoms.

A compound according to another embodiment of the present invention is represented by the following formula (2).

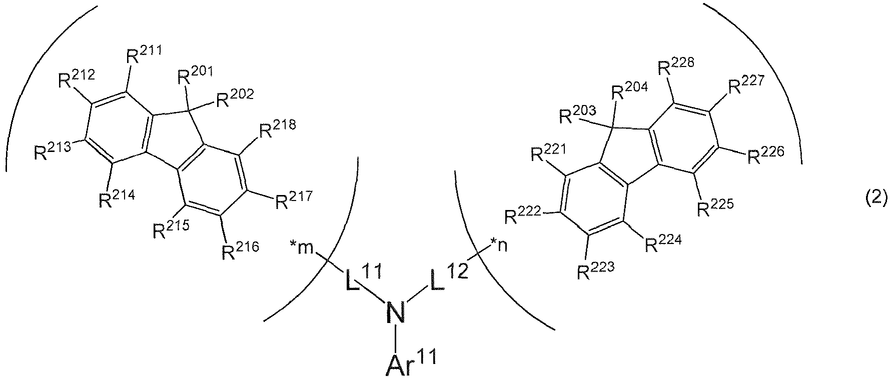

Hereinafter, descriptions will be made on symbols in the formula (2) and the formulas that are described below and are included in the formula (2). The same symbols have the same meanings.

In the formula (2),
one selected from R²⁰¹ to R²⁰⁴ is a methyl group, and the other three selected from R²⁰¹ to R²⁰⁴ are unsubstituted phenyl groups,
R²⁰¹ and R²⁰² are not bonded to each other and therefore do not form a ring structure, and
R²⁰³ and R²⁰⁴ are not bonded to each other and therefore do not form a ring structure.

R²¹¹ to R²¹⁸, and R²²¹ to R²²⁸ are hydrogen atoms.
provided that,
one selected from R²¹¹ to R²¹⁸ is a single bond bonded to *m, and
one selected from R²²¹ to R²²⁸ is a single bond bonded to *n.

It is preferable that one selected from R²¹², R²¹³, R²¹⁴, R²¹⁵, R²¹⁶, and R²¹⁷ is a single bond bonded to *m, and it is more preferable that one selected from R²¹² and R²¹⁷ is a single bond bonded to *m.

It is preferable that one selected from R²²², R²²³, R²²⁴, R²²⁵, R²²⁶, and R²²⁷ is a single bond bonded to *n, and it is more preferable that one selected from R²²², and R²²⁷ is a single bond bonded to *n.

L¹¹ and L¹² are each independently a single bond, or a substituted or unsubstituted phenylene group.

L¹¹ and L¹² are preferably single bonds.

Ar¹¹ is a group represented by any one of formulas (2-1) to (2-6):

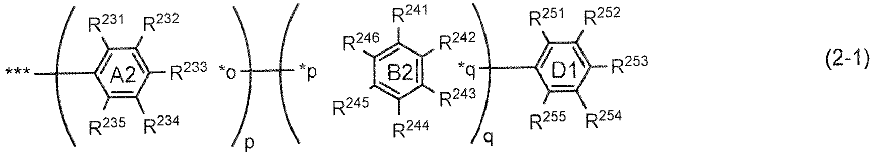

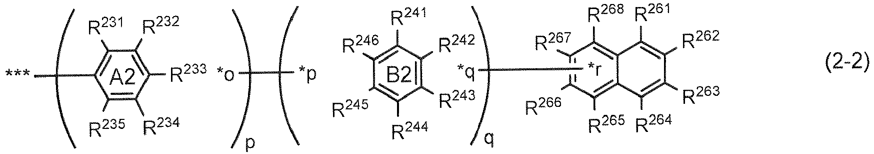

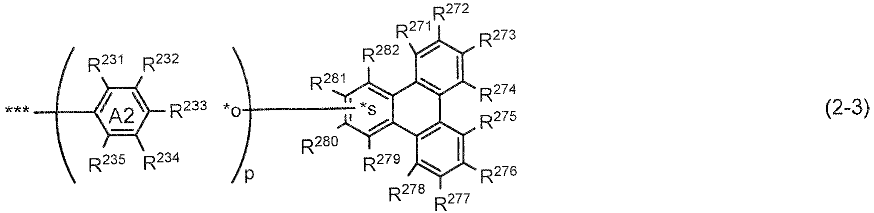

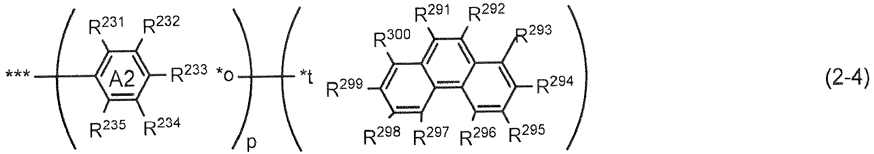

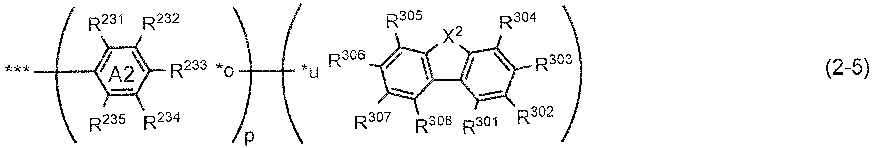

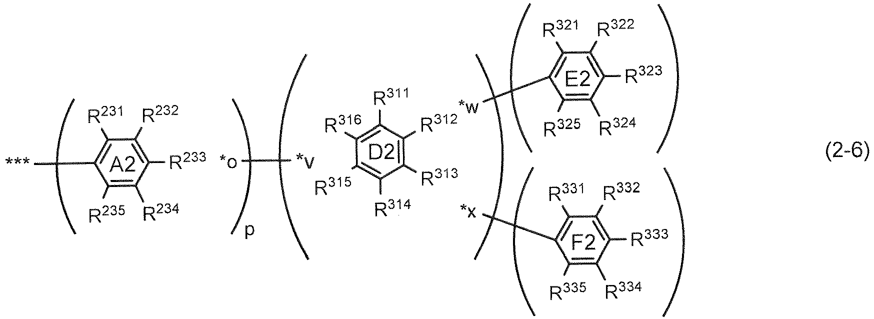

In the formulas (2-1) to (2-6),
R²³¹ to R²³⁵, R²⁴¹ to R²⁴⁶, R²⁵¹ to R²⁵⁵, R²⁶¹ to R²⁶⁸, R²⁷¹ to R²⁸², R²⁹¹ to R³⁰⁰, R³⁰¹ to R³⁰⁸, R³¹¹ to R³¹⁶, R³²¹ to R³²⁵, and R³³¹ to R³³⁵ are each independently a hydrogen atom, a substituted or unsubstituted alkyl group having 1 to 50 carbon atoms, a substituted or unsubstituted cycloalkyl group having 3 to 50 ring carbon atoms, a halogen atom, a cyano group, a nitro group, a substituted or unsubstituted aryl group having 6 to 50 ring carbon atoms, or a substituted or unsubstituted heterocyclic group having 5 to 50 ring atoms.

R²³¹ to R²³⁵, R²⁴¹ to R²⁴⁶ R²⁵¹ to R²⁵⁵, R²⁶¹ to R²⁶⁸, R²⁷¹ to R²⁸², R²⁹¹ to R³⁰⁰, R³⁰¹ to R³⁰⁸, R³¹¹ to R³¹⁶, R³²¹ to R³²⁵, and R³³¹ to R³³⁵ are each independently preferably a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms, more preferably a hydrogen atom, a substituted or unsubstituted alkyl group having 1 to 50 carbon atoms, a substituted or unsubstituted aryl group having 6 to 50 ring carbon atoms, or a substituted or unsubstituted heterocyclic group having 5 to 50 ring atoms, further preferably a hydrogen atom.

Details of the groups represented by R²³¹ to R²³⁵, R²⁴¹ to R²⁴⁶, R²⁵¹ to R²⁵⁵, R²⁶¹ to R²⁶⁸, R²⁷¹ to R²⁸², R²⁹¹ to R³⁰⁰, R³⁰¹ to R³⁰⁸, R³¹¹ to R³¹⁶, R³²¹ to R³²⁵, and R³³¹ to R³³⁵ are the same as details of the groups described for R³¹ to R³⁵, R⁴¹ to R⁴⁶, R⁵¹ to R⁵⁵, R⁶¹ to R⁶⁸, R⁷¹ to R⁸², R⁹¹ to R¹⁰⁰, R¹⁰¹ to R¹⁰⁸, R¹¹¹ to R¹¹⁶, R¹²¹ to R¹²⁵, and R¹³¹ to R¹³⁵, respectively, and all of the preferred groups are also the same.

X² is an oxygen atom, a sulfur atom, or CR^{c}R^{d}, and
R^{c} and R^{d} are each independently a hydrogen atom, or a methyl group, and R^{c} and R^{d} are not bonded to each other and therefore do not form a ring structure.

In one embodiment of the present invention, X² is preferably an oxygen atom. In another embodiment, X² is preferably a sulfur atom. In still another embodiment, X² is preferably CR^{c}R^{d}, and R^{c} and R^{d} are preferably a methyl groups, or a substituted or unsubstituted phenyl group.

One selected from R²³¹ to R²³⁵ is a single bond bonded to *o,
one selected from R²⁴¹ to R²⁴⁶ is a single bond bonded to *p, and the other one selected from R²⁴¹ to R²⁴⁶ is a single bond bonded to *q,
one selected from R²⁶⁵ to R²⁶⁸ is a single bond bonded to *r,
one selected from R²⁷⁹ to R²⁸² is a single bond bonded to *s,
one selected from R²⁹¹, and R²⁹⁷ to R³⁰⁰ is a single bond bonded to *t,
one selected from R³⁰⁵ to R³⁰⁸ is a single bond bonded to *u, and
one selected from R³¹¹ to R³¹⁶ is a single bond bonded to *v, the other one selected from R³¹¹ to R³¹⁶ is a single bond bonded to *w, and the other one selected from R³¹¹ to R³¹⁶ is a single bond bonded to *x.

Adjacent two selected from R²³¹ to R²³⁵ that are not the single bond, adjacent two selected from R²⁴¹ to R²⁴⁶ that are not the single bond, adjacent two selected from R²⁵¹ to R²⁵⁵, adjacent two selected from R²⁶¹ to R²⁶⁸ that are not the single bond, adjacent two selected from R²⁷¹ to R²⁸² that are not the single bond, adjacent two selected from R²⁹¹ to R³⁰⁰ that are not the single bond, adjacent two selected from R³⁰¹ to R³⁰⁸ that are not the single bond, adjacent two selected from R³¹¹ to R³¹⁶ that are not the single bond, adjacent two selected from R³²¹ to R³²⁵, and adjacent two selected from R³³¹ to R³³⁵ are not bonded to each other and therefore do not form ring structures.

The benzene ring A2 and the benzene ring B2, the benzene ring A2 and the benzene ring C2, the benzene ring A2 and the benzene ring D2, the benzene ring A2 and the benzene ring E2, the benzene ring A2 and the benzene ring F2, the benzene ring B2 and the benzene ring C2, the benzene ring D2 and the benzene ring E2, and the benzene ring D2 and the benzene ring F2 are not crosslinked.

*** represents a bonding position to the central nitrogen atom N,
p is 0 or 1, and q is 0 or 1,
in the formulas (2-1) and (2-2), when p is 0 and q is 0, *q is bonded to the central nitrogen atom N, when p is 0 and q is 1, *o is bonded to the central nitrogen atom N, and when p is 1 and q is 0, *q is bonded to one selected from R²³¹ to R²³⁵,
in the formulas (2-3) to (2-6), when p is 0, *o is bonded to the central nitrogen atom N,
in the formula (2-5), when X² is CR^{c}R^{d}, and p is 0, at least one selected from L¹¹ and L¹² is a substituted or unsubstituted phenylene group,
in the formula (2-5), when X² is an oxygen atom and p is 1, one selected from R³⁰⁶ to R³⁰⁸ is a single bond bonded to *u, and
when R²¹² or R²¹⁷ is a single bond bonded to *m, and R²²² or R²²⁷ is a single bond bonded to *n, the formula (2-1) excludes the p-biphenyl group.

That is, when R²¹² or R²¹⁷ is a single bond bonded to *m, R²²² or R²²⁷ is a single bond bonded to *n, m is 1 and n is 0, in the formula (2-1), one selected from R²³¹, R²³², R²³⁴, and R²³⁵ is a single bond bonded to *o, and preferably one selected from R²³¹ and R²³⁵ is a single bond bonded to *o.

Further, when R²¹² or R²¹⁷ is a single bond bonded to *m, R²²² or R²²⁷ is a single bond bonded to *n, m is 0 and n is 1, in the formula (2-1), R²⁴⁶ is a single bond bonded to *p, one selected from R²⁴¹, R²⁴², R²⁴⁴, and R²⁴⁵ is a single bond bonded to *q, and preferably one selected from R²⁴¹ and R²⁴⁵ is a single bond bonded to *q.

In the formulas (2-1) and (2-2), in one embodiment of the present invention, it is preferable that m is 0 and n is 0, in another embodiment, it is preferable that m is 0 and n is 1, in still another embodiment, it is preferable that m is 1 and n is 0, and in still another embodiment, it is preferable that m is 1 and n is 1.

In the formula (2-1), m or n is preferably 0.

In the formulas (1-3) to (1-6), in one embodiment of the present invention, m is preferably 0, and in another embodiment, m is preferably 1.

The preferred group represented by the formula (2-1) is the same as the preferred group represented by the formula (1-1).

The preferred group represented by the formula (2-2) is the same as the preferred group represented by the formula (1-2).

The preferred group represented by the formula (2-3) is the same as the preferred group represented by the formula (1-3).

The preferred group represented by the formula (2-4) is the same as the preferred group represented by the formula (1-4).

The preferred group represented by the formula (2-5) is the same as the preferred group represented by the formula (1-5).

The preferred group represented by the formula (1-6) is the same as the preferred group represented by the formula (1-6).

Ar¹¹ is preferably a group represented by any one of the formulas (2-1), (2-2), (2-5), and (2-6), more preferably a group represented by the formula (2-1).

In one embodiment of the present invention,
(2-1) R²³¹ to R²³⁵ that are not the single bond bonded to *o may be all hydrogen atoms,
(2-2) R²⁴¹ to R²⁴⁶ that are not the single bond bonded to *p, and are not the single bond bonded to *q may be all hydrogen atoms,
(2-3) R²⁵¹ to R²⁵⁵ may be all hydrogen atoms,
(2-4) R²⁶¹ to R²⁶⁴, and R²⁶⁵ to R²⁶⁸ that are not the single bond bonded to *r may be all hydrogen atoms,
(2-5) R²⁷¹ to R²⁷⁸, and R²⁷⁹ to R²⁸² that are not the single bond bonded to *s may be all hydrogen atoms,
(2-6) R²⁹² to R²⁹⁶, and R²⁹¹ and R²⁹⁷ to R³⁰⁰ that are not the single bond bonded to *t may be all hydrogen atoms,
(2-7) R³⁰¹ to R³⁰⁴, and R³⁰⁵ to R³⁰⁸ that are not the single bond bonded to *u may be all hydrogen atoms, and
(2-8) R³¹¹ to R³¹⁶ that are not the single bond bonded to *v, are not the single bond bonded to *w, and are not the single bond bonded to *x, and R³²¹ to R³²⁵, and R³³¹ to R³³⁵ may be all hydrogen atoms.

As described above, the "hydrogen atom" used in the description herein includes a protium atom, a deuterium atom, and a tritium atom. Therefore, the inventive compound may contain a naturally derived deuterium atom.

Further, a deuterium atom may be intentionally introduced into the inventive compound by using a deuterated compound for a part or all of raw material compounds. Therefore, in one embodiment of the present invention, the inventive compound contains at least one deuterium atom. That is, the inventive compound (1) is a compound represented by the formula (1), which may be a compound in which at least one of hydrogen atoms contained in the compound is a deuterium atom. The inventive compound (2) is a compound represented by the formula (2), which may be a compound in which at least one of hydrogen atoms contained in the compound is a deuterium atom.

In the compound represented by the formula (1),
at least one hydrogen atom selected from
a hydrogen atom included in the methyl group or the unsubstituted phenyl group represented by either R¹ or R²;
a hydrogen atom included in the methyl group or the unsubstituted phenyl group represented by either R³ or R⁴;
a hydrogen atom represented by any of R¹¹ to R¹⁸, and R²¹ to R²⁸;
a hydrogen atom included in the substituted or unsubstituted phenylene group represented by either L¹ or L²;
a hydrogen atom represented by any of R³¹ to R³⁵, R⁴¹ to R⁴⁶, R⁵¹ to R⁵⁵, R⁶¹ to R⁶⁸, R⁷¹ to R⁸², R⁹¹ to R¹⁰⁰, R¹⁰¹ to R¹⁰⁸, R¹¹¹ to R¹¹⁶, R¹²¹ to R¹²⁵, and R¹³¹ to R¹³⁵; a hydrogen atom included in the substituted or unsubstituted alkyl group, the cycloalkyl group, the aryl group, or the heterocyclic group represented by any of R³¹ to R³⁵, R⁴¹ to R⁴⁶, R⁵¹ to R⁵⁵, R⁶¹ to R⁶⁸, R⁷¹ to R⁸², R⁹¹ to R¹⁰⁰, R¹⁰¹ to R¹⁰⁸, R¹¹¹ to R¹¹⁶, R¹²¹ to R¹²⁵, and R¹³¹ to R¹³⁵; and
a hydrogen atom represented by either R^{a} or R^{b}; and a hydrogen atom included in the methyl group, or the substituted and unsubstituted phenyl group represented by either R^{a} or R^{b}
may be a deuterium atom.

In the compound represented by the formula (2),
at least one hydrogen atom selected from
a hydrogen atom included in the methyl group or the unsubstituted phenyl group represented by any of R²⁰¹ to R²⁰⁴;
a hydrogen atom represented by any of R²¹¹ to R²¹⁸, and R²²¹ to R²²⁸;
a hydrogen atom included in the substituted or unsubstituted phenylene group represented by either L¹¹ or L¹²;
a hydrogen atom represented by any of R²³¹ to R²³⁵, R²⁴¹ to R²⁴⁶, R²⁵¹ to R²⁵⁵, R²⁶¹ to R²⁶⁸, R²⁷¹ to R²⁸², R²⁹¹ to R³⁰⁰, R³⁰¹ to R³⁰⁸, R³¹¹ to R³¹⁶, R³²¹ to R³²⁵, and R³³¹ to R³³⁵; a hydrogen atom included in the substituted or unsubstituted alkyl group, the cycloalkyl group, the aryl group, or the heterocyclic group represented by any of R²³¹ to R²³⁵, R²⁴¹ to R²⁴⁶, R²⁵¹ to R²⁵⁵, R²⁶¹ to R²⁶⁸, R²⁷¹ to R²⁸², R²⁹¹ to R³⁰⁰, R³⁰¹ to R³⁰⁸, R³¹¹ to R³¹⁶, R³²¹ to R³²⁵, and R³³¹ to R³³⁵; and
a hydrogen atom represented by either R^{c} or R^{d}; and a hydrogen atom included in the methyl group or the substituted and unsubstituted phenyl group represented by either R^{c} or R^{d}
may be a deuterium atom.

The deuteration rate of the inventive compound depends on the deuteration rates of raw material compounds to be used. Even if a raw material with a predetermined deuteration rate is used, a certain proportion of naturally derived protium isotopes may be contained. Therefore, in the embodiment of the deuteration rate of the inventive compound as illustrated below, in addition to the rate obtained by simply counting the number of deuterium atoms represented by the chemical formula, the rate on which a trace amount of naturally derived isotopes are reflected is included.

The deuteration rate of the inventive compound is preferably 1% or more, more preferably 3% or more, further preferably 5% or more, still further preferably 10% or more, still further preferably 50% or more.

The inventive compound may be a mixture containing a deuterated compound and a non-deuterated compound, or a mixture of two or more compounds having different deuteration rates. The deuteration rate of such a mixture is preferably 1% or more, more preferably 3% or more, further preferably 5% or more, still further preferably 10% or more, still further preferably 50% or more, and is less than 100%.

Further, the ratio of each of the numbers of deuterium atoms to the total number of hydrogen atoms in the inventive compound is preferably 1% or more, more preferably 3% or more, further preferably 5% or more, still further preferably 10% or more, and is 100% or less.

In the case of "substituted or unsubstituted" included in the definition of each of the above formulas, details of substituents (optional substituents) are as described in "Substituents in the case of "substituted or unsubstituted"".

The inventive compound can be easily produced by those skilled in the art with reference to the following synthesis examples and known synthesis methods.

Hereinafter, specific examples of the inventive compound will be illustrated, but are not limited to the following exemplary compounds.

In the following specific examples, D represents a deuterium atom.

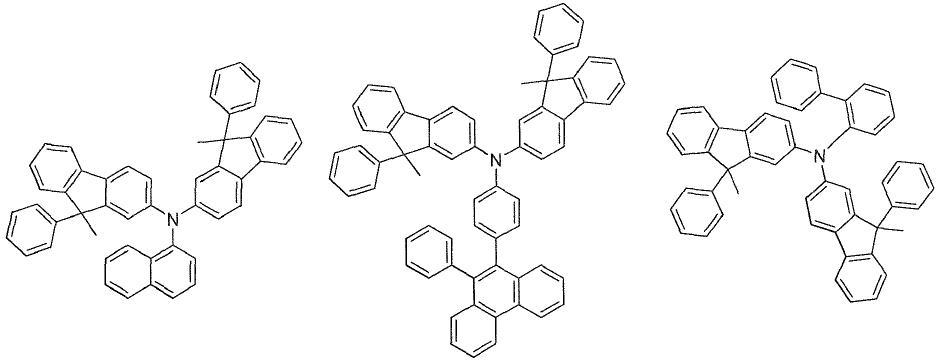

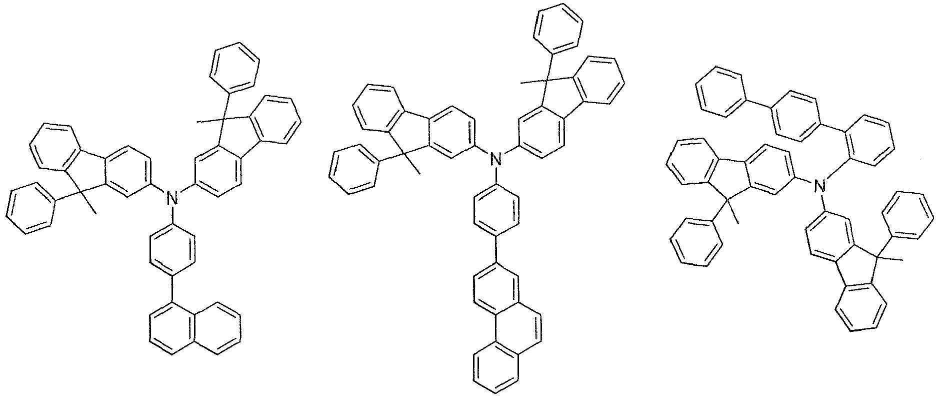

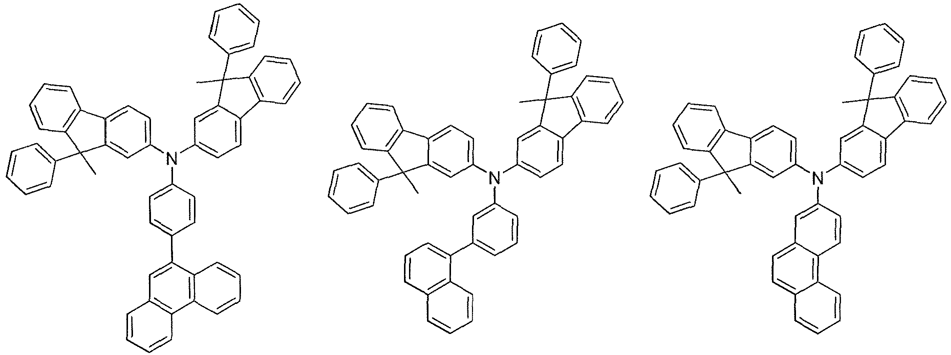

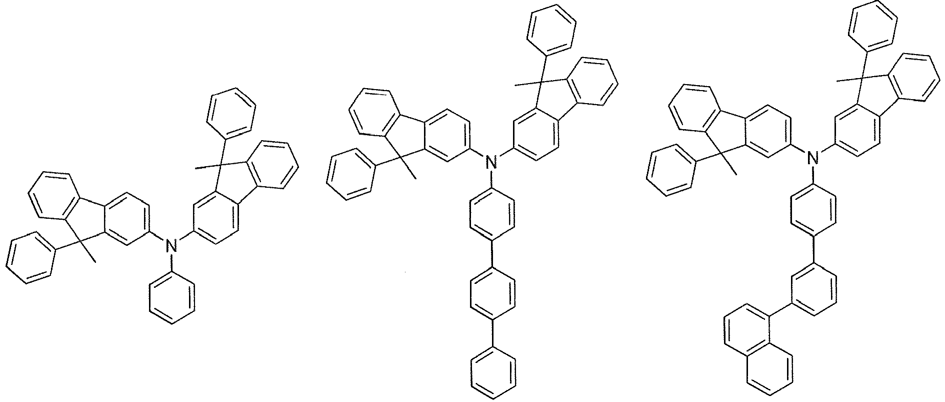

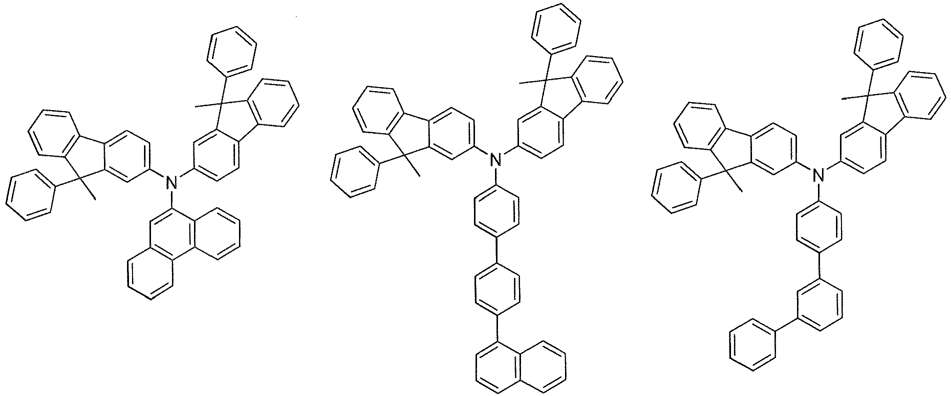

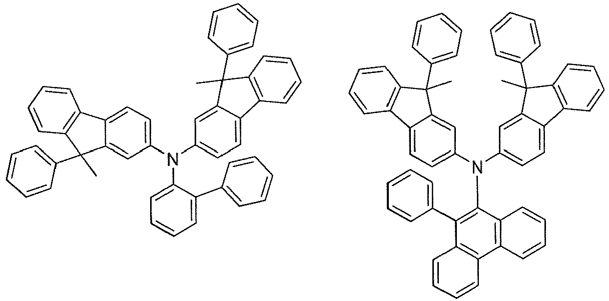

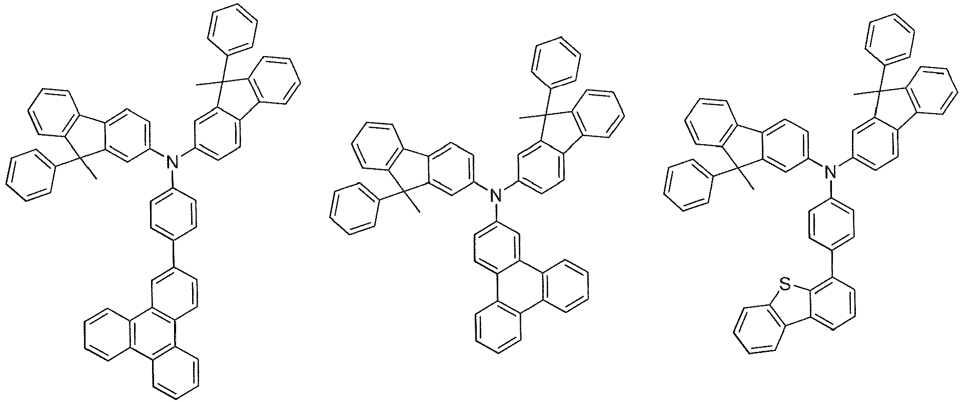

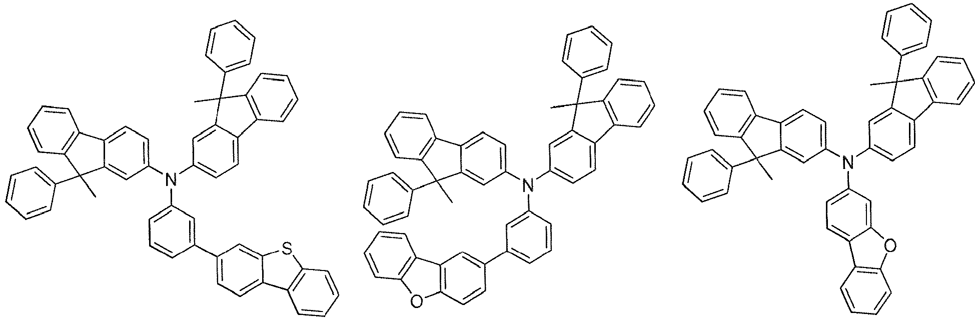

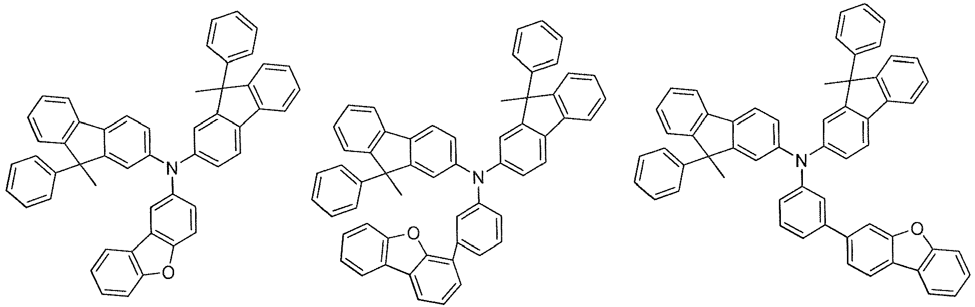

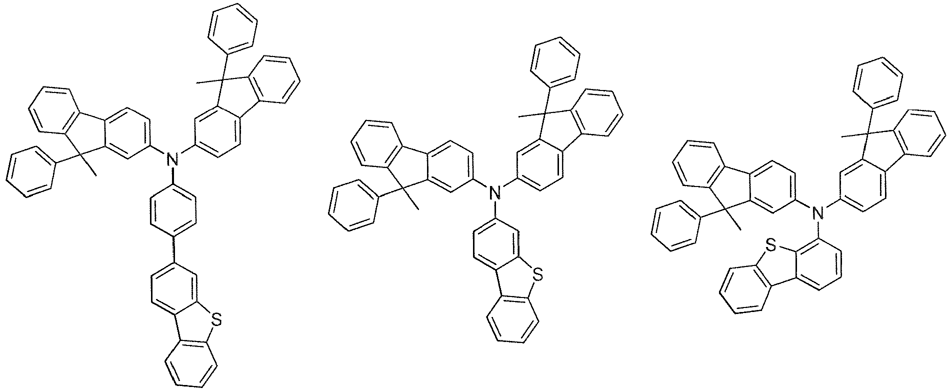

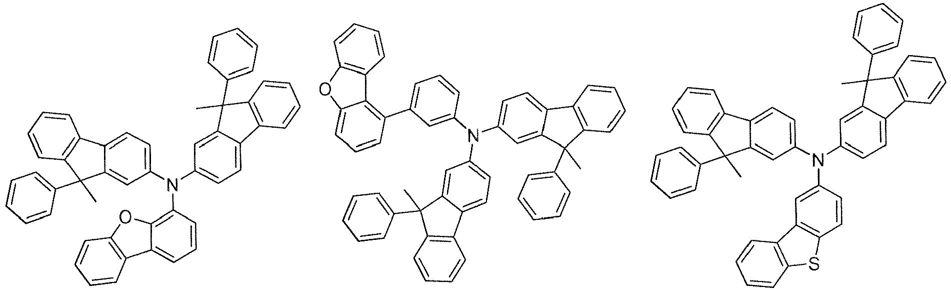

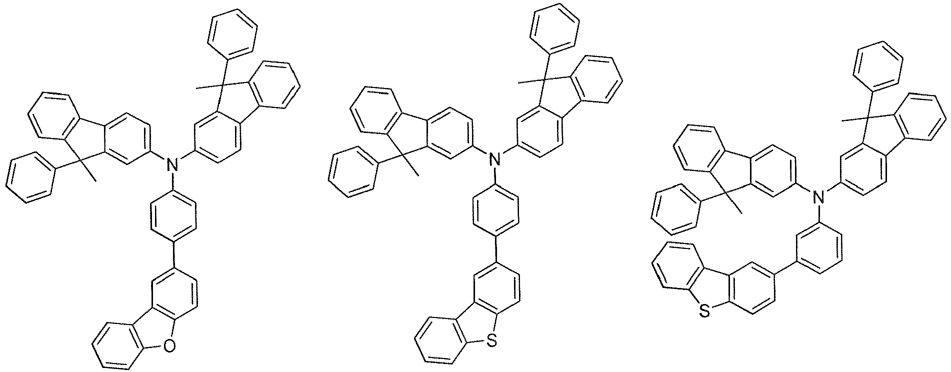

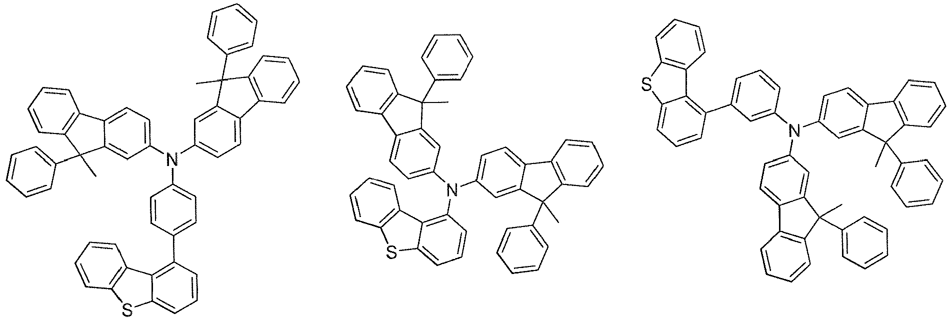

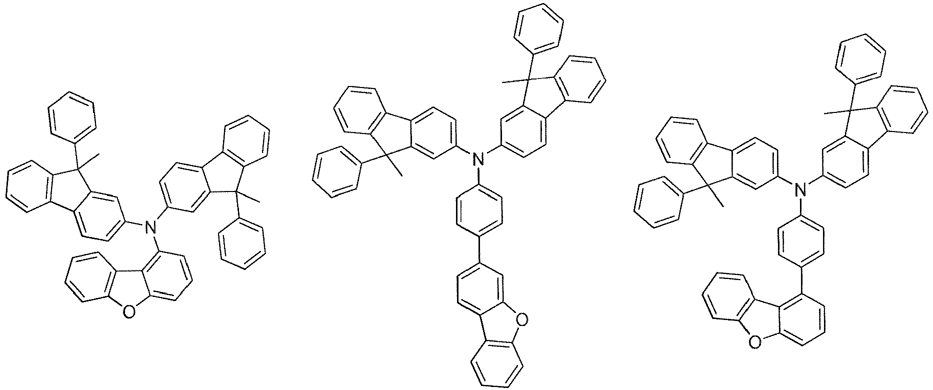

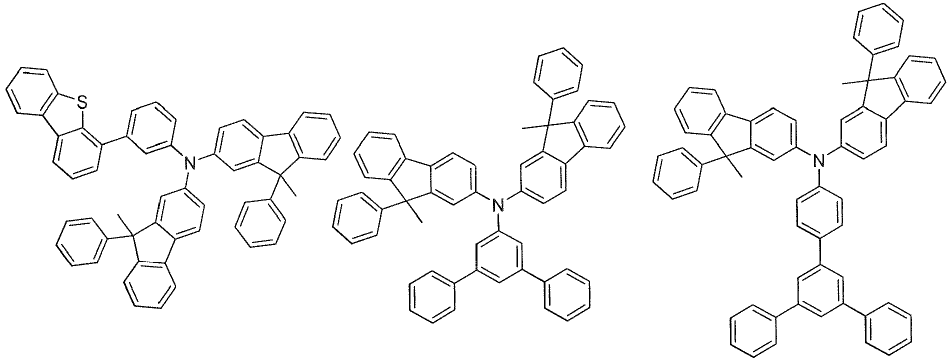

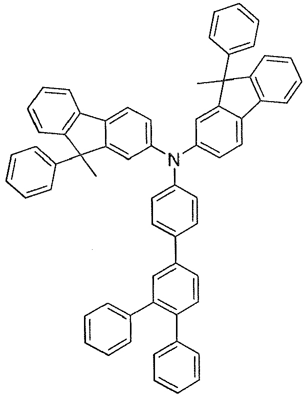

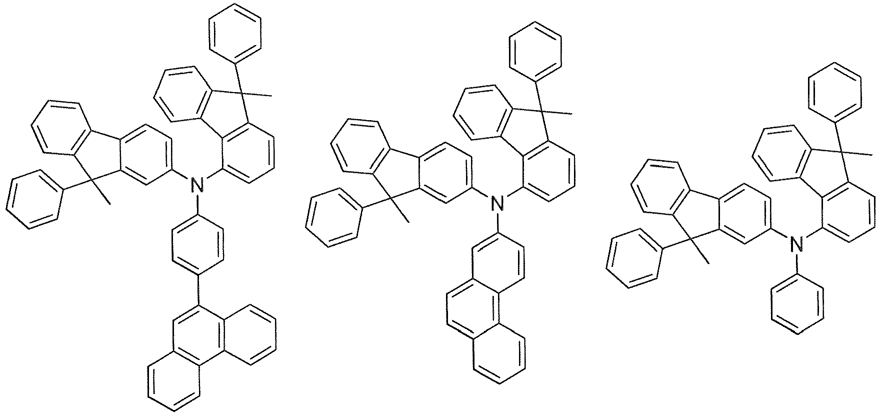

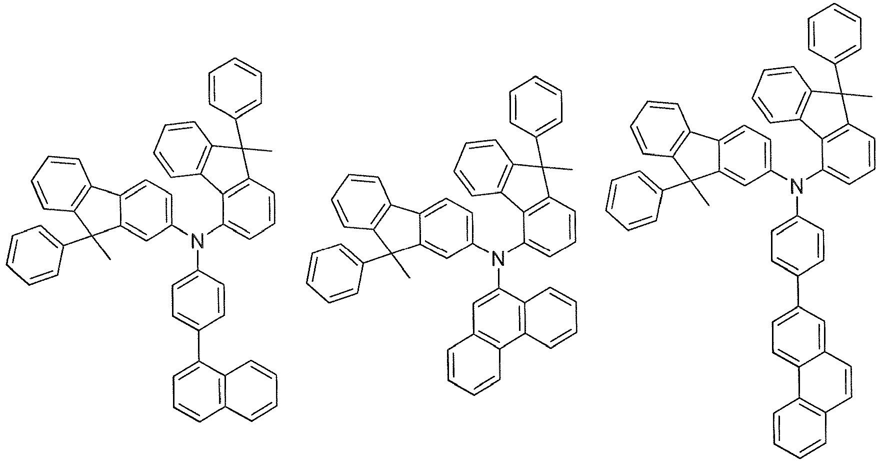

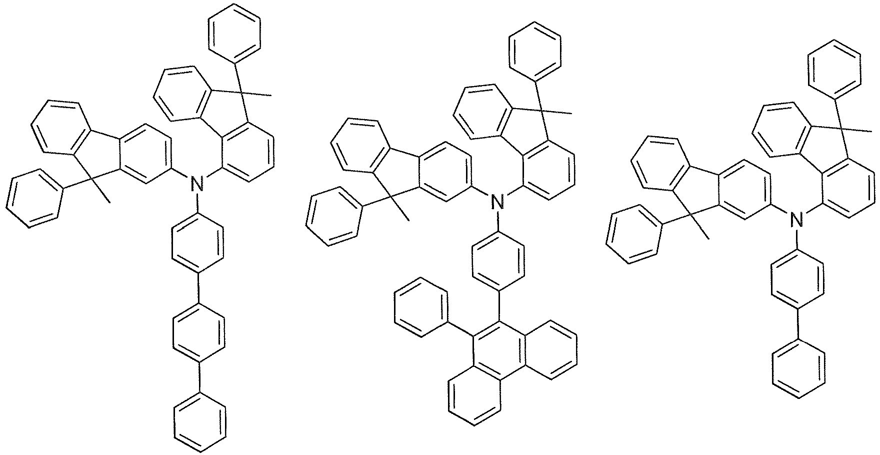

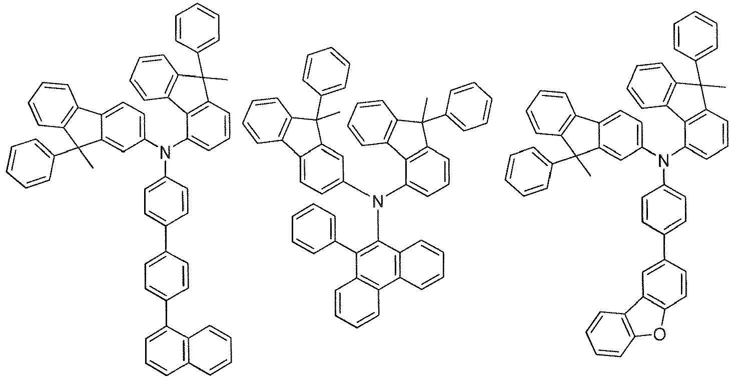

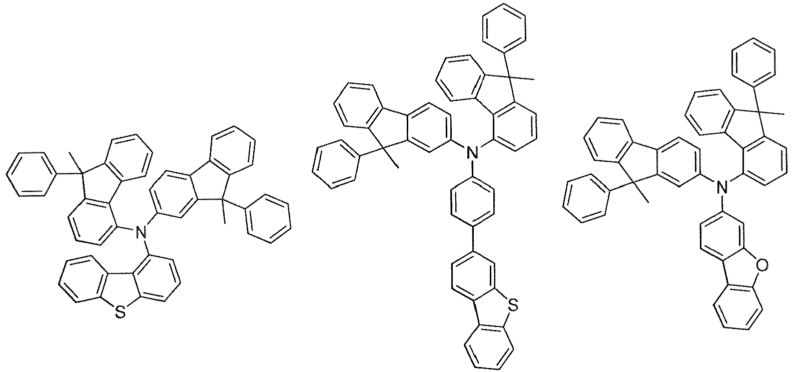

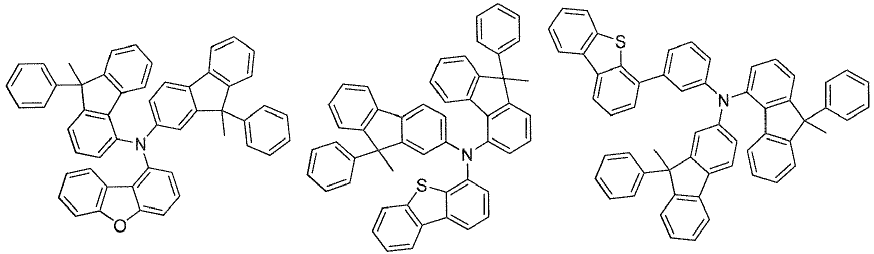

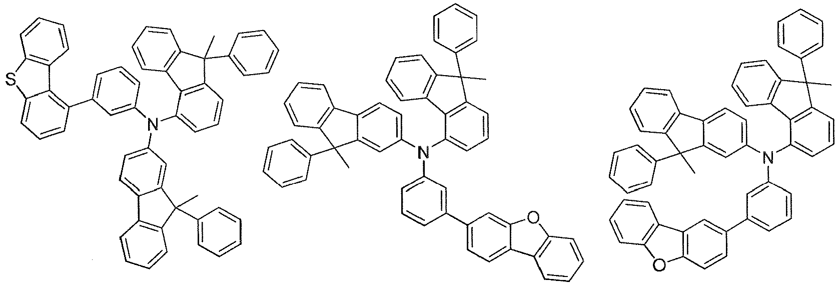

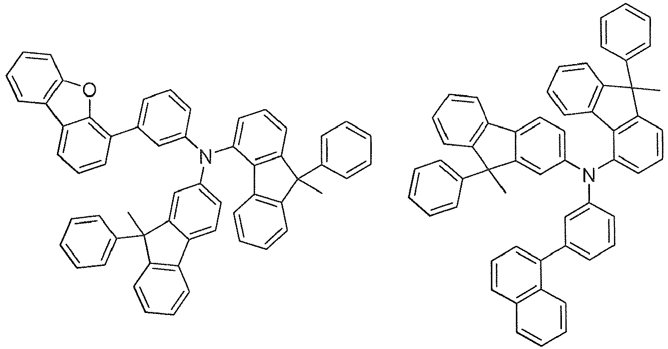

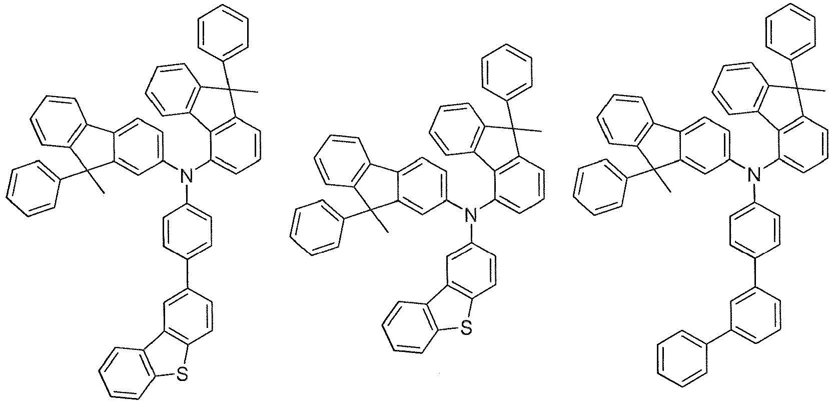

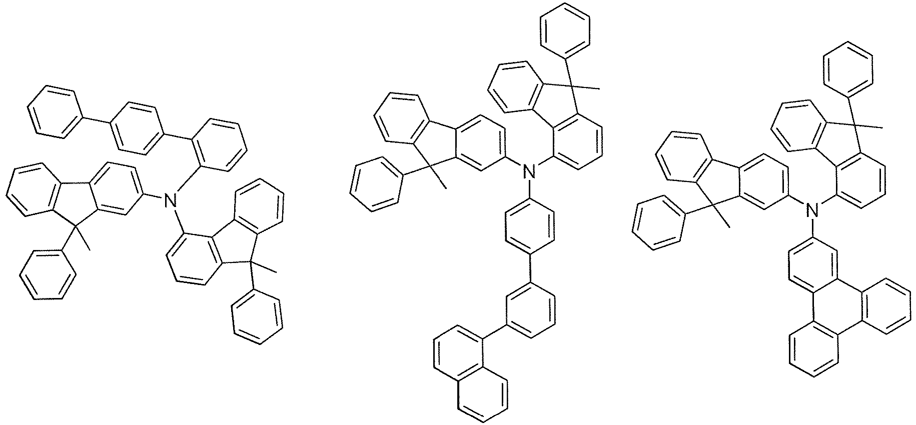

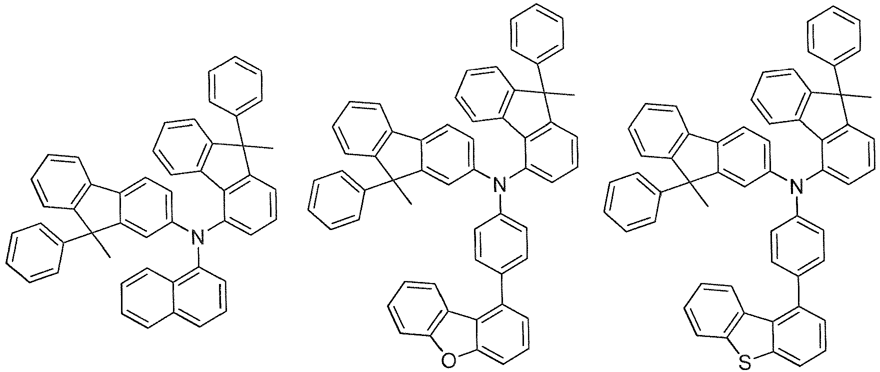

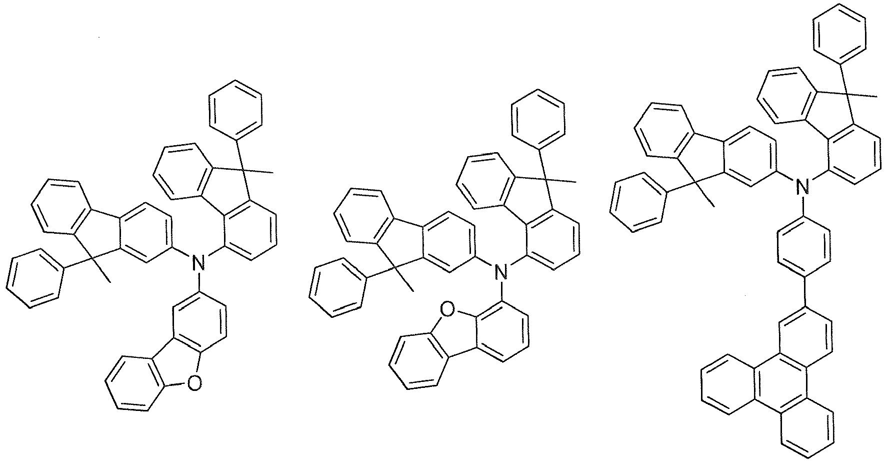

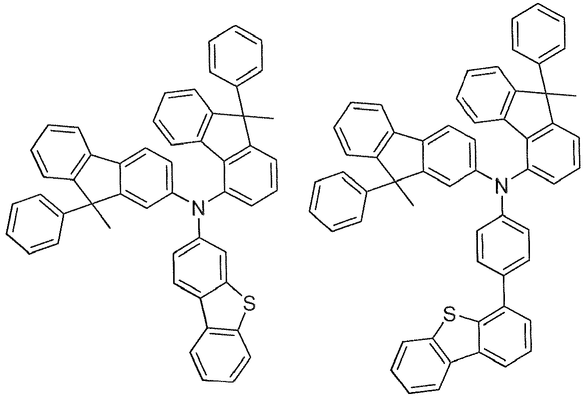

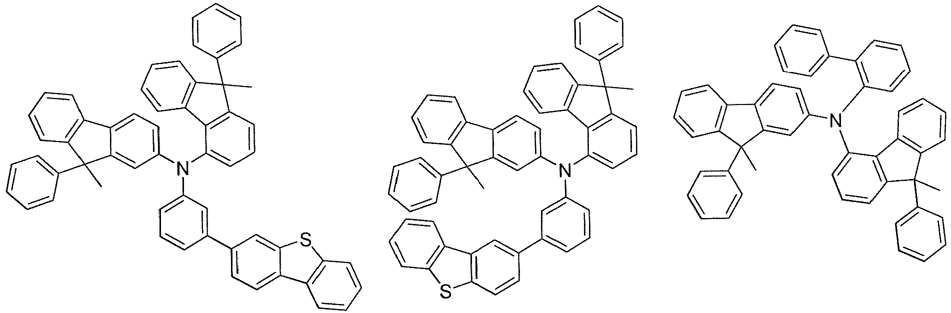

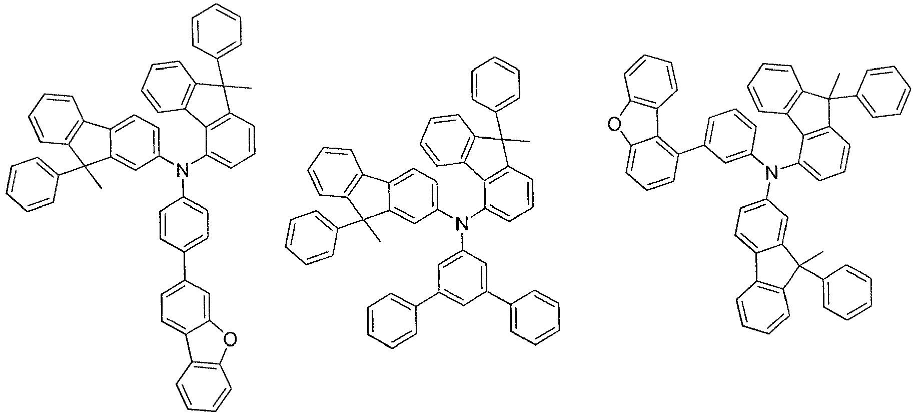

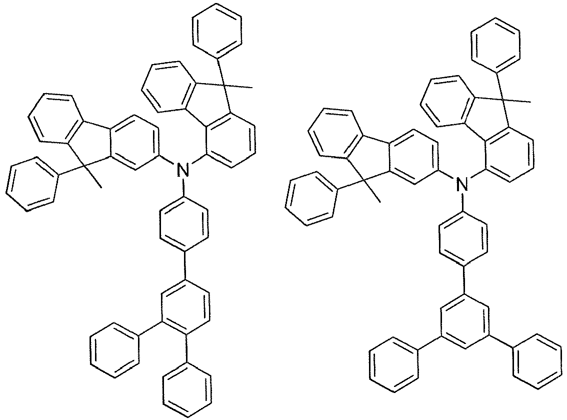

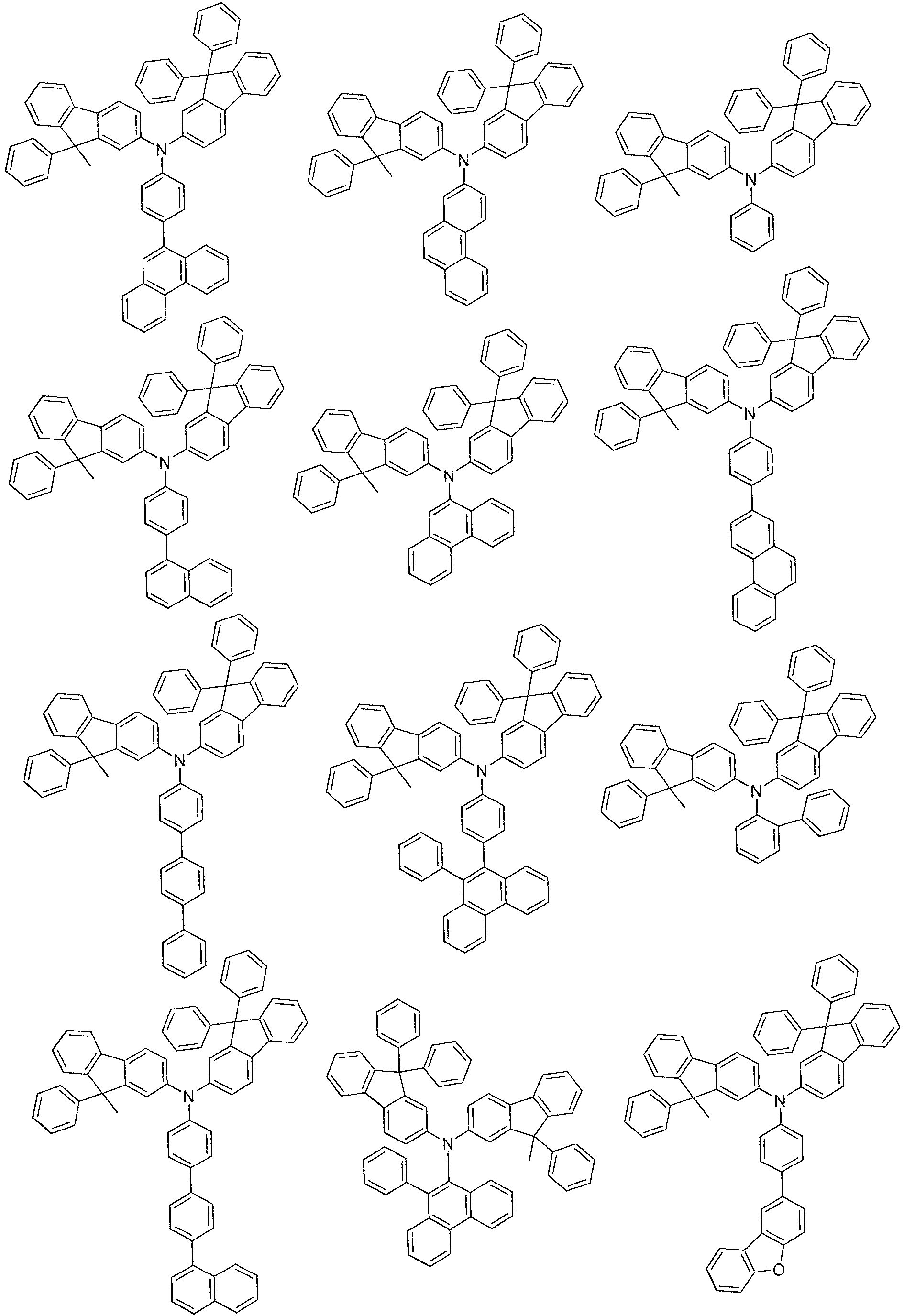

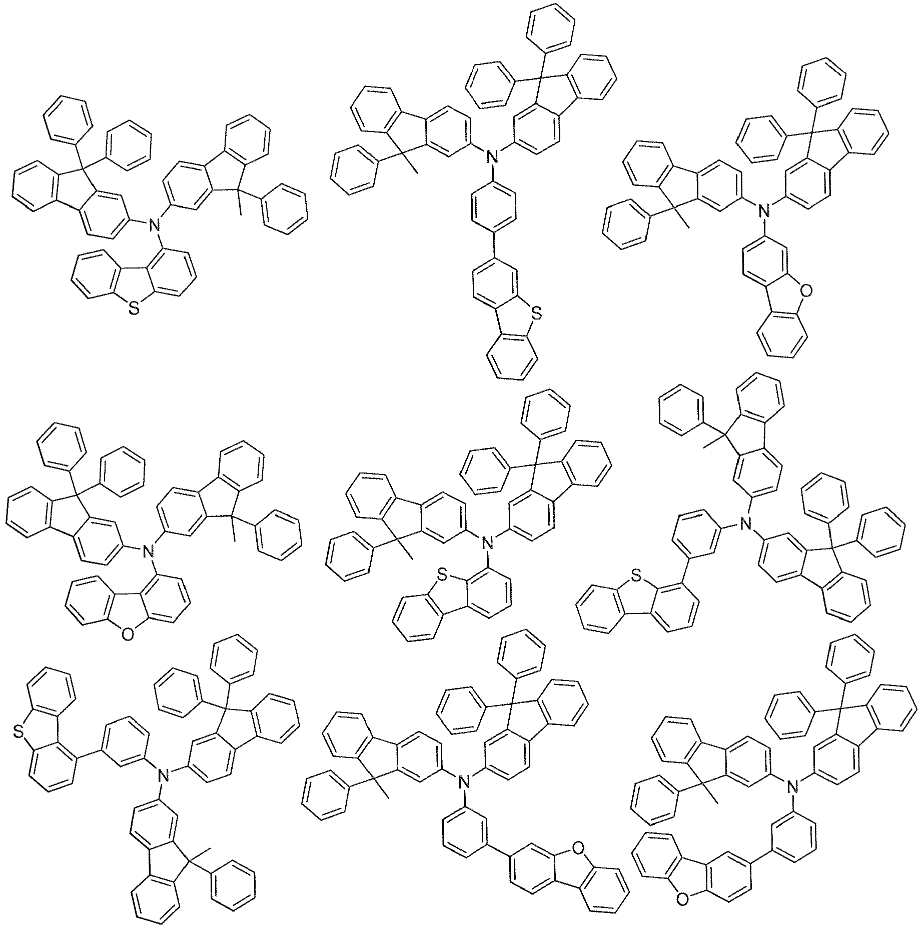

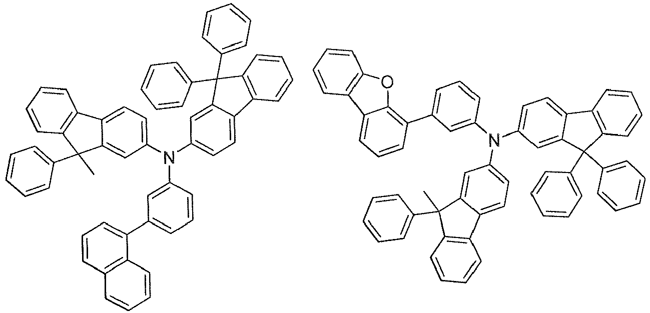

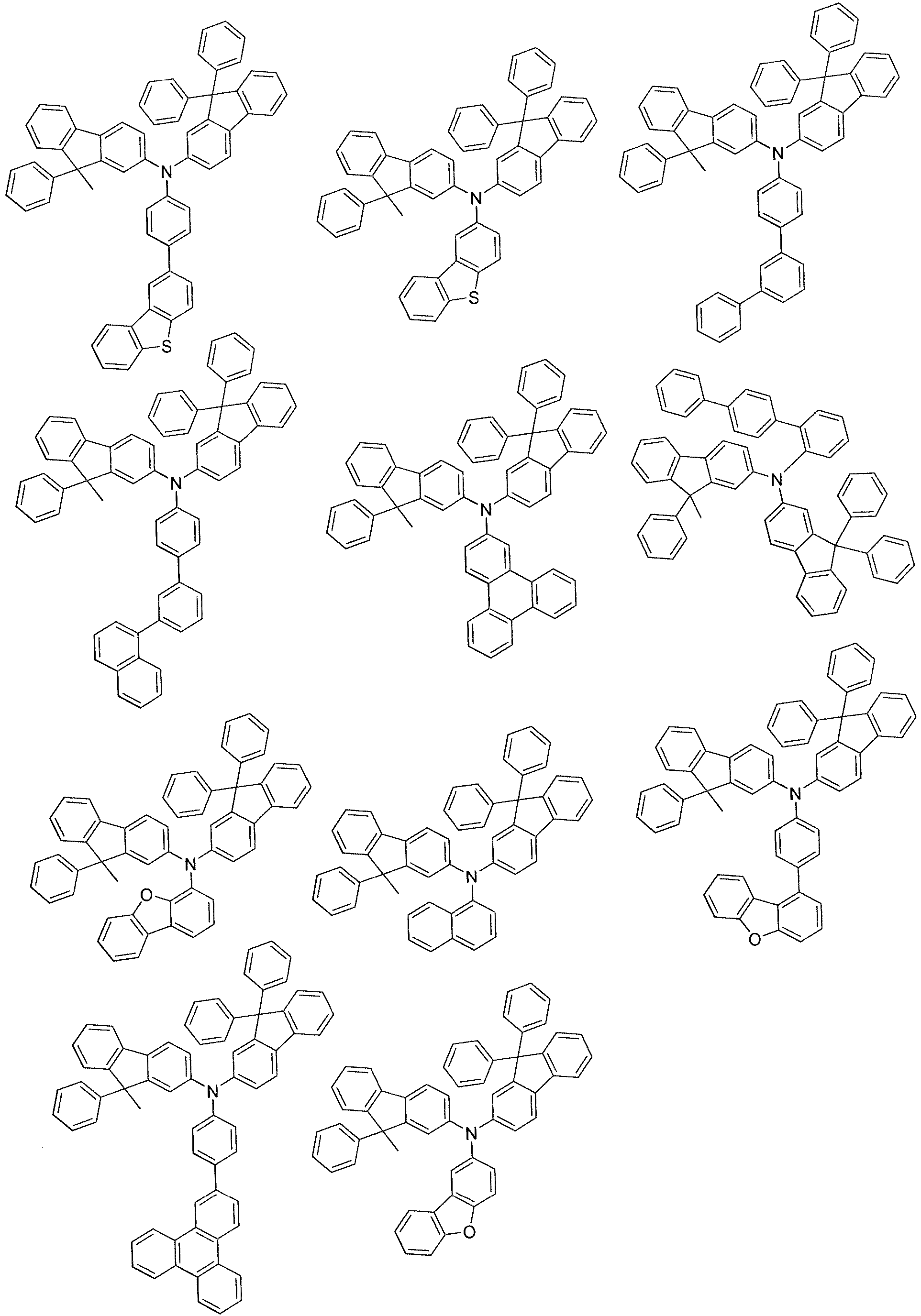

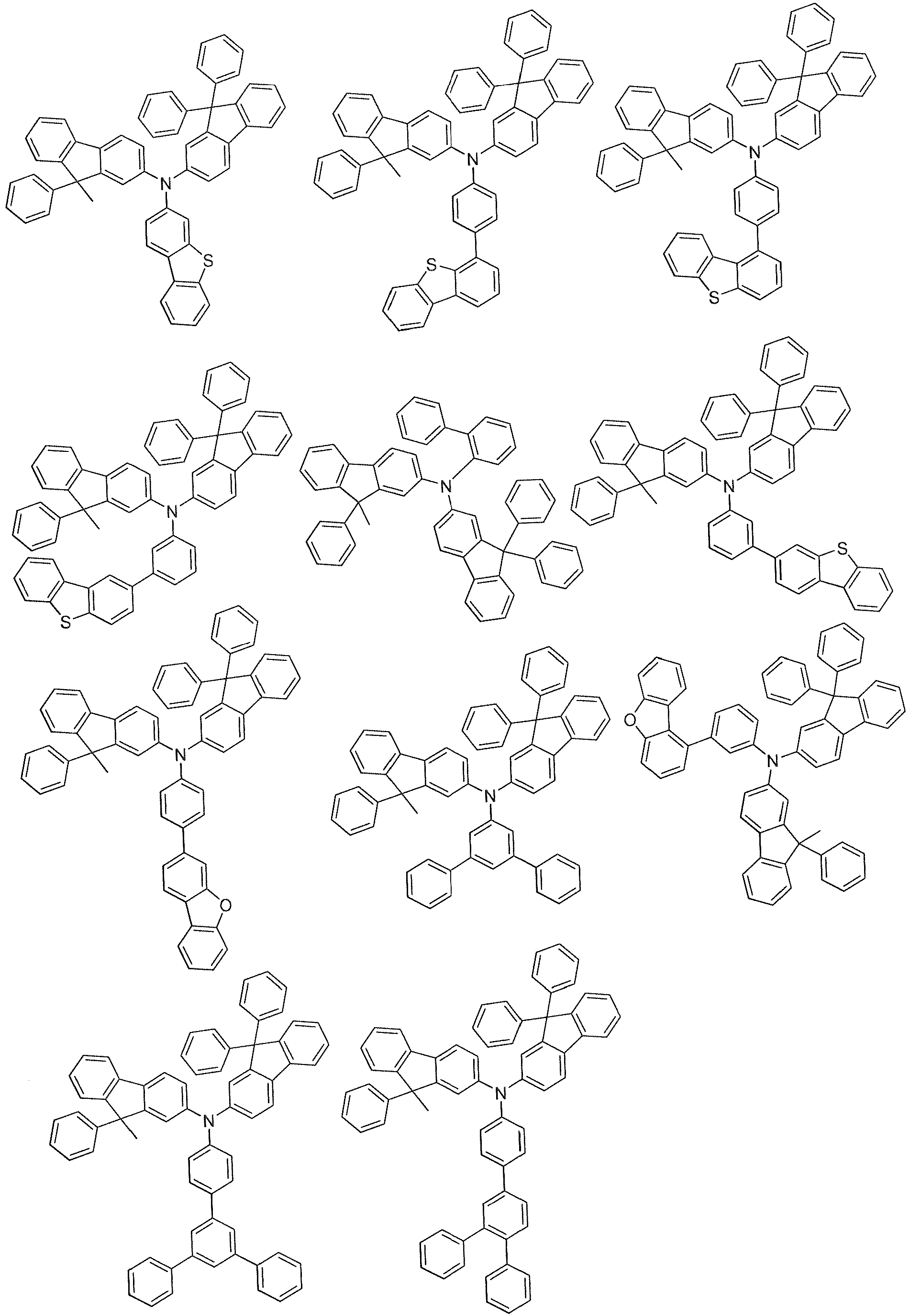

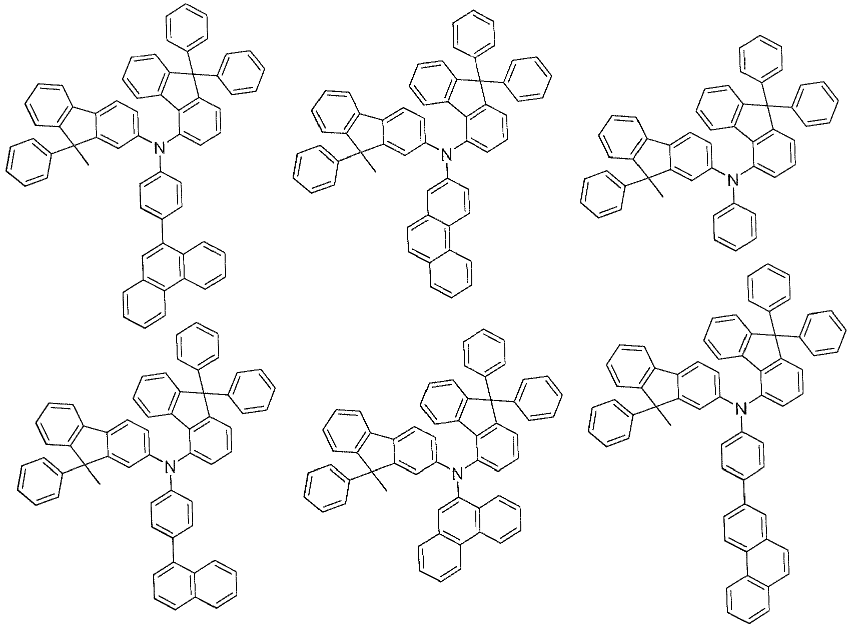

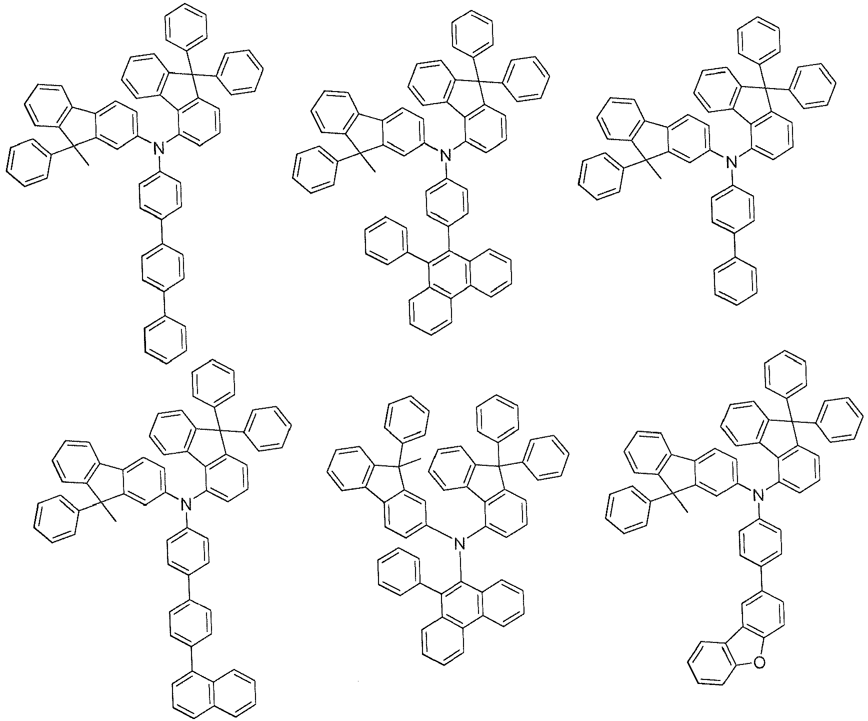

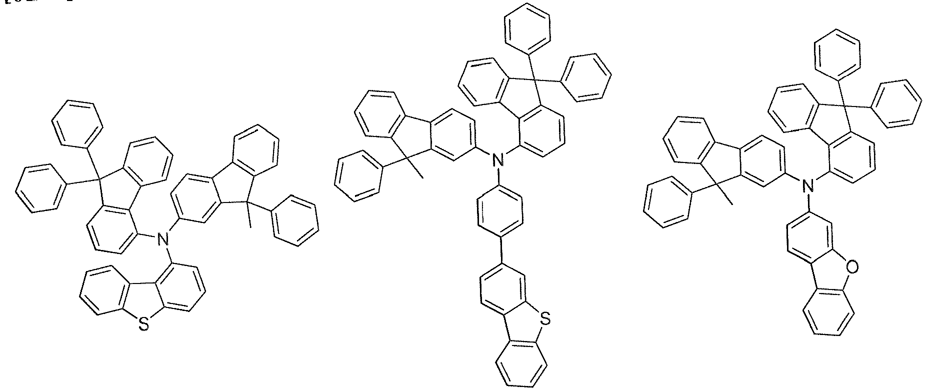

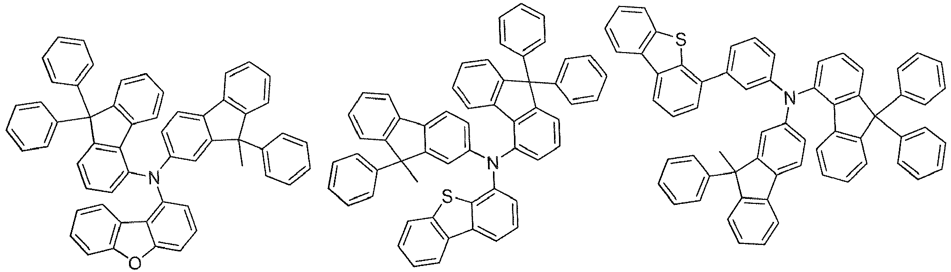

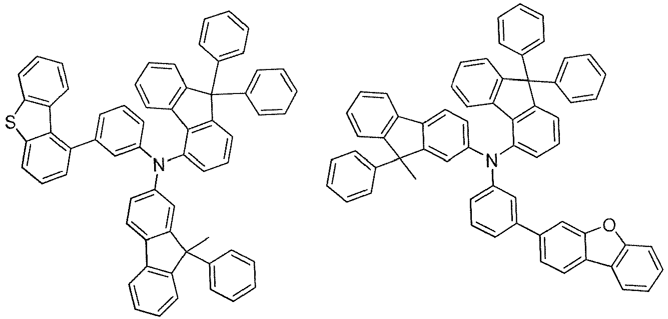

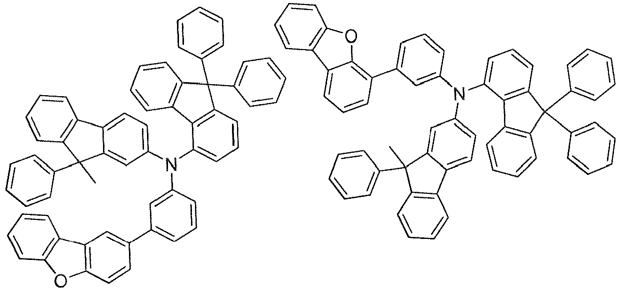

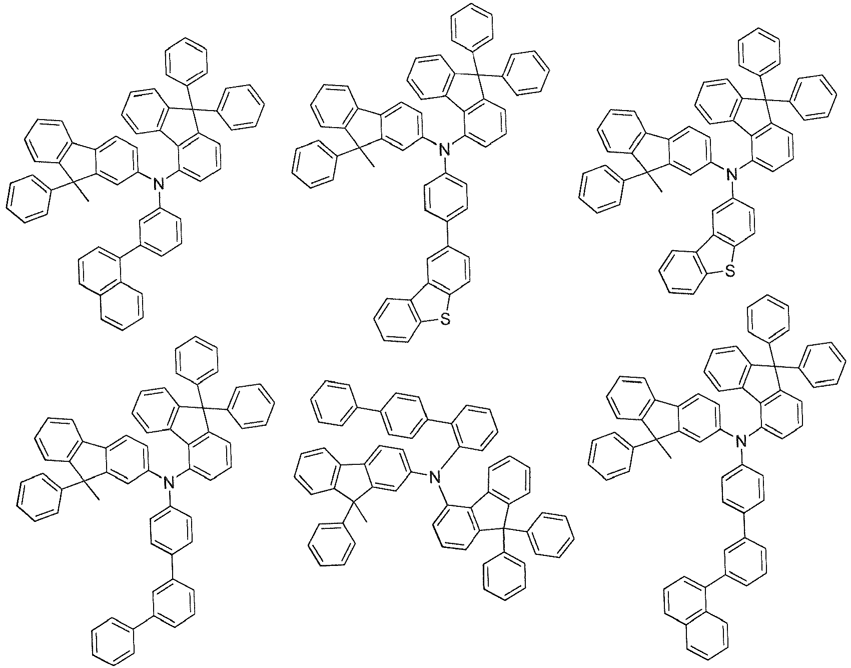

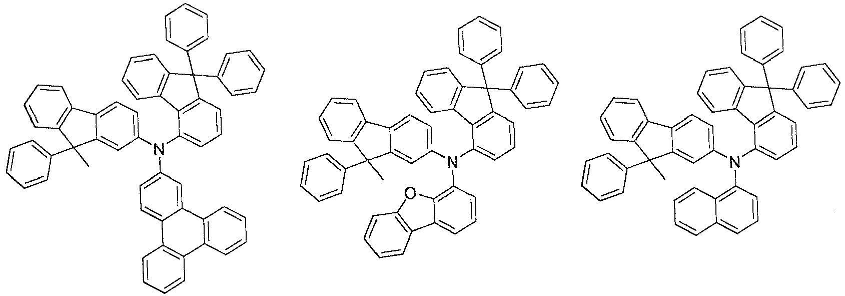

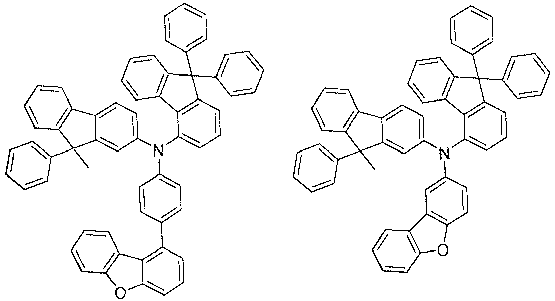

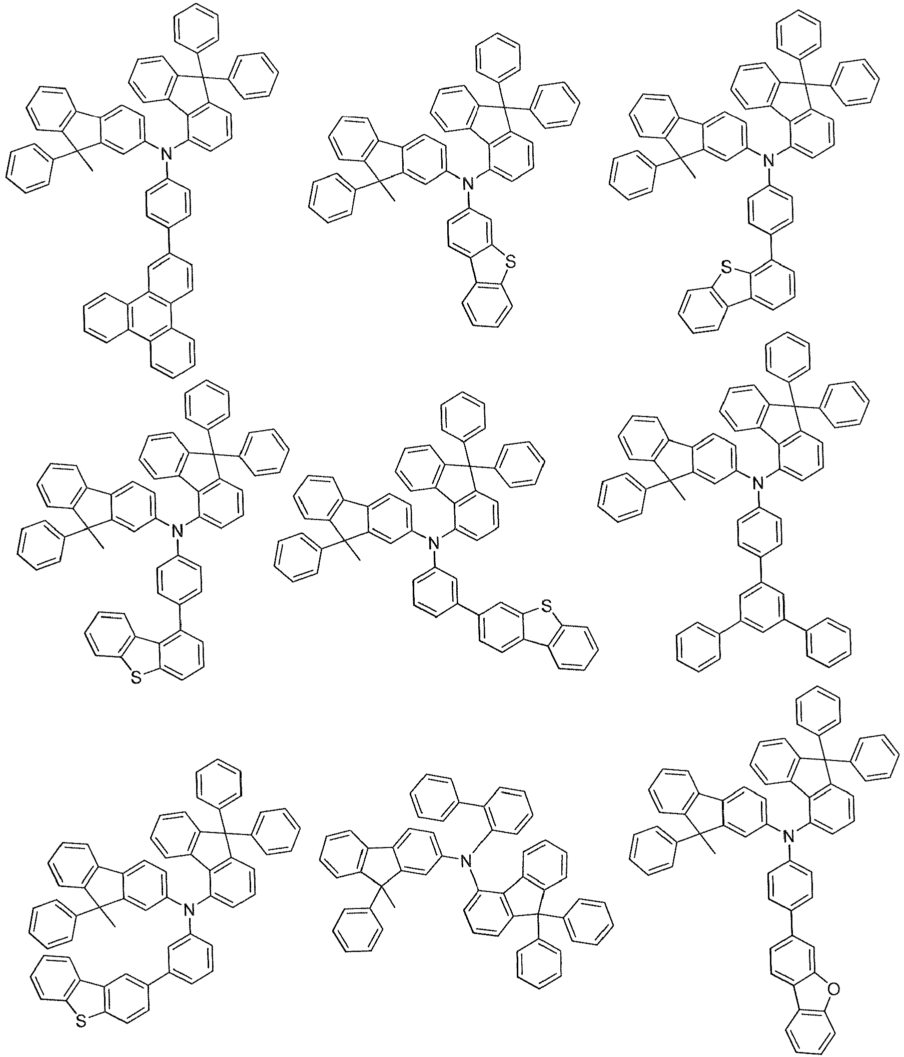

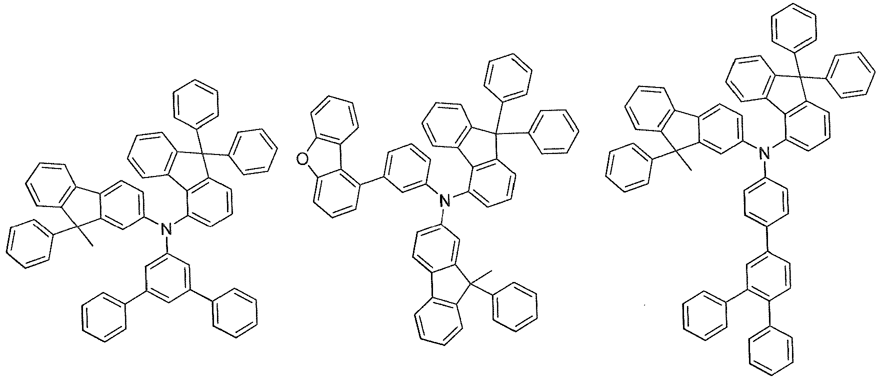

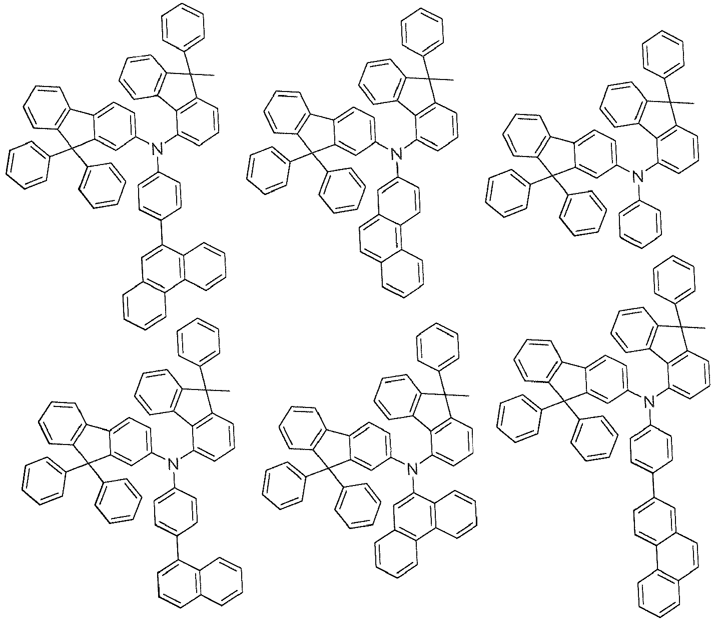

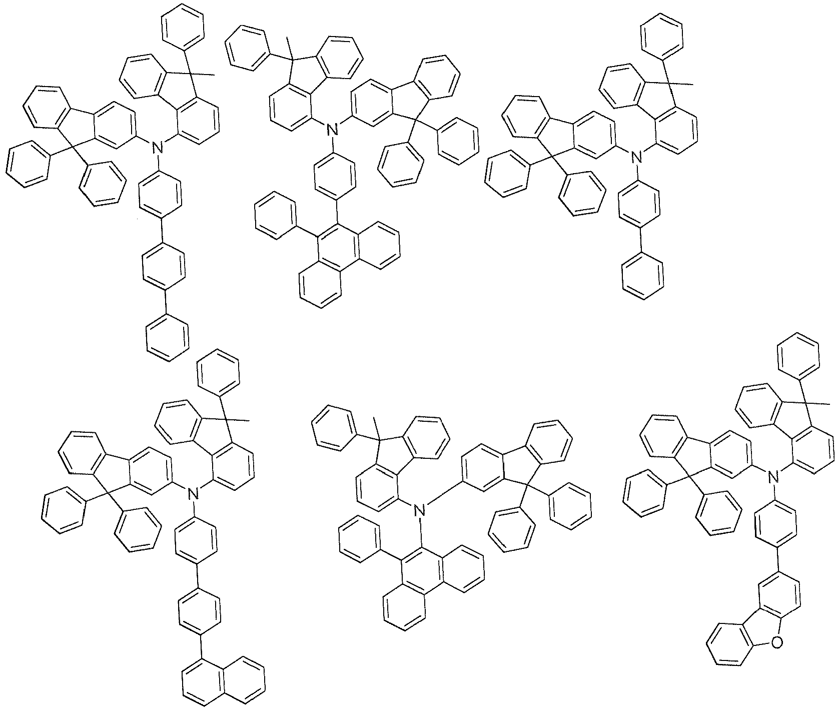

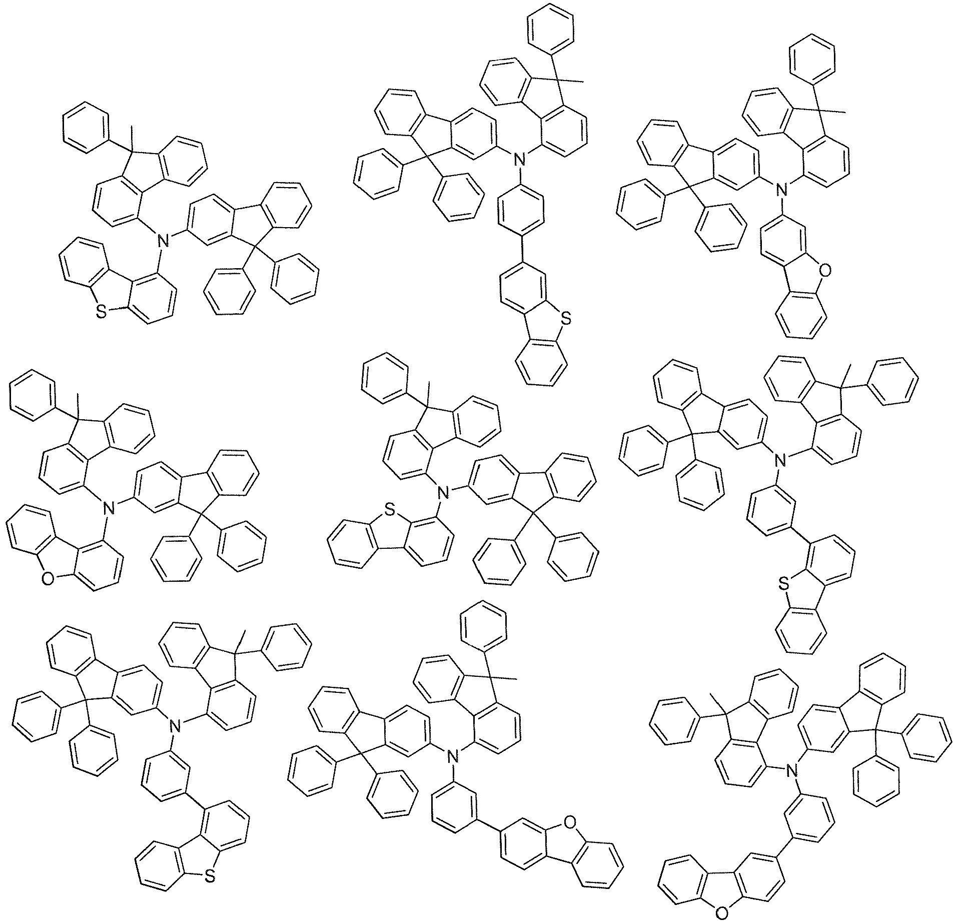

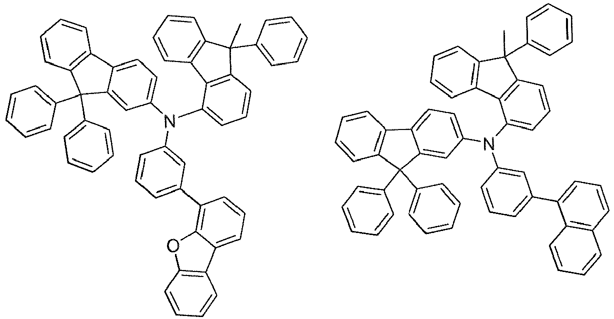

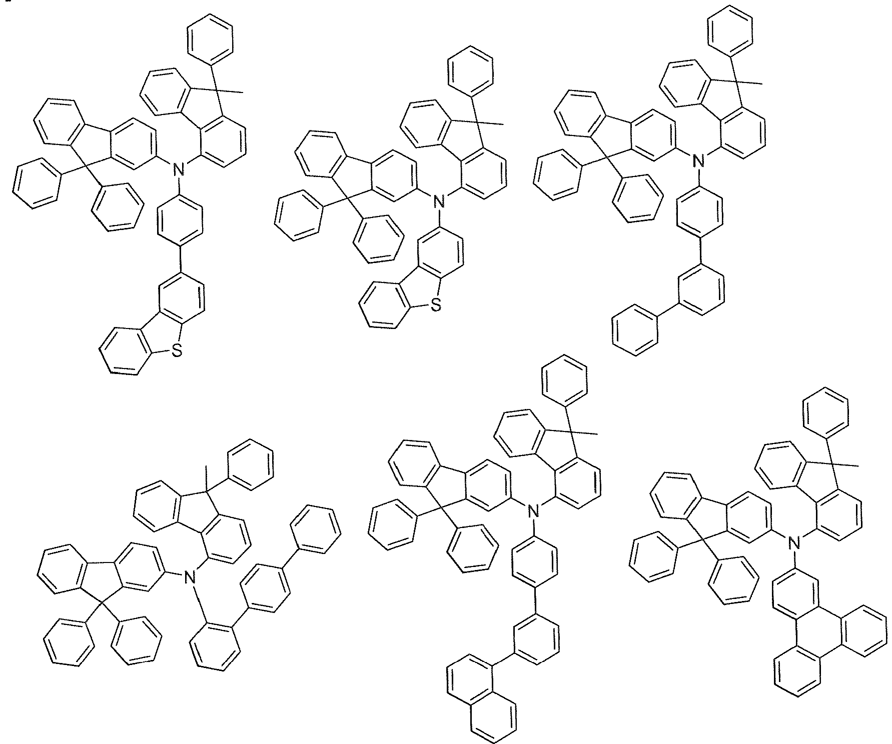

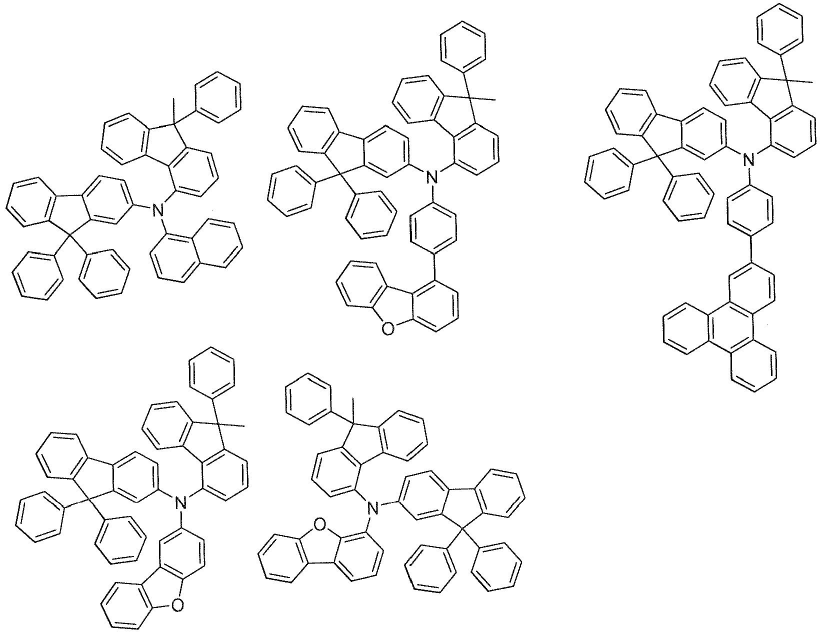

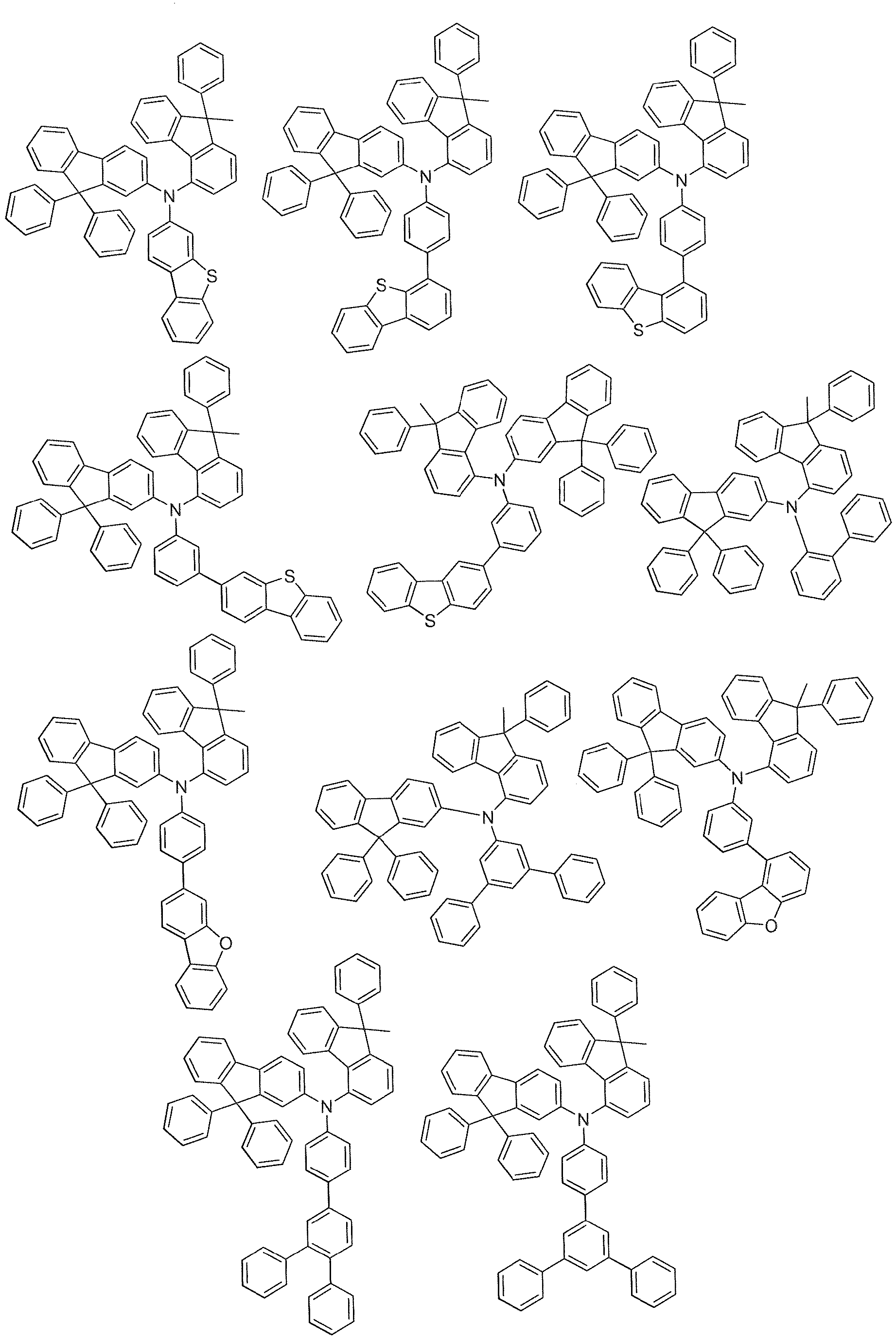

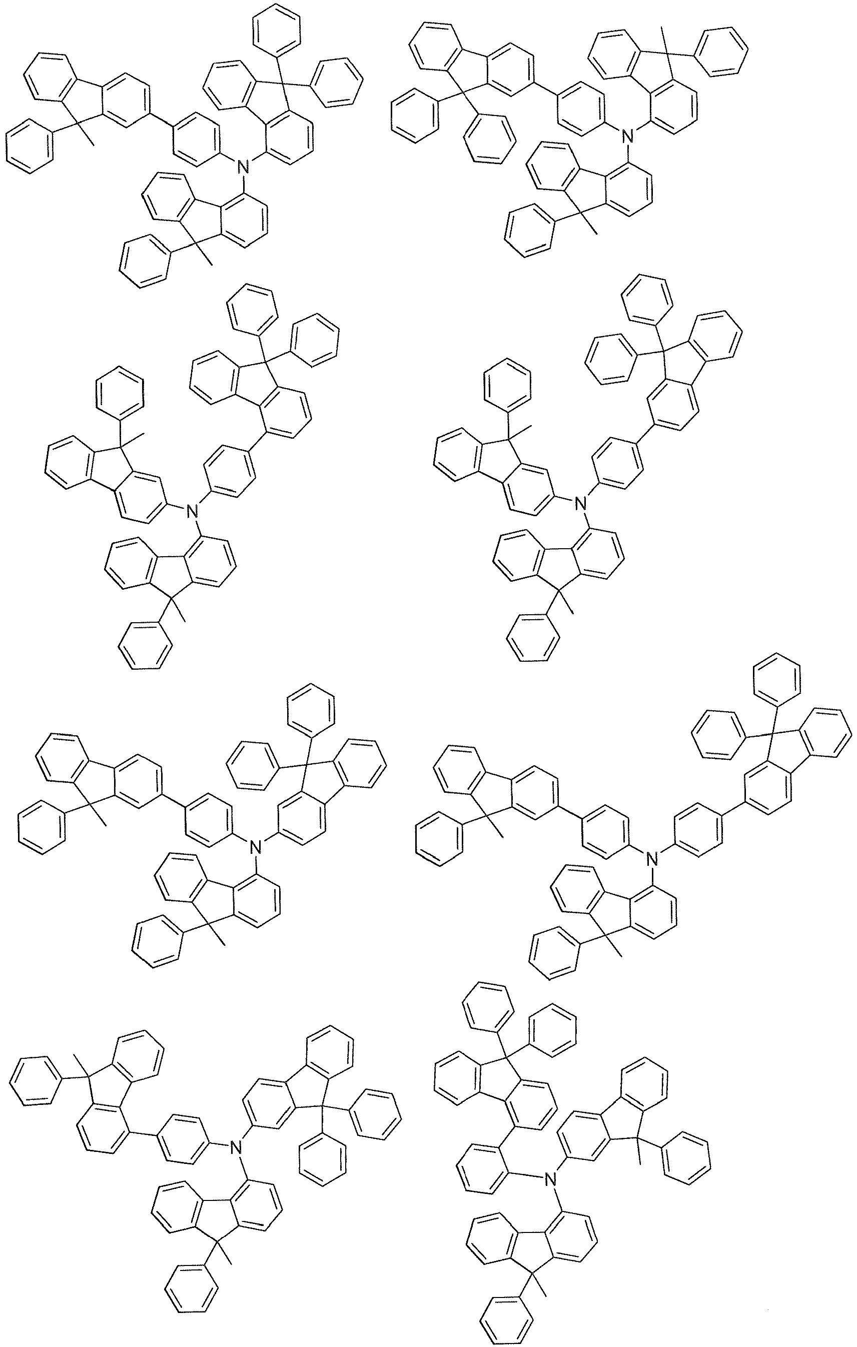

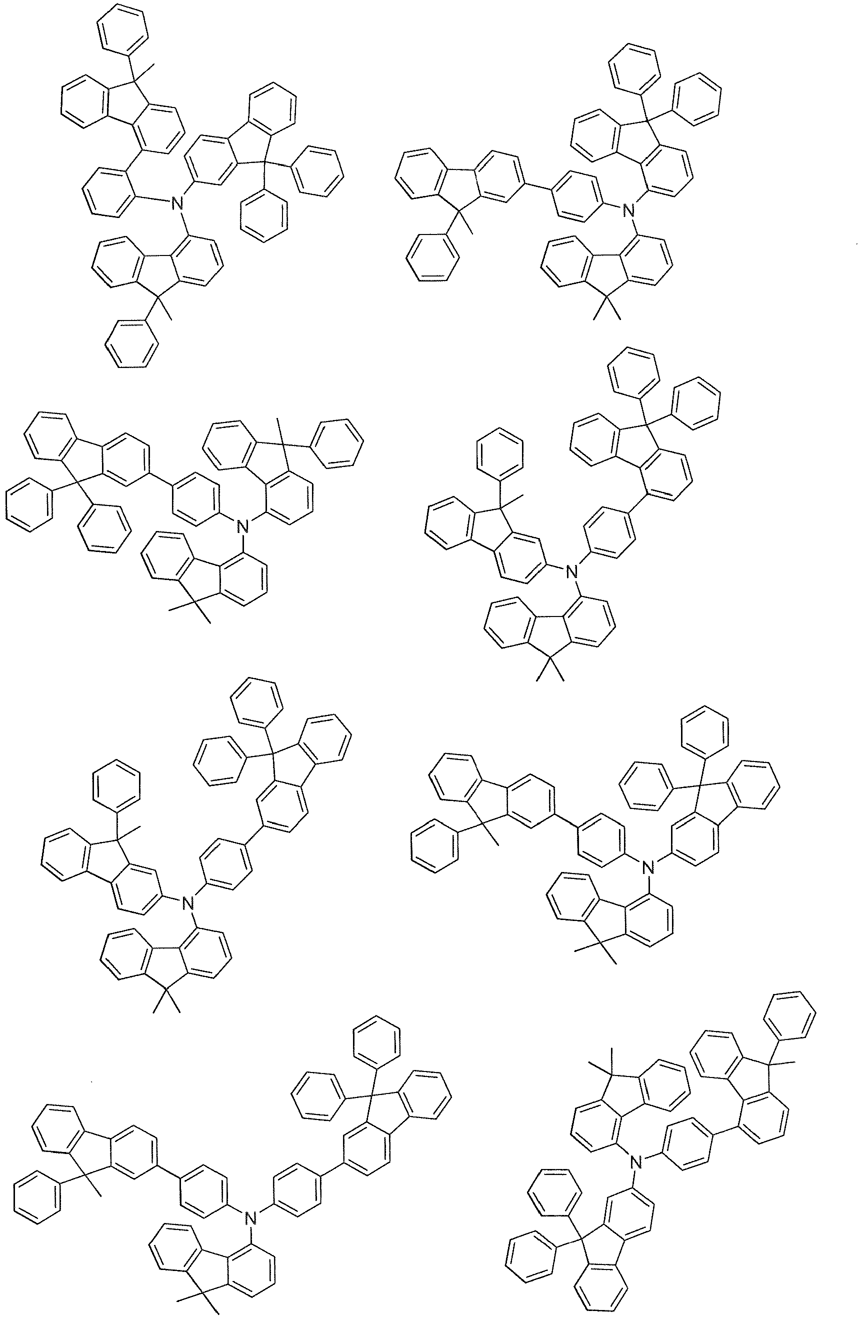

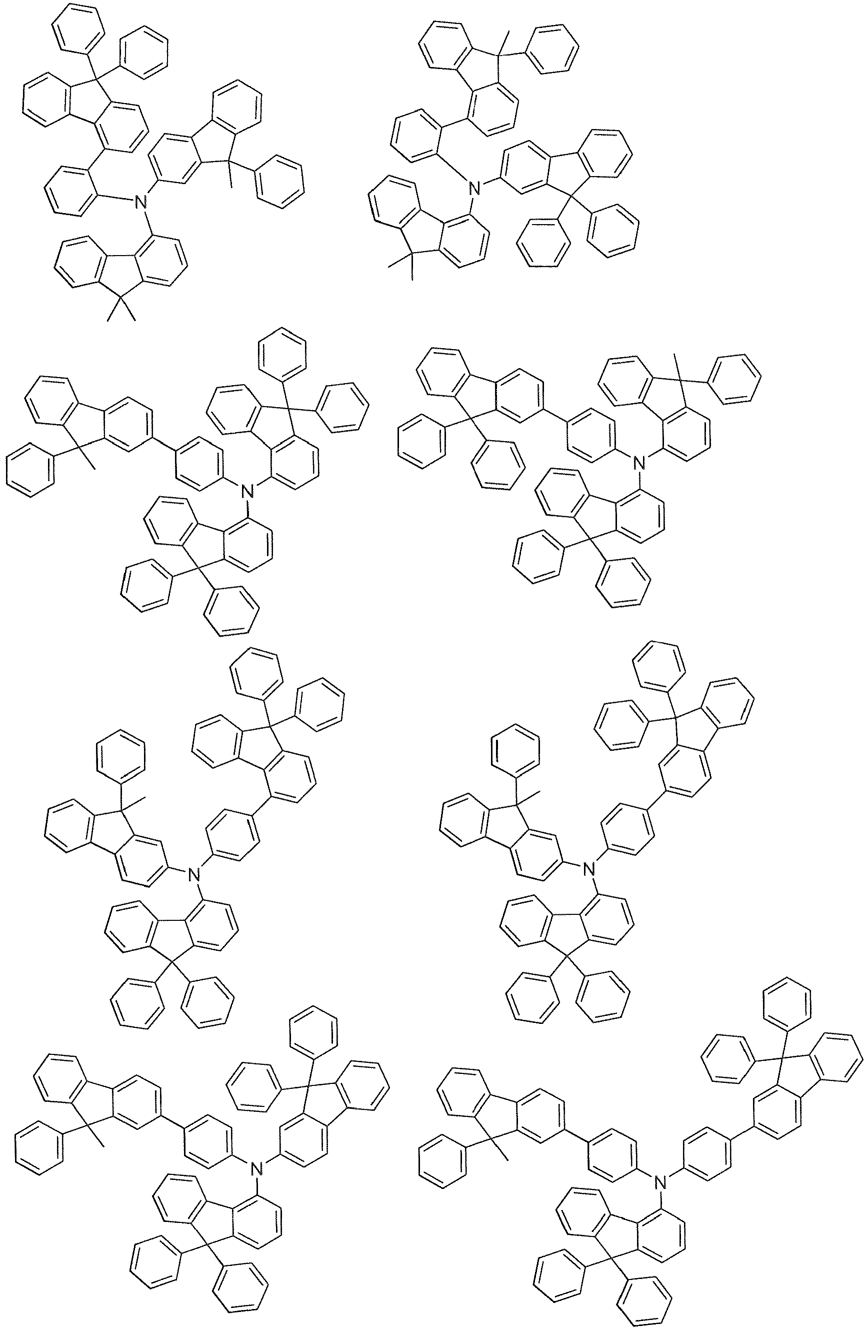

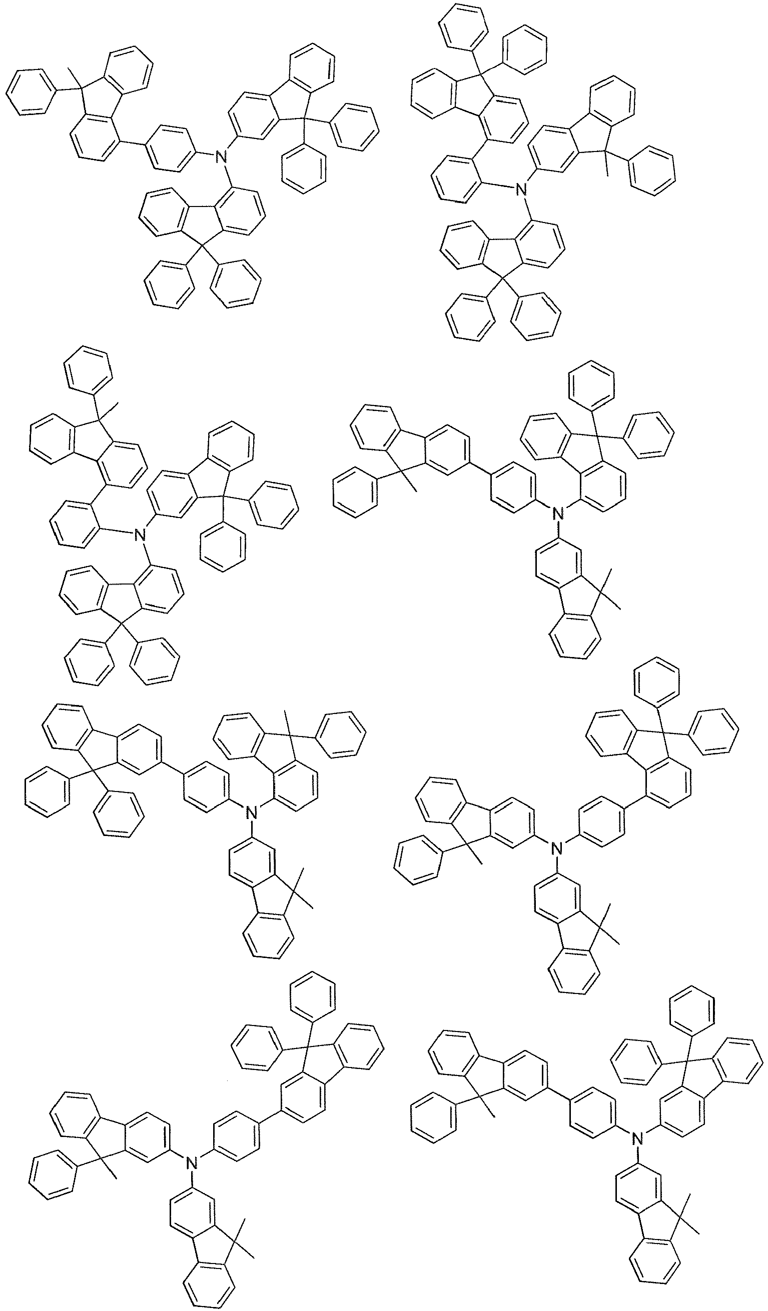

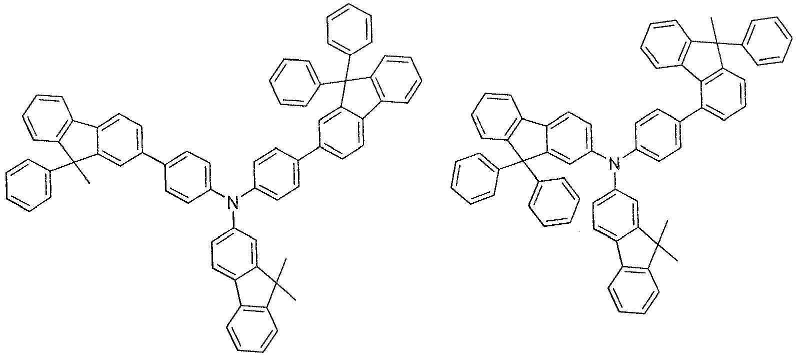

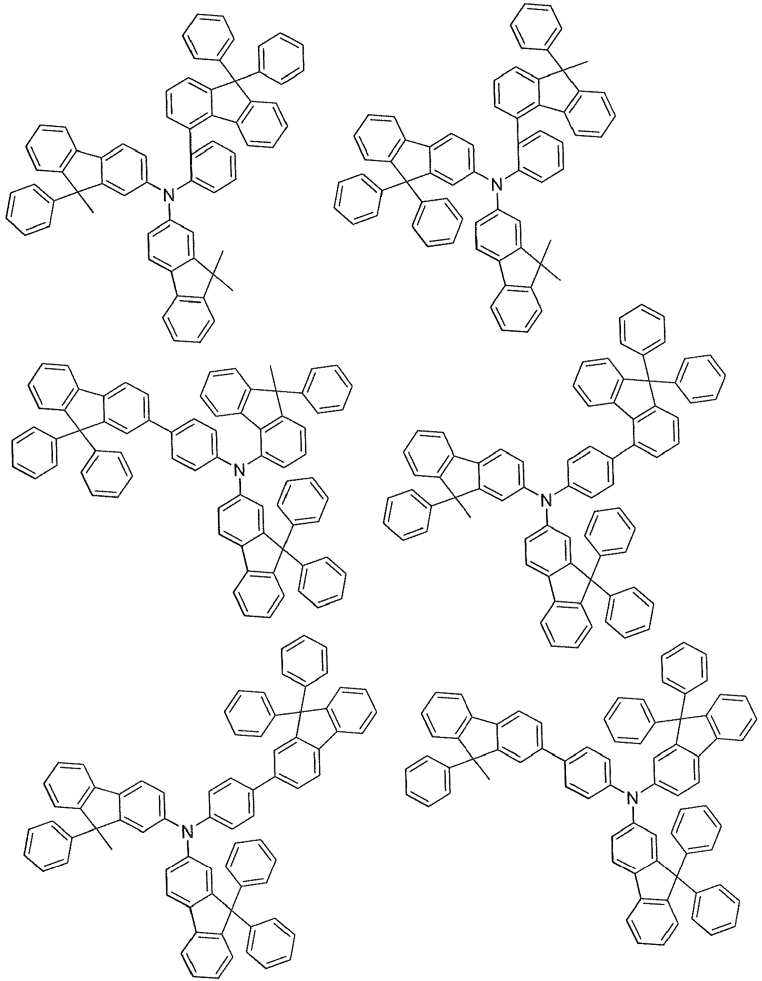

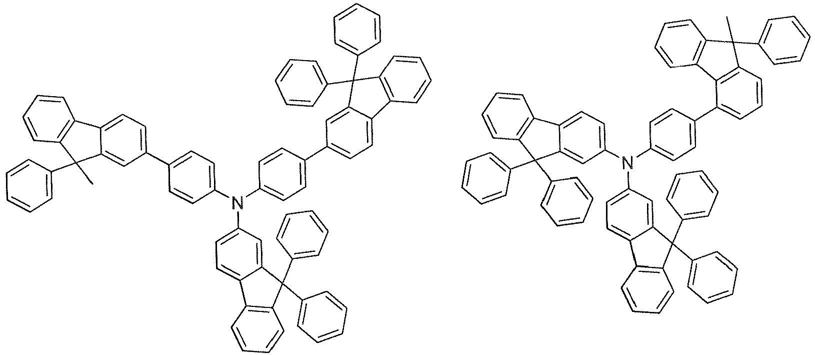

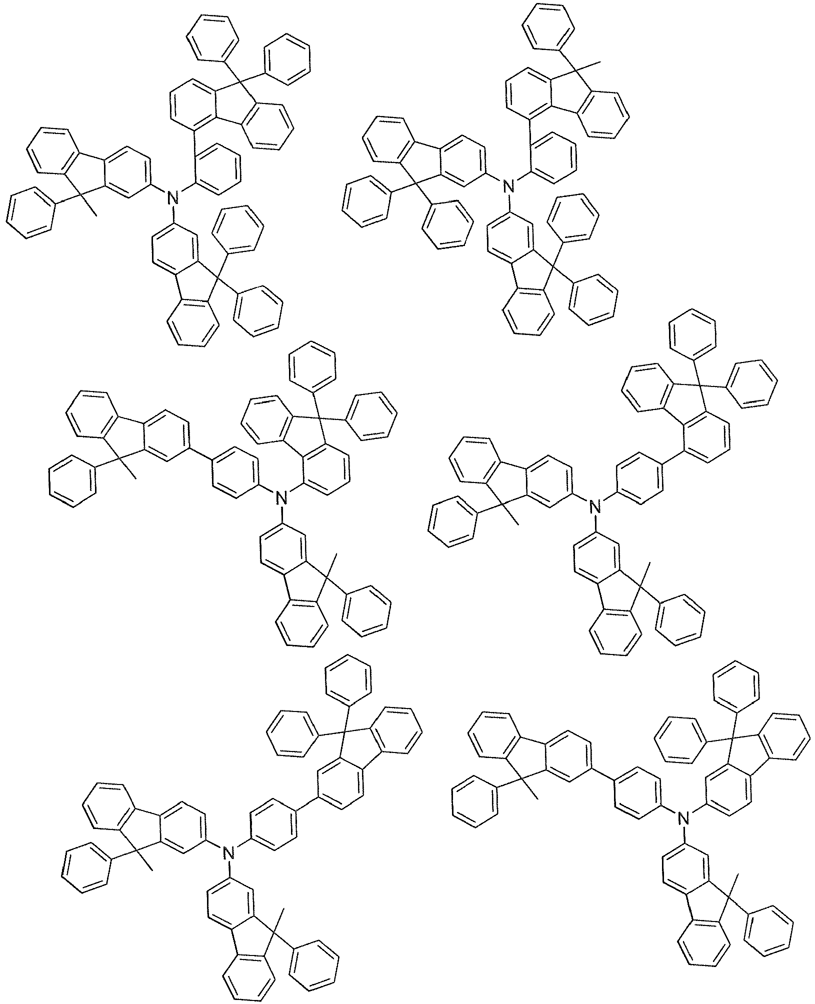

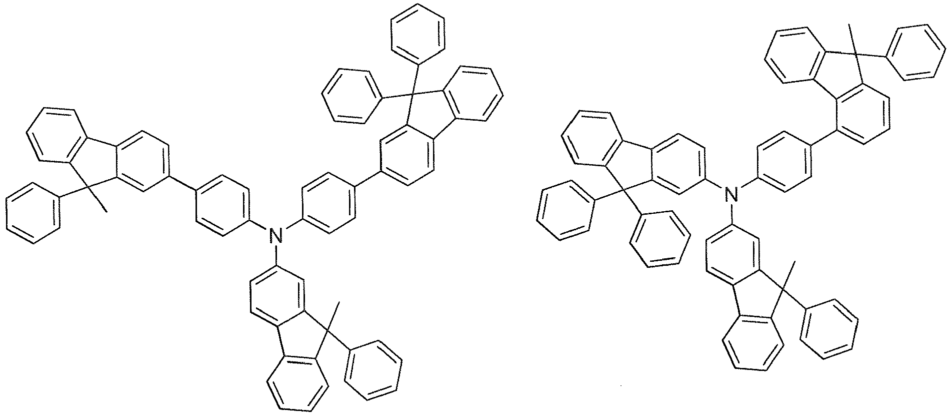

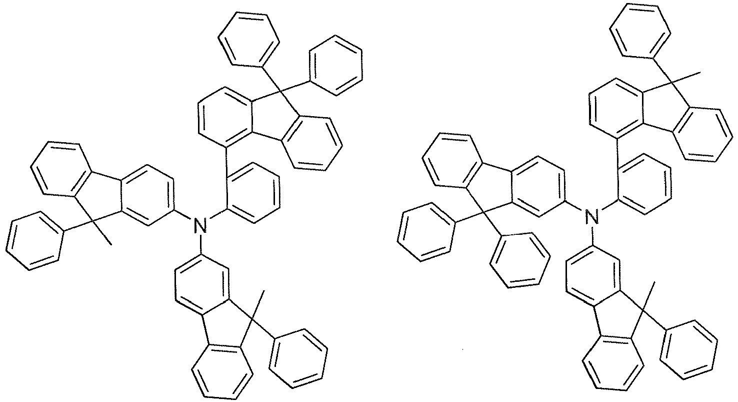

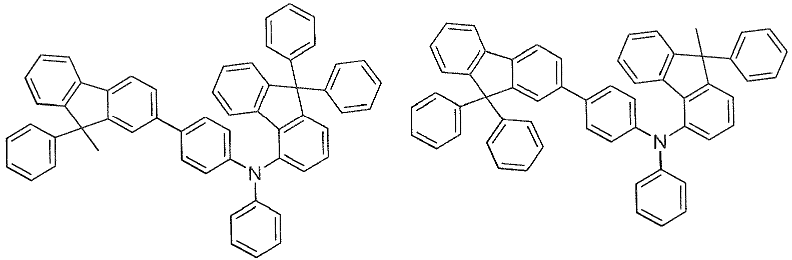

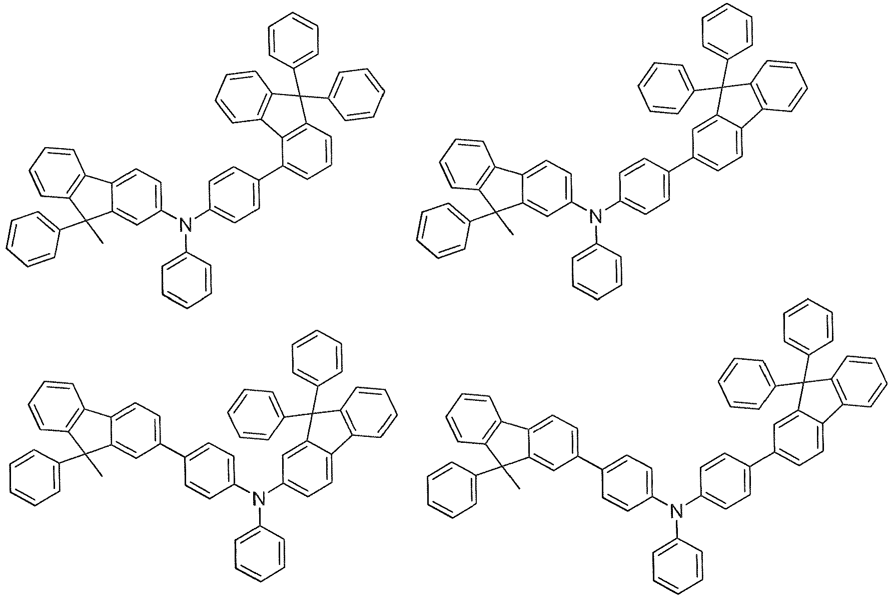

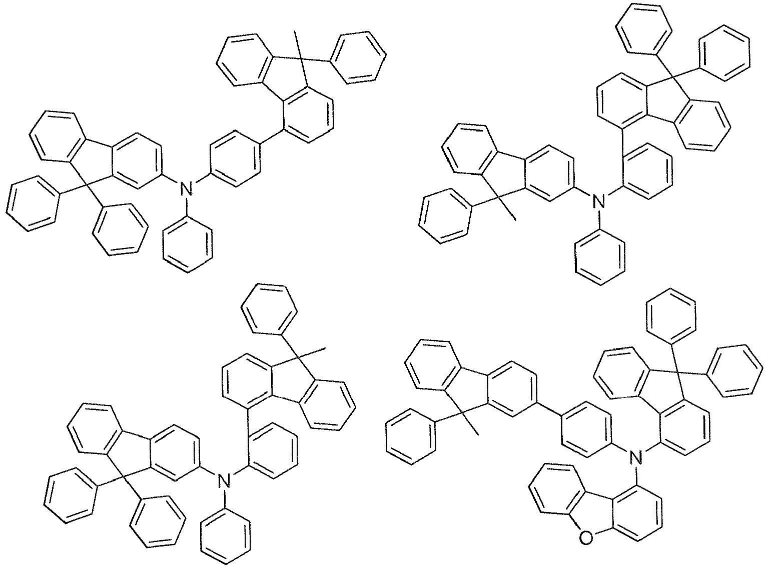

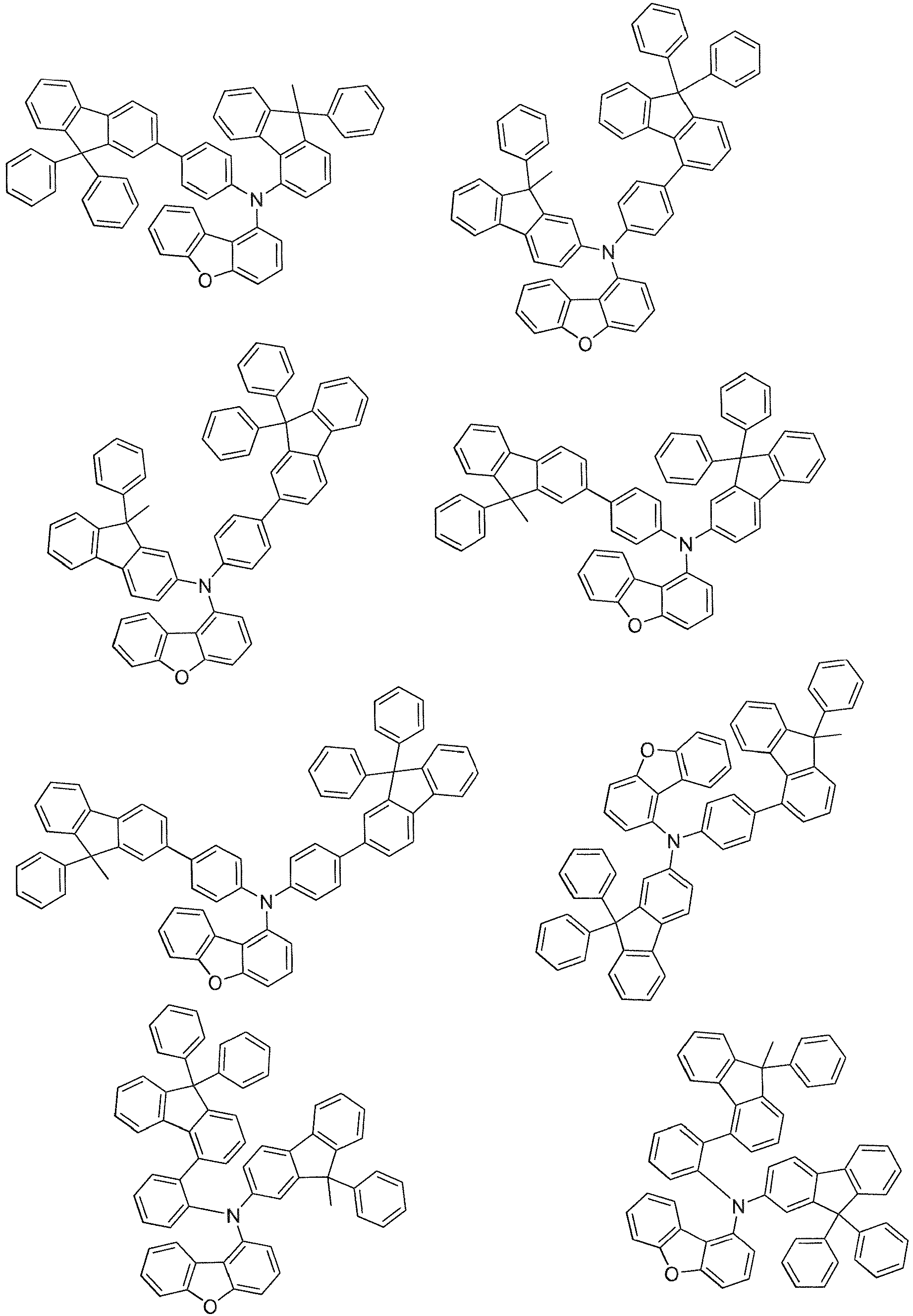

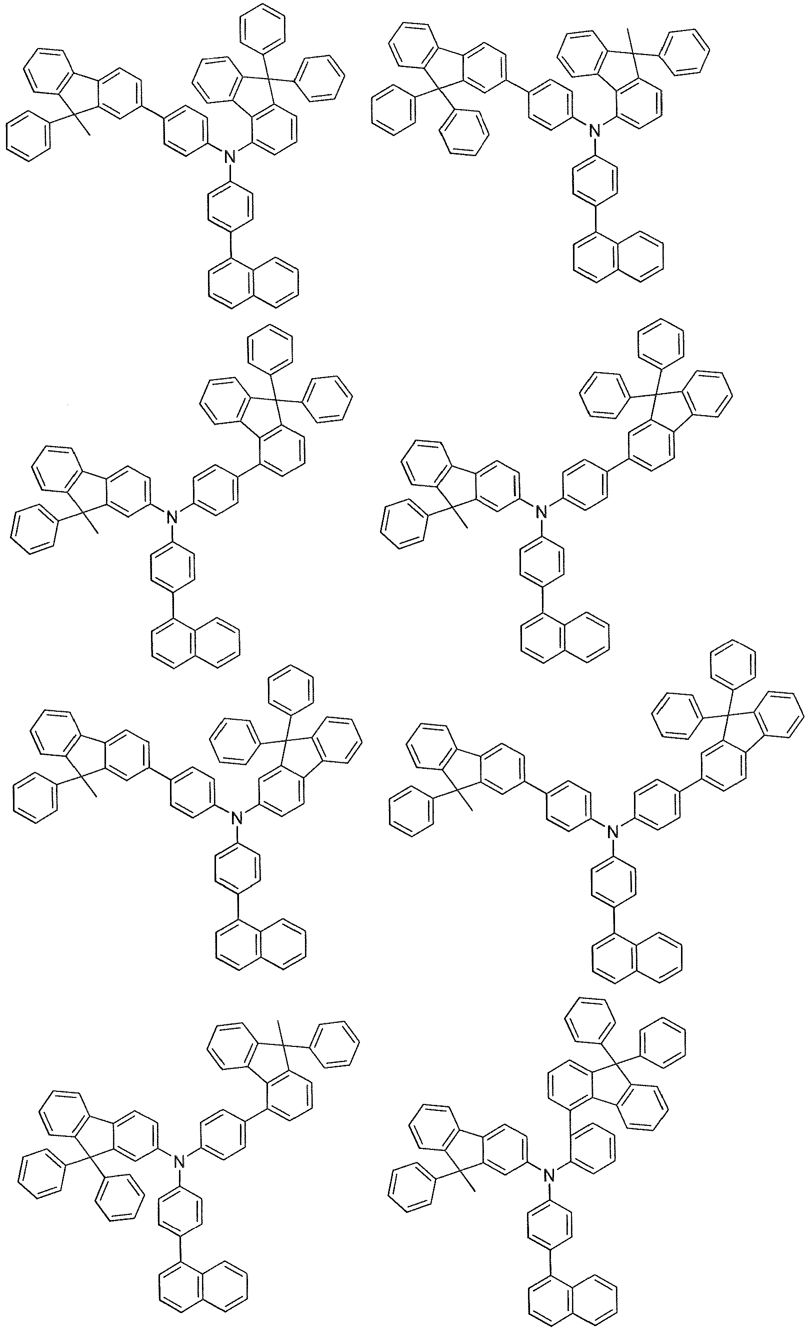

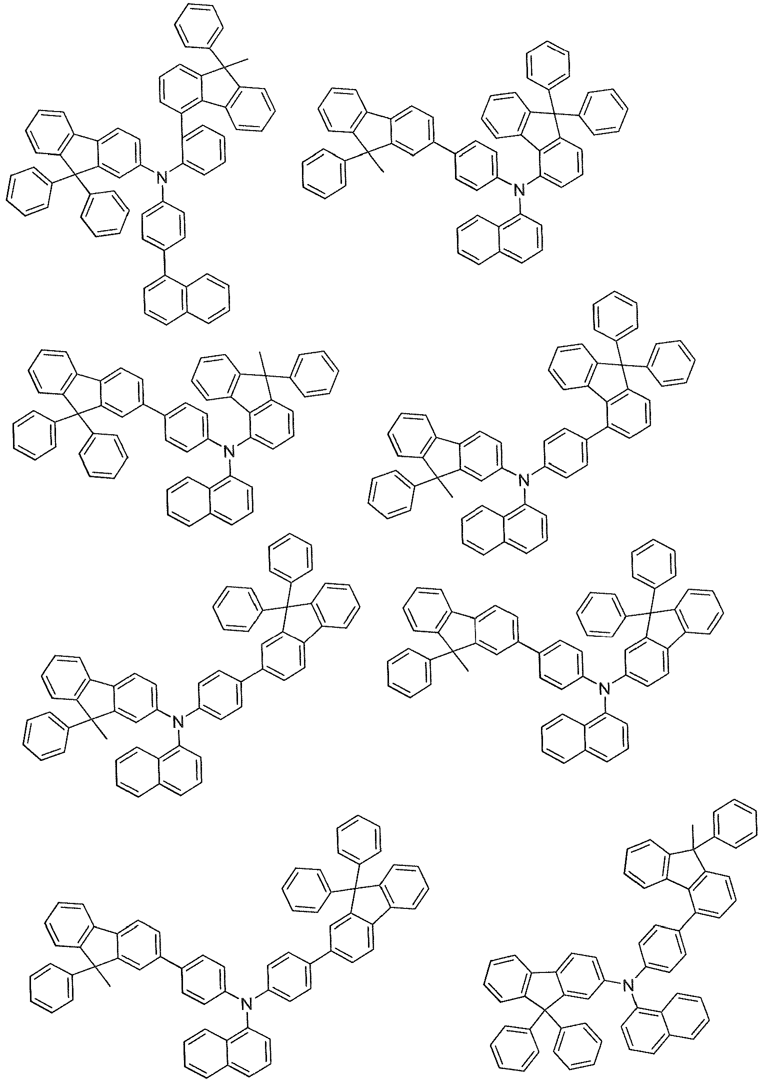

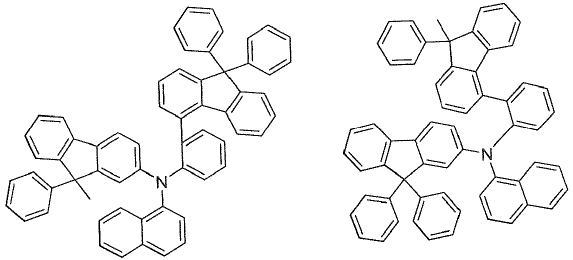

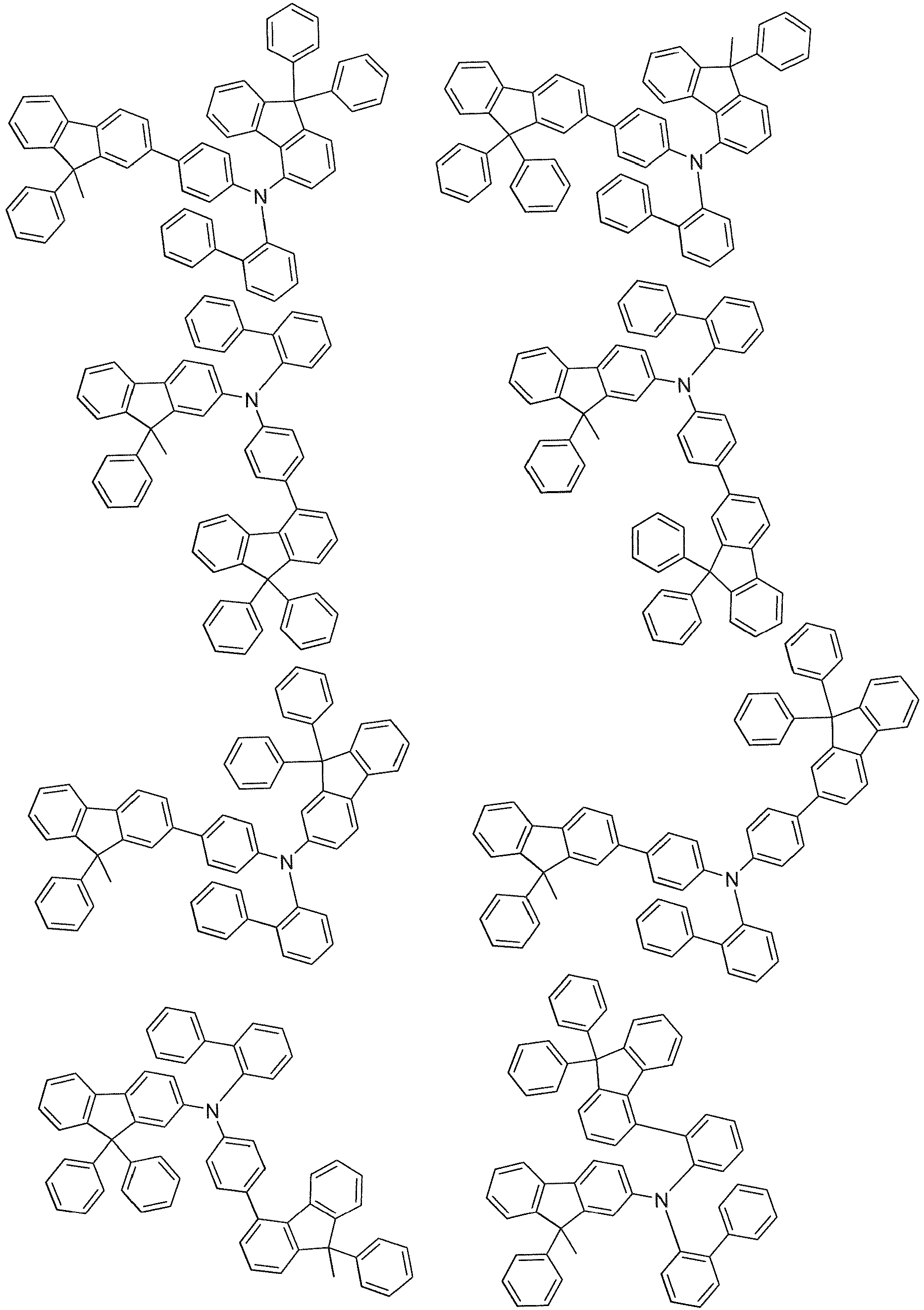

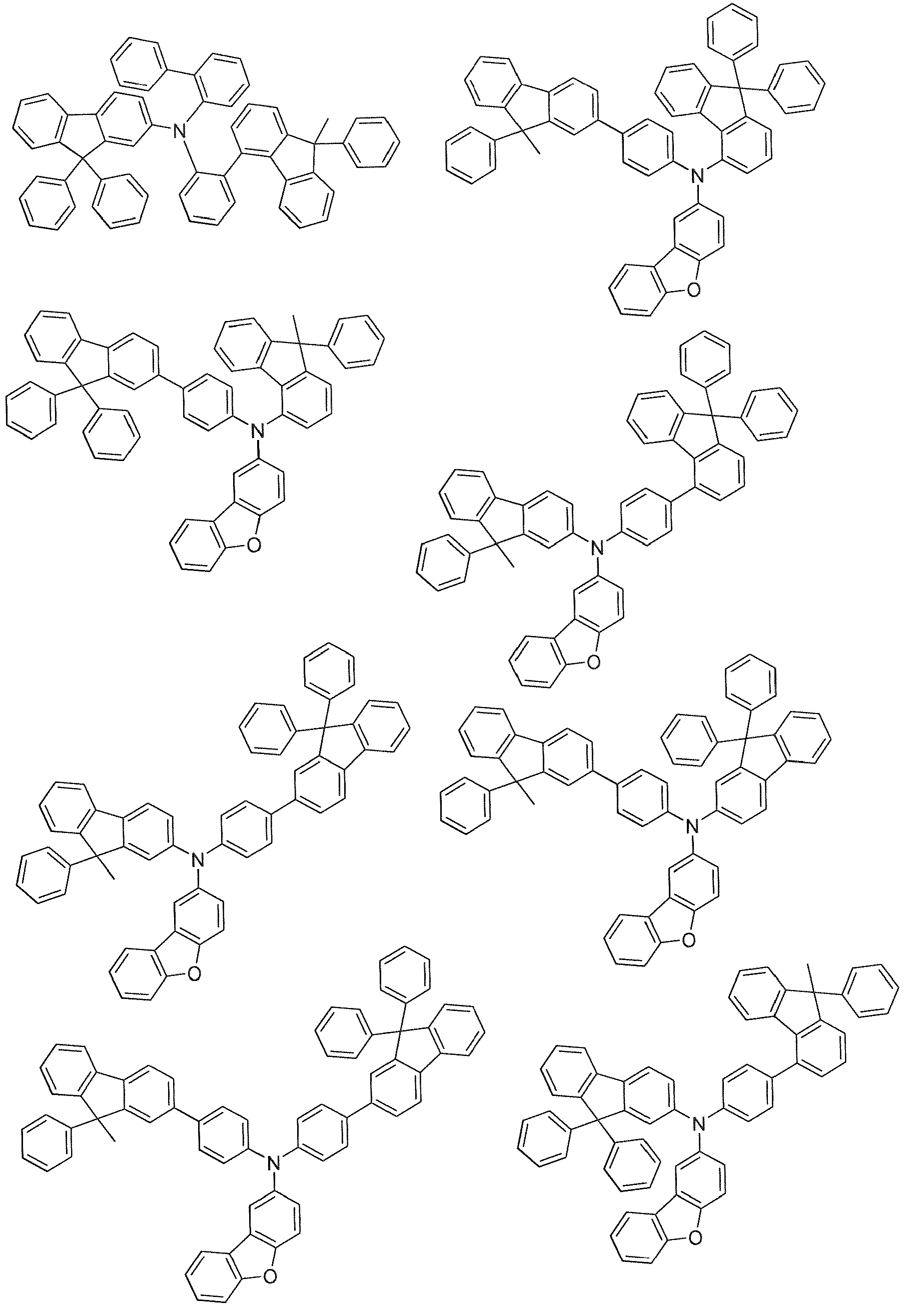

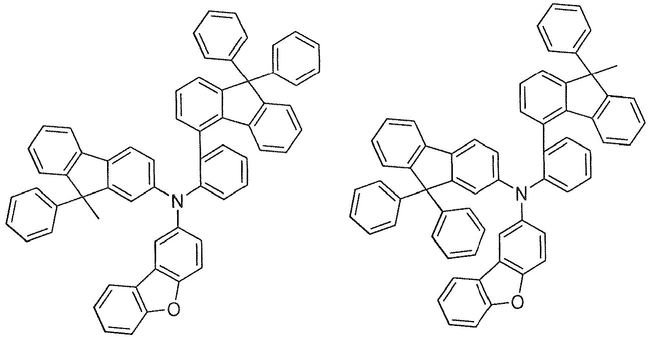

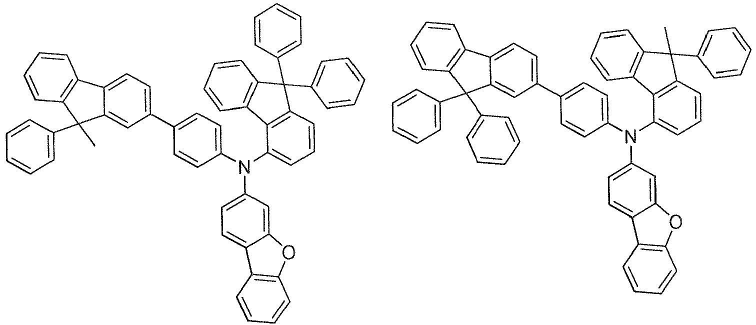

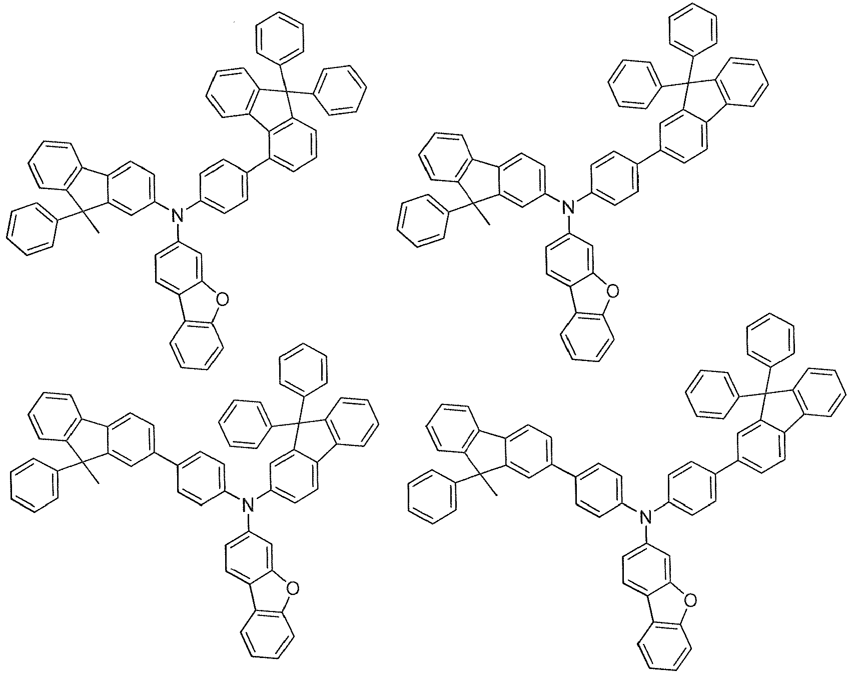

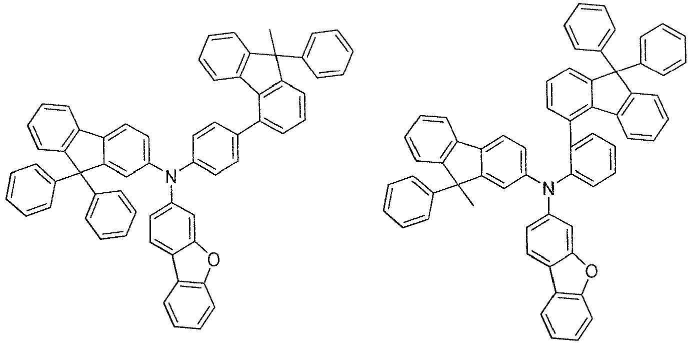

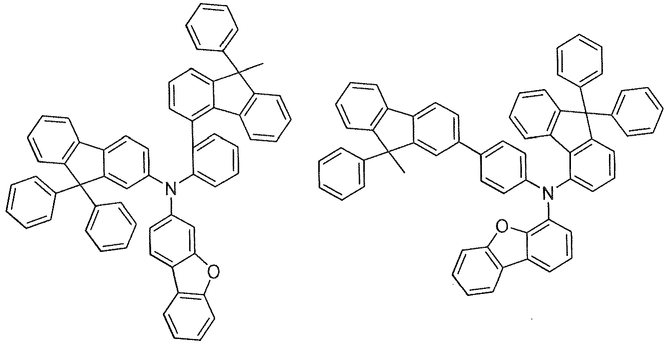

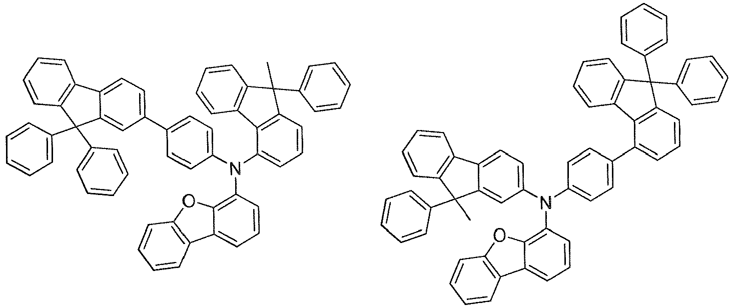

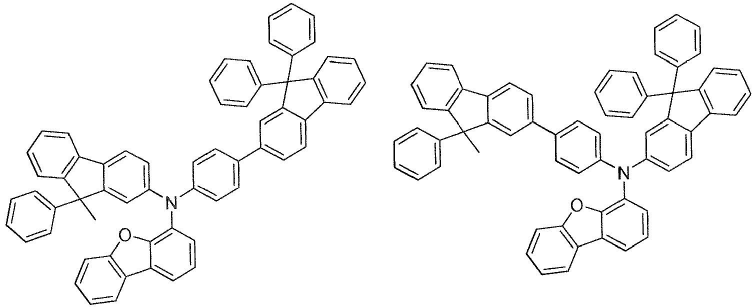

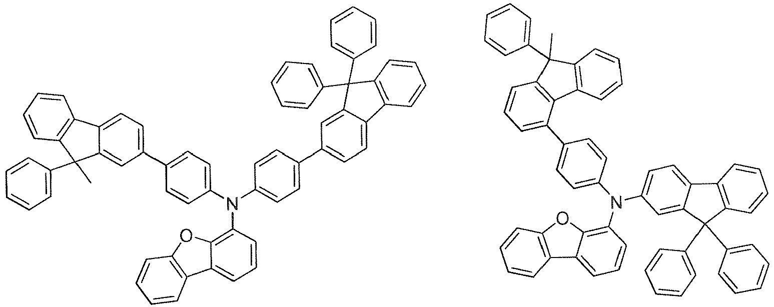

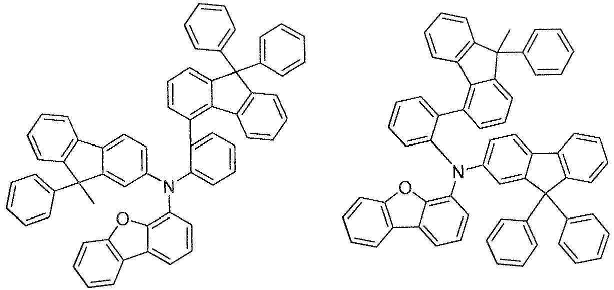

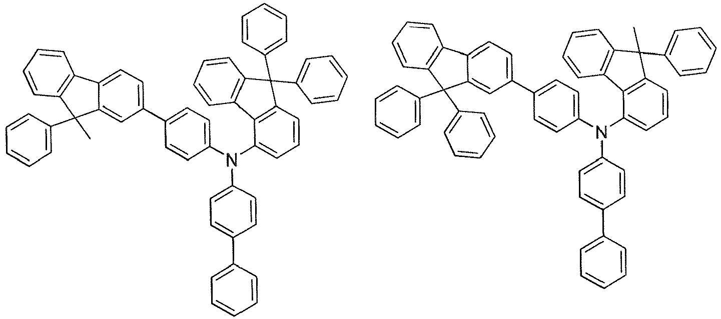

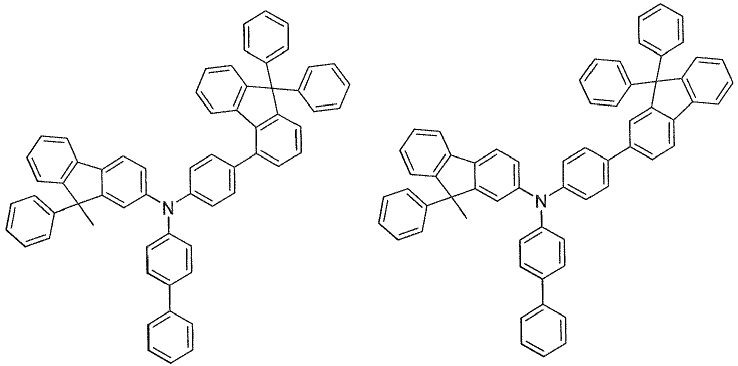

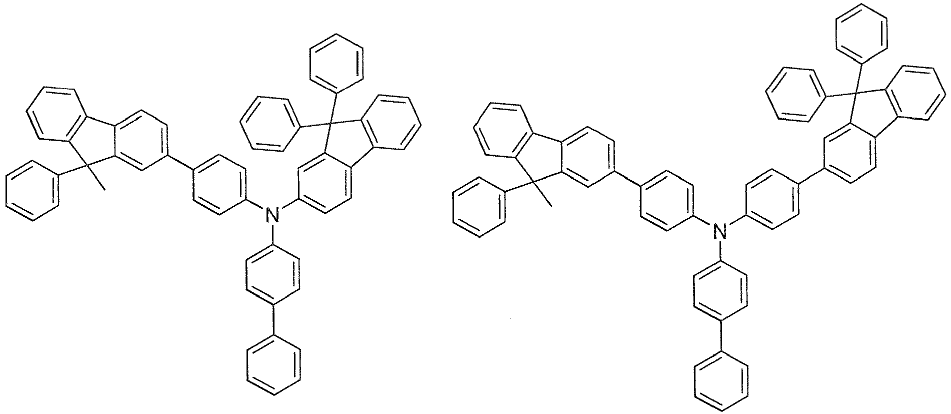

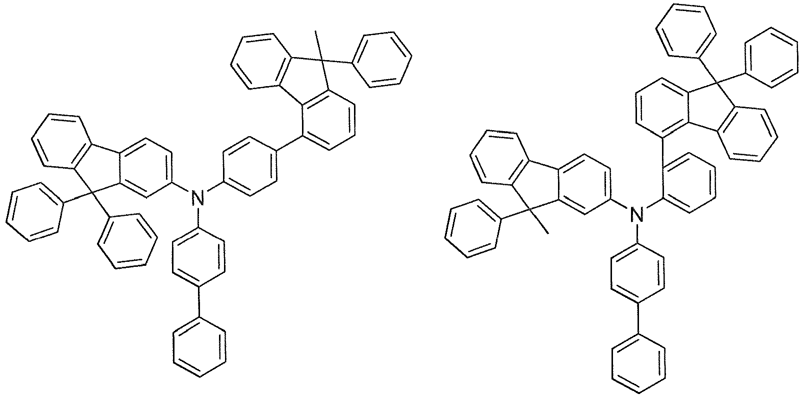

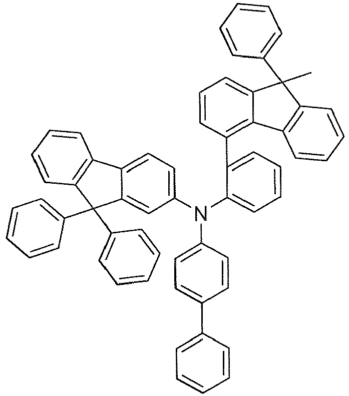

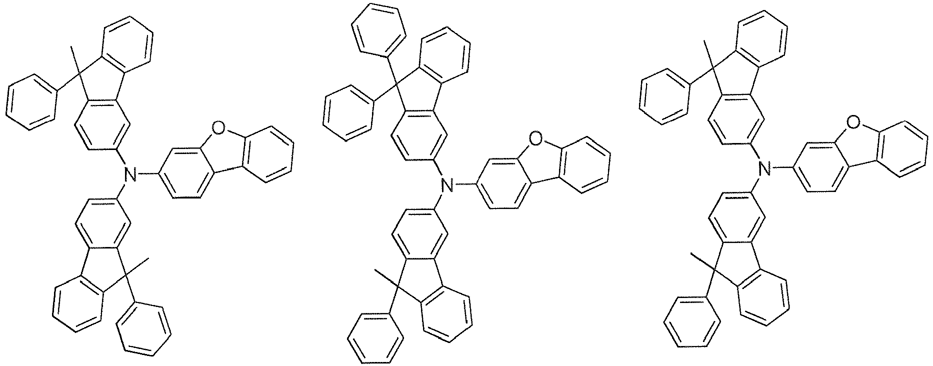

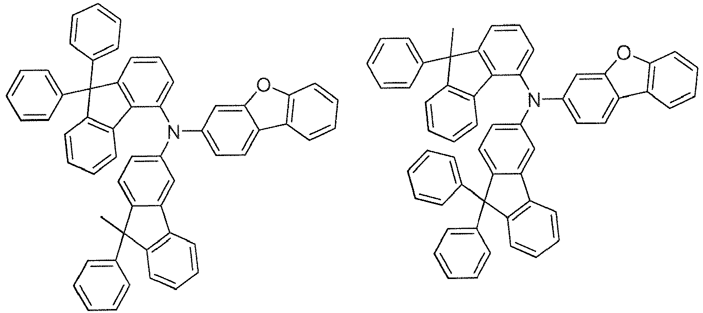

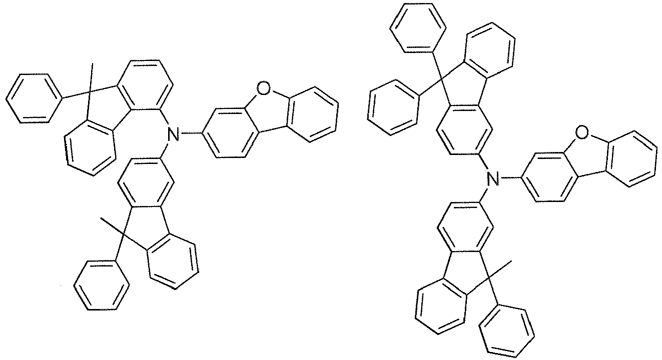

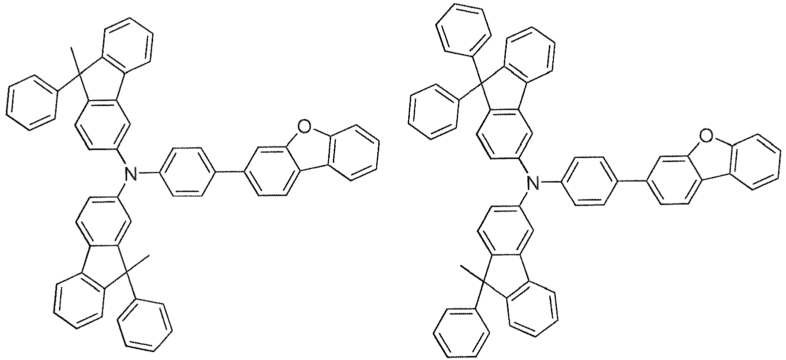

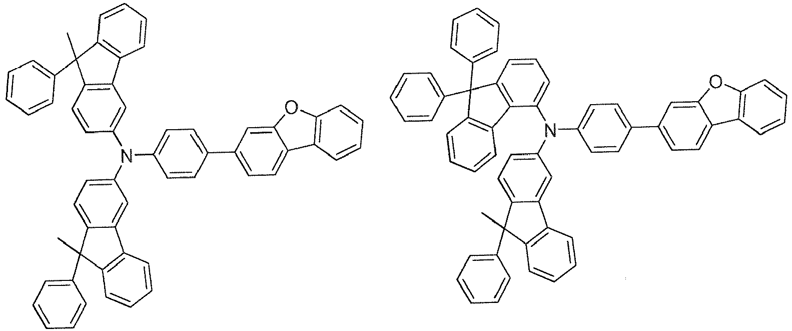

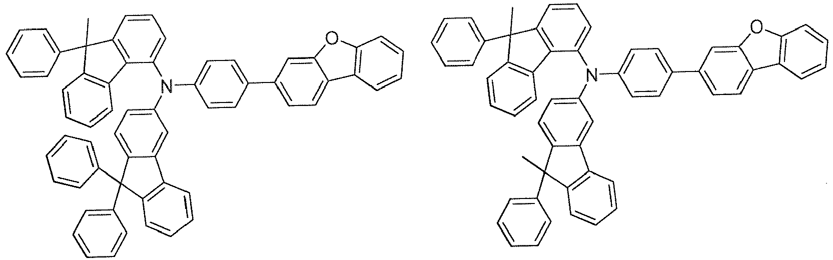

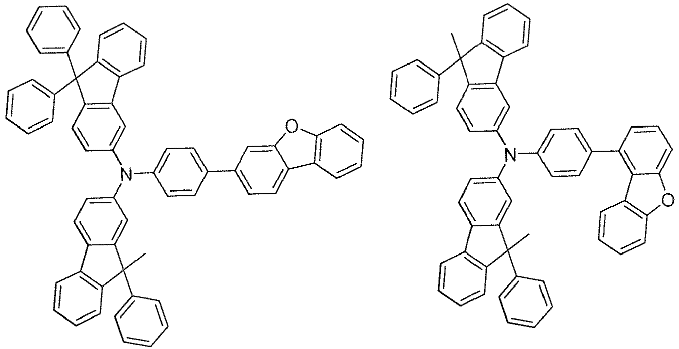

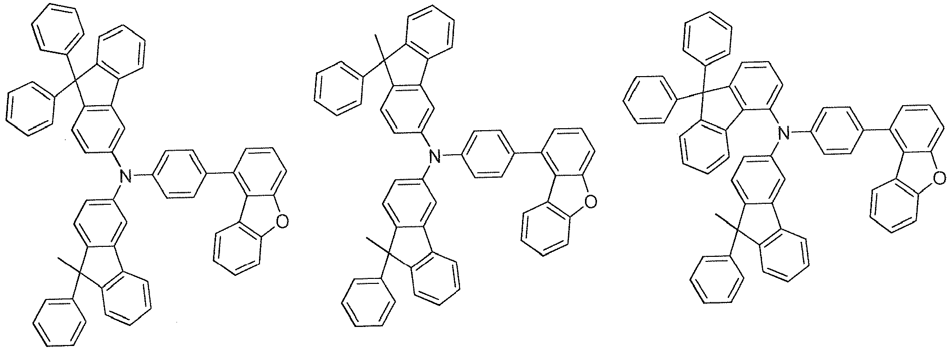

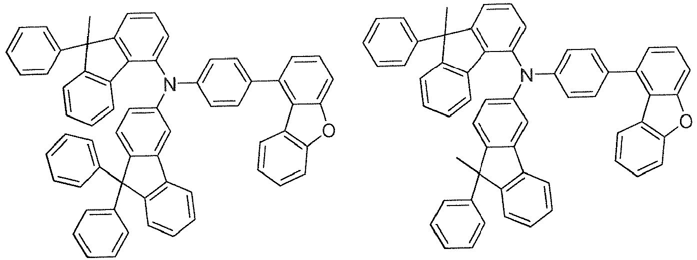

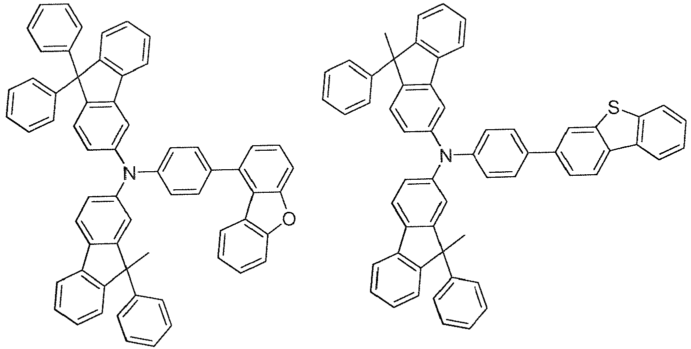

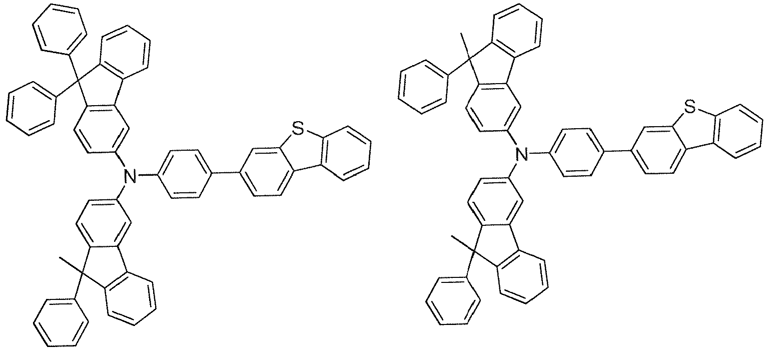

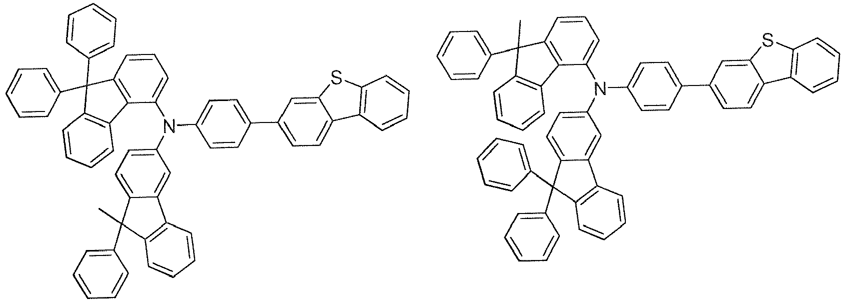

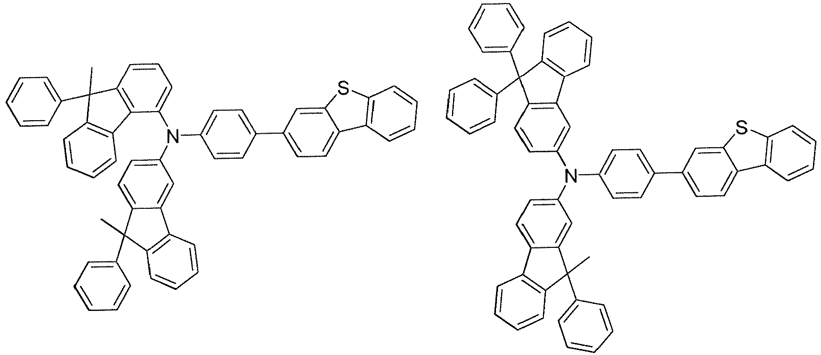

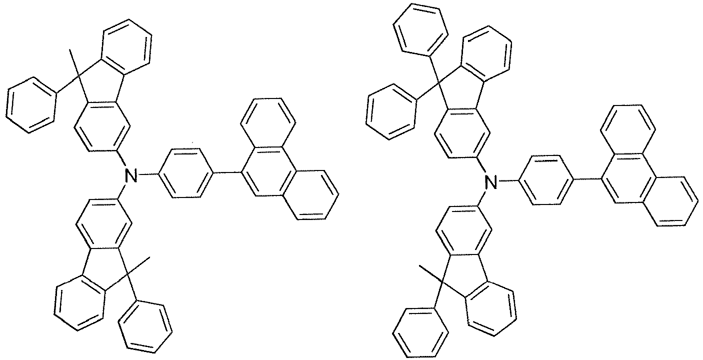

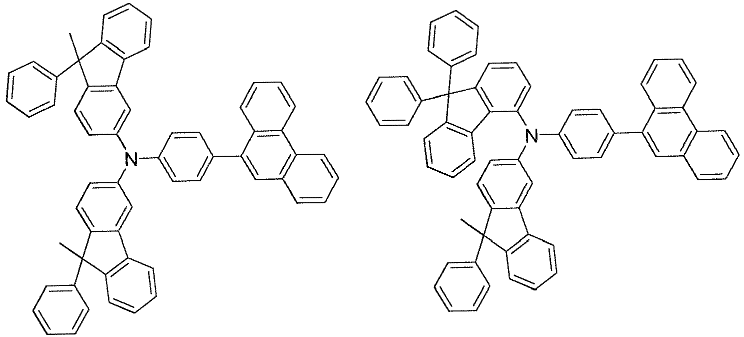

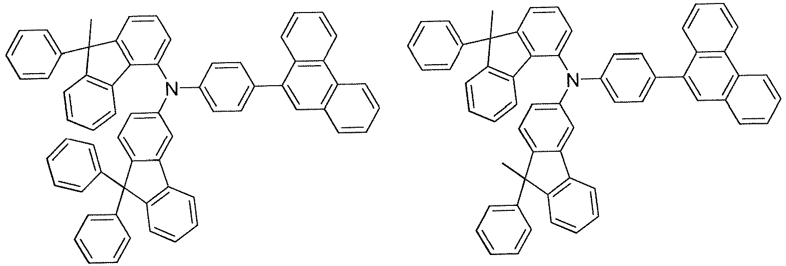

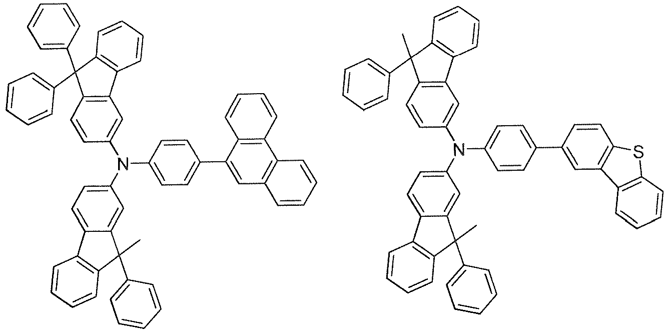

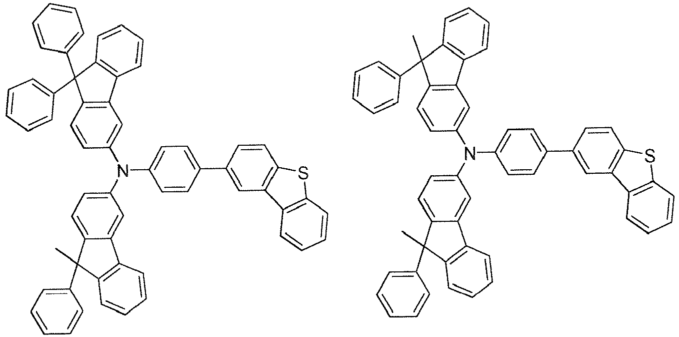

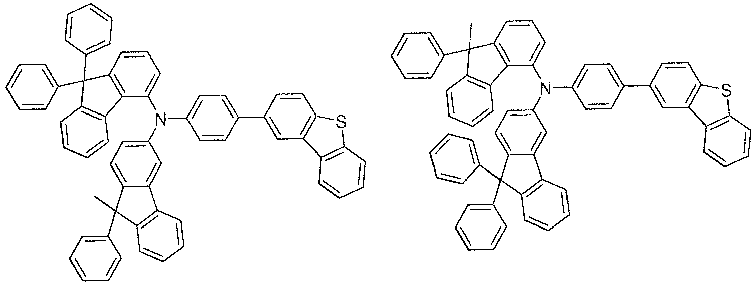

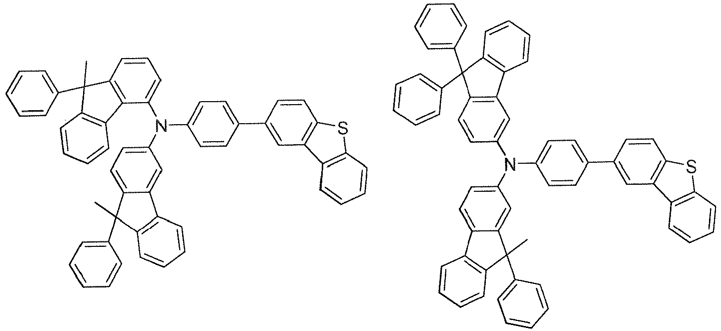

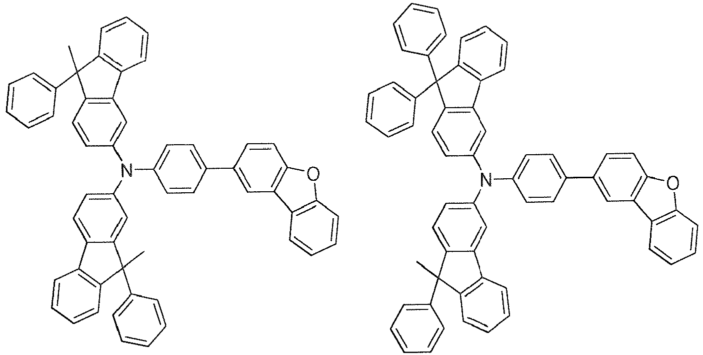

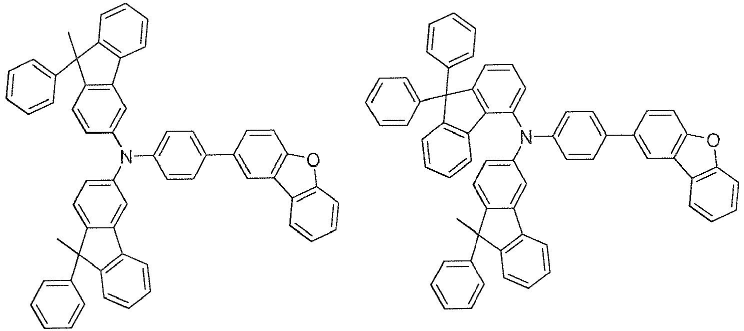

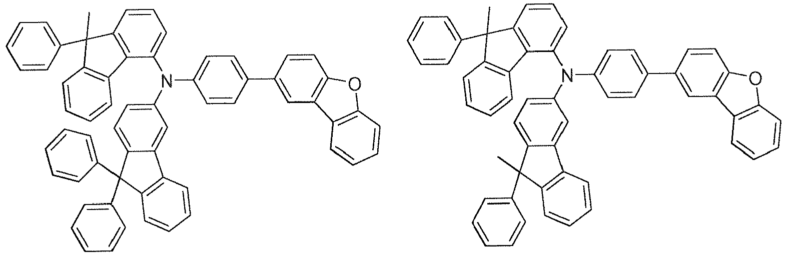

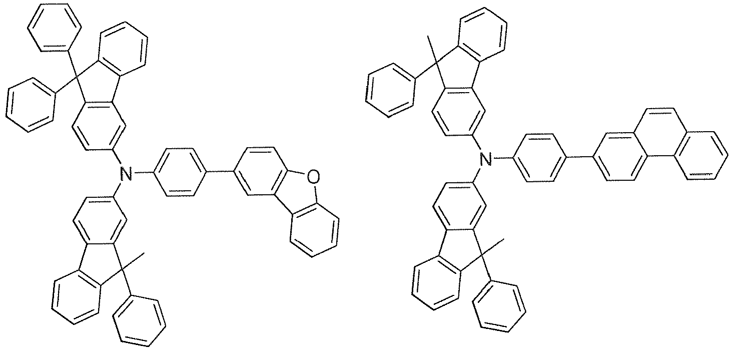

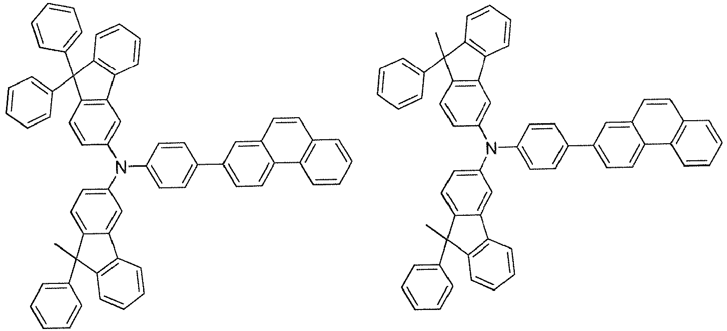

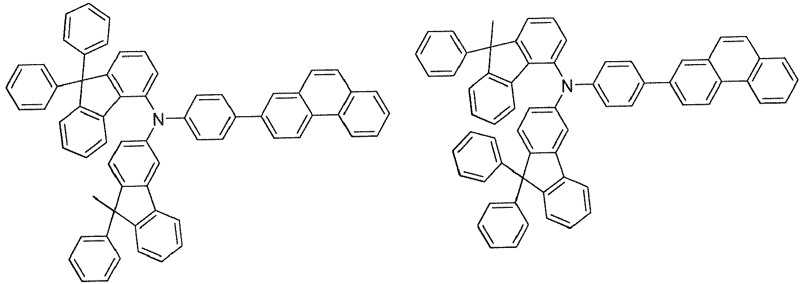

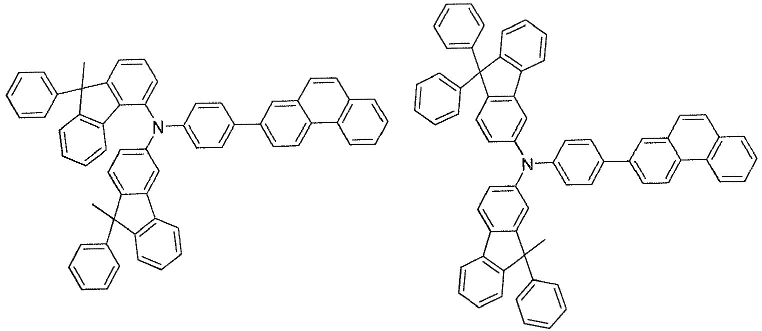

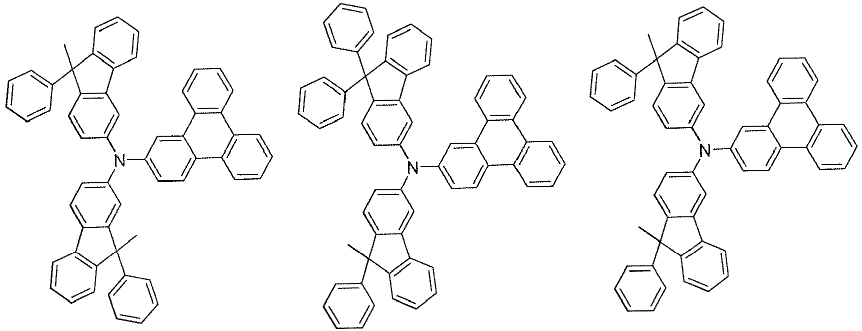

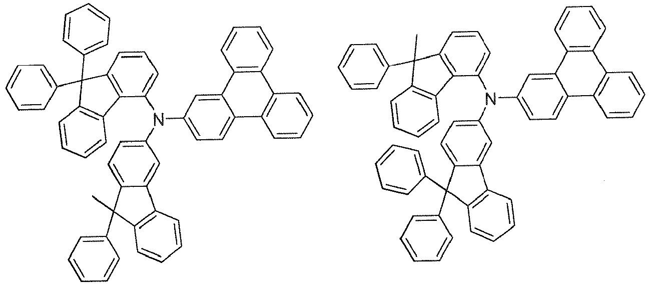

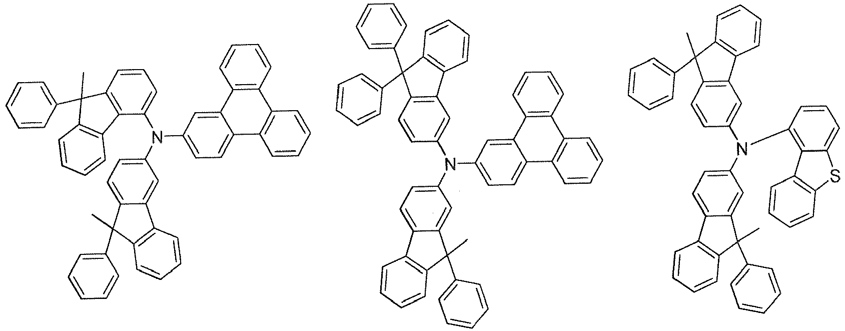

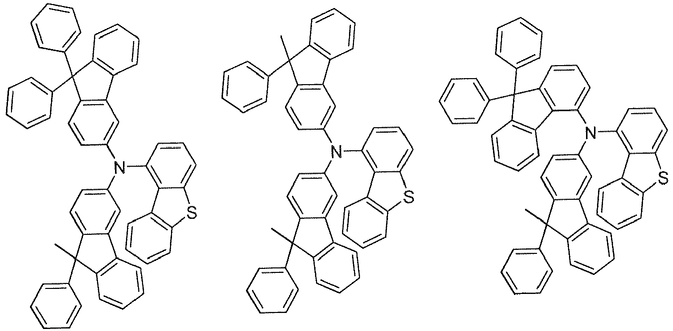

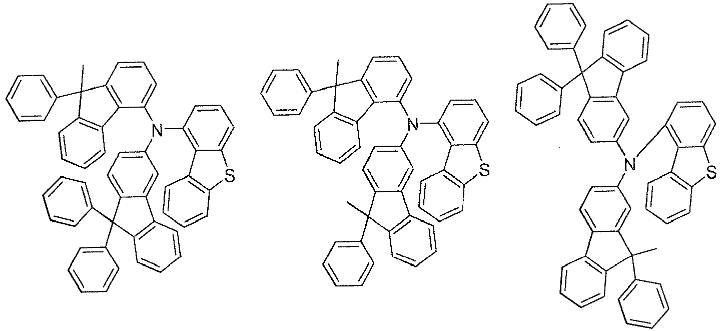

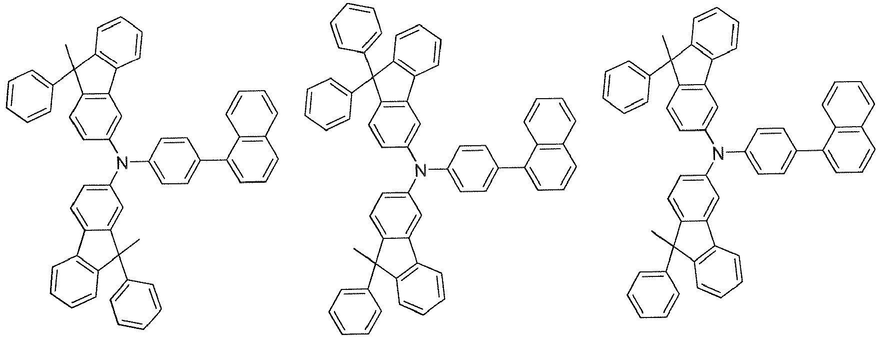

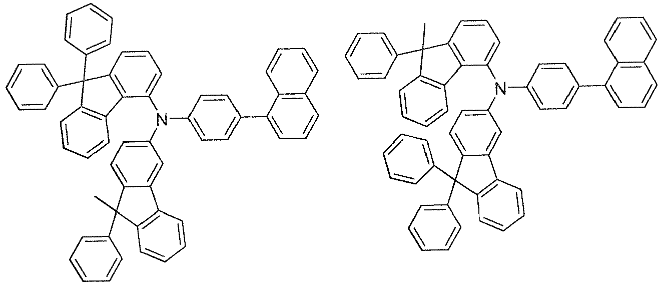

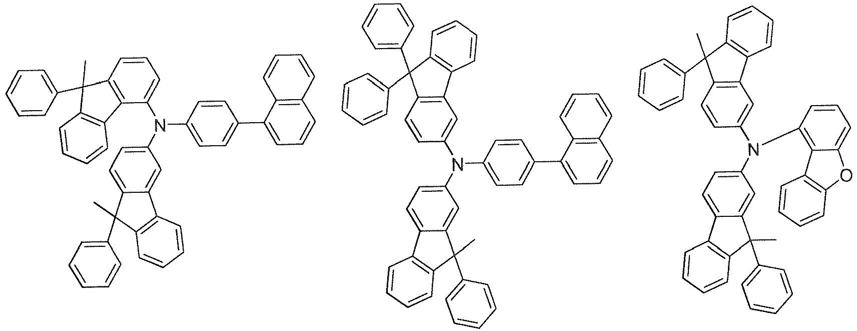

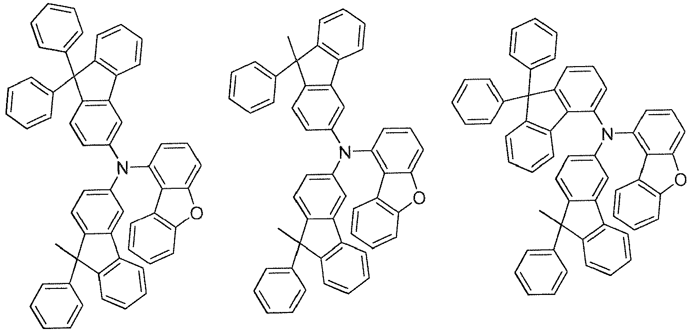

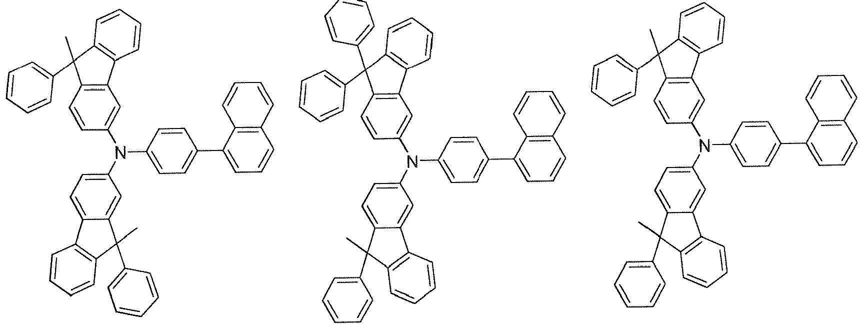

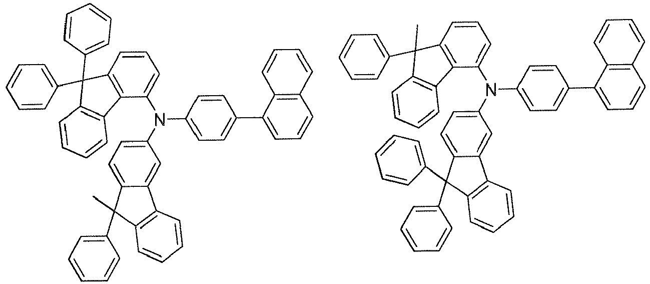

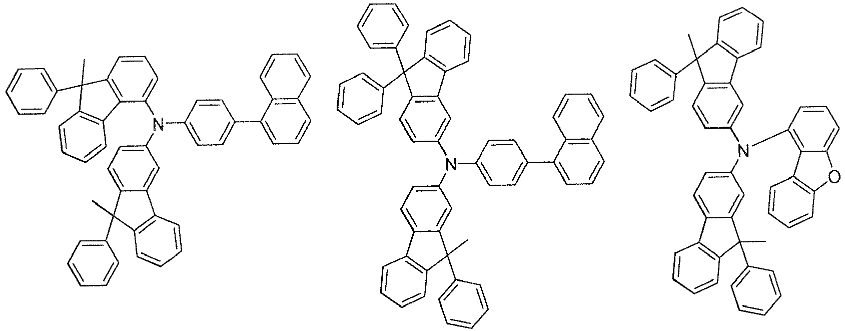

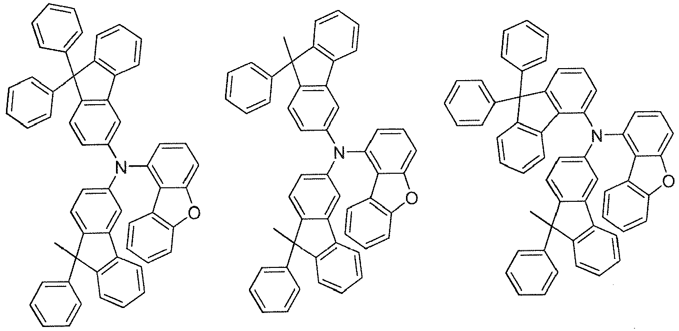

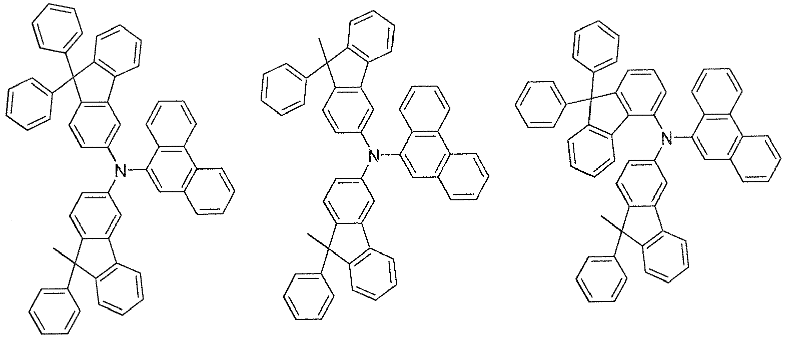

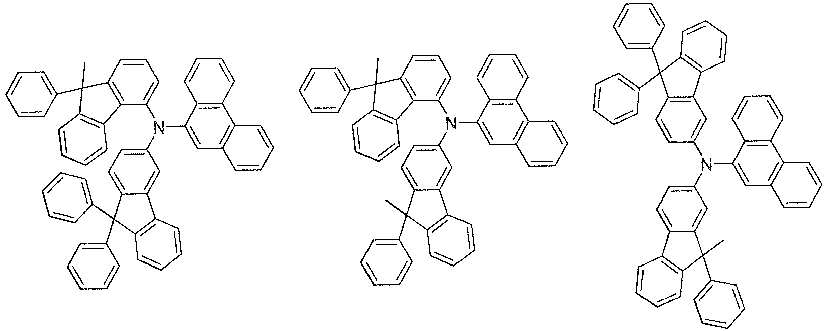

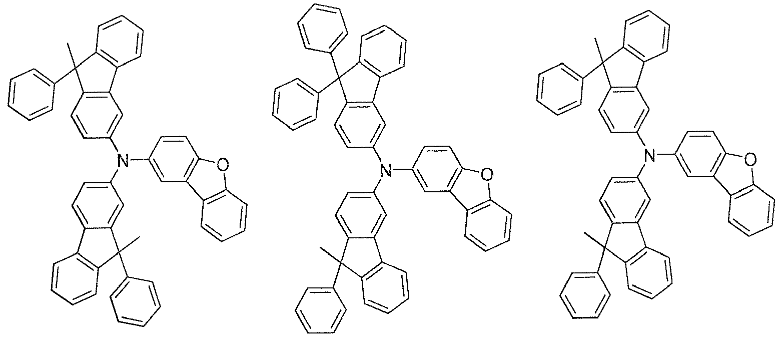

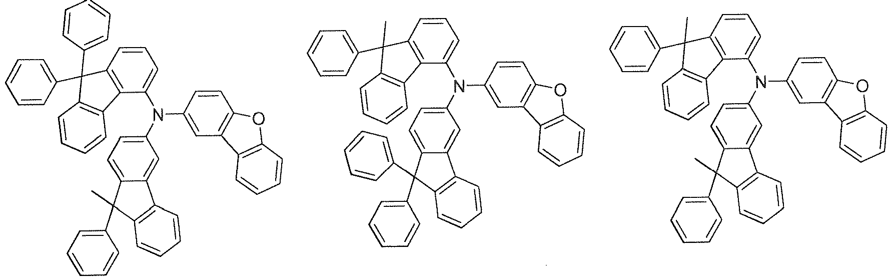

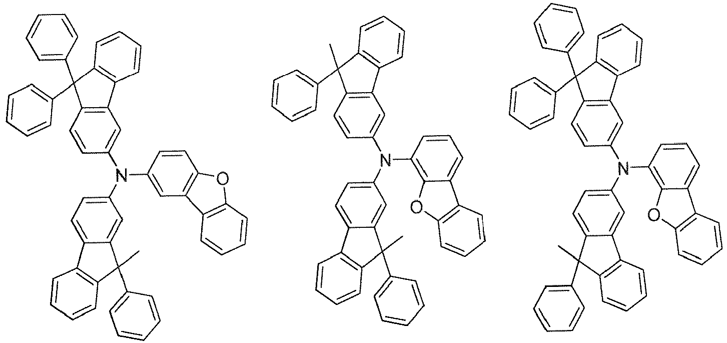

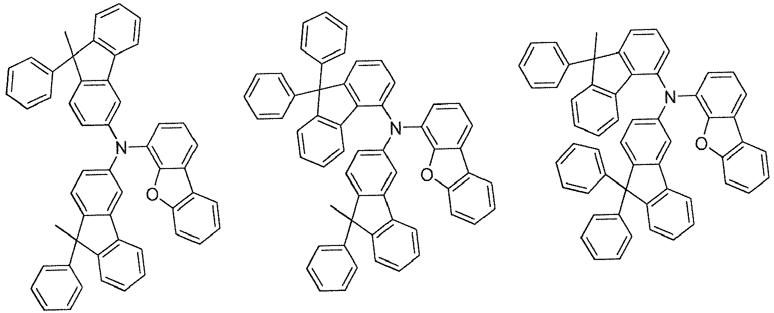

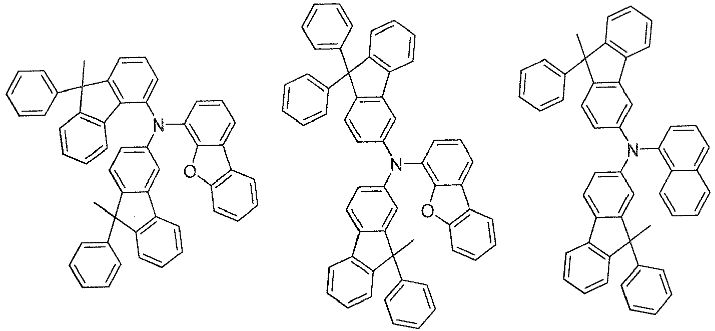

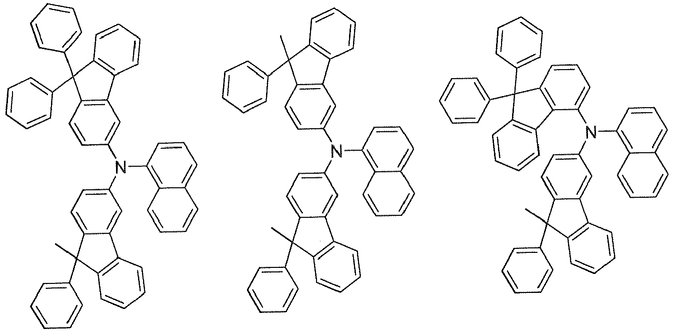

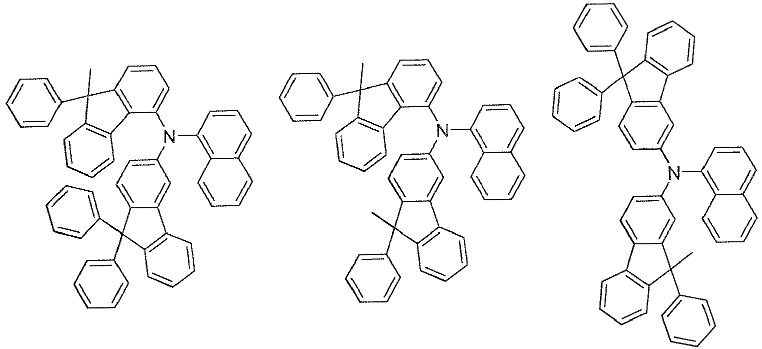

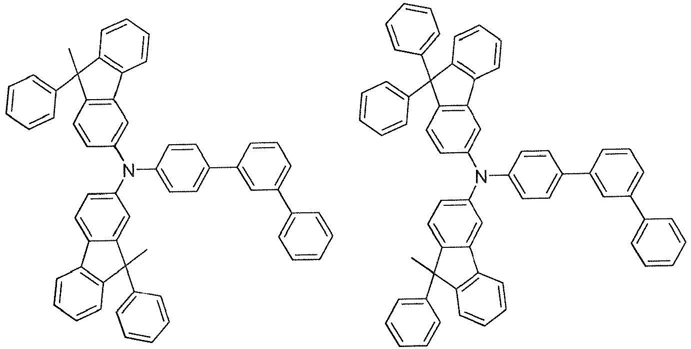

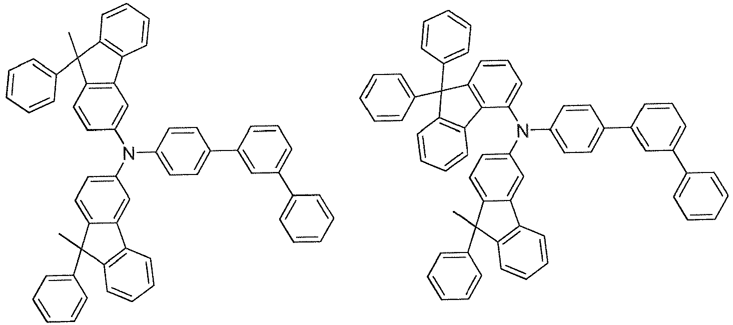

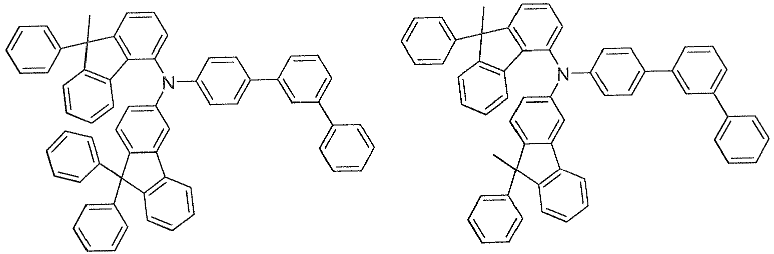

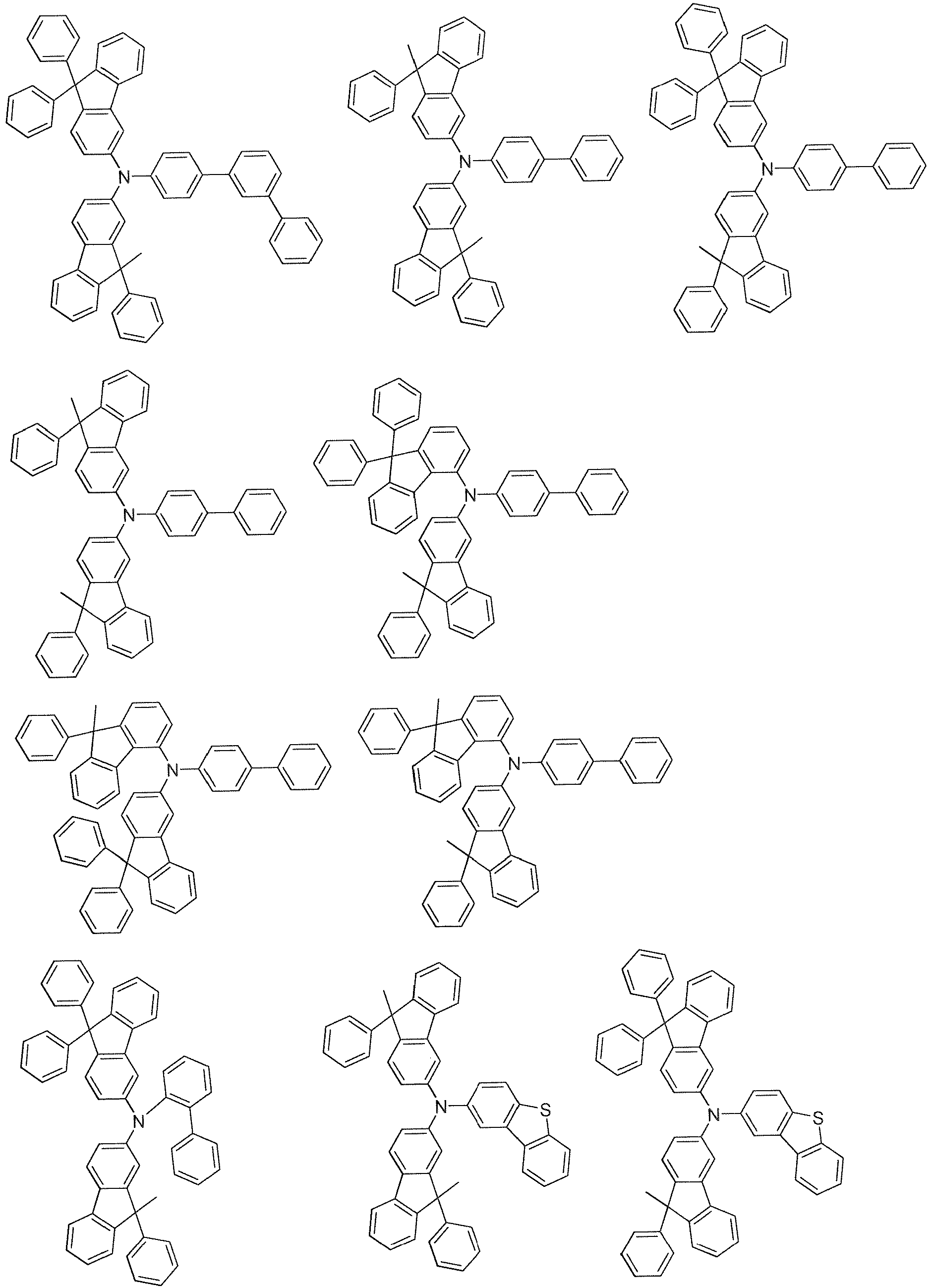

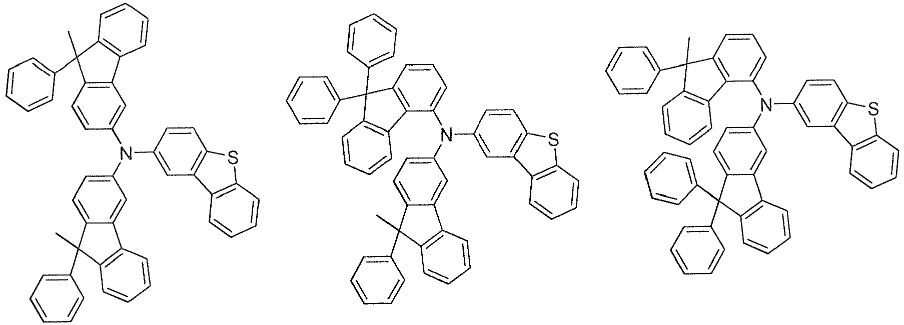

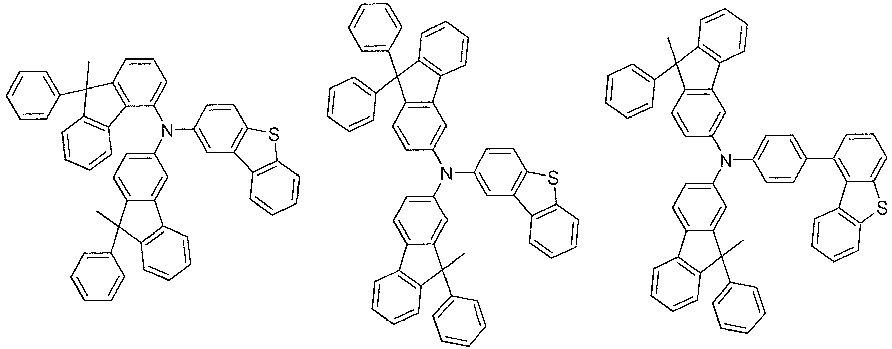

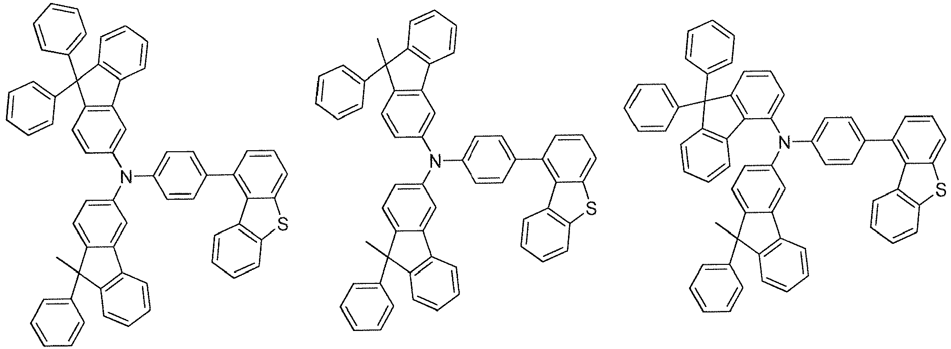

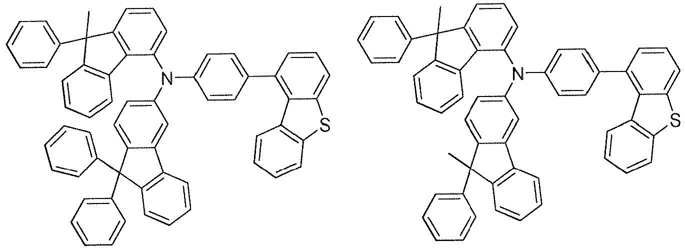

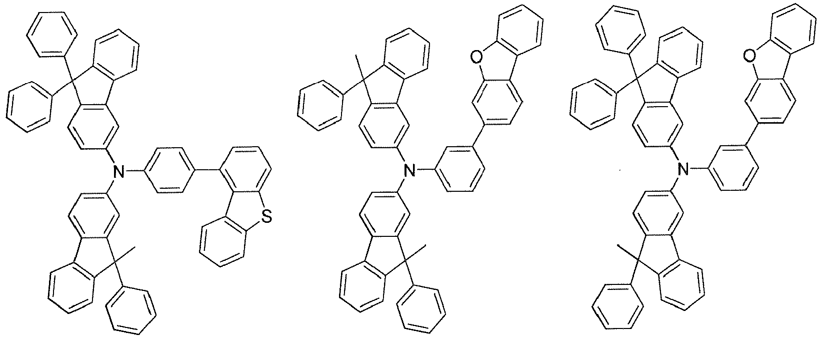

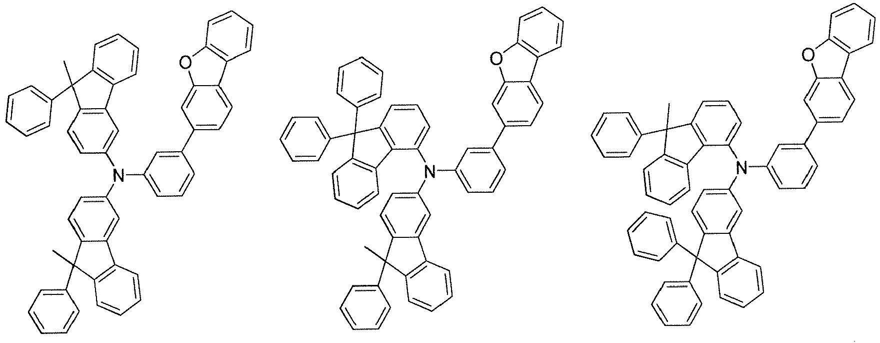

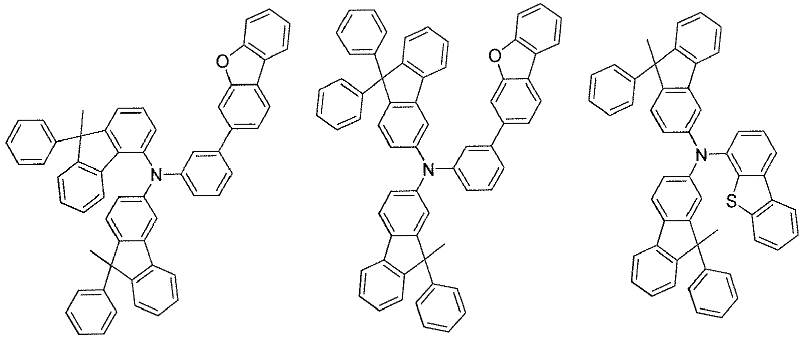

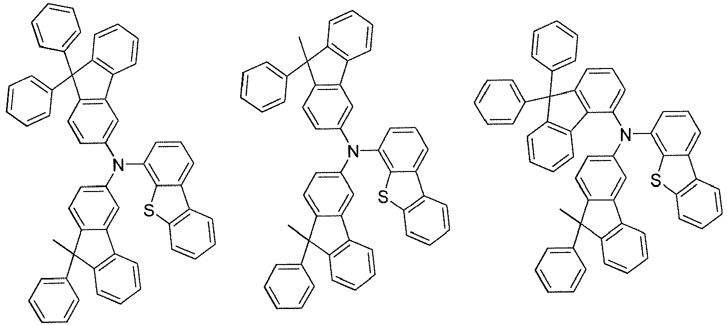

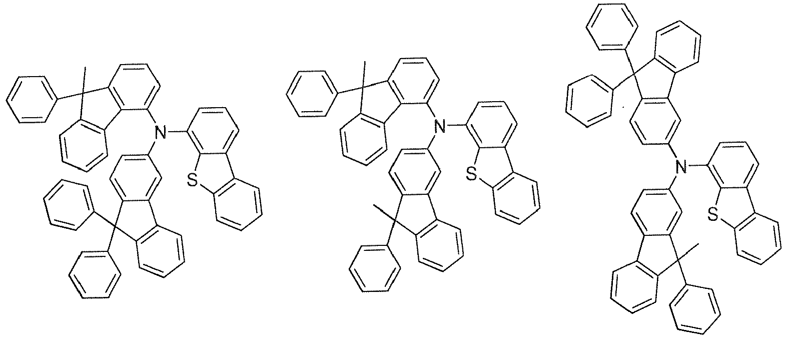

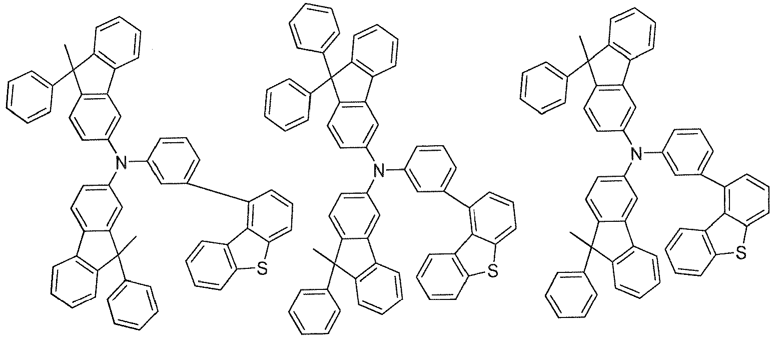

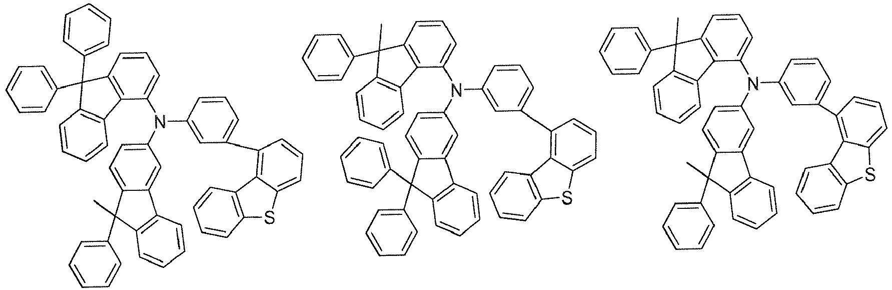

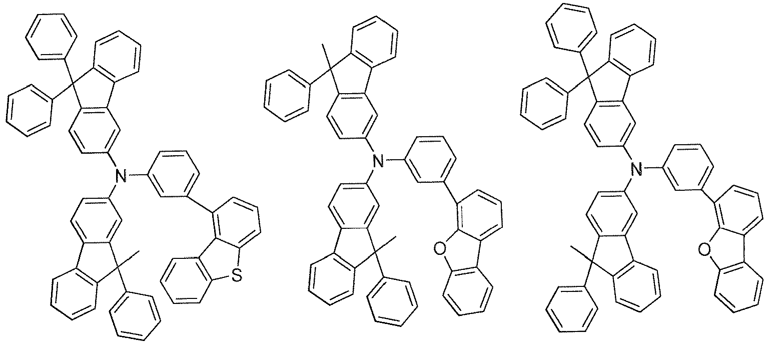

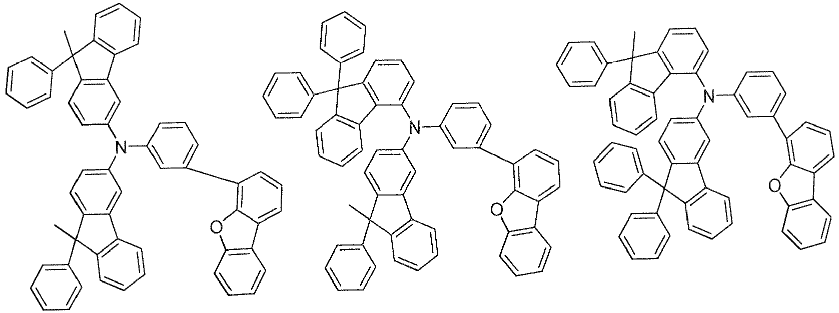

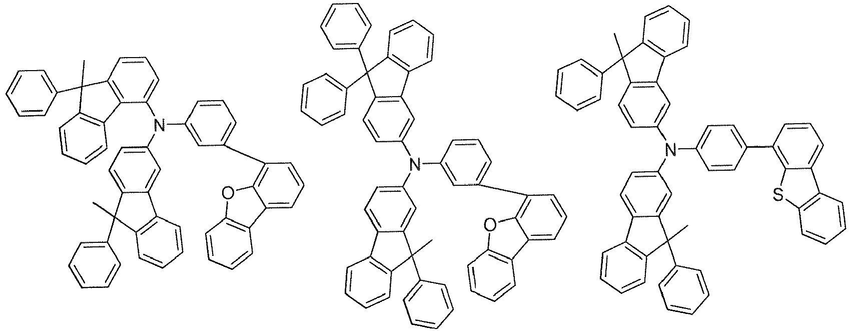

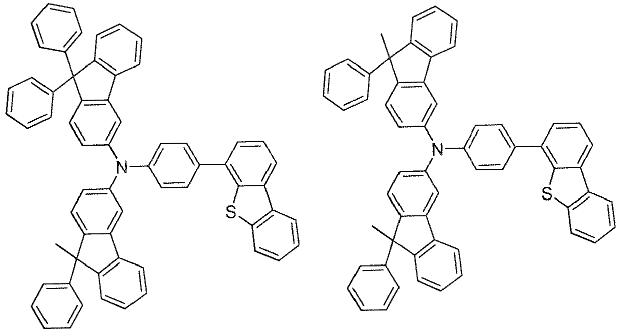

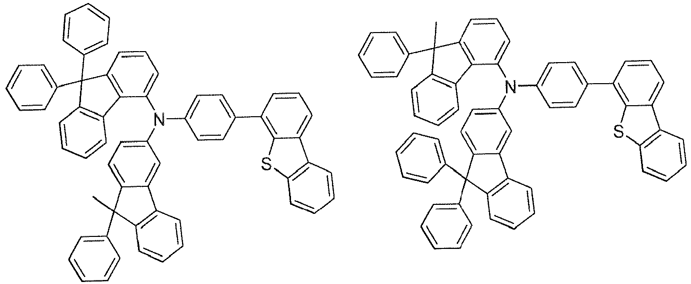

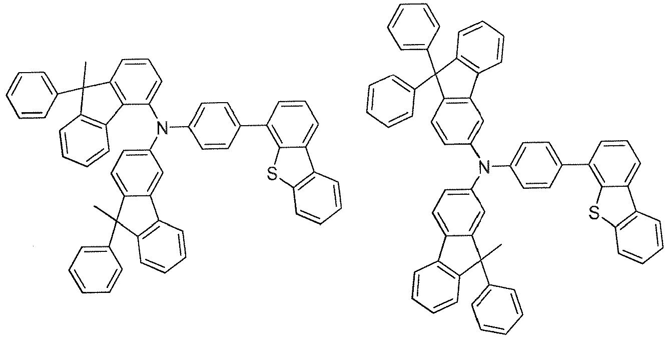

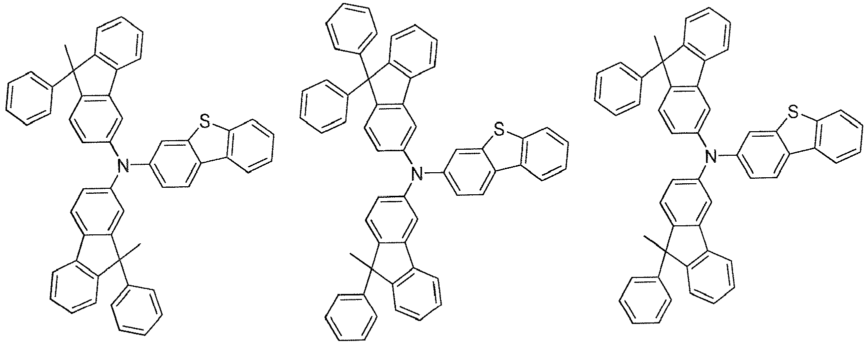

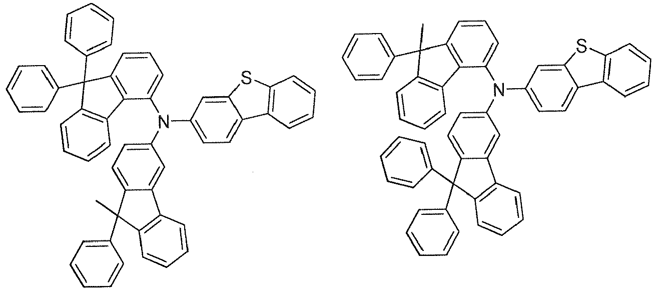

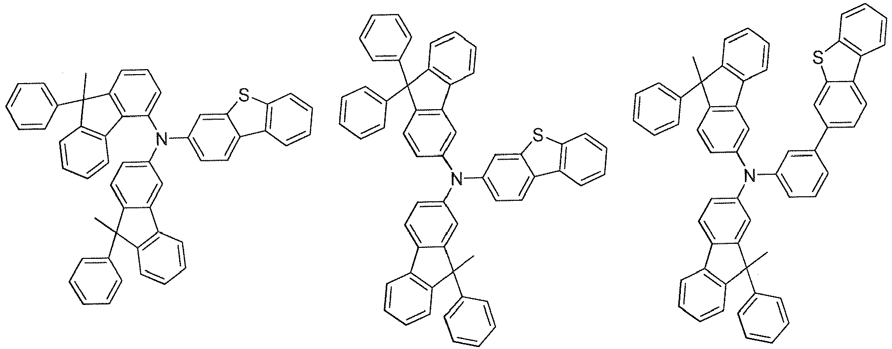

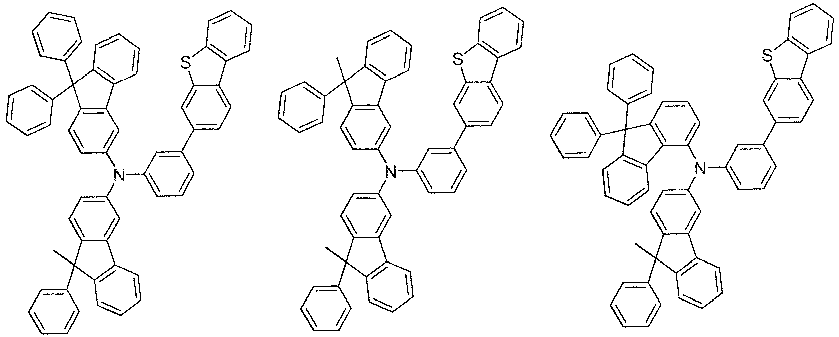

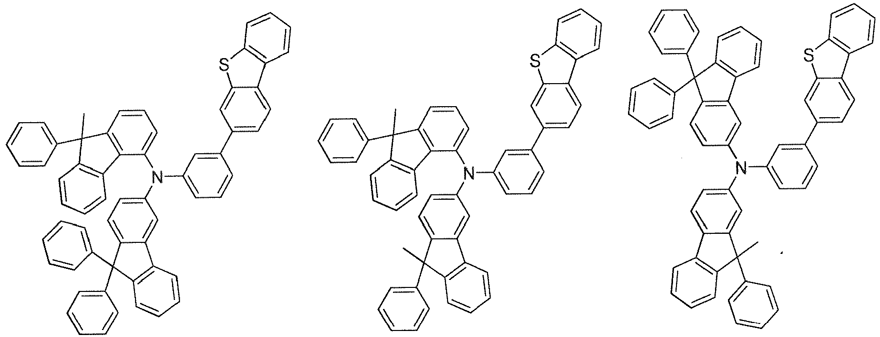

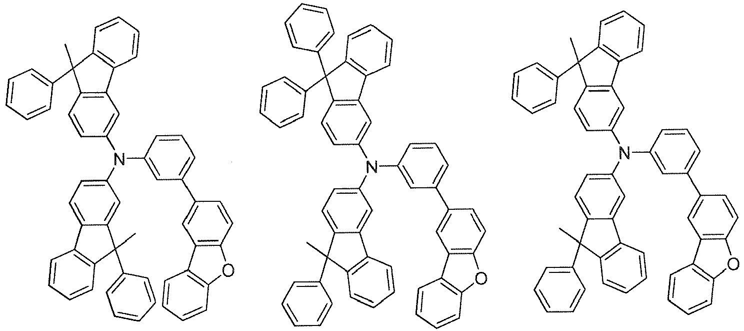

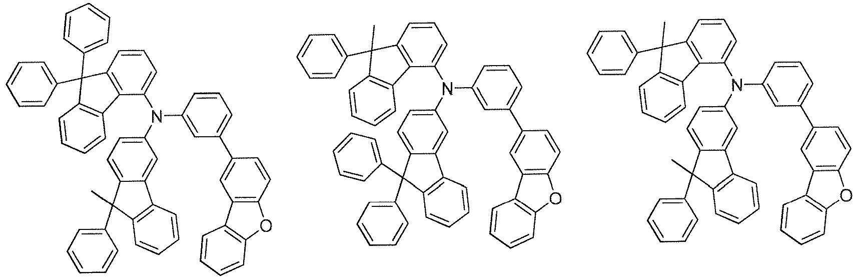

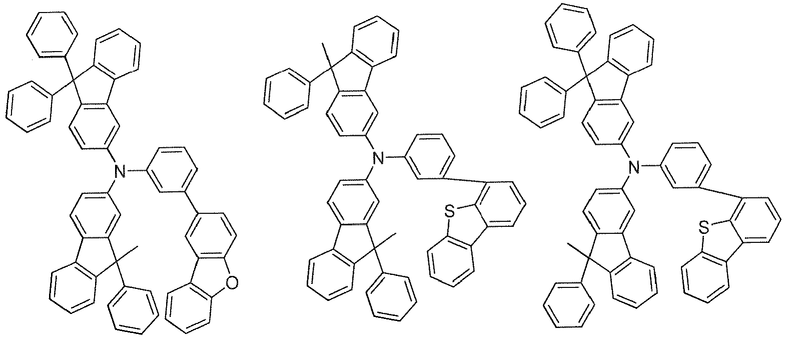

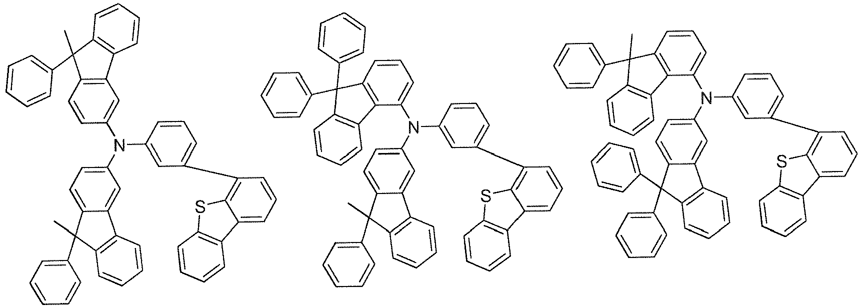

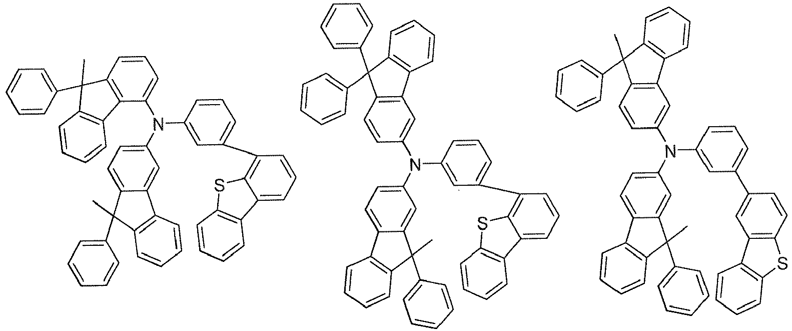

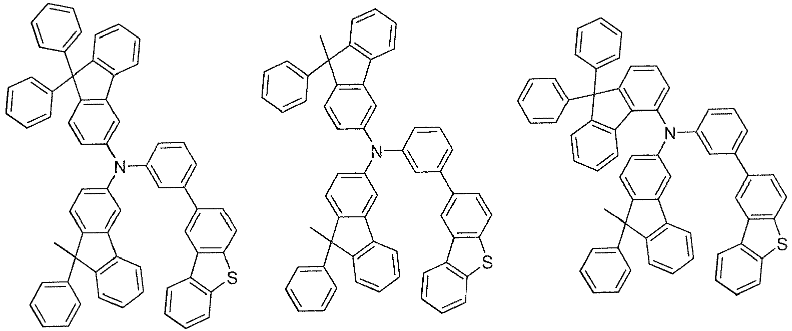

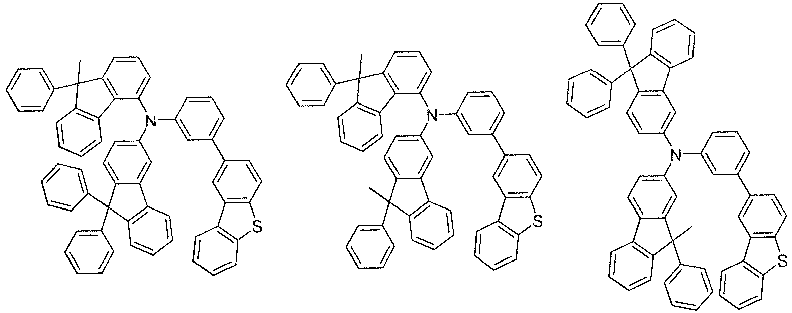

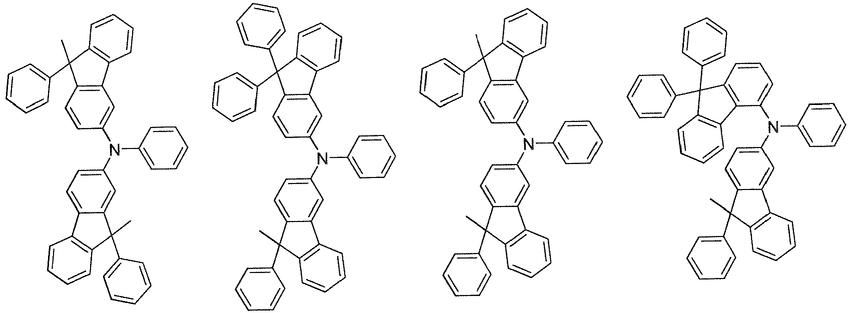

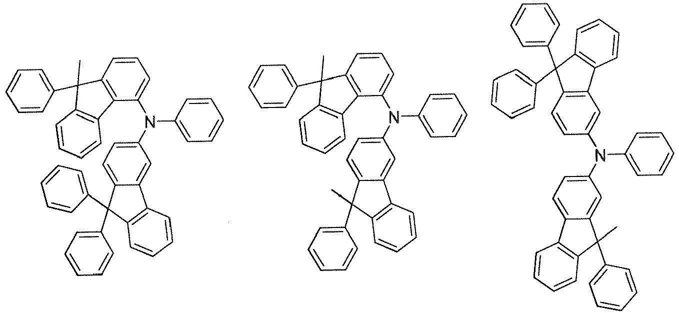

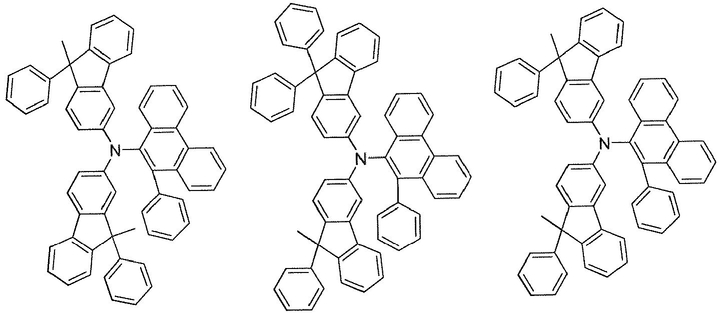

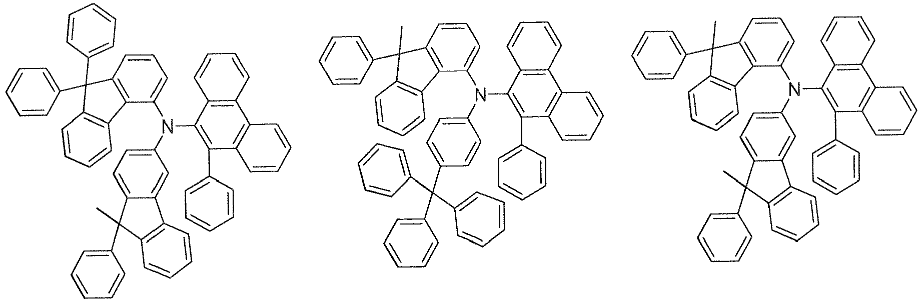

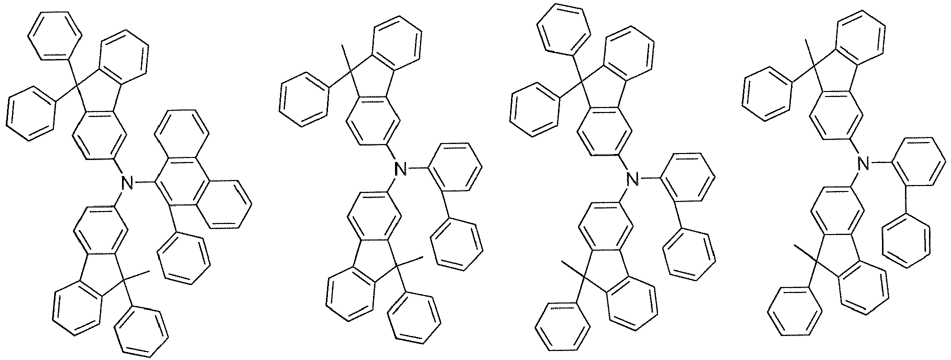

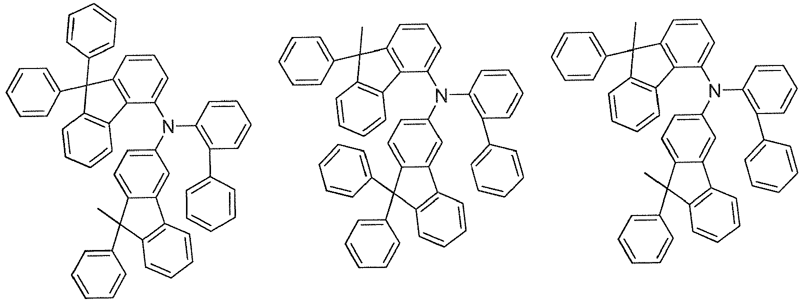

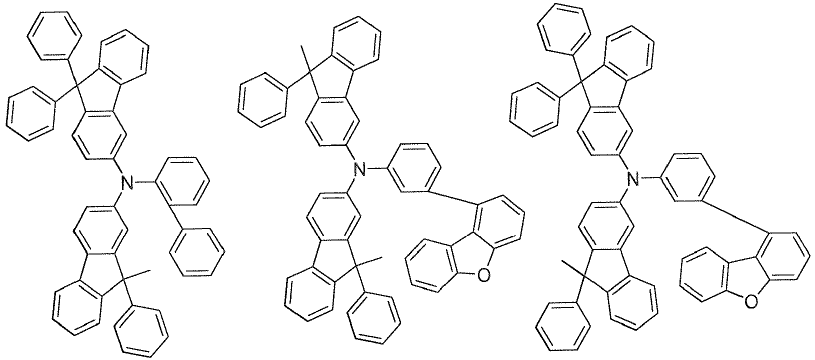

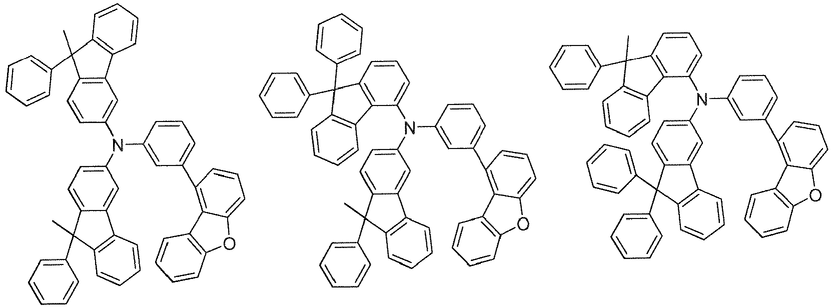

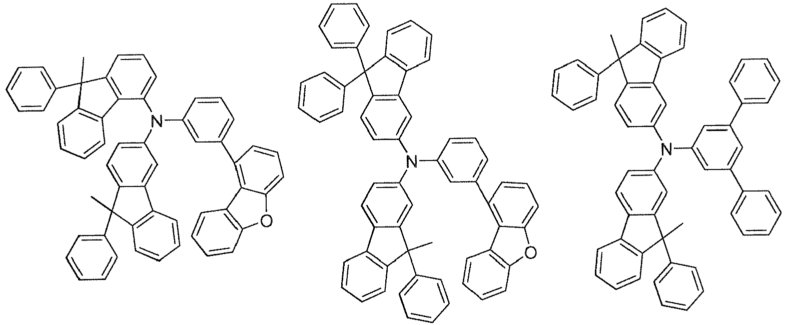

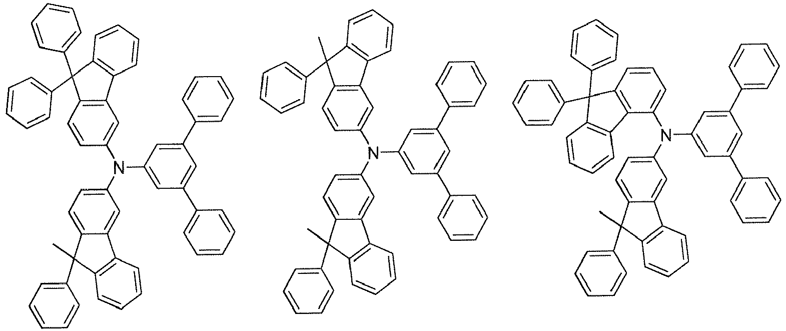

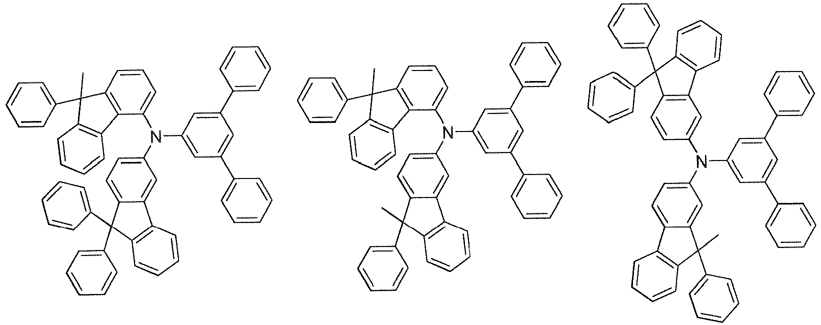

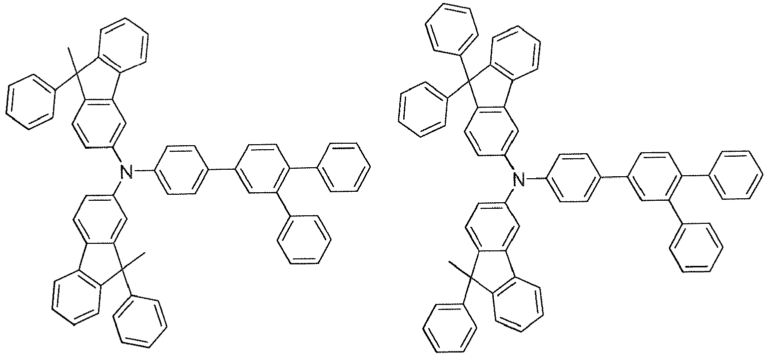

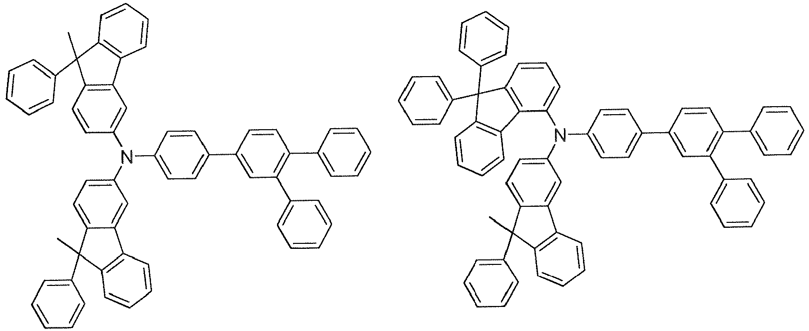

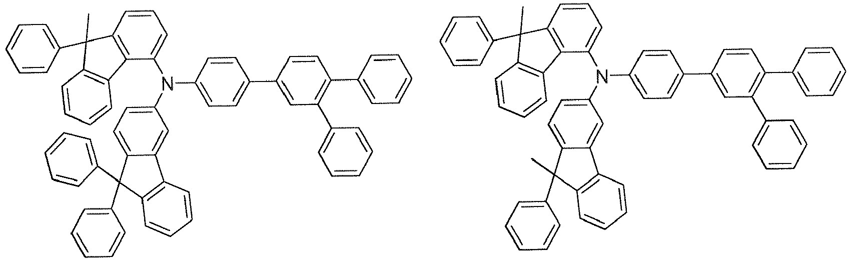

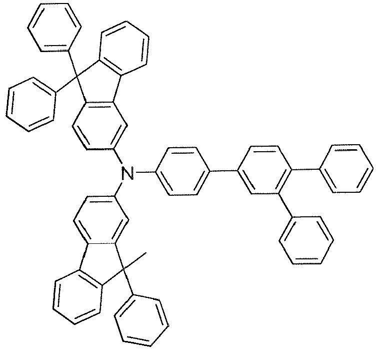

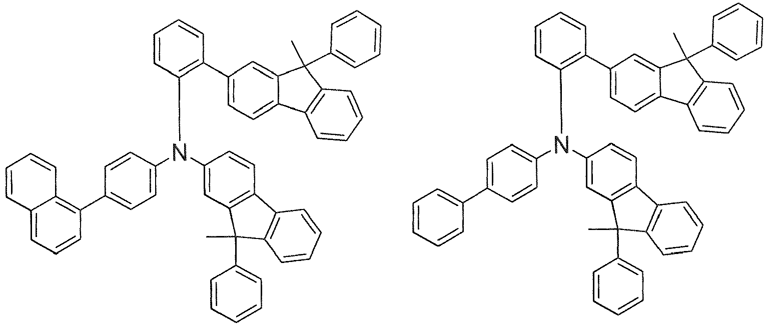

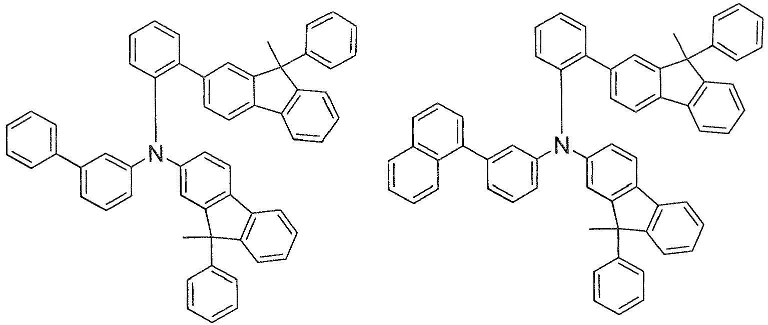

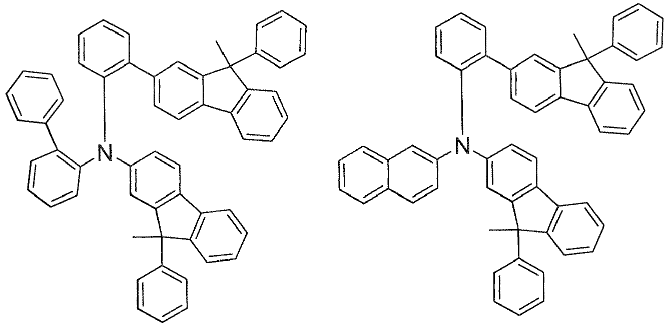

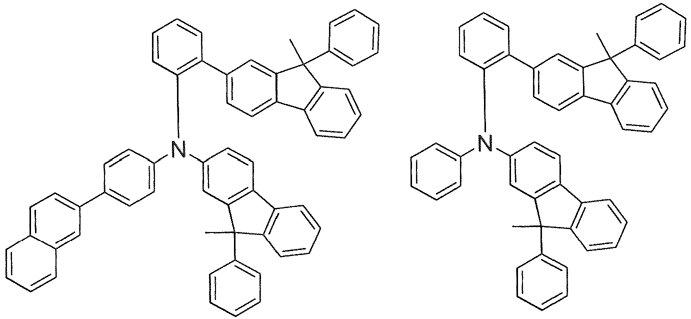

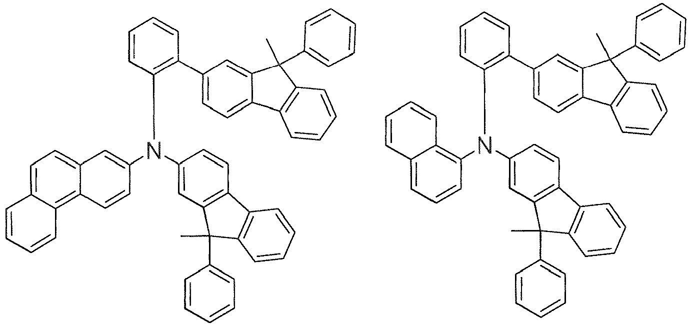

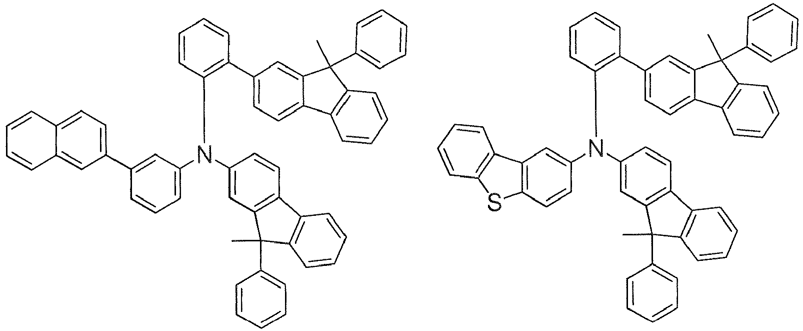

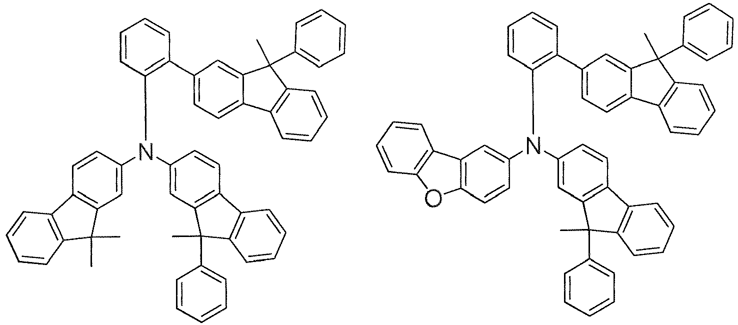

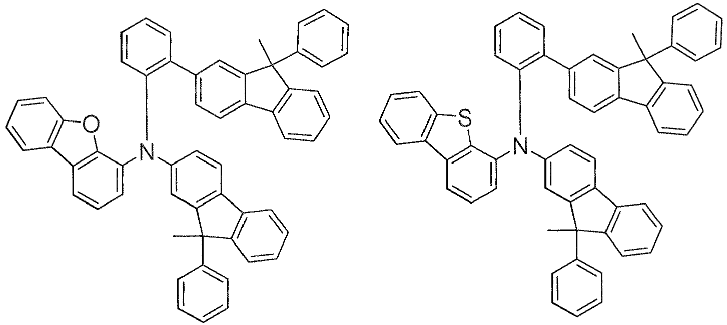

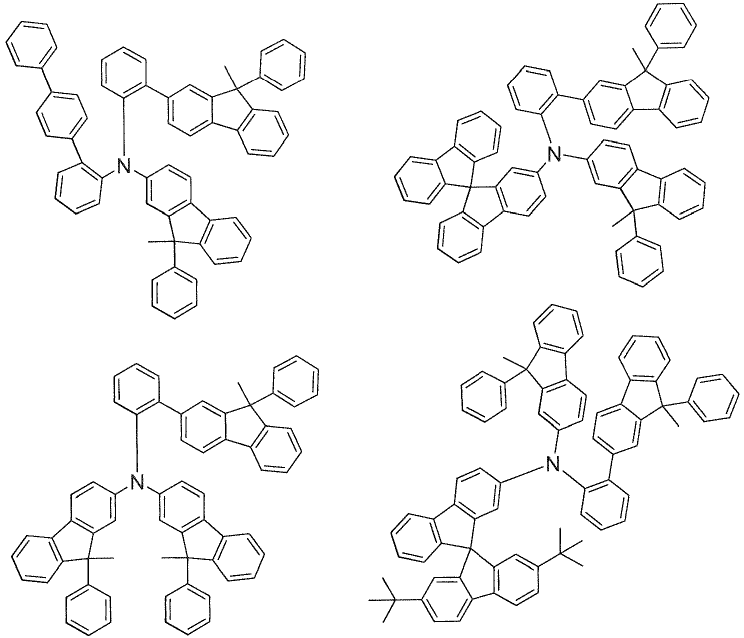

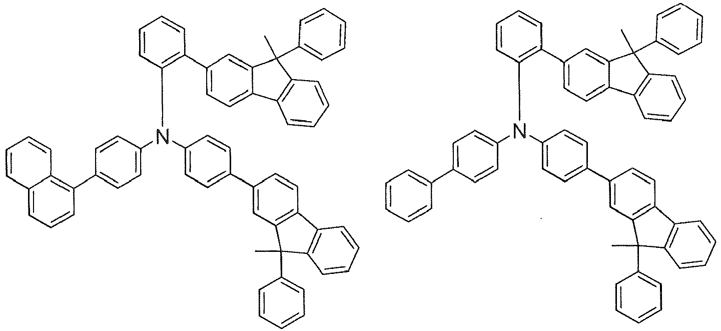

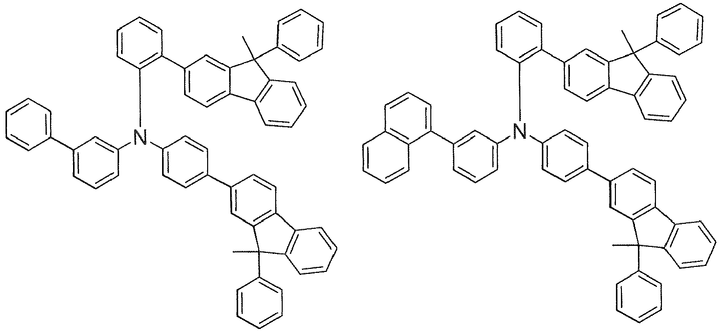

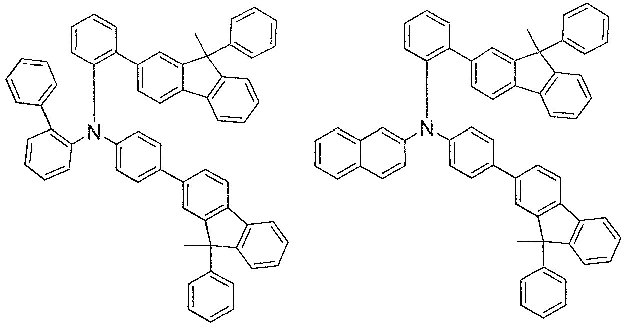

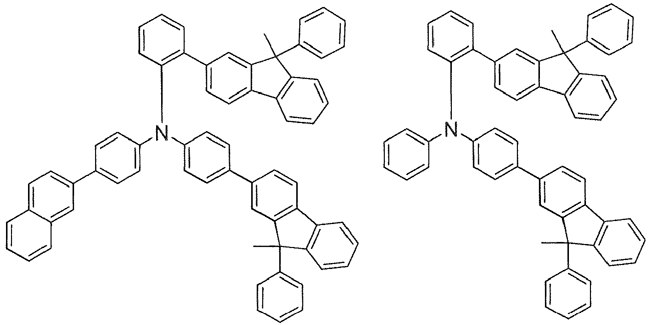

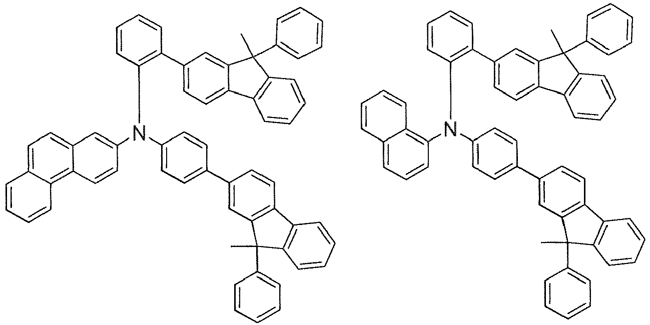

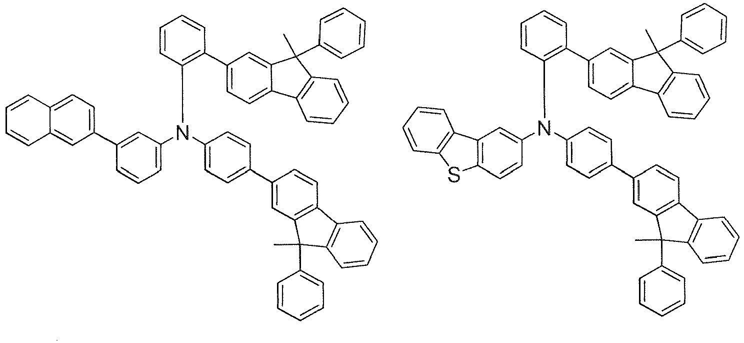

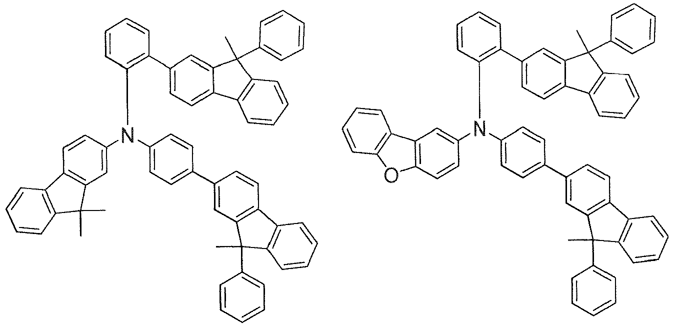

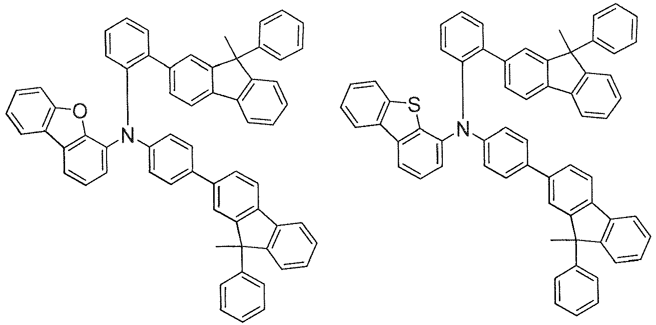

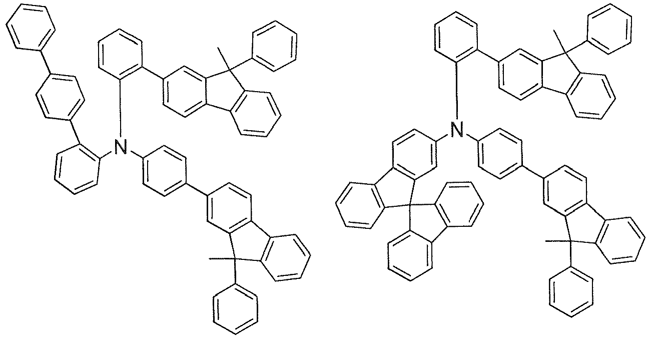

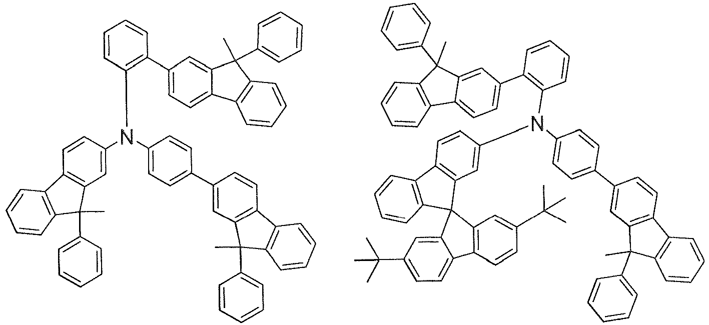

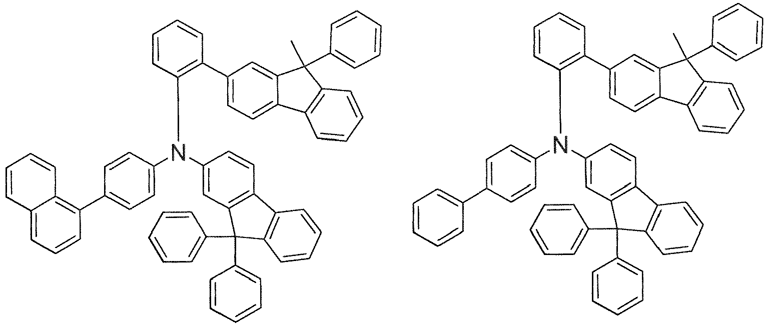

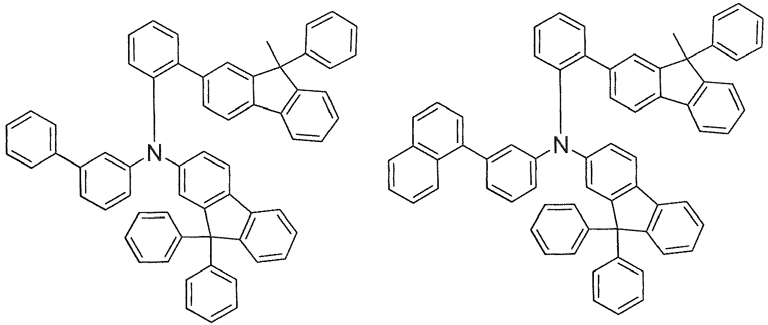

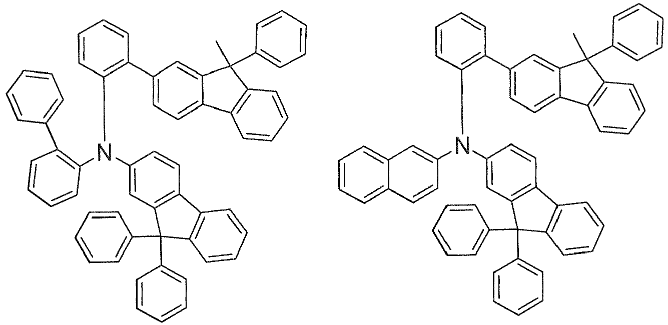

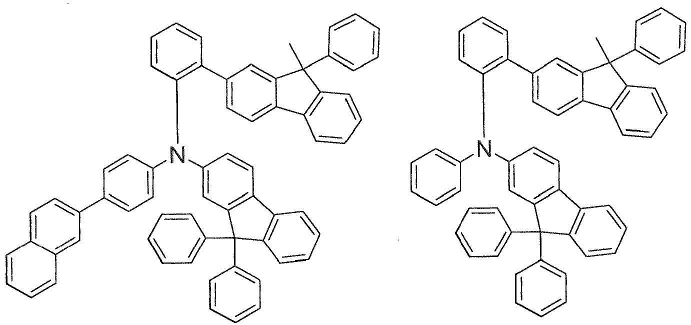

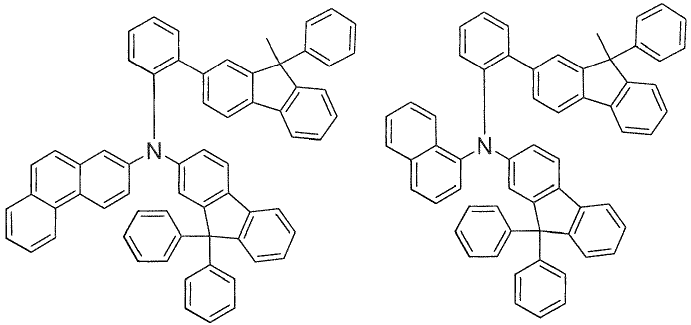

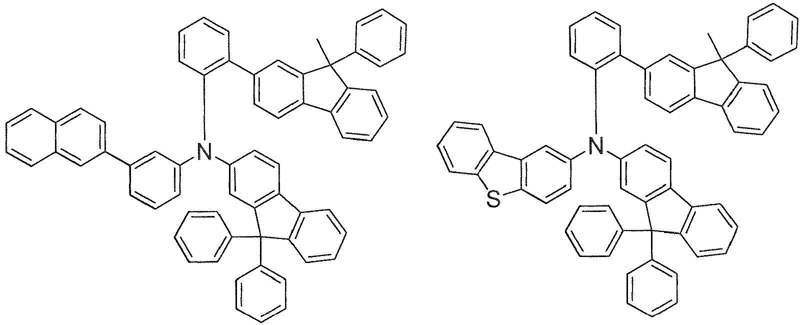

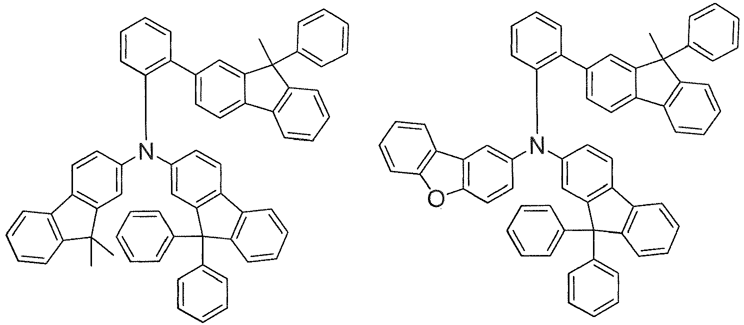

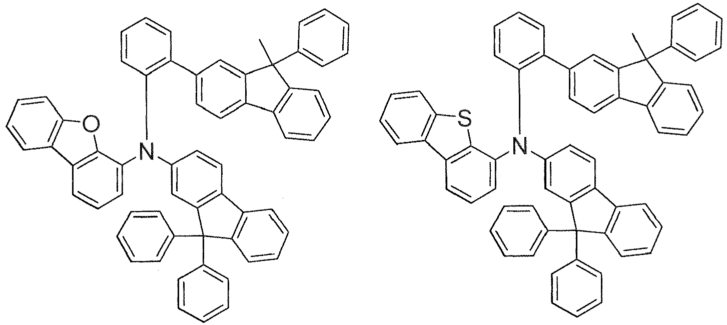

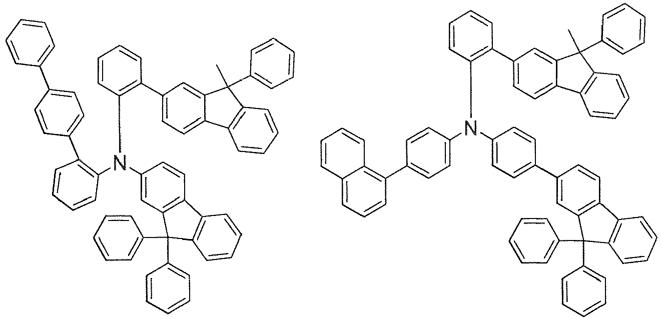

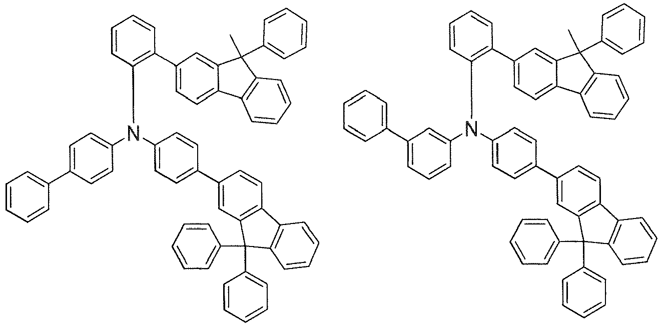

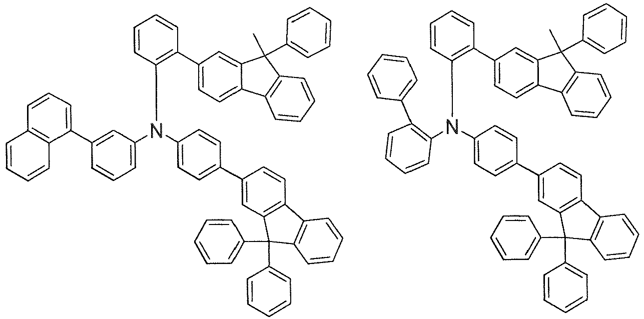

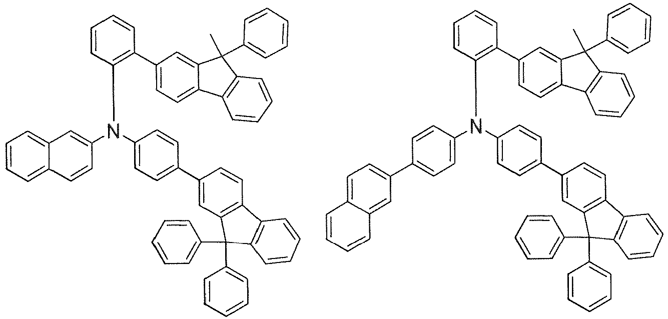

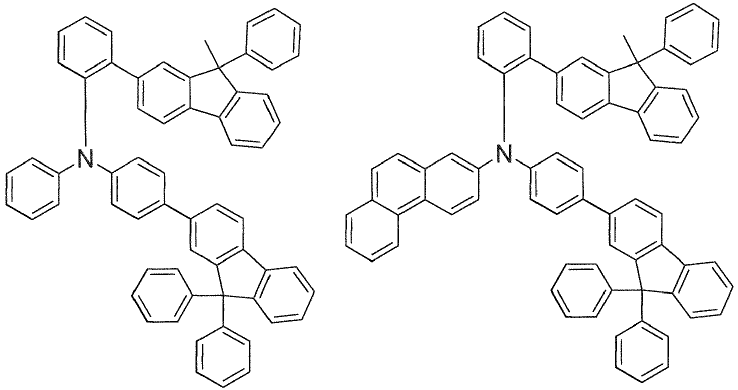

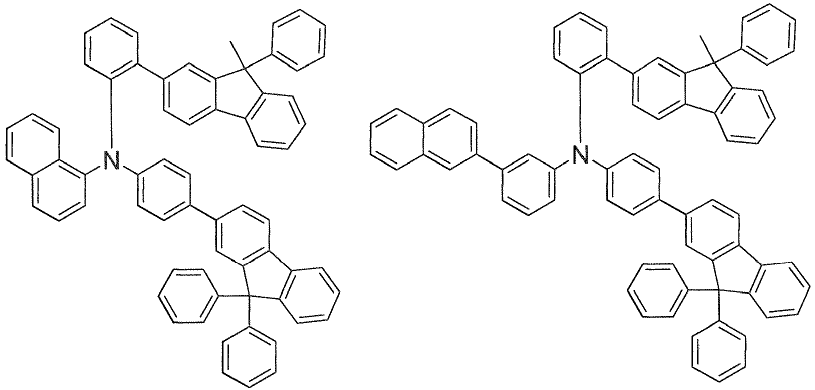

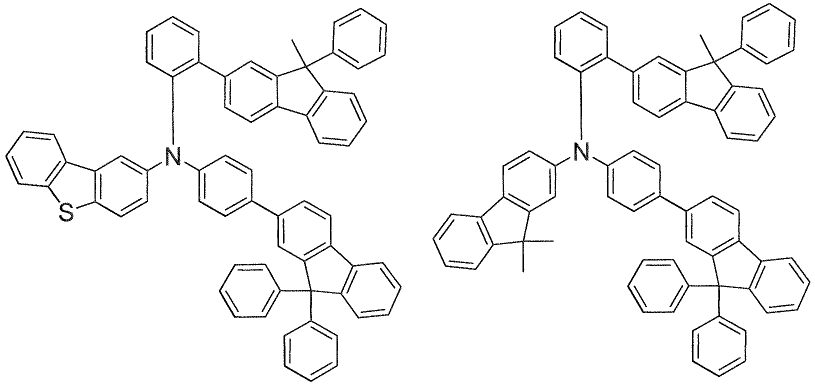

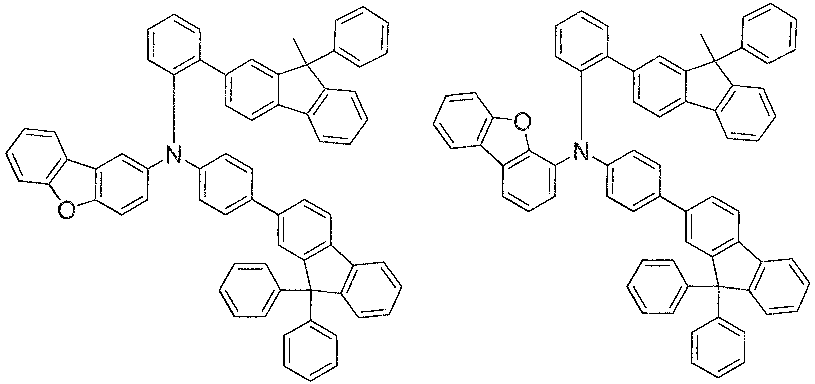

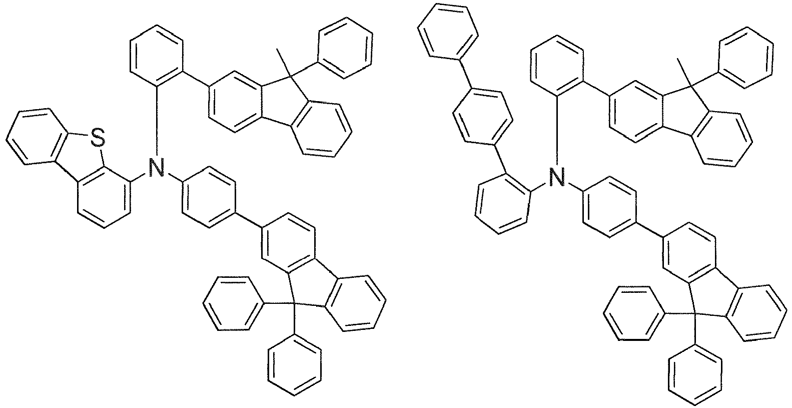

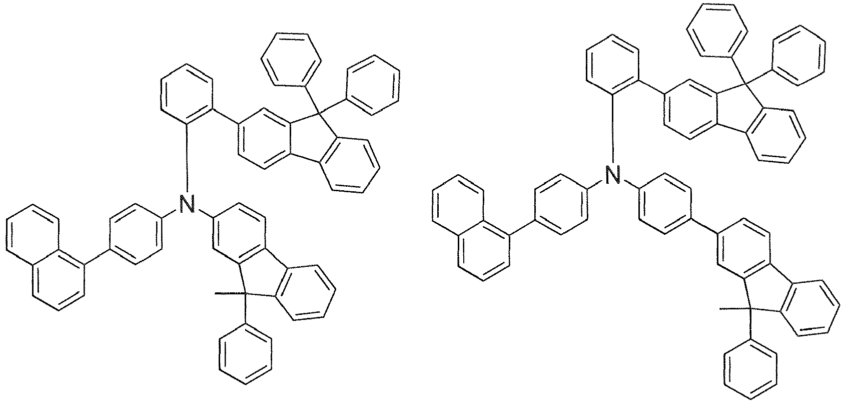

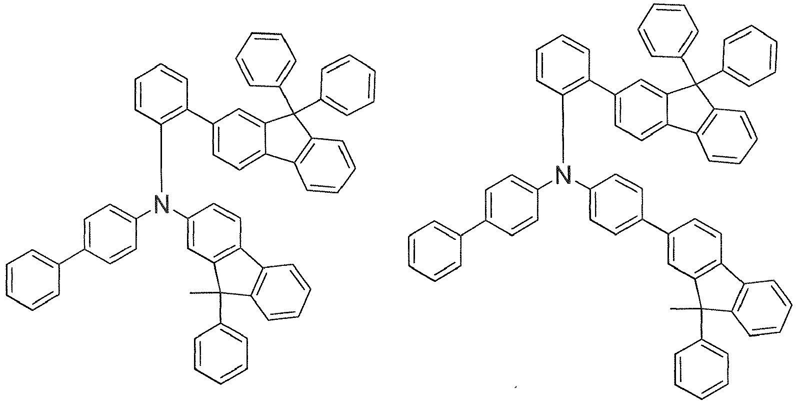

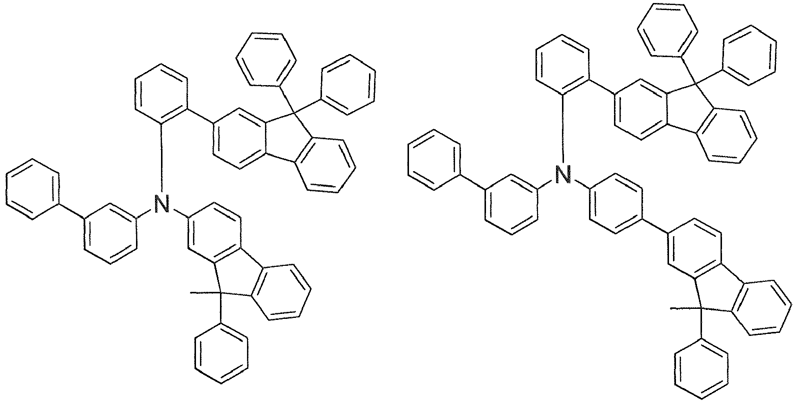

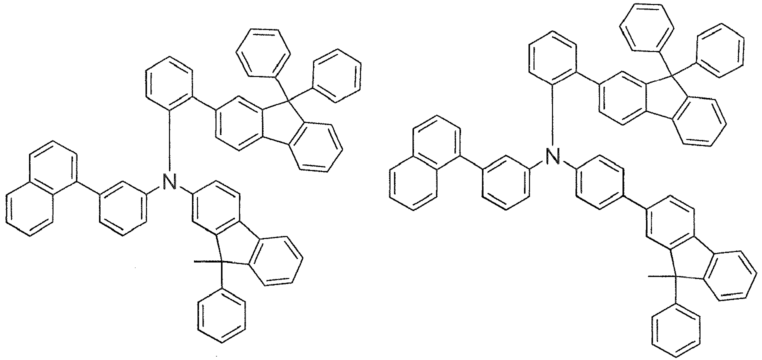

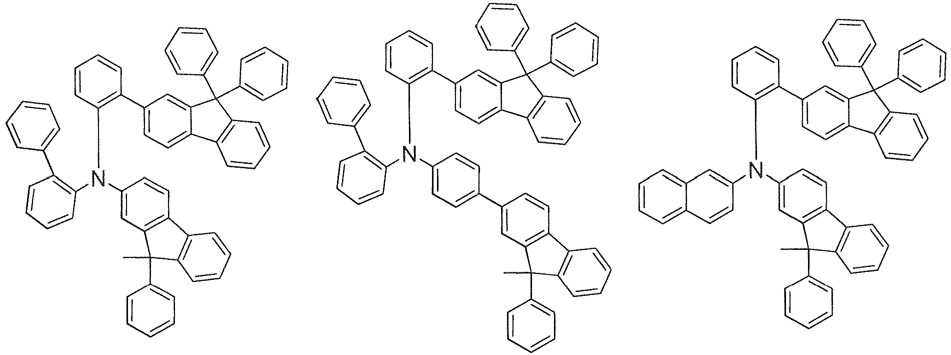

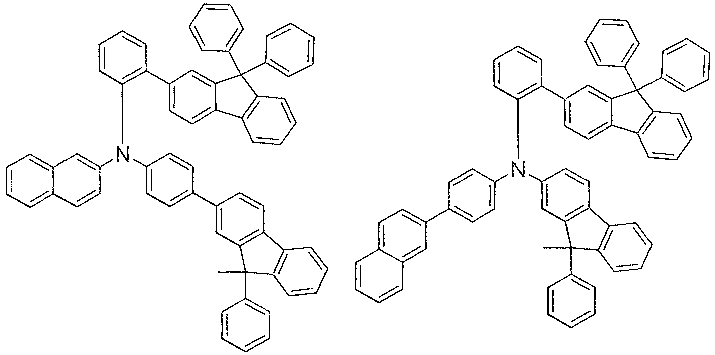

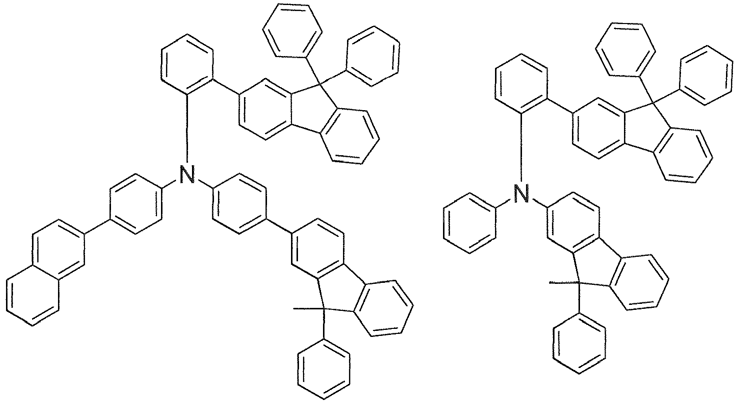

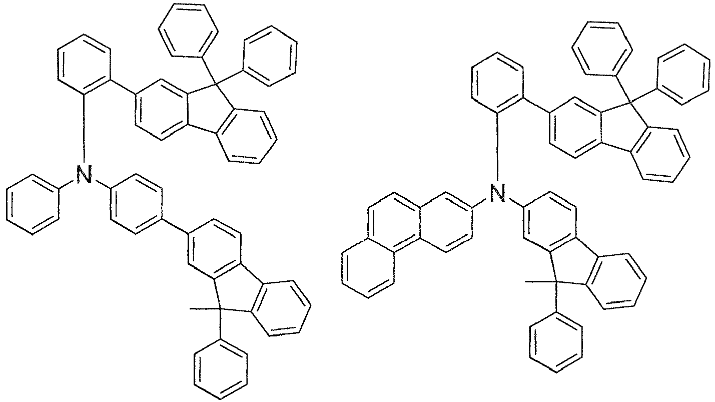

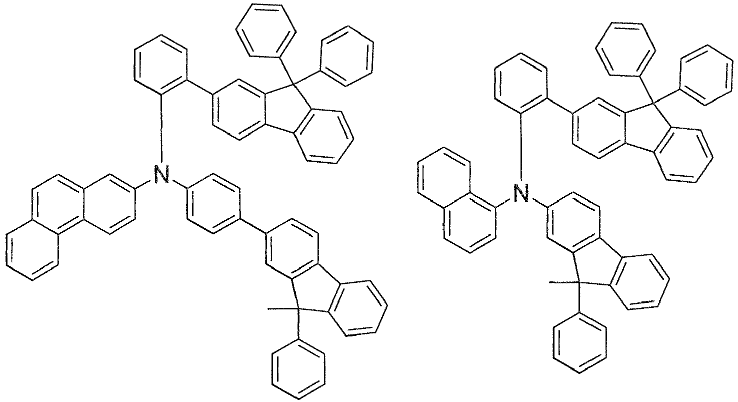

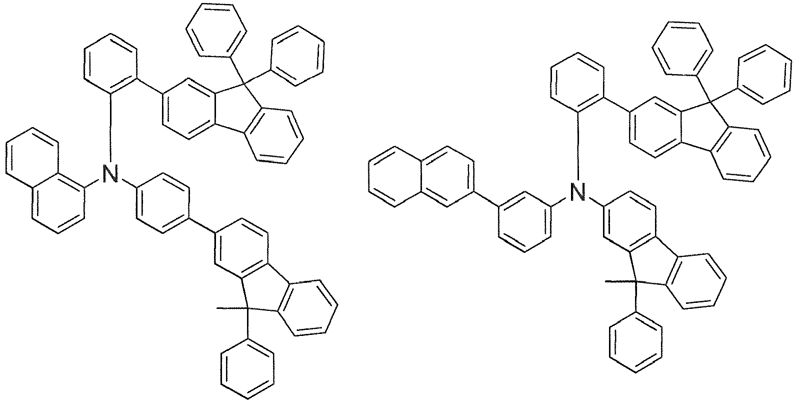

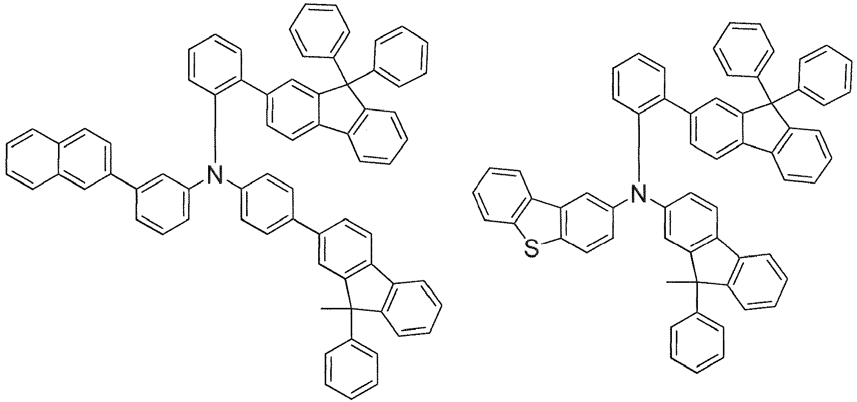

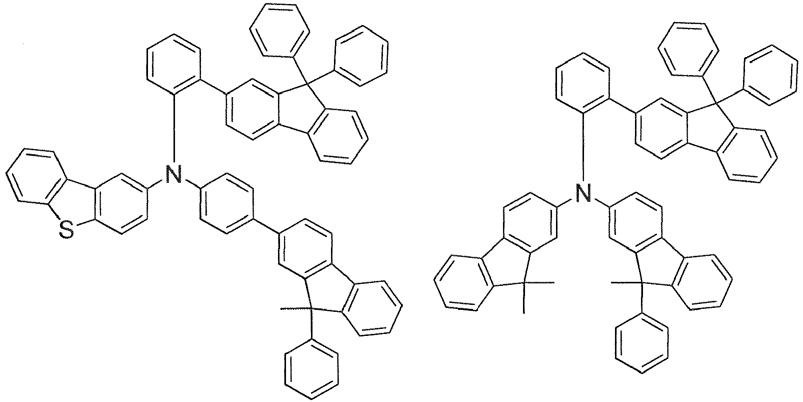

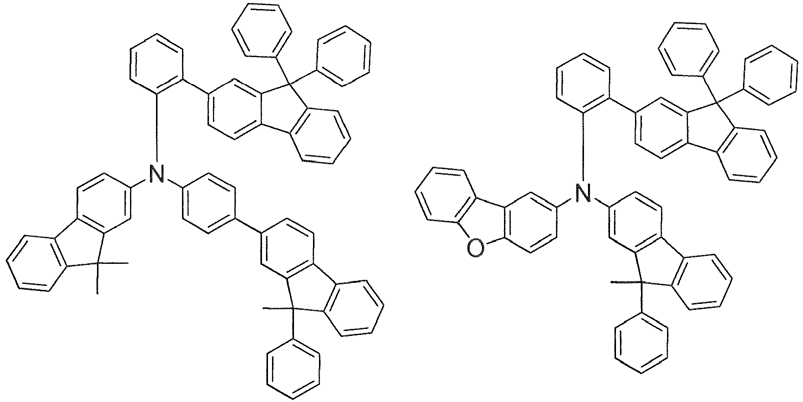

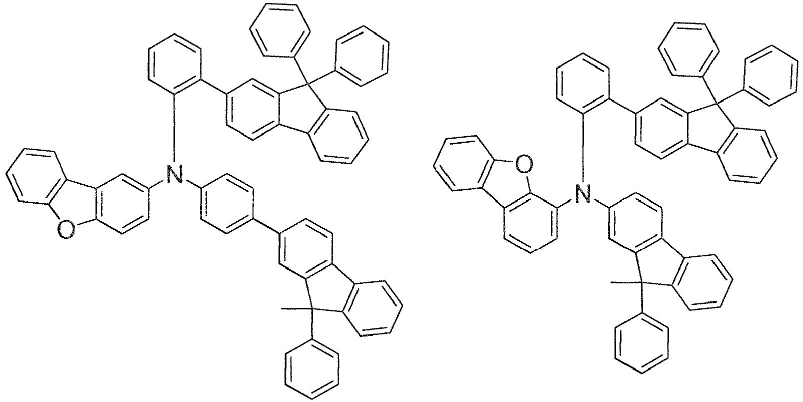

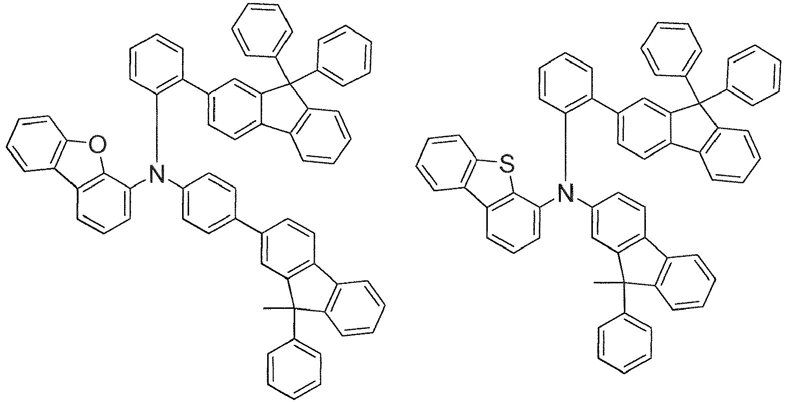

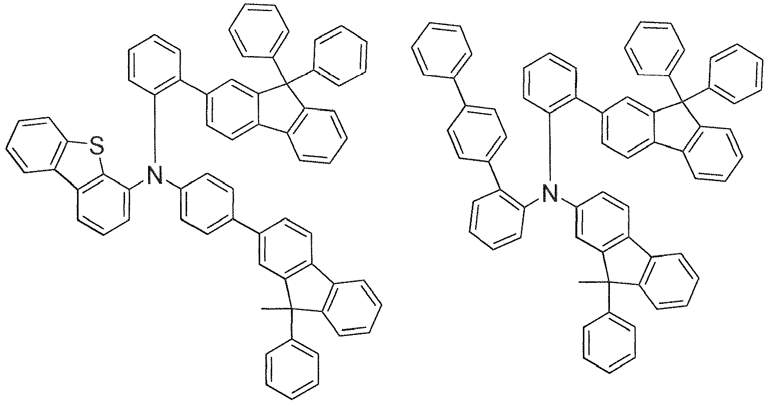

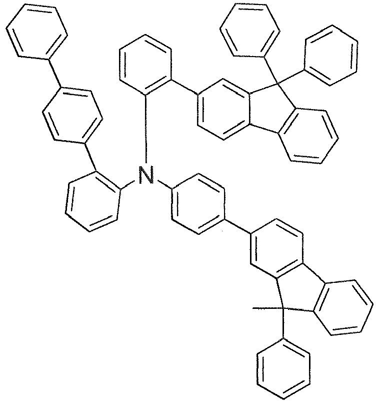

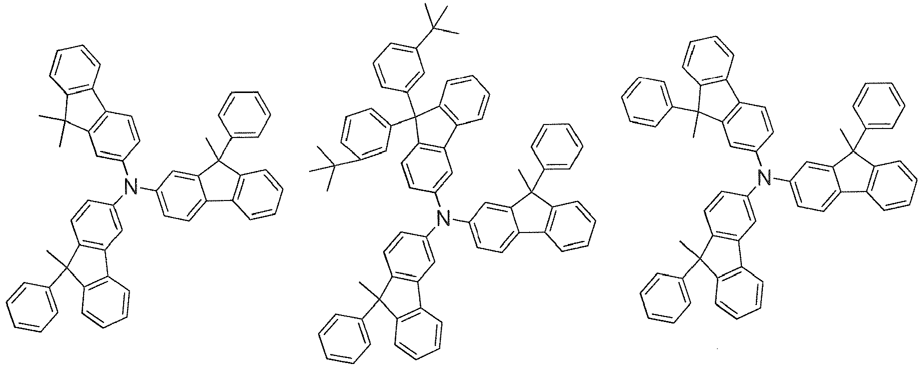

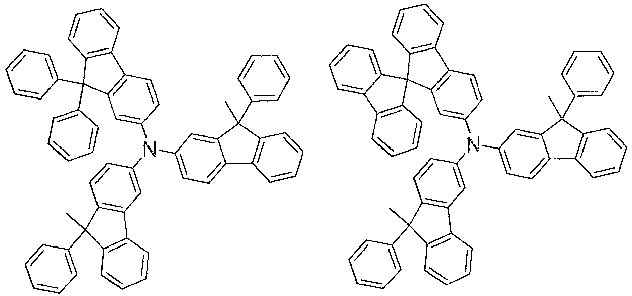

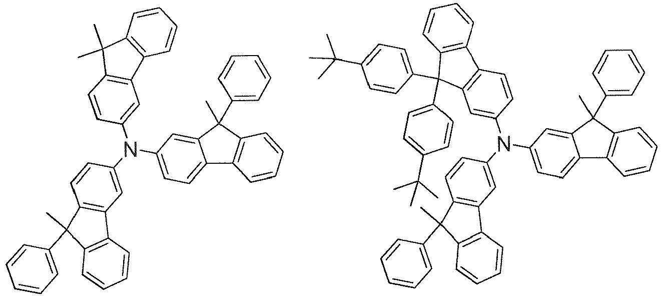

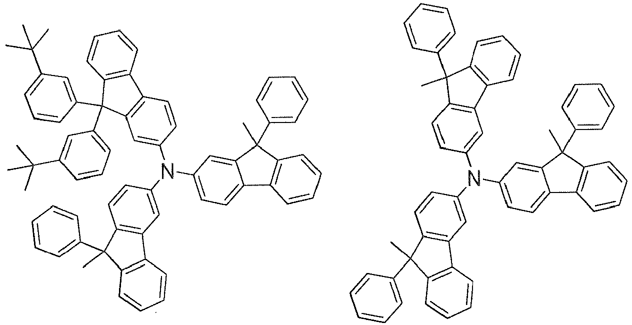

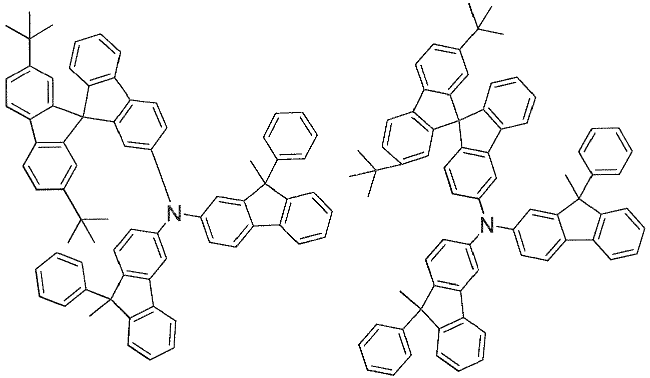

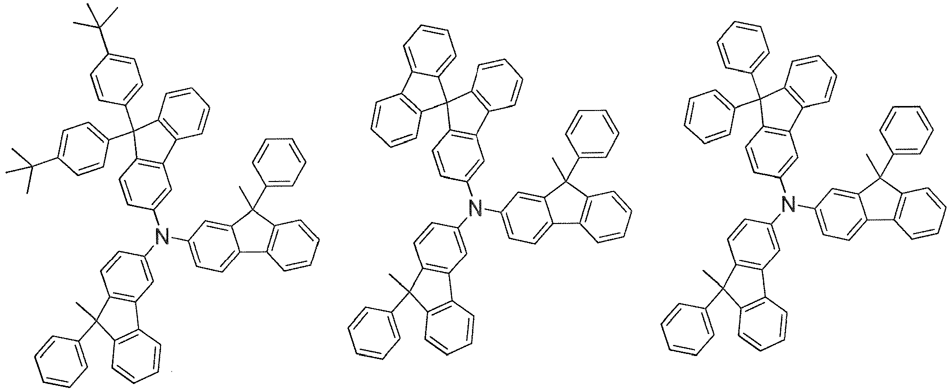

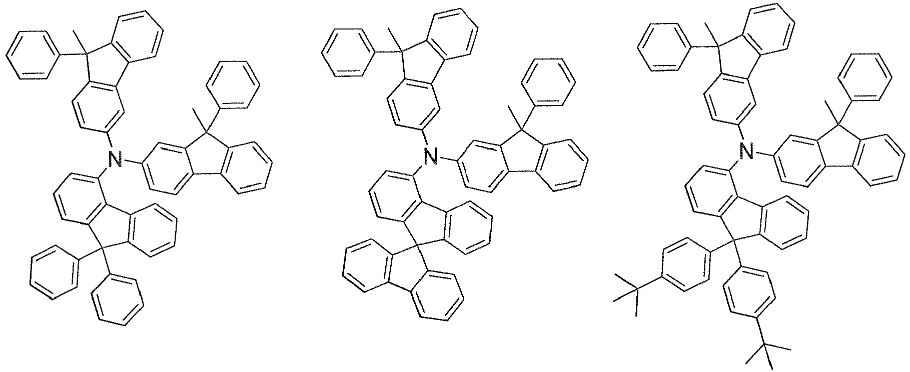

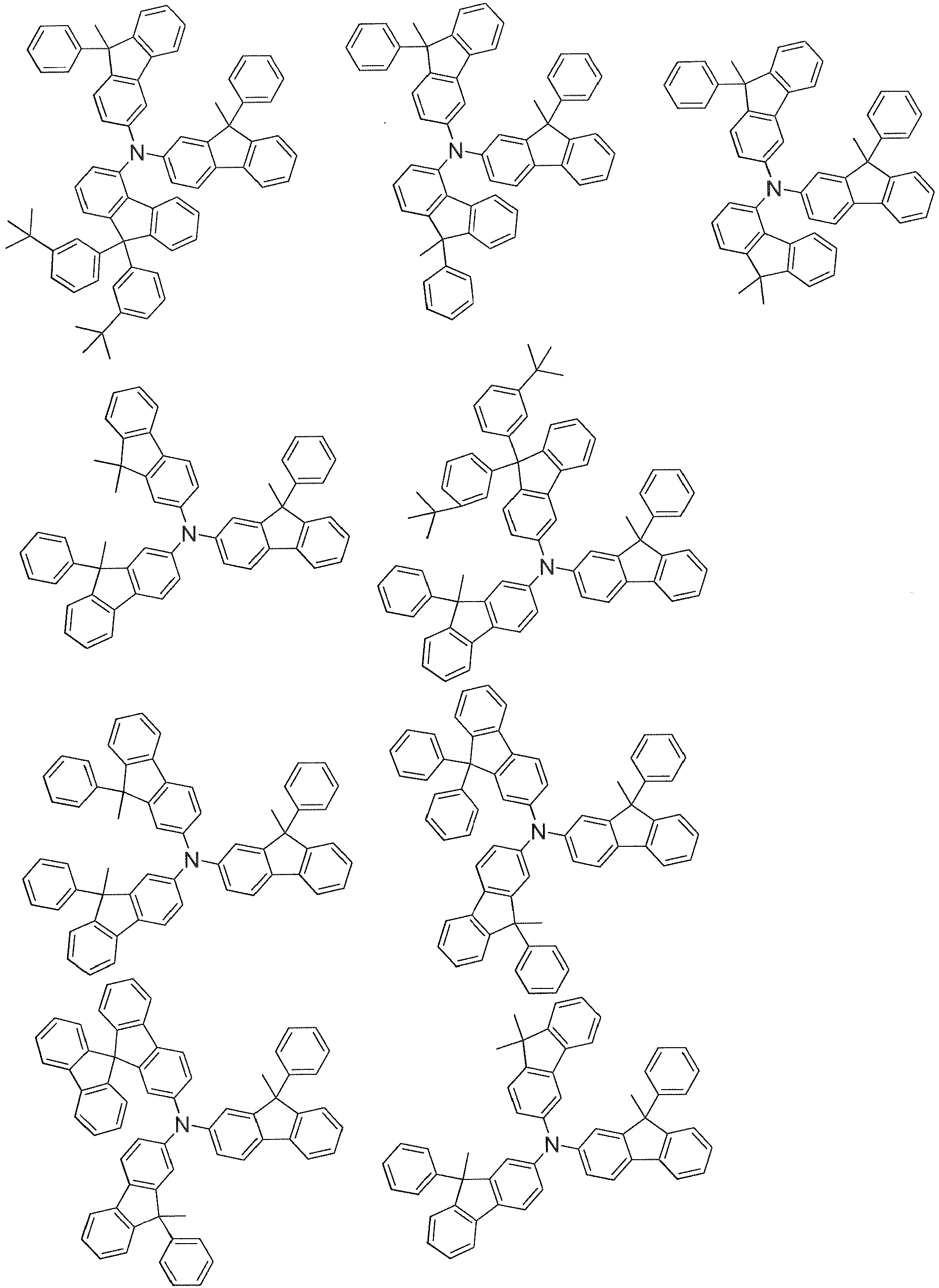

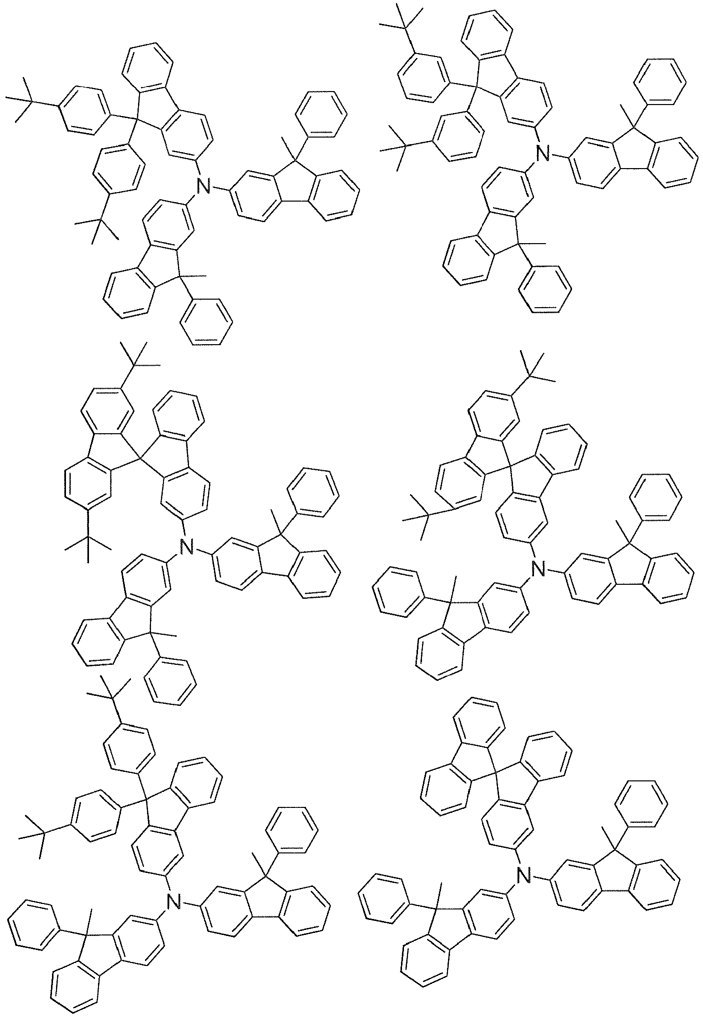

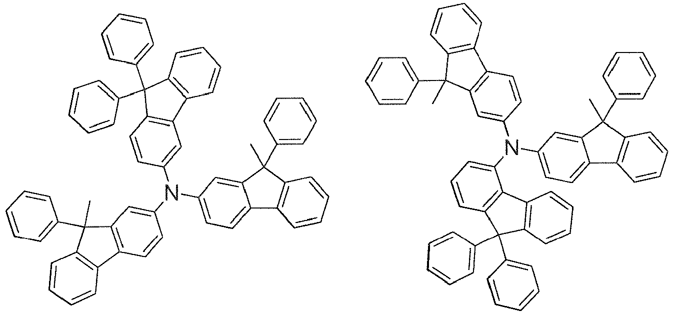

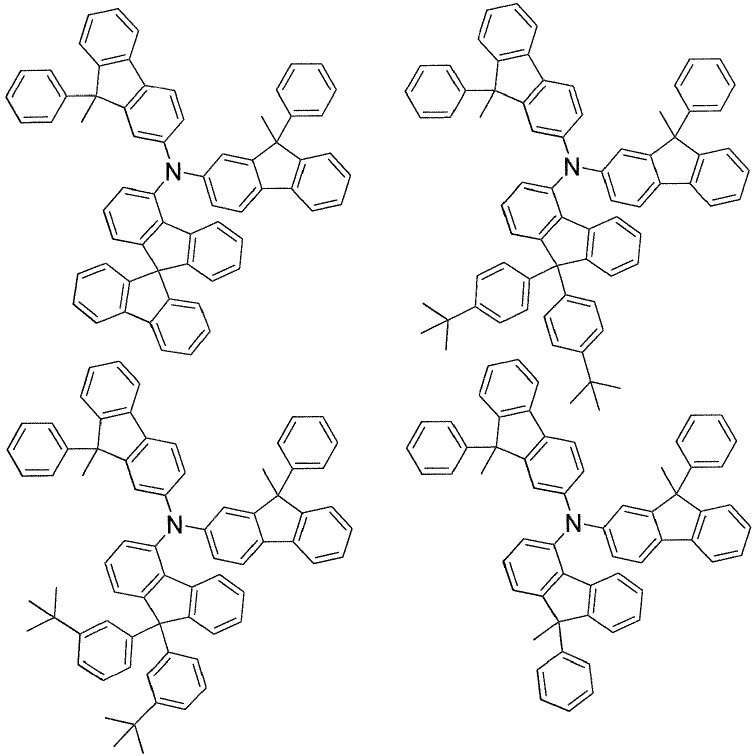

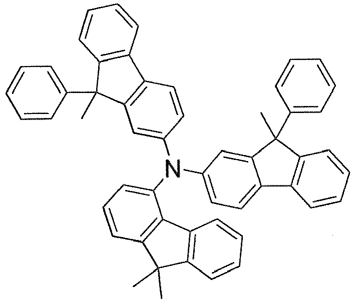

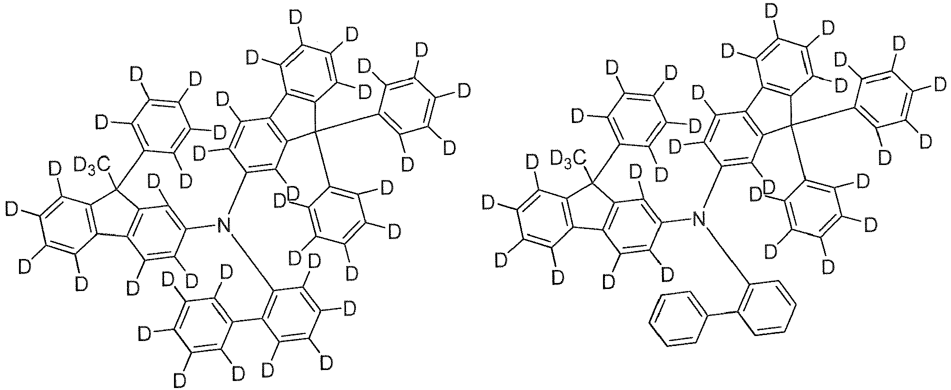

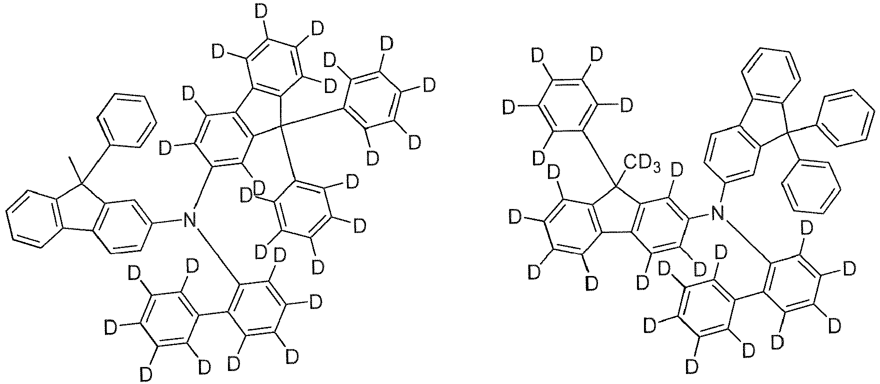

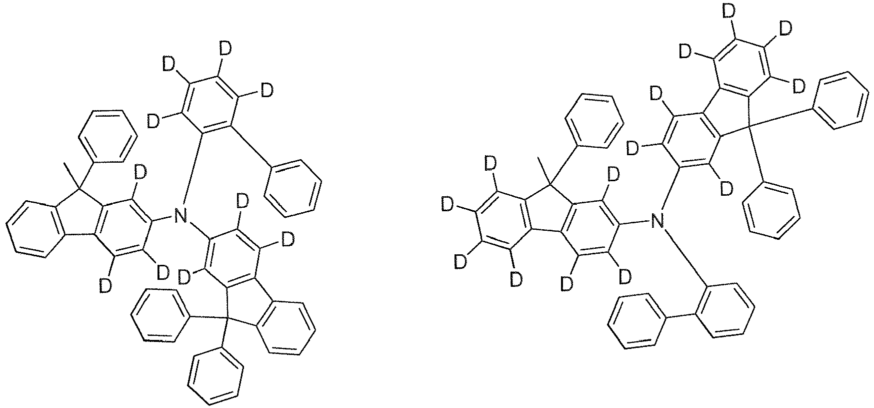

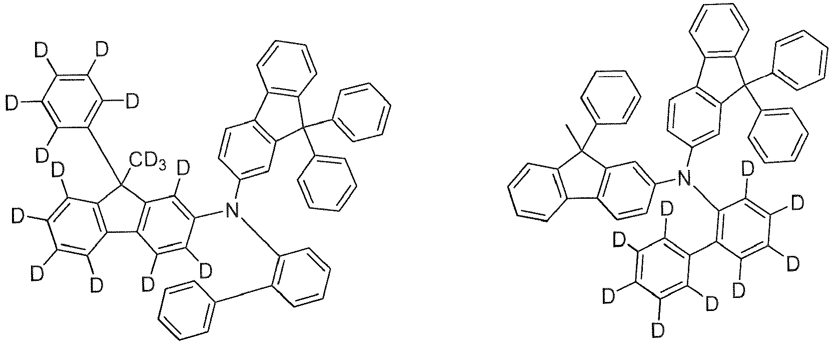

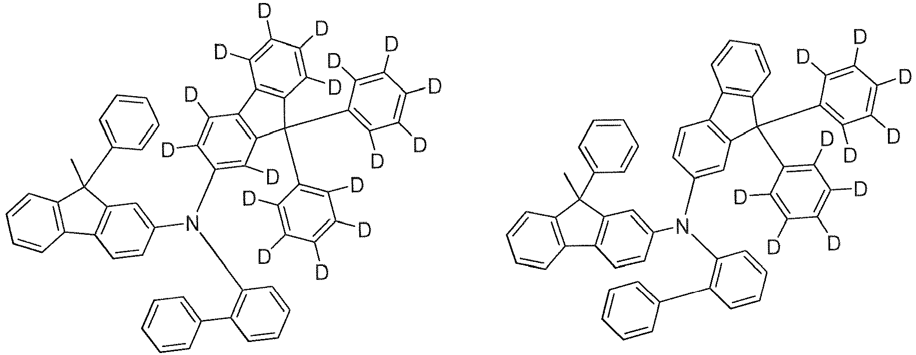

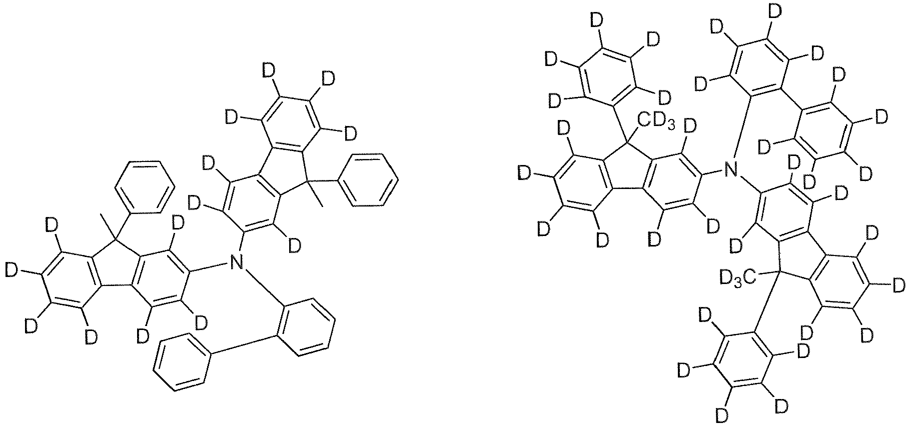

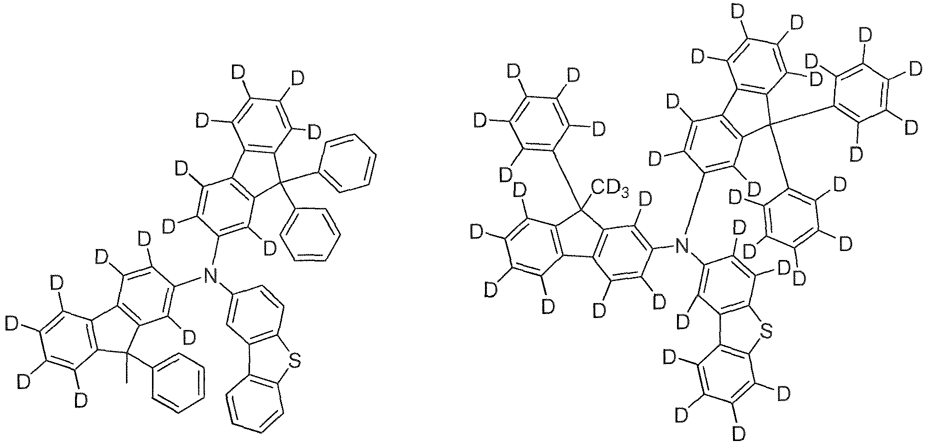

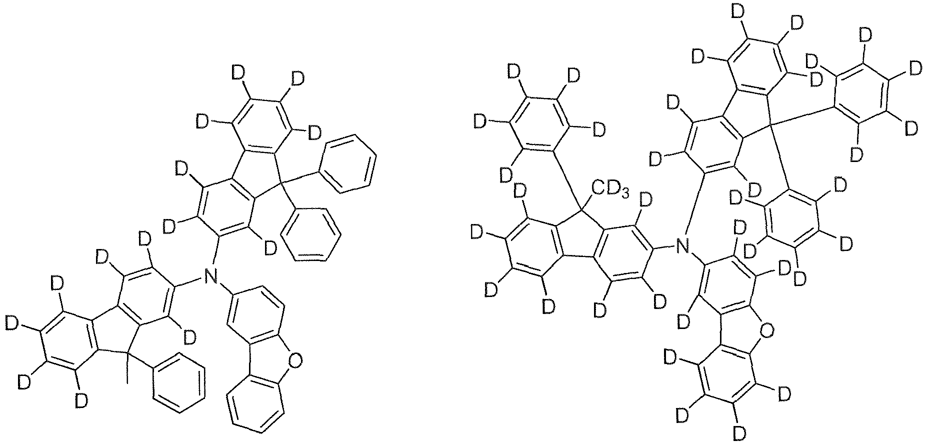

### Material for Organic EL Devices

The material for organic EL devices of the present invention contains the inventive compound. The content of the inventive compound in the material for organic EL devices is 1% by mass or more (including 100%), preferably 10% by mass or more (including 100%), more preferably 50% by mass or more (including 100%), further preferably 80% by mass or more (including 100%), particularly preferably 90% by mass or more (including 100%). The material for organic EL devices of the present invention is useful for production of an organic EL device.

### Organic EL Device

The organic EL device of the present invention includes an anode, a cathode, and organic layers arranged between the anode and the cathode. The organic layers include a light emitting layer, and at least one layer of the organic layers contains the inventive compound.

Examples of the organic layer containing the inventive compound include a hole transporting zone (a hole injecting layer, a hole transporting layer, an electron blocking layer, an exciton blocking layer, etc.) provided between the anode and the light emitting layer, the light emitting layer, a space layer, and an electron transporting zone (an electron injecting layer, an electron transporting layer, a hole blocking layer, etc.) provided between the cathode and the light emitting layer, but are not limited thereto. The inventive compound is preferably used as a material for the hole transporting zone or the light emitting layer in a fluorescent or phosphorescent EL device, more preferably as a material for the hole transporting zone, further preferably as a material for the hole injecting layer, the hole transporting layer, the electron blocking layer, or the exciton blocking layer, particularly preferably as a material for the hole injecting layer or the hole transporting layer.

The organic EL device of the present invention may be a fluorescent or phosphorescent emission-type monochromatic light emitting device, or a fluorescent/phosphorescent hybrid type white light emitting device, and may be a simple type having a single light emitting unit, or a tandem type having a plurality of light emitting units. Above all, the fluorescent light emission-type device is preferred. Herein, the "light emitting unit" refers to a minimum unit including organic layers among which at least one layer is a light emitting layer, in which light is emitted through recombination of injected holes and electrons.

For example, as a representative device configuration of the simple type organic EL device, the following device configuration may be exemplified.

### (1) Anode/Light Emitting Unit/Cathode

Further, the light emitting unit may be a multilayer type having a plurality of phosphorescent light emitting layers or fluorescent light emitting layers. In this case, a space layer may be provided between the light emitting layers for the purpose of preventing excitons generated in the phosphorescent light emitting layer from diffusing into the fluorescent light emitting layer. Representative layer configurations of the simple type light emitting unit are illustrated below. Layers in parentheses are optional.
(a) (hole injecting layer/) hole transporting layer/fluorescent light emitting layer/electron transporting layer (/electron injecting layer)
(b) (hole injecting layer/) hole transporting layer/phosphorescent light emitting layer electron transporting layer (/electron injecting layer)
(c) (hole injecting layer/) hole transporting layer/first fluorescent light emitting layer/second fluorescent light emitting layer/electron transporting layer (/electron injecting layer)
(d) (hole injecting layer/) hole transporting layer/first phosphorescent light emitting layer/second phosphorescent light emitting layer/electron transporting layer (/electron injecting layer)
(e) (hole injecting layer/) hole transporting layer/phosphorescent light emitting layer/space layer/fluorescent light emitting layer/electron transporting layer (/electron injecting layer)
(f) (hole injecting layer/) hole transporting layer/first phosphorescent light emitting layer/second phosphorescent light emitting layer/space layer/fluorescent light emitting layer/electron transporting layer (/electron injecting layer)
(g) (hole injecting layer/) hole transporting layer/first phosphorescent light emitting layer/space layer/second phosphorescent light emitting layer/space layer/fluorescent light emitting layer/electron transporting layer (/electron injecting layer)
(h) (hole injecting layer/) hole transporting layer/phosphorescent light emitting layer/space layer/first fluorescent light emitting layer/second fluorescent light emitting layer/electron transporting layer (/electron injecting layer)
(i) (hole injecting layer/) hole transporting layer/electron blocking layer/fluorescent light emitting layer/electron transporting layer (/electron injecting layer)
(j) (hole injecting layer/) hole transporting layer/electron blocking layer/phosphorescent light emitting layer/electron transporting layer (/electron injecting layer)
(k) (hole injecting layer/) hole transporting layer/exciton blocking layer/fluorescent light emitting layer/electron transporting layer (/electron injecting layer)
(l) (hole injecting layer/) hole transporting layer/exciton blocking layer/phosphorescent light emitting layer/electron transporting layer (/electron injecting layer)
(m) (hole injecting layer/) first hole transporting layer/second hole transporting layer/fluorescent light emitting layer/electron transporting layer (/electron injecting layer)
(n) (hole injecting layer/) first hole transporting layer/second hole transporting layer/phosphorescent light emitting layer/electron transporting layer (/electron injecting layer)
(o) (hole injecting layer/) first hole transporting layer/second hole transporting layer/fluorescent light emitting layer/first electron transporting layer/second electron transporting layer (/electron injecting layer)
(p) (hole injecting layer/) first hole transporting layer/second hole transporting layer/phosphorescent light emitting layer/first electron transporting layer/second electron transporting layer (/electron injecting layer)
(q) (hole injecting layer/) hole transporting layer/fluorescent light emitting layer/hole blocking layer/electron transporting layer (/electron injecting layer)
(r) (hole injecting layer/) hole transporting layer/phosphorescent light emitting layer/hole blocking layer/electron transporting layer (/electron injecting layer)
(s) (hole injecting layer/) hole transporting layer/fluorescent light emitting layer/exciton blocking layer/electron transporting layer (/electron injecting layer)
(t) (hole injecting layer/) hole transporting layer/phosphorescent light emitting layer/exciton blocking layer/electron transporting layer (/electron injecting layer)
(m) (hole injecting layer/) first hole transporting layer/second hole transporting layer/third hole transporting layer/first fluorescent light emitting layer/second fluorescent light emitting layer/first electron transporting layer/second electron transporting layer (/electron injecting layer)
(n) (hole injecting layer/) first hole transporting layer/second hole transporting layer/third hole transporting layer/fluorescent light emitting layer/first electron transporting layer/second electron transporting layer (/electron injecting layer)

The phosphorescent or fluorescent light emitting layers can exhibit different emission colors, respectively. Specifically, in the light emitting unit (f), a layer configuration such as (hole injecting layer/) hole transporting layer/first phosphorescent light emitting layer (red light emission)/second phosphorescent light emitting layer (green light emission)/space layer/fluorescent light emitting layer (blue light emission)/electron transporting layer, may be exemplified.

The electron blocking layer may be appropriately provided between each light emitting layer and the hole transporting layer or the space layer. Further, the hole blocking layer may be appropriately provided between each light emitting layer and the electron transporting layer. By providing the electron blocking layer or the hole blocking layer, it is possible to confine electrons or holes within the light emitting layer and to increase the probability of charge recombination in the light emitting layer, thereby improving the emission efficiency.

As a representative device configuration of the tandem type organic EL device, the following device configuration may be exemplified.

### (2) Anode/First Light Emitting Unit/Intermediate Layer/Second Light Emitting Unit/Cathode

Herein, for example, each of the first light emitting unit and the second light emitting unit can be independently selected from the above-described light emitting units.

The intermediate layer is also generally called an intermediate electrode, an intermediate conductive layer, a charge generation layer, an electron withdrawing layer, a connecting layer, or an intermediate insulating layer, and it is possible to use a known material configuration in which electrons and holes are supplied to the first light emitting unit and the second light emitting unit, respectively.

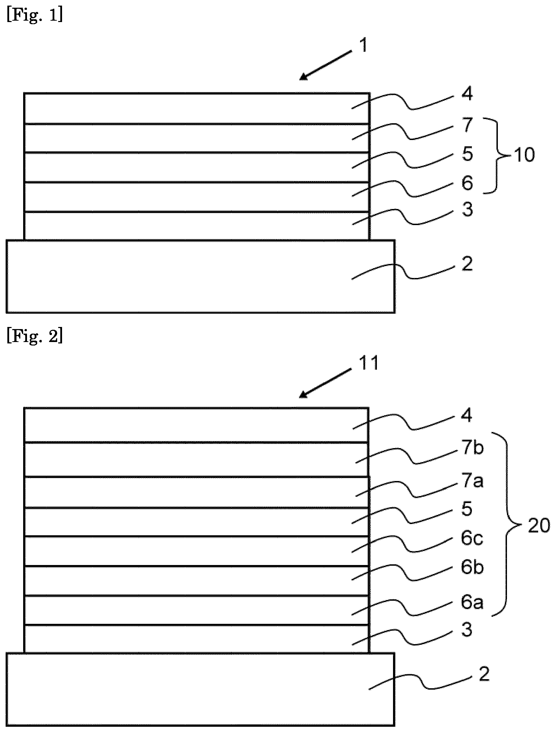

FIG. 1 is a schematic view illustrating an example of the configuration of the organic EL device of the present invention. An organic EL device 1 includes a substrate 2, an anode 3, a cathode 4, and a light emitting unit 10 arranged between the anode 3 and the cathode 4. The light emitting unit 10 has a light emitting layer 5, and has a hole transporting zone 6 (a hole injecting layer, a hole transporting layer, etc.) between the light emitting layer 5 and the anode 3, and an electron transporting zone 7 (an electron injecting layer, an electron transporting layer, etc.) between the light emitting layer 5 and the cathode 4. Further, an electron blocking layer (not illustrated) and a hole blocking layer (not illustrated) may be provided on the anode 3 side of the light emitting layer 5 and on the cathode 4 side of the light emitting layer 5, respectively. Accordingly, electrons or holes can be confined in the light emitting layer 5, so that the exciton generation efficiency in the light emitting layer 5 can be further increased.

FIG. 2 is a schematic view illustrating another configuration of the organic EL device of the present invention. An organic EL device 11 includes a substrate 2, an anode 3, a cathode 4, and a light emitting unit 20 arranged between the anode 3 and the cathode 4. The light emitting unit 20 has a light emitting layer 5. A hole transporting zone arranged between the anode 3 and the light emitting layer 5 is formed by a hole injecting layer 6a, a first hole transporting layer 6b and a second hole transporting layer 6c. Further, an electron transporting zone arranged between the light emitting layer 5 and the cathode 4 is formed by a first electron transporting layer 7a and a second electron transporting layer 7b.

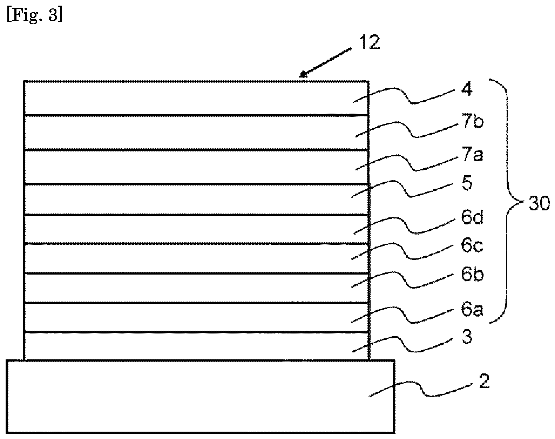

FIG. 3 is a schematic view illustrating another configuration of the organic EL device of the present invention. An organic EL device 12 includes a substrate 2, an anode 3, a cathode 4, and a light emitting unit 30 arranged between the anode 3 and the cathode 4. The light emitting unit 30 has a light emitting layer 5. A hole transporting zone arranged between the anode 3 and the light emitting layer 5 is formed by a hole injecting layer 6a, a first hole transporting layer 6b, a second hole transporting layer 6c, and a third hole transporting layer 6d. Further, an electron transporting zone arranged between the light emitting layer 5 and the cathode 4 is formed by a first electron transporting layer 7a and a second electron transporting layer 7b.

In the present invention, a host combined with a fluorescent dopant material (a fluorescence emitting material) is called a fluorescent host, and a host combined with a phosphorescent dopant material is called a phosphorescent host. The fluorescent host and the phosphorescent host are not distinguished from each other merely by molecular structures thereof. That is, the phosphorescent host means a material that forms a phosphorescent light emitting layer containing a phosphorescent dopant, but does not mean that the use as a material for forming a fluorescent light emitting layer is not possible. The same also applies to the fluorescent host.

### Substrate

The substrate is used as a support for the organic EL device. As the substrate, for example, a plate of glass, quartz, plastic or the like can be used. Further, a flexible substrate may be used. Examples of the flexible substrate include plastic substrates made of polycarbonate, polyarylate, polyethersulfone, polypropylene, polyester, polyvinyl fluoride, and polyvinyl chloride. Further, an inorganic vapor deposition film can also be used.

### Anode

For the anode to be formed on the substrate, it is desirable to use a metal, an alloy, an electrically conductive compound, a mixture thereof or the like with a large work function (specifically 4.0 eV or more). Specifically, examples thereof include indium oxide-tin oxide (ITO: Indium Tin Oxide), indium oxide-tin oxide containing silicon or silicon oxide, indium oxide-zinc oxide, indium oxide containing tungsten oxide and zinc oxide, and graphene. In addition, gold (Au), platinum (Pt), nickel (Ni), tungsten (W), chromium (Cr), molybdenum (Mo), iron (Fe), cobalt (Co), copper (Cu), palladium (Pd), titanium (Ti), nitrides of the metals (for example, titanium nitride) or the like may be exemplified.

These materials are usually deposited by a sputtering method. For example, indium oxide-zinc oxide, and indium oxide containing tungsten oxide and zinc oxide can be formed through a sputtering method by using a target obtained by adding 1 to 10 wt% of zinc oxide to indium oxide, and a target containing 0.5 to 5 wt% of tungsten oxide and 0.1 to 1 wt% of zinc oxide relative to indium oxide, respectively. In addition, a vacuum deposition method, a coating method, an inkjet method, a spin coating method or the like may be used for the production.

The hole injecting layer formed in contact with the anode is formed by using a material that facilitates hole injection regardless of the work function of the anode. Thus, it is possible to use materials generally used as electrode materials (e.g., metals, alloys, electrically conductive compounds, and mixtures thereof, and elements belonging to Group 1 or Group 2 in the periodic table of elements).

It is also possible to use materials with a small work function, e.g., elements belonging to Group 1 or Group 2 in the periodic table of elements, i.e., alkali metals such as lithium (Li) or cesium (Cs), alkaline earth metals such as magnesium (Mg), calcium (Ca), and strontium (Sr), and alloys containing these (e.g., MgAg, AlLi), and rare earth metals such as europium (Eu), and ytterbium (Yb) and alloys containing these. When the anode is formed by using an alkali metal, an alkaline earth metal, or an alloy containing these, a vacuum deposition method or a sputtering method can be used. Further, when silver paste or the like is used, a coating method, an inkjet method or the like can be used.

### Hole Injecting Layer

The hole injecting layer is a layer containing a material having a high hole injection capability (a hole injecting material), and is formed between the anode and the light emitting layer, or between the hole transporting layer (if this exists) and the anode.

As for the hole injecting material other than the inventive compound, it is possible to use molybdenum oxide, titanium oxide, vanadium oxide, rhenium oxide, ruthenium oxide, chromium oxide, zirconium oxide, hafnium oxide, tantalum oxide, silver oxide, tungsten oxide, manganese oxide, etc.

Examples of the hole injecting layer material include aromatic amine compounds, which are low-molecular weight organic compounds, such as 4,4',4"-tris(N,N-diphenylamino)triphenylamine (abbreviation: TDATA), 4,4',4"-tris[N-(3-methylphenyl)-N-phenylamino]triphenylamine (abbreviation: MTDATA), 4,4'-bis[N-(4-diphenylaminophenyl)-N-phenylamino]biphenyl (abbreviation: DPAB), 4,4'-bis(N-{4-[N'-(3-methylphenyl)-N'-phenylamino]phenyl}-N-phenylamino)biphenyl (abbreviation: DNTPD), 1,3,5-tris[N-(4-diphenylaminophenyl)-N-phenylamino]benzene (abbreviation: DPA3B), 3-[N-(9-phenylcarbazole-3-yl)-N-phenylaminol-9-phenylcarbazole (abbreviation: PCzPCA1), 3,6-bis[N-(9-phenylcarbazole-3-yl)-N-phenylamino]-9-phenylcarbazole (abbreviation: PCzPCA2), and 3-[N-(1-naphthyl)-N-(9-phenylcarbazole-3-yl)amino]-9-phenylcarbazole (abbreviation: PCzPCN1).

High-molecular weight compounds (oligomers, dendrimers, polymers, etc.) can also be used. Examples thereof include high-molecular weight compounds such as poly(N-vinylcarbazole) (abbreviation: PVK), poly(4-vinyltriphenylamine) (abbreviation: PVTPA), poly[N-(4-{N'-[4-(4-diphenylamino)phenyl]phenyl-N'-phenylamino}phenyl)methacrylamide] (abbreviation: PTPDMA), and poly[N,N'-bis(4-butylphenyl)-N,N'-bis(phenyl)benzidine] (abbreviation: Poly-TPD). Further, high-molecular weight compounds to which acids are added, such as poly(3,4-ethylenedioxythiophene)/poly(styrene sulfonic acid) (PEDOT/PSS), and polyaniline/poly(styrene sulfonic acid)(PAni/PSS), can also be used.

Further, it is also preferable to use an acceptor material represented by the following formula (K), such as a hexaazatriphenylene (HAT) compound.

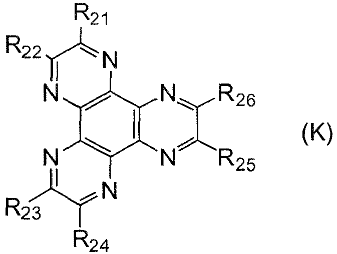

In the formula, R₂₁ to R₂₆ each independently represent a cyano group, - CONH₂, a carboxy group, or -COOR₂₇ (R₂₇ represents an alkyl group having 1 to 20 carbon atoms or a cycloalkyl group having 3 to 20 carbon atoms). Further, adjacent two selected from R₂₁ and R₂₂, R₂₃ and R₂₄, and R₂₅ and R₂₆ may be bonded to each other to form a group represented by -CO-O-CO-.)

Examples of R₂₇ include a methyl group, an ethyl group, a n-propyl group, an isopropyl group, a n-butyl group, an isobutyl group, a t-butyl group, a cyclopentyl group, and a cyclohexyl group.

### Hole Transporting Layer

The hole transporting layer is a layer containing a material with a high hole transporting capability (a hole transporting material), and is formed between the anode and the light emitting layer, or between the hole injecting layer (if this exists) and the light emitting layer. The inventive compound may be used alone or in combination with the following compounds, in the hole transporting layer.

The hole transporting layer may have a single layer structure or a multilayer structure including two or more layers. For example, the hole transporting layer may have a two-layer structure including a first hole transporting layer (anode side) and a second hole transporting layer (cathode side). That is, the hole transporting zone may include the first hole transporting layer on the anode side and the second hole transporting layer on the cathode side. Further, the hole transporting layer may have a three-layer structure including a first hole transporting layer, a second hole transporting layer, and a third hole transporting layer in order from the anode side. That is, the third hole transporting layer may be arranged between the second hole transporting layer and the light emitting layer.

In one embodiment of the present invention, the hole transporting layer with the single layer structure is preferably adjacent to the light emitting layer, and the hole transporting layer in the multilayer structure closest to the cathode, for example, the second hole transporting layer in the two-layer structure, is preferably adjacent to the light emitting layer. Further, for example, the third hole transporting layer in the three-layer structure is preferably adjacent to the light emitting layer.

In another embodiment of the present invention, the electron blocking layer to be described below may be interposed between the hole transporting layer with the single layer structure and the light emitting layer, or between the light emitting layer and the hole transporting layer in the multilayer structure closest to the light emitting layer.

In the hole transporting layer with the two-layer structure, the inventive compound may be contained in either the first hole transporting layer or the second hole transporting layer, or may be contained in both.

In one embodiment of the present invention, the inventive compound is preferably contained in only the first hole transporting layer, in another embodiment, the inventive compound is preferably contained in only the second hole transporting layer, and in yet another embodiment, the inventive compound is preferably contained in the first hole transporting layer and the second hole transporting layer.

In the hole transporting layer with the three-layer structure, the inventive compound may be contained in at least one of the first to third hole transporting layers. That is, the inventive compound may be contained in only one layer selected from the first to third hole transporting layers (only the first hole transporting layer, only the second hole transporting layer, or only the third hole transporting layer), only two layers selected from the first to third hole transporting layers (only the first hole transporting layer and the second hole transporting layer, only the first hole transporting layer and the third hole transporting layer, or only the second hole transporting layer and the third hole transporting layer), or all layers of the first to third hole transporting layers.

In one embodiment of the present invention, the inventive compound contained in either or both of the first hole transporting layer and the second hole transporting layer is preferably a protium compound from the viewpoint of a production cost.

In another embodiment of the present invention, the inventive compound contained in at least one of the first to third hole transporting layers is preferably a protium compound from the viewpoint of a production cost.

The protium compound is the inventive compound in which all hydrogen atoms in the inventive compound are protium atoms.

Therefore, the present invention includes an organic EL device, in which either or both of the first hole transporting layer and the second hole transporting layer contains the inventive compound substantially composed of only the protium compound. "The inventive compound substantially composed of only the protium compound" means that the content of the protium compound in the total amount of the inventive compound is 90 mol% or more, preferably 95 mol% or more, more preferably 99 mol% or more (each including 100%).

Other than the inventive compound, for example, an aromatic amine compound, a carbazole derivative, and an anthracene derivative can be used for the hole transporting layer material.

Examples of the aromatic amine compound include 4,4'-bis[N-(1-naphthyl)-N-phenylamino]biphenyl (abbreviation: NPB) or N,N'-bis(3-methylphenyl)-N,N'-diphenyl-[1,1'-biphenyl]-4,4'-diamine (abbreviation: TPD), 4-phenyl-4'-(9-phenylfluorene-9-yl)triphenylamine (abbreviation: BAFLP), 4,4'-bis[N-(9,9-dimethylfluorene-2-yl)-N-phenylamino]biphenyl (abbreviation: DFLDPBi), 4,4',4"-tris(N,N-diphenylamino)triphenylamine (abbreviation: TDATA), 4,4',4"-tris[N-(3-methylphenyl)-N-phenylamino]triphenylamine (abbreviation: MTDATA), and 4,4'-bis[N-(spiro-9,9'-bifluorene-2-yl)-N-phenylamino]biphenyl (abbreviation: BSPB). The compounds have a hole mobility of 10⁻⁶ cm²/Vs or more.

Examples of the carbazole derivative include 4,4'-di(9-carbazolyl)biphenyl (abbreviation: CBP), 9-[4-(9-carbazolyl)phenyl]-10-phenylanthracene (abbreviation: CzPA), and 9-phenyl-3-[4-(10-phenyl-9-anthryl)phenyl]-9H-carbazole (abbreviation: PCzPA).

Examples of the anthracene derivative include 2-t-butyl-9,10-di(2-naphthyl)anthracene (abbreviation: t-BuDNA), 9,10-di(2-naphthyl)anthracene (abbreviation: DNA), and 9,10-diphenylanthracene (abbreviation: DPAnth).

High-molecular weight compounds such as poly(N-vinylcarbazole) (abbreviation: PVK) or poly(4-vinyltriphenylamine) (abbreviation: PVTPA) can also be used.

Meanwhile, a compound other than those mentioned above may also be used as long as the compound has a hole transporting capability higher than an electron transporting capability.

### Dopant Material of Light Emitting Layer

The light emitting layer is a layer containing a material having a high light emitting property (a dopant material), and various materials can be used. For example, a fluorescence emitting material or a phosphorescence emitting material can be used as the dopant material. The fluorescence emitting material is a compound that emits light from a singlet excited state, and the phosphorescence emitting material is a compound that emits light from a triplet excited state.

As for a blue-based fluorescence emitting material that can be used for the light emitting layer, a pyrene derivative, a styrylamine derivative, a chrysene derivative, a fluoranthene derivative, a fluorene derivative, a diamine derivative, a triarylamine derivative or the like can be used. Specific examples thereof include N,N'-bis[4-(9H-carbazole-9-yl)phenyl]-N,N'-diphenylstilbene-4,4'-diamine (abbreviation: YGA2S), 4-(9H-carbazole-9-yl)-4'-(10-phenyl-9-anthryl)triphenylamine (abbreviation: YGAPA), and 4-(10-phenyl-9-anthryl)-4'-(9-phenyl-9H-carbazole-3-yl)triphenylamine (abbreviation: PCBAPA).

As for a green-based fluorescence emitting material that can be used for the light emitting layer, an aromatic amine derivative or the like can be used. Specific examples thereof include N-(9,10-diphenyl-2-anthryl)-N,9-diphenyl-9H-carbazole-3-amine (abbreviation: 2PCAPA), N-[9,10-bis(1,1'-biphenyl-2-yl)-2-anthryl]-N,9-diphenyl-9H-carbazole-3-amine (abbreviation: 2PCABPhA), N-(9,10-diphenyl-2-anthryl)-N,N',N'-triphenyl-1,4-phenylenediamine (abbreviation: 2DPAPA), N-[9,10-bis(1,1'-biphenyl-2-yl)-2-anthryl]-N,N',N'-triphenyl-1,4-phenylenediamine (abbreviation: 2DPABPhA), N-[9,10-bis(1,1'-biphenyl-2-yl)]-N-[4-(9H-carbazole-9-yl)phenyl]-N-phenylanthracene-2-amine (abbreviation: 2YGABPhA), and N,N,9-triphenylanthracene-9-amine (abbreviation: DPhAPhA).

As for a red-based fluorescence emitting material that can be used for the light emitting layer, a tetracene derivative, a diamine derivative or the like can be used. Specific examples thereof include N,N,N',N'-tetrakis(4-methylphenyl)tetracene-5,11-diamine (abbreviation: p-mPhTD), and 7,14-diphenyl-N,N,N',N'-tetrakis(4-methylphenyl)acenaphtho[1,2-a]fluoranthene-3,10-diamine (abbreviation: p-mPhAFD).

In one embodiment of the present invention, it is desirable that the light emitting layer contains the fluorescence emitting material (fluorescent dopant material).

As for a blue-based phosphorescence emitting material that can be used for the light emitting layer, a metal complex such as an iridium complex, an osmium complex, or a platinum complex is used. Specific examples thereof include bis[2-(4',6'-difluorophenyl)pyridinato-N, C2']iridium(III)tetrakis(1-pyrazolyl)borate (abbreviation: FIr6), bis[2-(4',6'-difluorophenyl)pyridinato-N,C2']iridium(III)picolinate (abbreviation: FIrpic), bis[2-(3',5'bistrifluoromethylphenyl)pyridinato-N,C2']iridium(III)picolinate (abbreviation: Ir(CF3ppy)2(pic)), and bis[2-(4',6'-difluorophenyl)pyridinato-N,C2']iridium(III)acetylacetonate (abbreviation: FIracac).

As for a green-based phosphorescence emitting material that can be used for the light emitting layer, an iridium complex or the like is used. Examples thereof include tris(2-phenylpyridinato-N,C2')iridium(III) (abbreviation: Ir(ppy)3), bis(2-phenylpyridinato-N,C2')iridium(III)acetylacetonate (abbreviation: Ir(ppy)2(acac)), bis(1,2-diphenyl-1H-benzoimidazolato)iridium(III)acetylacetonate (abbreviation: Ir(pbi)2(acac)), and bis(benzo[h]quinolinato)iridium(III)acetylacetonate (abbreviation: Ir(bzq)2(acac)).

As for a red-based phosphorescence emitting material that can be used for the light emitting layer, a metal complex such as an iridium complex, a platinum complex, a terbium complex, or an europium complex is used. Specific examples thereof include organic metal complexes such as bis[2-(2'-benzo[4,5-α]thienyl)pyridinato-N,C3']iridium(III)acetylacetonate(abbreviation: Ir(btp)2(acac)), bis(1-phenylisoquinolinato-N,C2')iridium(III)acetylacetonate (abbreviation: Ir(piq)2(acac)), (acetylacetonate)bis[2,3-bis(4-fluorophenyl) quinoxalinato]iridium(III) (abbreviation: Ir(Fdpq)2(acac)), and 2,3,7,8,12,13,17,18-octaethyl-21H,23H-porphyrin platinum(II) (abbreviation: PtOEP).

Further, rare earth metal complexes such as tris(acetylacetonate)(monophenanthroline)terbium(III) (abbreviation: Tb(acac)3(Phen)), tris(1,3-diphenyl-1,3-propanedionato)(monophenanthroline)europium(III) (abbreviation: Eu(DBM)3(Phen)), and tris[1-(2-thenoyl)-3,3,3-trifluoroacetonato](monophenanthroline)europium(III) (abbreviation: Eu(TTA)3(Phen)) emit light from rare earth metal ions (electron transition between different multiplicities), and thus can be used as the phosphorescence emitting material.

In one embodiment of the present invention, it is preferable that the light emitting layer contains the phosphorescence emitting material (phosphorescent dopant material).

### Host Material for Light Emitting Layer

The light emitting layer may have a configuration in which the above-described dopant material is dispersed in another material (a host material). It is desirable to use a material that has a higher lowest unoccupied orbital level (LUMO level) and a lower highest occupied orbital level (HOMO level) than the dopant material.

Examples of the host material include:
(1) a metal complex such as an aluminum complex, a beryllium complex, or a zinc complex,
(2) a heterocyclic compound such as an oxadiazole derivative, a benzoimidazole derivative, or a phenanthroline derivative,
(3) a fused aromatic compound such as a carbazole derivative, an anthracene derivative, a phenanthrene derivative, a pyrene derivative, or a chrysene derivative, or
(4) an aromatic amine compound such as a triarylamine derivative or a fused polycyclic aromatic amine derivative.

For example, it is possible to use metal complexes such as tris(8-quinolinolato)aluminum(III) (abbreviation: Alq), tris(4-methyl-8-quinolinolato)aluminum(III) (abbreviation: Almq3), bis(10-hydroxybenzo[h]quinolinato)beryllium(II) (abbreviation: BeBq2), bis(2-methyl-8-quinolinolato)(4-phenylphenolato)aluminum(III) (abbreviation: BAlq), bis(8-quinolinolato)zinc(II) (abbreviation: Znq), bis[2-(2-benzooxazolyl)phenolato]zinc(II) (abbreviation: ZnPBO), and bis[2-(2-benzothiazolyl)phenolato]zinc(II) (abbreviation: ZnBTZ);
heterocyclic compounds such as 2-(4-biphenylyl)-5-(4-tert-butylphenyl)-1,3,4-oxadiazole (abbreviation: PBD), 1,3-bis[5-(p-tert-butylphenyl)-1,3,4-oxadiazole 2-yl]benzene (abbreviation: OXD-7), 3-(4-biphenylyl)-4-phenyl-5-(4-tert-butylphenyl)-1,2,4-triazole (abbreviation: TAZ), 2,2',2"-(1,3,5-benzenetriyl)tris(1-phenyl-1H-benzoimidazole) (abbreviation: TPBI), bathophenanthroline(abbreviation: BPhen), and bathocuproine (abbreviation: BCP);
fused aromatic compounds such as 9-[4-(10-phenyl-9-anthryl)phenyl]-9H-carbazole (abbreviation: CzPA), 3,6-diphenyl-9-[4-(10-phenyl-9-anthryl)phenyl]-9H-carbazole (abbreviation: DPCzPA), 9,10-bis(3,5-diphenylphenyl)anthracene (abbreviation: DPPA), 9,10-di(2-naphthyl)anthracene (abbreviation: DNA), 2-tert-butyl-9,10-di(2-naphthyl)anthracene (abbreviation: t-BuDNA), 9,9'-bianthryl (abbreviation: BANT), 9,9'-(stilbene-3,3'-diyl)diphenanthrene (abbreviation: DPNS), 9,9'-(stilbene-4,4'-diyl)diphenanthrene (abbreviation: DPNS2), 3,3',3"-(benzene-1,3,5-triyl)tripyrene (abbreviation: TPB3), 9,10-diphenylanthracene (abbreviation: DPAnth), and 6, 12-dimethoxy-5, 11-diphenylchrysene; and
aromatic amine compounds such as N,N-diphenyl-9-[4-(10-phenyl-9-anthryl)phenyl]-9H-carbazole-3-amine (abbreviation: CzA1PA), 4-(10-phenyl-9-anthryl)triphenylamine (abbreviation: DPhPA), N,9-diphenyl-N-[4-(10-phenyl-9-anthryl)phenyl]-9H-carbazole-3-amine (abbreviation: PCAPA), N,9-diphenyl-N-14-[4-(10-phenyl-9-anthryl)phenyl]phenyl}-9H-carbazole-3-amine (abbreviation: PCAPBA), N-(9,10-diphenyl-2-anthryl)-N,9-diphenyl-9H-carbazole-3-amine (abbreviation: 2PCAPA), 4,4'-bis[N-(1-naphthyl)-N-phenylamino]biphenyl (abbreviation: NPB or α-NPD), N,N'-bis(3-methylphenyl)-N,N'-diphenyl-[1,1'-biphenyl]-4,4'-diamine (abbreviation: TPD), 4,4'-bis[N-(9,9-dimethylfluorene-2-yl)-N-phenylamino]biphenyl (abbreviation: DFLDPBi), and 4,4'-bis[N-(spiro-9,9'-bifluorene-2-yl)-N-phenylamino]biphenyl (abbreviation: BSPB). A plurality of types of host materials may be used.

In particular, in the case of a blue fluorescent device, it is preferable to use the following anthracene compounds as the host material.

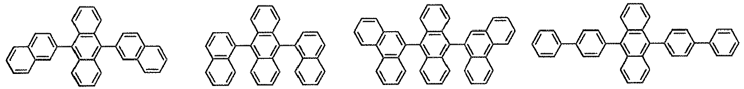

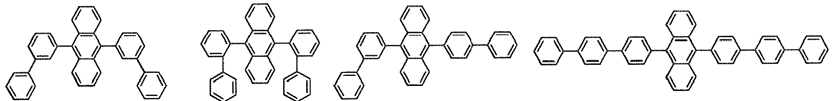

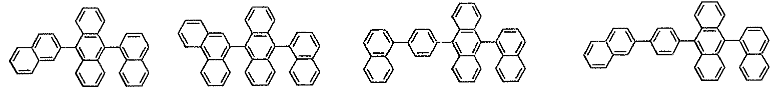

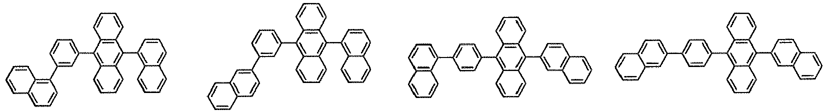

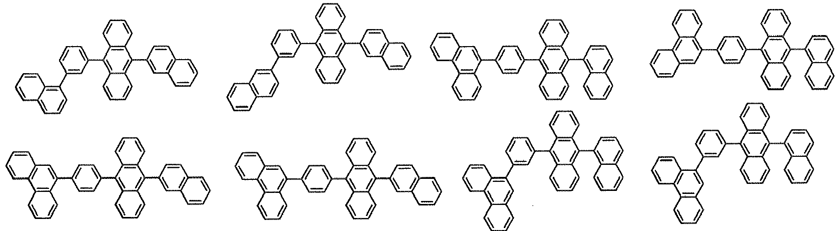

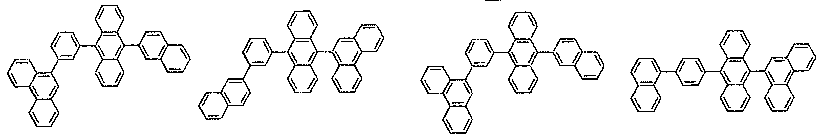

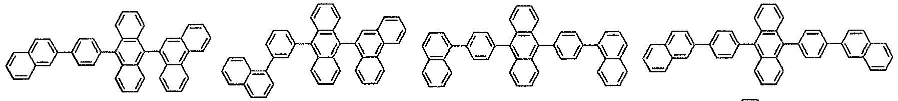

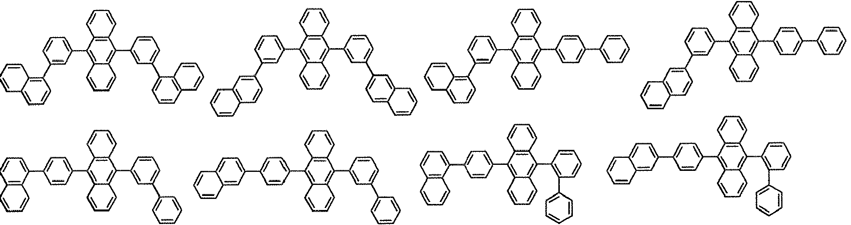

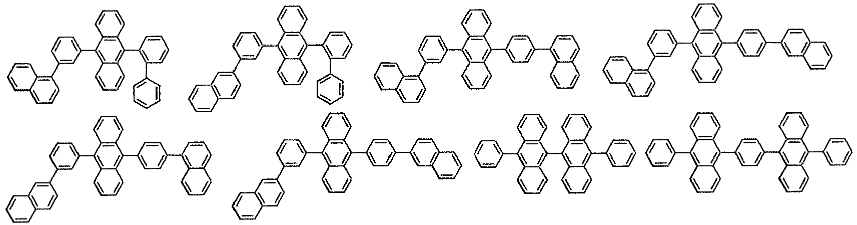

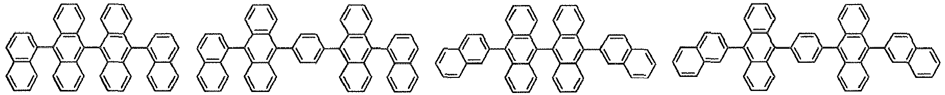

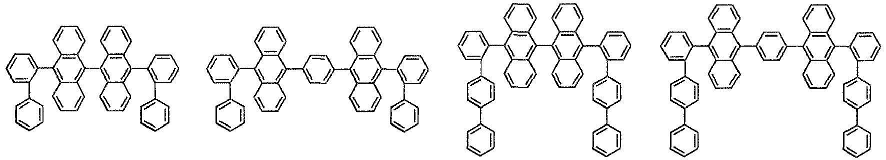

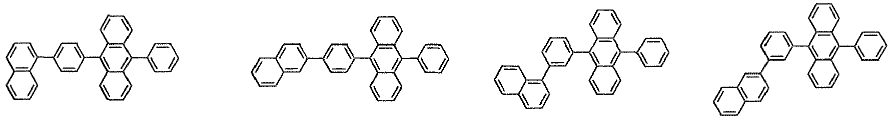

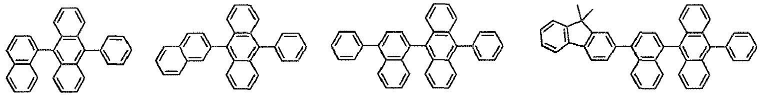

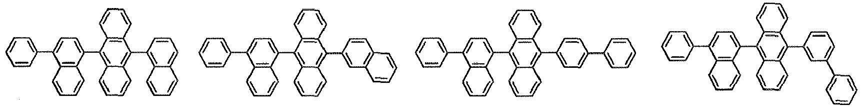

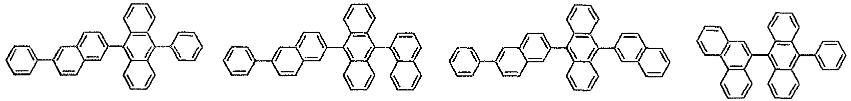

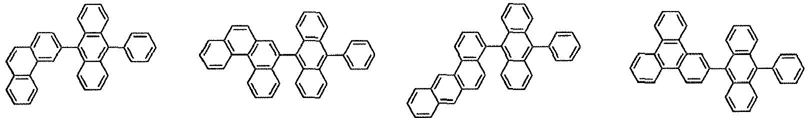

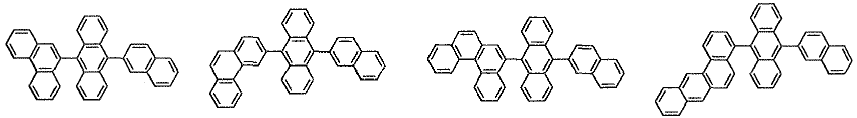

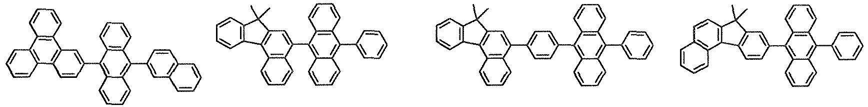

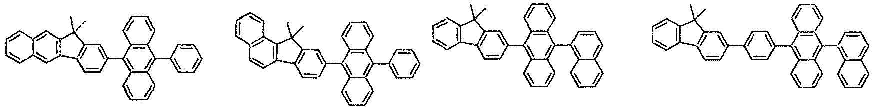

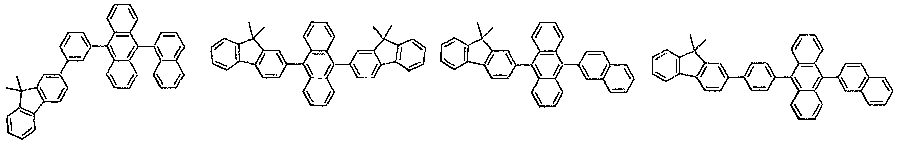

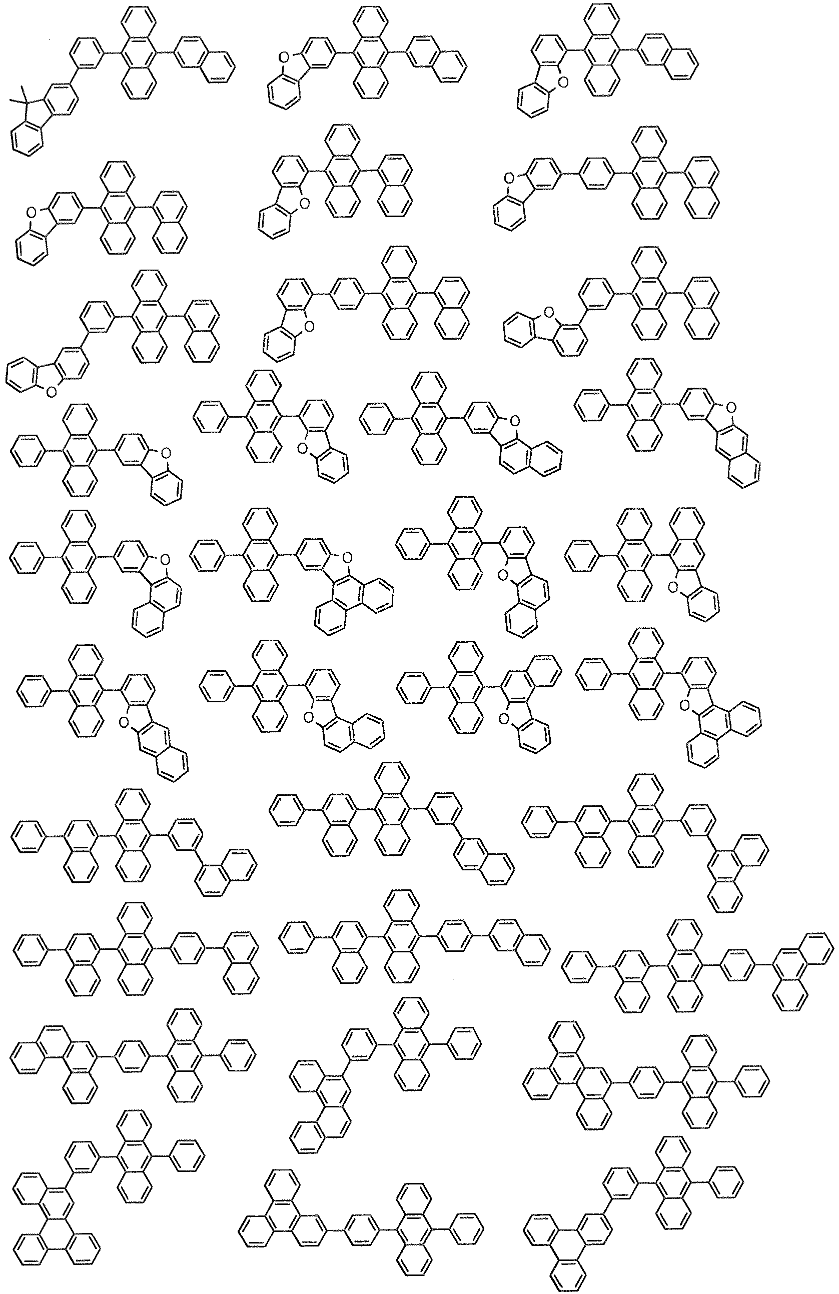

### Electron Transporting Layer

The electron transporting layer is a layer containing a material having a high electron transporting capability (an electron transporting material), and is formed between the light emitting layer and the cathode, or between the light emitting layer and the electron injecting layer (if this exists).

The electron transporting layer may have a single layer structure, or may have a multilayer structure including two or more layers. For example, the electron transporting layer may have a two-layer structure including a first electron transporting layer (anode side) and a second electron transporting layer (cathode side). In one embodiment of the present invention, the electron transporting layer with the single layer structure is preferably adjacent to the light emitting layer, and the electron transporting layer in the multilayer structure closest to the anode, for example, the first electron transporting layer in the two-layer structure, is preferably adjacent to the light emitting layer. In another embodiment of the present invention, a hole blocking layer to be described below may be interposed between the electron transporting layer with the single layer structure and the light emitting layer, or between the light emitting layer and the electron transporting layer in the multilayer structure closest to the light emitting layer.

For the electron transporting layer, for example,
(1) a metal complex such as an aluminum complex, a beryllium complex, or a zinc complex,
(2) a heteroaromatic compound such as an imidazole derivative, a benzoimidazole derivative, an azine derivative, a carbazole derivative, or a phenanthroline derivative, and
(3) a high-molecular weight compound can be used.

Examples of the metal complex include tris(8-quinolinolato)aluminum(III) (abbreviation: Alq), tris(4-methyl-8-quinolinolato)aluminum (abbreviation: Almq3), bis(10-hydroxybenzo[h]quinolinato)beiyllium (abbreviation: BeBq₂), bis(2-methyl-8-quinolinolato)(4-phenylphenolato)aluminum(III) (abbreviation: BAlq), bis(8-quinolinolato)zinc(II) (abbreviation: Znq), bis[2-(2-benzooxazolyl)phenolato]zinc(II) (abbreviation: ZnPBO), and bis[2-(2-benzothiazolyl)phenolato]zinc(II) (abbreviation: ZnBTZ).

Examples of the heteroaromatic compound include 2-(4-biphenylyl)-5-(4-tert-butylphenyl)-1,3,4-oxadiazole (abbreviation: PBD), 1,3-bis[5-(ptert-butylphenyl)-1,3,4-oxadiazole-2-yl]benzene (abbreviation: OXD-7), 3-(4-tert-butylphenyl)-4-phenyl-5-(4-biphenylyl)-1,2,4-triazole (abbreviation: TAZ), 3-(4-tert-butylphenyl)-4-(4-ethylphenyl)-5-(4-biphenylyl)-1,2,4-triazole (abbreviation: p-EtTAZ), bathophenanthroline (abbreviation: BPhen), bathocuproine (abbreviation: BCP), and 4,4'-bis(5-methylbenzooxazole-2-yl)stilbene (abbreviation: BzOs).

Examples of the high-molecular weight compound include poly[(9,9-dihexylfluorene-2, 7-diyl)-co-(pyridine-3, 5-diyl)] (abbreviation: PF-Py), and poly[(9,9-dioctylfluorene-2,7-diyl)-co-(2,2'-bipyridine-6,6'-diyl)] (abbreviation: PF-BPy).

The material is a material having an electron mobility of 10⁻⁶ cm²/Vs or more. A material other than those mentioned above may also be used for the electron transporting layer as long as the material has an electron transporting capability higher than a hole transporting capability.

### Electron Injecting Layer

The electron injecting layer is a layer containing a material with a high electron injection capability. For the electron injecting layer, alkali metals such as lithium (Li), and cesium (Cs), alkaline earth metals such as magnesium (Mg), calcium (Ca), and strontium (Sr), rare earth metals such as europium (Eu), and ytterbium (Yb), and compounds containing these metals can be used. Examples of such compounds include alkali metal oxides, alkali metal halides, alkali metal-containing organic complexes, alkaline earth metal oxides, alkaline earth metal halides, alkaline earth metal-containing organic complexes, rare earth metal oxides, rare earth metal halides, and rare earth metal-containing organic complexes. Further, these compounds can also be mixed and then used.

Besides, a material having an electron transporting capability, in which an alkali metal, an alkaline earth metal, or a compound thereof is contained, specifically, Alq in which magnesium (Mg) is contained, may be used. In this case, electron injection from the cathode can be more efficiently performed.

Otherwise, for the electron injecting layer, a composite material obtained by mixing an organic compound with an electron donor may be used. Such a composite material is excellent in the electron injection capability and the electron transporting capability because the organic compound accepts electrons from the electron donor. In this case, as for the organic compound, a material excellent in transporting accepted electrons is preferred, and specifically, for example, the above-described materials (a metal complex, a heteroaromatic compound, or the like) constituting the electron transporting layer can be used. The electron donor only has to be a material exhibiting an electron donation property for the organic compound. Specifically, alkali metals, alkaline earth metals and rare earth metals are preferred, and lithium, cesium, magnesium, calcium, erbium, ytterbium, etc. may be exemplified. Further, alkali metal oxide or alkaline earth metal oxide is preferred, and lithium oxide, calcium oxide, barium oxide, etc. may be exemplified. Further, Lewis bases such as magnesium oxide can also be used. Further, organic compounds such as tetrathiafulvalene (abbreviation: TTF) can also be used.

### Cathode

For the cathode, it is preferable to use a metal, an alloy, an electrically conductive compound, a mixture thereof or the like which has a small work function (specifically 3.8 eV or less). Specific examples of these cathode materials include elements belonging to Group 1 or Group 2 of the periodic table of elements, i.e., alkali metals such as lithium (Li) or cesium (Cs), alkaline earth metals such as magnesium (Mg), calcium (Ca), and strontium (Sr), and alloys containing these (e.g., MgAg, AlLi), and rare earth metals such as europium (Eu), and ytterbium (Yb) and alloys containing these.

When the cathode is formed by using the alkali metals, the alkaline earth metals, or the alloys containing these, a vacuum deposition method or a sputtering method can be used. Further, when a silver paste or the like is used, a coating method, an inkjet method, or the like can be used.

By providing the electron injecting layer, regardless of the magnitude of the work function, it is possible to form the cathode by using various conductive materials such as Al, Ag, ITO, graphene, and indium oxide-tin oxide containing silicon or silicon oxide. These conductive materials can be deposited by using a sputtering method, an inkjet method, a spin coating method or the like.

### Insulating Layer

In the organic EL device, since an electric field is applied to an ultra-thin film, pixel defects are likely to occur due to leaks or short-circuiting. In order to prevent this, an insulating layer including an insulating thin film layer may be inserted between a pair of electrodes.

As for the material used for the insulating layer, for example, aluminum oxide, lithium fluoride, lithium oxide, cesium fluoride, cesium oxide, magnesium oxide, magnesium fluoride, calcium oxide, calcium fluoride, aluminum nitride, titanium oxide, silicon oxide, germanium oxide, silicon nitride, boron nitride, molybdenum oxide, ruthenium oxide, vanadium oxide etc. may be exemplified. A mixture or a laminate of these may also be used.

### Space Layer

The space layer is, for example, a layer provided between the fluorescent light emitting layer and the phosphorescent light emitting layer for the purpose of preventing excitons generated in the phosphorescent light emitting layer from diffusing into the fluorescent light emitting layer or adjusting a carrier balance in a case where the fluorescent light emitting layer and the phosphorescent light emitting layer are stacked. Further, the space layer can also be provided between phosphorescent light emitting layers.

Since the space layer is provided between the light emitting layers, a material having both an electron transporting capability and a hole transporting capability is preferred. Further, the triplet energy is preferably 2.6 eV or more in order to prevent the diffusion of triplet energy in the adjacent phosphorescent light emitting layer. As for the material used for the space layer, the same as those used for the above-described hole transporting layer may be exemplified.

### Blocking Layer

The blocking layer such as the electron blocking layer, the hole blocking layer, or the exciton blocking layer may be provided adjacent to the light emitting layer. The electron blocking layer is a layer that prevents electrons from leaking from the light emitting layer to the hole transporting layer, and the hole blocking layer is a layer that prevents holes from leaking from the light emitting layer to the electron transporting layer. The exciton blocking layer has a function of preventing excitons generated in the light emitting layer from diffusing into the surrounding layers, and confining the excitons within the light emitting layer.

Each layer of the organic EL device can be formed by a conventionally known vapor deposition method, a coating method, etc. For example, formation can be performed by a known method using a vapor deposition method such as a vacuum deposition method, or a molecular beam vapor deposition method (MBE method), or a coating method in which a solution of a compound for forming a layer is used, such as a dipping method, a spin-coating method, a casting method, a bar-coating method, or a roll-coating method.

The film thickness of each layer is not particularly limited, but is usually 5 nm to 10 µm, more preferably 10 nm to 0.2 µm because in general, when the film thickness is too small, defects such as pinholes are likely to occur, and conversely, when it is too large, a high driving voltage is required and the efficiency becomes poor.

In the organic EL device of the present invention, in which the hole transporting layer with the two-layer structure or the three-layer structure is included, the sum of the thickness of the first hole transporting layer and the thickness of the second hole transporting layer is preferably 30 nm to 150 nm, more preferably 40 nm to 130 nm.

Further, in one embodiment of the present invention, the thickness of the second hole transporting layer in the two-layer structure or the three-layer structure is preferably 5 nm or more, more preferably 20 nm or more, further preferably 25 nm or more, particularly preferably 35 nm or more, and also is preferably 100 nm or less.

Further, in one embodiment of the present invention, the thickness of the hole transporting layer adjacent to the light emitting layer is preferably 5 nm or more, more preferably 10 nm or more, and also is preferably 20 nm or less, more preferably 17 nm or less.

Further, in another embodiment of the present invention, the thickness of the hole transporting layer adjacent to the light emitting layer is preferably 5 nm or more, more preferably 20 nm or more, further preferably 25 nm or more, particularly preferably 30 nm or more, and also is preferably 100 nm or less.

Further, in the organic EL device of the present invention, in which the hole transporting layer with the two-layer structure or the three-layer structure is included, the ratio of the film thickness D2 of the second hole transporting layer to the film thickness D 1 of the first hole transporting layer is preferably 0.3<D2/D1<4.0, more preferably 0.5<D2/D1<3.5, further preferably 0.75<D2/D1<3.0.

As preferred embodiments of the organic EL device of the present invention, for example,
(1) an organic EL device having a hole transporting layer with a two-layer configuration
   · a first embodiment in which the second hole transporting layer contains the inventive compound, and the first hole transporting layer does not contain the inventive compound;
   · a second embodiment in which both the first hole transporting layer and the second hole transporting layer contain the inventive compound; and
   · a third embodiment in which the first hole transporting layer contains the inventive compound, and the second hole transporting layer does not contain the inventive compound; and
(2) an organic EL device having a hole transporting layer with a three-layer configuration
   · a fourth embodiment in which the first hole transporting layer contains the inventive compound, and the second and third hole transporting layers do not contain the inventive compound;
   · a fifth embodiment in which the second hole transporting layer contains the inventive compound, and the first and third hole transporting layers do not contain the inventive compound;
   · a sixth embodiment in which the third hole transporting layer contains the inventive compound, and the first and second hole transporting layers do not contain the inventive compound;
   · a seventh embodiment in which the first and second hole transporting layers contain the inventive compound, and the third hole transporting layer does not contain the inventive compound;
   · an eighth embodiment in which the first and third hole transporting layers contain the inventive compound, and the second hole transporting layer does not contain the inventive compound;
   · a tenth embodiment in which the second and third hole transporting layers contain the inventive compound, and the first hole transporting layer does not contain the inventive compound; and
   · a tenth embodiment in which all of the first to third hole transporting layers contain the inventive compound; may be exemplified.

The organic EL device can be used for electronic devices, e.g., display components such as organic EL panel modules, display devices for televisions, mobile phones, personal computers, etc. and light emitting devices for lightings and vehicular lamps.

### Examples

Hereinafter, the present invention will be described in more detail by using Examples, but the present invention is not limited to the following Examples.

### Inventive Compound used for Production of Organic EL device of Example 1

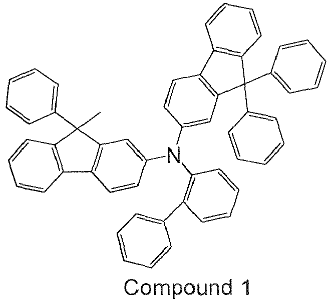

### Comparative Compound used for Production of Organic EL Device of Comparative Example 1

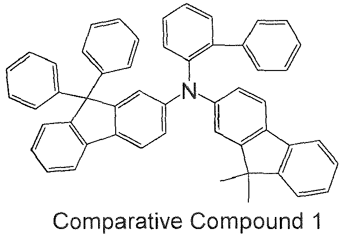

### Other Compounds used for Production of Organic EL Devices of Example 1 and Comparative Example 1

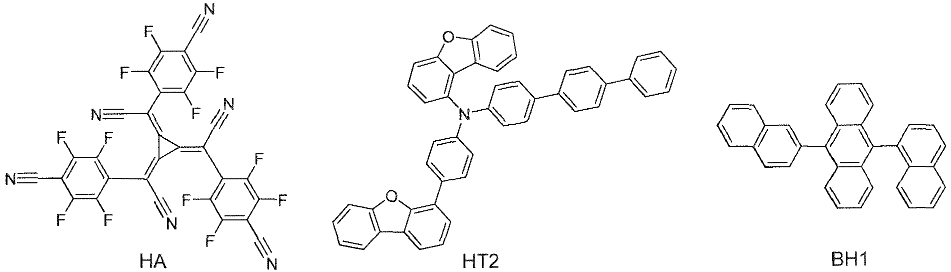

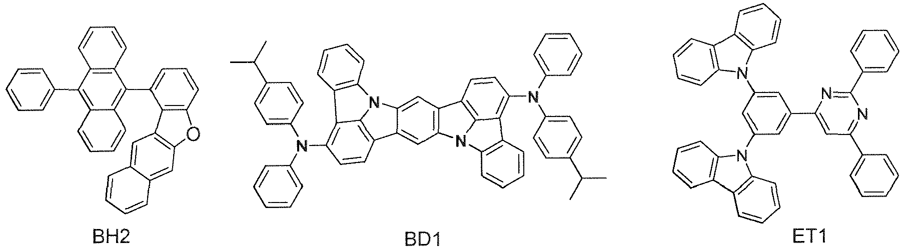

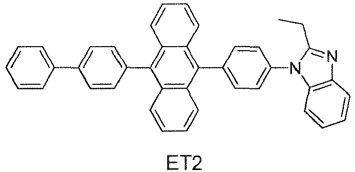

### Production of Organic EL Device (I)

### Example 1

A glass substrate of 25 mm × 75 mm × 1.1 mm with an ITO transparent electrode (anode) (manufactured by GEOMATEC Co., Ltd.) was ultrasonically cleaned in isopropyl alcohol for 5 min, and then was subjected to UV ozone cleaning for 30 min. The film thickness of the ITO was set as 130 nm.

The cleaned glass substrate provided with the transparent electrode was mounted on a substrate holder of a vacuum deposition device. First, Compound 1 and Compound HA were co-deposited on the surface on the side on which the transparent electrode was formed such that the transparent electrode was covered, and then a hole injecting layer with a film thickness of 10 nm was formed. The mass ratio (Compound 1:HA) of Compound 1 to Compound HA was 97:3.

Next, on the hole injecting layer, Compound 1 was vapor-deposited to form a first hole transporting layer with a film thickness of 75 nm.

Next, on the first hole transporting layer, Compound HT2 was vapor-deposited to form a second hole transporting layer with a film thickness of 15 nm.

Next, on the second hole transporting layer, a BH (host material):BD1 (dopant material) film with a film thickness of 20 nm was formed. This BH:BD1 film functions as a light emitting layer. The BH compounds [BH1 and BH2 (both are host materials)] contained in the light emitting layer have a mass ratio of 1:1, and the concentration of BD1 is 2% by mass with respect to the entire light emitting layer.

Next, on the light emitting layer, Compound ET1 was vapor-deposited to form a first electron transporting layer with a film thickness of 3 nm.

Next, on the first electron transporting layer, Compound ET2 and Liq were co-deposited to form a second electron transporting layer with a film thickness of 30 nm. The mass ratio (ET2:Liq) of Compound ET2 to Liq was 67:33.

Next, on the second electron transporting layer, LiF and Yb were co-deposited to form an electron injecting electrode with a film thickness of 1 nm. The mass ratio (LiF:Yb) of LiF to Yb was 50:50.

Then, on the electron injecting electrode, a metal Al was vapor-deposited to form a metal cathode with a film thickness of 50 nm.

The layer configuration of the organic EL device (I) of Example 1 obtained in this manner is illustrated below.
ITO(130)/ Compound 1:HA=97:3 (10)/Compound 1 (75)/ HT2(15)/ BH1:BH2:BD=50:50:2 (25)/ET1 (3)/ET2:Liq=67.33 (30)/LiF:Yb=50:50 (1)/Al (50)

In the layer configuration, numbers in parentheses are film thicknesses (nm), and the ratio is a mass ratio.

### Measurement of External Quantum Efficiency (EQE)

The obtained organic EL device (I) was driven at room temperature with a constant DC current at a current density of 10 mA/cm². The luminance was measured by using a luminance meter (spectroradiometer CS-1000 manufactured by Minolta), and the external quantum efficiency (%) was obtained from the result. The result is noted in Table 1.

### Comparative Example 1

An organic EL device (I) was produced in the same manner as in Example 1 except that a material for the hole injecting layer and the first hole transporting layer was changed to the compound illustrated in Table 1 below instead of Compound 1, and the external quantum efficiency (EQE) was measured. The result is noted in Table 1.

**Table 1**

| | Material for hole injecting layer and first hole transporting layer | EQE(%) @10 mA/cm² |
|---|---|---|
| Example 1 | Compound 1 | 10.14 |
| Comparative Example 1 | Comparative Compound 1 | 9.31 |

As is clear from the results in Table 1, it can be found that the organic EL device containing the inventive compound (Compound 1) is excellent in the external quantum efficiency as compared to the organic EL device containing the comparative compound 1.

### Inventive Compounds used for Production of Organic EL Devices (II) of Examples 2 and 3

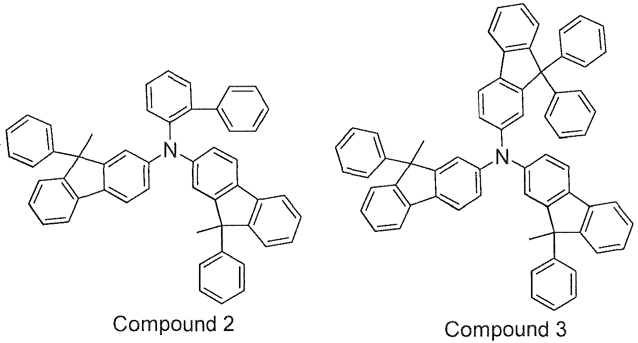

### Comparative Compounds used for Production of Organic EL Devices (II) of Comparative Examples 2 and 3

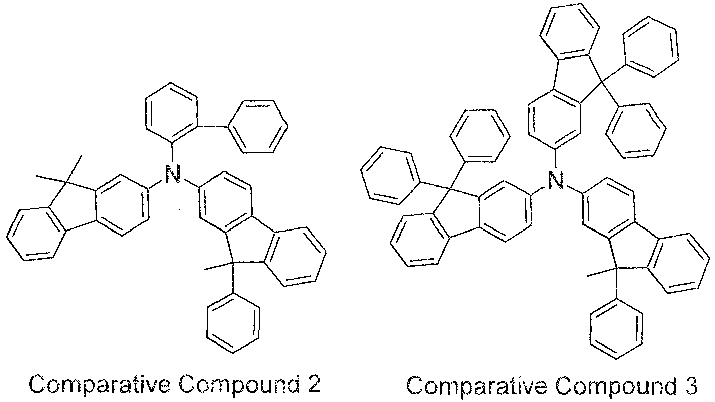

### Other Compounds used for Production of Organic EL Devices (II)

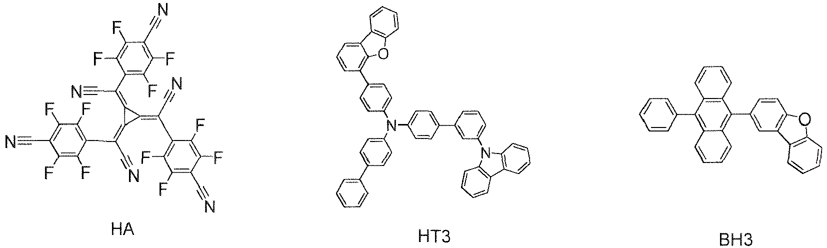

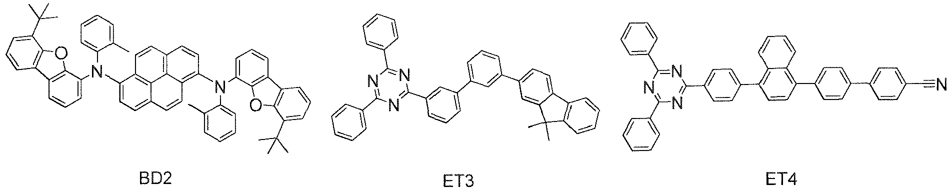

### Production of Organic EL Device (II)

### Example 2

A glass substrate of 25 mm × 75 mm × 1.1 mm with an ITO transparent electrode (anode) (manufactured by GEOMATEC Co., Ltd.) was ultrasonically cleaned in isopropyl alcohol for 5 min, and then was subjected to UV ozone cleaning for 30 min. The film thickness of the ITO was set as 130 nm.

The cleaned glass substrate provided with the transparent electrode was mounted on a substrate holder of a vacuum deposition device. First, Compound 2 and Compound HA were co-deposited on the surface on the side on which the transparent electrode was formed such that the transparent electrode was covered, and then a hole injecting layer with a film thickness of 10 nm was formed. The mass ratio (Compound 2:HA) of Compound 2 to Compound HA was 97:3.

Next, on the hole injecting layer, Compound 2 was vapor-deposited to form a first hole transporting layer with a film thickness of 80 nm.

Next, on the first hole transporting layer, Compound HT3 was vapor-deposited to form a second hole transporting layer with a film thickness of 10 nm.

Next, on the second hole transporting layer, Compound BH3 (host material) and Compound BD2 (dopant material) were co-deposited to form a light emitting layer with a film thickness of 25 nm. The mass ratio (BH3:BD2) of Compound BH3 to Compound BD2 was 96:4.

Next, on the light emitting layer, Compound ET3 was vapor-deposited to form a first electron transporting layer with a film thickness of 5 nm.

Next, on the first electron transporting layer, Compound ET4 and Liq were co-deposited to form a second electron transporting layer with a film thickness of 20 nm. The mass ratio (ET4:Liq) of Compound ET4 to Liq was 50:50.

Next, on the second electron transporting layer, LiF was vapor-deposited to form an electron injecting electrode with a film thickness of 1 nm.

Then, on the electron injecting electrode, a metal Al was vapor-deposited to form a metal cathode with a film thickness of 50 nm.

The layer configuration of the organic EL device (II) of Example 2 obtained in this manner is illustrated below.
ITO (130)/Compound 2:HA=97:3(10)/Compound 2(80)/HT3 (10)/BH3:BD2 = 96:4 (25)]/ET3 (5)/ET4:Liq = 50:50 (20)/Lif (1)/Al (50)

In the layer configuration, numbers in parentheses are film thicknesses (nm), and the ratio is a mass ratio.

### Measurement of 95% Lifetime

The obtained organic EL device (II) was driven with a constant DC current at a current density of 50 mA/cm², and the time (h) until the luminance was reduced to 95% of the initial luminance was measured, and then this was set as 95% lifetime (LT95). The result is noted in Table 2.

### Example 3, and Comparative Examples 2 and 3

Organic EL devices (II) were produced in the same manner as in Example 2 except that a material for the hole injecting layer and the first hole transporting layer was changed to the compounds illustrated in Table 2 below instead of Compound 2, and the 95% lifetime was measured. The results are noted in Table 2.

**Table 2**

| | Material for hole injecting layer and first hole transporting layer | LT95 (h) @50 mA/cm² |
|---|---|---|
| Example 2 | Compound 2 | 140 |
| Example 3 | Compound 3 | 144 |
| Comparative Example 2 | Comparative Compound 2 | 87 |
| Comparative Example 3 | Comparative Compound 1 | 122 |

As is clear from the results in Table 2, it can be found that the organic EL devices containing the inventive compounds (Compounds 2 and 3) have a longer lifetime than the organic EL devices containing the comparative compounds 2 and 3.

### Inventive Compounds used for Production of Organic EL Devices (III) of Examples 4 and 5

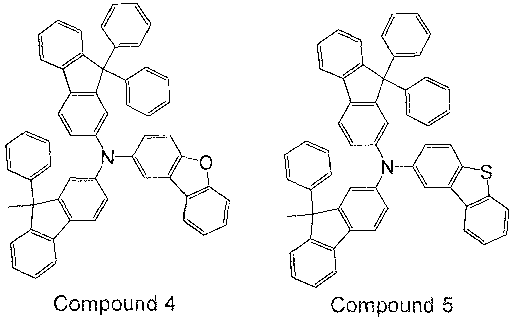

### Comparative Compound used for Production of Organic EL Device (III) of Comparative Example 4

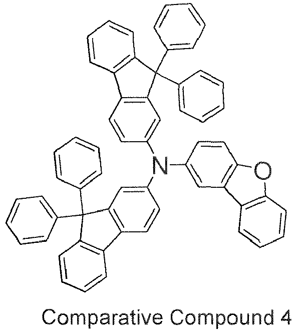

### Other Compounds used for Production of Organic EL Devices (III)

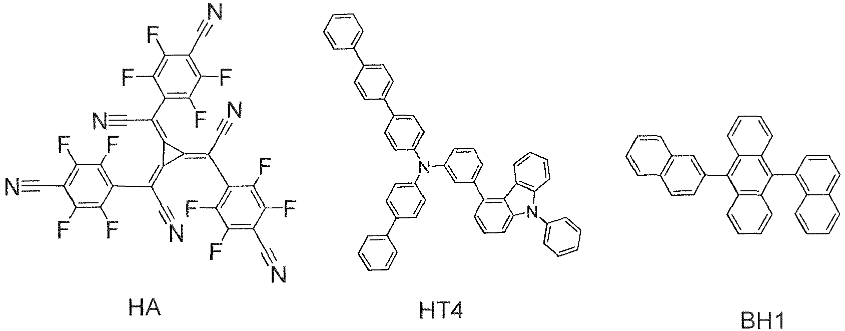

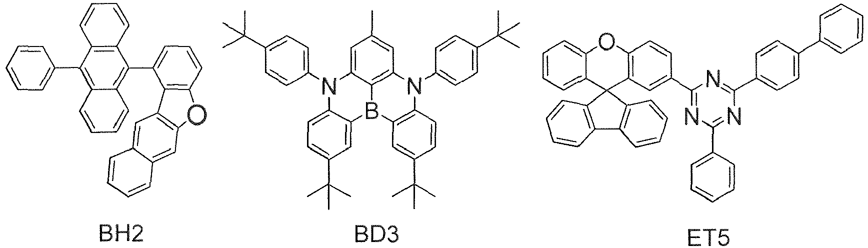

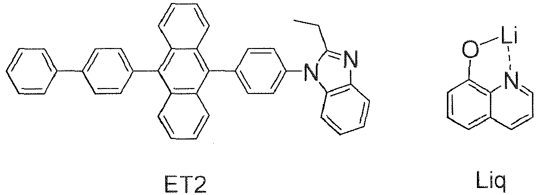

### Production of Organic EL Device (III)

### Example 4

A glass substrate of 25 mm × 75 mm × 1.1 mm with an ITO transparent electrode (anode) (manufactured by GEOMATEC Co., Ltd.) was ultrasonically cleaned in isopropyl alcohol for 5 min, and then was subjected to UV ozone cleaning for 30 min. The film thickness of the ITO was set as 130 nm.

The cleaned glass substrate provided with the transparent electrode was mounted on a substrate holder of a vacuum deposition device. First, Compound 4 and Compound HA were co-deposited on the surface on the side on which the transparent electrode was formed such that the transparent electrode was covered, and then a hole injecting layer with a film thickness of 10 nm was formed. The mass ratio (Compound 4:HA) of Compound 4 to Compound HA was 97:3.

Next, on the hole injecting layer, Compound 4 was vapor-deposited to form a first hole transporting layer with a film thickness of 75 nm.

Next, on the first hole transporting layer, Compound HT4 was vapor-deposited to form a second hole transporting layer with a film thickness of 15 nm.

Next, on the second hole transporting layer, a BH (host material):BD3(dopant material) film with a film thickness of 20 nm was formed. This BH:BD3 film functions as a light emitting layer. The BH compounds [BH1 and BH2 (both are host materials)] contained in the light emitting layer have a mass ratio of 3:2, and the concentration of BD3 is 2% by mass with respect to the entire light emitting layer.

Next, on the light emitting layer, Compound ET5 was vapor-deposited to form a first electron transporting layer with a film thickness of 3 nm.

Next, on the first electron transporting layer, Compound ET2 and Liq were co-deposited to form a second electron transporting layer with a film thickness of 30 nm. The mass ratio (ET2:Liq) of Compound ET2 to Liq was 50:50.

Next, on the second electron transporting layer, LiF and Yb were co-deposited to form an electron injecting electrode with a film thickness of 1 nm. The mass ratio (LiF:Yb) of LiF to Yb was 50:50.

Then, on the electron injecting electrode, a metal Al was vapor-deposited to form a metal cathode with a film thickness of 50 nm.

The layer configuration of the organic EL device (III) of Example 4 obtained in this manner is illustrated below.

ITO (130)/Compound4:HA=97:3 (10)/Compound 4 (75)/HT4 (15)/BH1:BH2:BD3=60:40:2 (20)/ET5 (3)/ET2:Liq=50:50 (30)/LiF:Yb=50=50(1)/Al (50)

In the layer configuration, numbers in parentheses are film thicknesses (nm), and the ratio is a mass ratio.

### Measurement of External Quantum Efficiency (EQE)

The external quantum efficiency (%) of the obtained organic EL device (III) was obtained in the same manner as in Example 1. The result is noted in Table 3.

### Example 5 and Comparative Example 4

Organic EL devices (III) were produced in the same manner as in Example 4 except that a material for the hole injecting layer and the first hole transporting layer was changed to the compounds illustrated in Table 3 below instead of Compound 4, and the external quantum efficiency (EQE) was measured. The results are noted in Table 3.

**Table 3**

| | Material for hole injecting layer and first hole transporting layer | EQE(%) @10 mA/cm² |
|---|---|---|
| Example 4 | Compound 4 | 10.26 |
| Example 5 | Compound 5 | 10.31 |
| Comparative Example 4 | Comparative Compound 4 | 9.44 |

As is clear from the results in Table 3, it can be found that the organic EL devices containing the inventive compounds (Compounds 4 and 5) are excellent in the external quantum efficiency as compared to the organic EL device containing the comparative compound 4.

### Inventive Compounds used for Production of Organic EL Devices (IV) of Examples 6 and 7

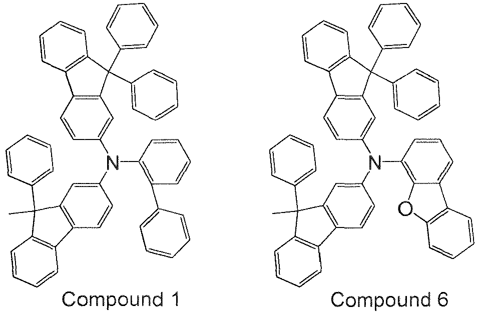

### Comparative Compound used for Production of Organic EL Device (IV) of Comparative Example 5

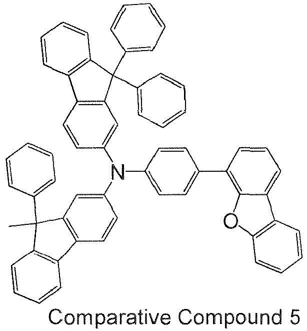

### Other Compounds used for producing Organic EL Devices (IV)

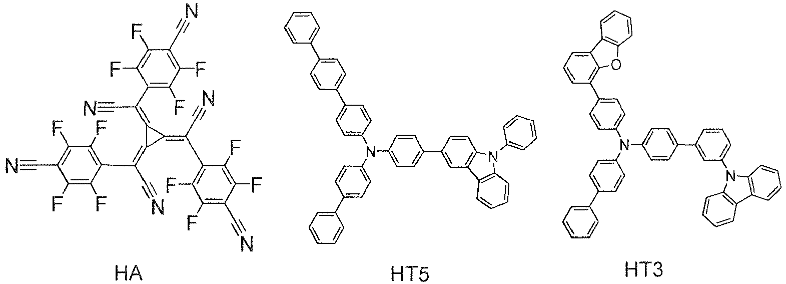

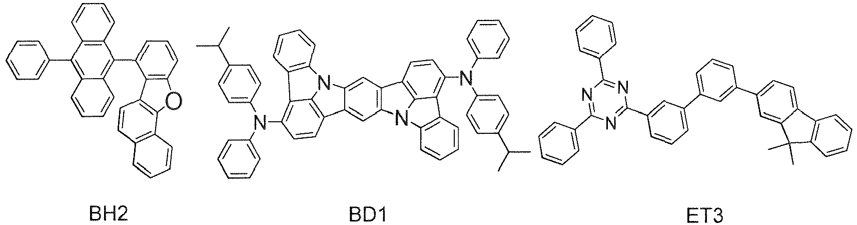

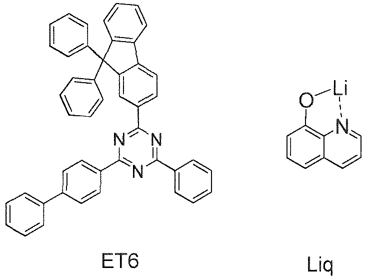

### Production of Organic EL Device (IV)

### Example 6

A glass substrate of 25 mm × 75 mm × 1.1 mm with an ITO transparent electrode (anode) (manufactured by GEOMATEC Co., Ltd.) was ultrasonically cleaned in isopropyl alcohol for 5 min, and then was subjected to UV ozone cleaning for 30 min. The film thickness of the ITO was set as 130 nm.

The cleaned glass substrate provided with the transparent electrode was mounted on a substrate holder of a vacuum deposition device. First, Compound HT5 and Compound HA were co-deposited on the surface on the side on which the transparent electrode was formed such that the transparent electrode was covered, and then a hole injecting layer with a film thickness of 10 nm was formed. The mass ratio (Compounds HT5:HA) of Compound HT5 to Compound HA was 97:3.

Next, on the hole injecting layer, Compound HT5 was vapor-deposited to form a first hole transporting layer with a film thickness of 40 nm.

Next, on the first hole transporting layer, Compound 1 was vapor-deposited to form a second hole transporting layer with a film thickness of 40 nm.

Next, on the second hole transporting layer, Compound HT3 was vapor-deposited to form a third hole transporting layer with a film thickness of 5 nm.

Next, on the third hole transporting layer, Compound BH4 (host material) and Compound BD1 (dopant material) were co-deposited to form a light emitting layer with a film thickness of 20 nm. The mass ratio(BH4:BD1) of Compound BH4 to Compound BD1 was 99:1.

Next, on the light emitting layer, Compound ET3 was vapor-deposited to form a first electron transporting layer with a film thickness of 5 nm.

Next, on the first electron transporting layer, Compound ET6 and Liq were co-deposited to form a second electron transporting layer with a film thickness of 25 nm. The mass ratio (ET6:Liq) of Compound ET6 to Liq was 50:50.

Next, on the second electron transporting layer, Yb was vapor-deposited to form an electron injecting electrode with a film thickness of 1 nm.

Then, on the electron injecting electrode, a metal Al was vapor-deposited to form a metal cathode with a film thickness of 50 nm.

The layer configuration of the organic EL device (IV) of Example 6 obtained in this manner is illustrated below.
ITO (130)/HT5:HA=97:3 (10)/HT5 (40)/Compound 1 (40)/HT3 (5)/BH4:BD1=99:1 (20) /ET3 (5)/ET6:Liq=50:50 (25)/Yb (1)/Al (50)

In the layer configuration, numbers in parentheses are film thicknesses (nm), and the ratio is a mass ratio.

### Measurement of External Quantum Efficiency (EQE)

The external quantum efficiency (%) of the obtained organic EL device (IV) was obtained in the same manner as in Example 1. The result is noted in Table 4.

### Example 7 and Comparative Example 5

Organic EL devices (IV) were produced in the same manner as in Example 6 except that a material for the second hole transporting layer was changed to the compounds illustrated in Table 4 below instead of Compound 1, and the external quantum efficiency (EQE) was measured. The results are noted in Table 4.

**Table 4**

| | Second hole transporting layer material | EQE(%) @10 mA/cm² |
|---|---|---|
| Example 6 | Compound 1 | 10.92 |
| Example 7 | Compound 6 | 11.36 |
| Comparative Example 5 | Comparative Compound 5 | 9.88 |

As is clear from the results in Table 4, it can be found that the organic EL devices containing the inventive compounds (Compounds 1 and 6) are excellent in the external quantum efficiency as compared to the organic EL device containing the comparative compound 5.

### Inventive Compounds synthesized in Synthesis Examples

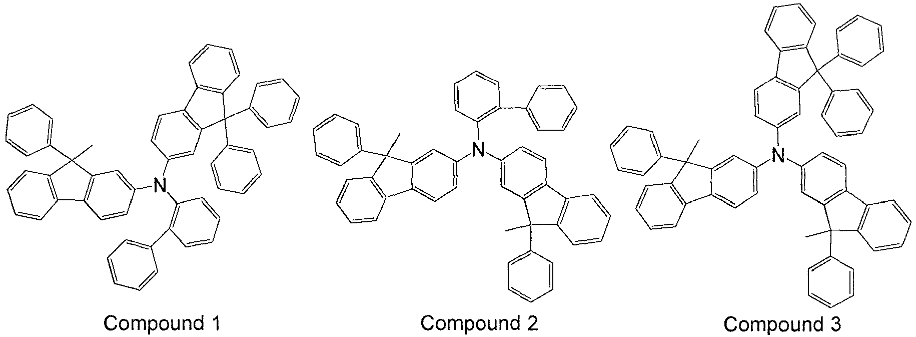

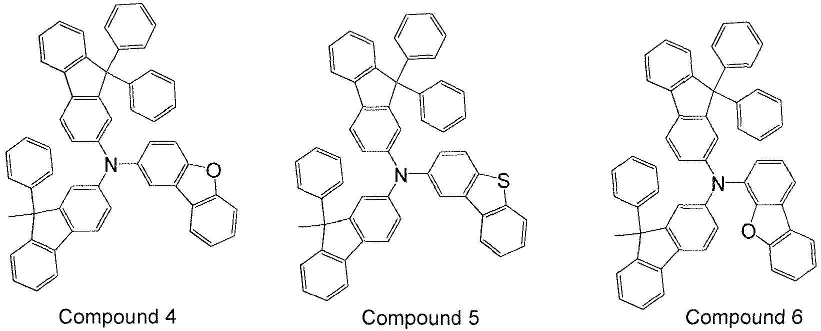

### Intermediate Synthesis Example 1: Synthesis of Intermediate A

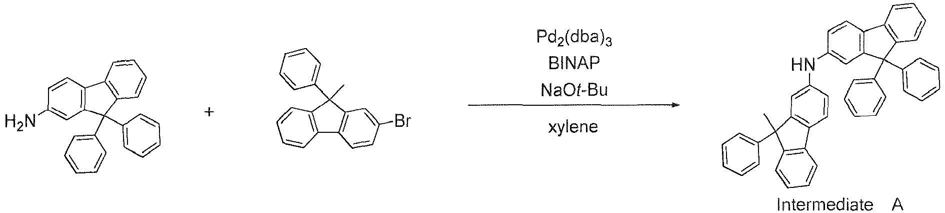

Under an argon atmosphere, a mixture of 29.2 g of 2-amino-9,9-diphenylfluorene (87.6 mmol), 28.8 g of 2-bromo-9-methyl-9-phenyl-9H-fluorene (85.9 mmol), 1.57 g of tris(dibenzylideneacetone)dipalladium(0) (1.71 mmol), 2.14 g of BINAP (3.44 mmol), 20.6 g of sodium-t-butoxide (214 mmol), and 580 mL of xylene was stirred at 120°C for 5 h. The reaction solution was cooled to room temperature, and concentrated under reduced pressure. The resulting residue was purified by silica gel column chromatography and recrystallization to obtain 15.1 g of Intermediate A as a white solid. The yield was 30%.

### Intermediate Synthesis Example 2: Synthesis of Intermediate B

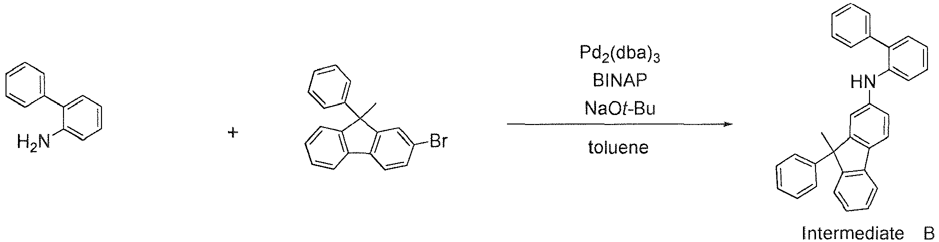

Under an argon atmosphere, a mixture of 6.77 g of 2-aminobiphenyl (40.0 mmol), 13.4 g of 2-bromo-9-methyl-9-phenyl-9H-fluorene (40.0 mmol), 0.730 g of tris(dibenzylideneacetone)dipalladium(0) (0.797 mmol), 1.00 g of BINAP (1.61 mmol), 4.23 g of sodium-t-butoxide ( 44.0 mmol), and 200 mL of toluene was stirred at 90°C for 7 h. The reaction solution was cooled to room temperature, and concentrated under reduced pressure. The resulting residue was purified by silica gel column chromatography to obtain 16.6 g of Intermediate B as a white solid. The yield was 97%.

### Synthesis Example 1: Synthesis of Compound 1

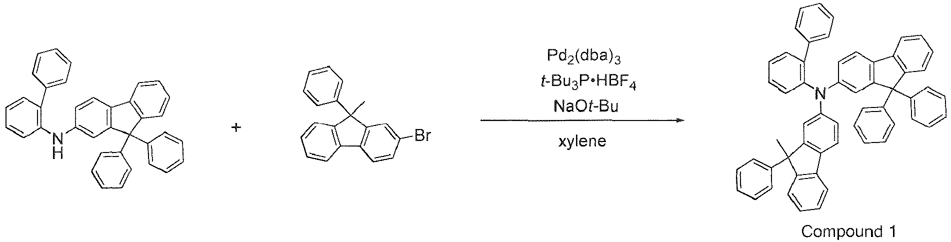

Under an argon atmosphere, a mixture of 7.28 g of N-[1,1'-biphenyl]-2-yl-9,9-diphenyl-9H-fluorene-2-amine (15.0 mmol), 6.03 g of 2-bromo-9-methyl-9-phenyl-9H-fluorene (18.0 mmol), 0.275 g of tris(dibenzylideneacetone)dipalladium(0) (0.30 mmol), 0.348 g of tri-t-butylphosphonium tetrafluoroborate (1.20 mmol), 2.02 g of sodium-t-butoxide (21.0 mmol), and 150 mL of xylene was stirred at 120 °C for 7 h. The reaction solution was cooled to room temperature, and concentrated under reduced pressure. The resulting residue was purified by silica gel column chromatography and recrystallization to obtain 5.20 g of a white solid. The yield was 47%.

The resulting product was Compound 1 in the result of mass spectroscopy, and m/e was 740 for a molecular weight of 739.96.

### Synthesis Example 2: Synthesis of Compound 2

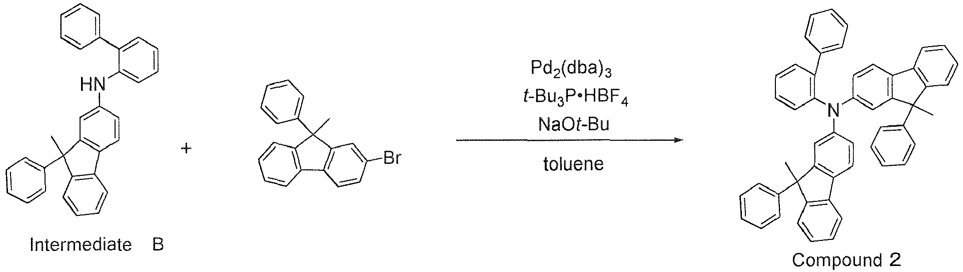

A white solid was obtained by performing the same operation as in Synthesis Example 1 except that Intermediate B and toluene were used instead of N-[1,1'-biphenyl]-2-yl-9,9-diphenyl-9H-fluorene-2-amine and xylene, respectively. The yield was 46%.

The resulting product was Compound 2 in the result of mass spectroscopy (m/e=678 for a molecular weight of 677.89).

### Synthesis Example 3: Synthesis of Compound 3

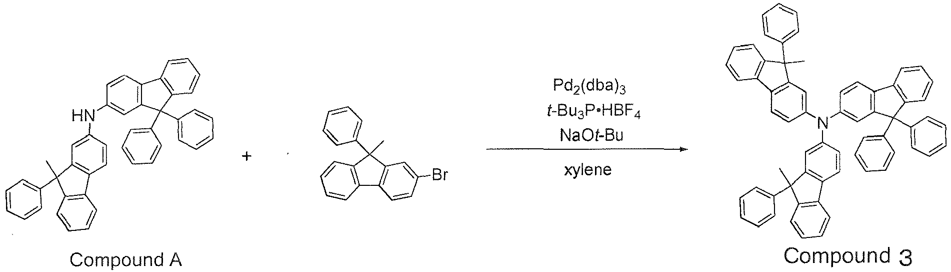

A white solid was obtained by performing the same operation as in Synthesis Example 1 except that Intermediate A was used instead of N-[1,1'-biphenyl]-2-yl-9,9-diphenyl-9H-fluorene-2-amine, The yield was 44%.

The resulting product was Compound 3 in the result of mass spectroscopy (m/e=842 for a molecular weight of 842.10).

### Synthesis Example 4: Synthesis of Compound 4

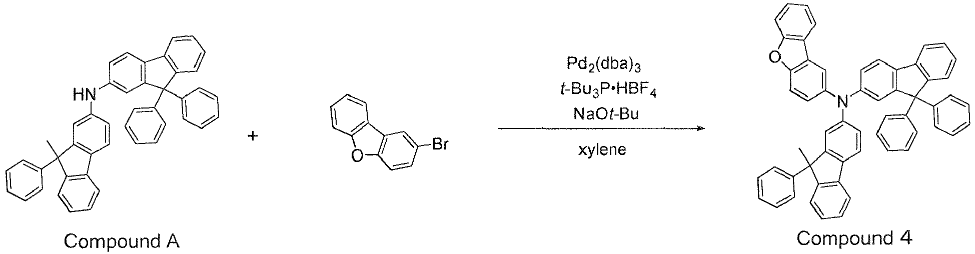

A white solid was obtained by performing the same operation as in synthesis example 1 except that Intermediate A was used instead of N-[1,1'-biphenyl]-2-yl-9,9-diphenyl-9H-fluorene-2-amine, and 2-bromodibenzofuran was used instead of 2-bromo-9-methyl-9-phenyl-9H-fluorene. The yield was 39%.

The resulting product was Compound 4 in the result of mass spectroscopy (m/e=754 for a molecular weight of 753.95).

### Synthesis Example 5: Synthesis Compound 5

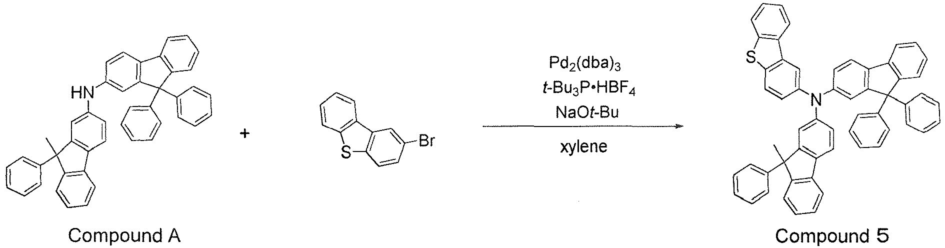

A white solid was obtained by performing the same operation as in Synthesis Example 1 except that Intermediate A and 2-bromodibenzothiophene were used instead of N-[1,1'-biphenyl]-2-yl-9,9-diphenyl-9H-fluorene-2-amine, and 2-bromo-9-methyl-9-phenyl-9H-fluorene, respectively. The yield was 43%.

The resulting product was Compound 5 in the result of mass spectroscopy (m/e=770 for a molecular weight of 770.01).

### Synthesis Example 6: Synthesis of Compound 6

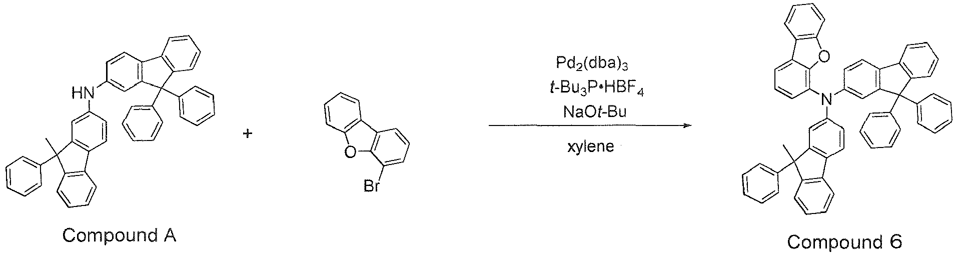

A white solid was obtained by performing the same operation as in Synthesis Example 1 except that Intermediate A was used instead of N-[1,1'-biphenyl]-2-yl-9,9-diphenyl-9H-fluorene-2-amine, and 4-bromodibenzofuran was used instead of 2-bromo-9-methyl-9-phenyl-9H-fluorene. The yield was 48%.

The resulting product was Compound 6 in the result of mass spectroscopy (m/e=754 for a molecular weight of 753.95).

### Reference Signs List

1, 11, 12: Organic EL device
2: Substrate
3: Anode
4: Cathode
5: Light emitting layer
6: Hole transporting zone (hole transporting layer)
6a: Hole injecting layer
6b: First hole transporting layer
6c: Second hole transporting layer
6d: Third hole transporting layer
7: Electron transporting zone (electron transporting layer)
7a: First electron transporting layer
7b: Second electron transporting layer
10, 20, 30: Light emitting unit

## Claims

1. A compound represented by the following formula (1): 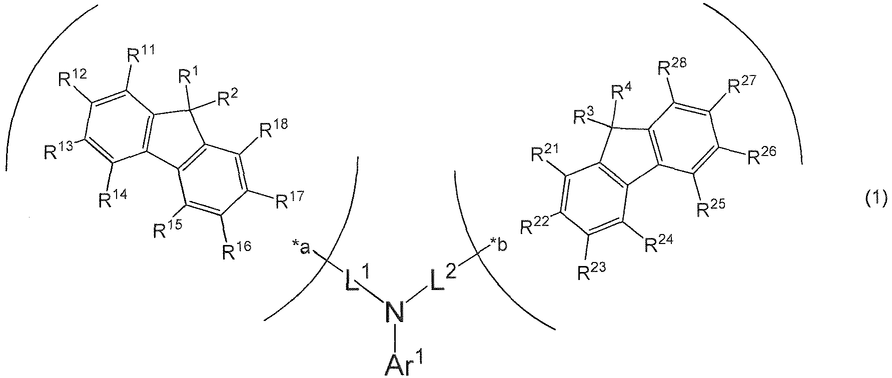 wherein
one of R¹ and R² is a methyl group, and the other is an unsubstituted phenyl group, and
R¹ and R² are not bonded to each other and therefore do not form a ring structure,
one of R³ and R⁴ is a methyl group, and the other is an unsubstituted phenyl group, and
R³ and R⁴ are not bonded to each other and therefore do not form a ring structure,
R¹¹ to R¹⁸, and R²¹ to R²⁸ are hydrogen atoms.
provided that,
one selected from R¹¹ to R¹⁸ is a single bond bonded to *a, and
one selected from R²¹ to R²⁸ is a single bond bonded to *b,
L¹ and L² are each independently a single bond, or a substituted or unsubstituted phenylene group,
Ar¹ is a group represented by any one of formulas (1-1) to (1-6): 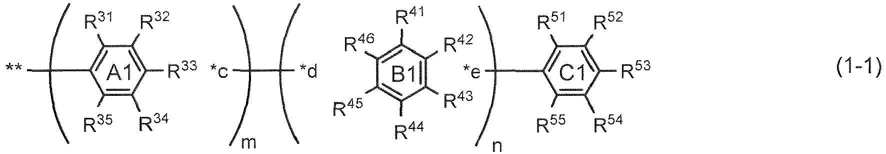 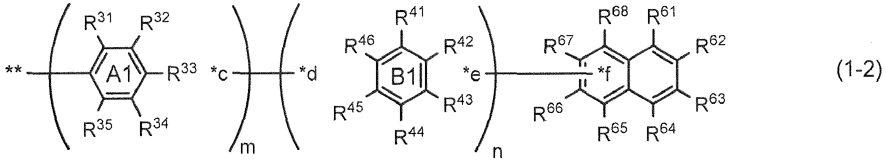 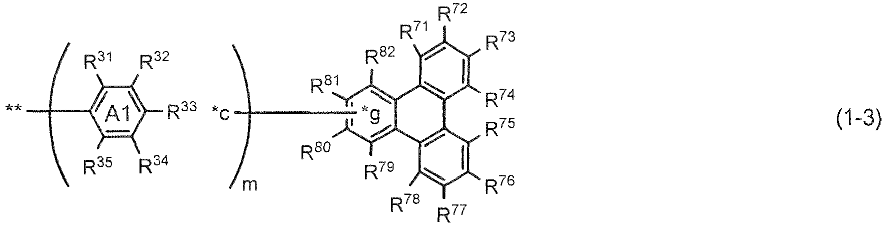 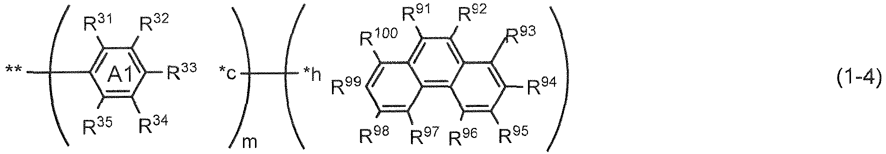 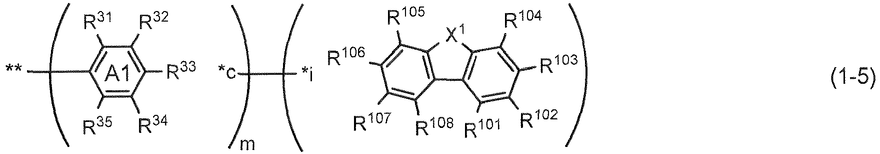 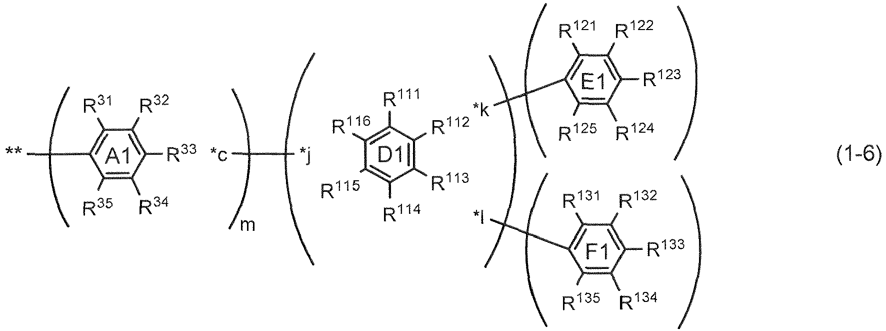 wherein,
R³¹ to R³⁵, R⁴¹ to R⁴⁶, R⁵¹ to R⁵⁵, R⁶¹ to R⁶⁸, R⁷¹ to R⁸², R⁹¹ to R¹⁰⁰, R¹⁰¹ to R¹⁰⁸, R¹¹¹ to R¹¹⁶, R¹²¹ to R¹²⁵, and R¹³¹ to R¹³⁵ are each independently a hydrogen atom, a substituted or unsubstituted alkyl group having 1 to 50 carbon atoms, a substituted or unsubstituted cycloalkyl group having 3 to 50 ring carbon atoms, a halogen atom, a cyano group, a nitro group, a substituted or unsubstituted aryl group having 6 to 50 ring carbon atoms, or a substituted or unsubstituted heterocyclic group having 5 to 50 ring atoms.
X¹ is an oxygen atom, a sulfur atom, or CR^{a}R^{b}, and
R^{a} and R^{b} are each independently a hydrogen atom, a methyl group, or a substituted or unsubstituted phenyl group, and R^{a} and R^{b} may be bonded to each other to form a substituted or unsubstituted ring.
provided that,
one selected from R³¹ to R³⁵ is a single bond bonded to *c,
one selected from R⁴¹ to R⁴⁶ is a single bond bonded to *d, and the other one selected from R⁴¹ to R⁴⁶ is a single bond bonded to *e,
one selected from R⁶⁵ to R⁶⁸ is a single bond bonded to *f,
one selected from R⁷⁹ to R⁸² is a single bond bonded to *g,
one selected from R⁹¹, and R⁹⁷ to R¹⁰⁰ is a single bond bonded to *h,
one selected from R¹⁰⁵ to R¹⁰⁸ is a single bond bonded to *i,
one selected from R¹¹¹ to R¹¹⁶ is a single bond bonded to *j, the other one selected from R¹¹¹ to R¹¹⁶ is a single bond bonded to *k, and the other one selected from R¹¹¹ to R¹¹⁶ is a single bond bonded to *l,
adjacent two selected from R³¹ to R³⁵ that are not the single bond, adjacent two selected from R⁴¹ to R⁴⁶ that are not the single bond, adjacent two selected from R⁵¹ to R⁵⁵, adjacent two selected from R⁶¹ to R⁶⁸ that are not the single bond, adjacent two selected from R⁷¹ to R⁸² that are not the single bond, adjacent two selected from R⁹¹ to R¹⁰⁰ that are not the single bond, adjacent two selected from R¹⁰¹ to R¹⁰⁸ that are not the single bond, adjacent two selected from R¹¹¹ to R¹¹⁶ that are not the single bond, adjacent two selected from R¹²¹ to R¹²⁵, and adjacent two selected from R¹³¹ to R¹³⁵ are not bonded to each other and therefore do not form ring structures,
the benzene ring A1 and the benzene ring B1, the benzene ring A1 and the benzene ring C1, the benzene ring A1 and the benzene ring D1, the benzene ring A1 and the benzene ring E1, the benzene ring A1 and the benzene ring F1, the benzene ring B1 and the benzene ring C1, the benzene ring D1 and the benzene ring E1, and the benzene ring D1 and the benzene ring F1 are not crosslinked,
** represents a bonding position to the central nitrogen atom N,
m is 0 or 1, and n is 0 or 1,
in the formulas (1-1) and (1-2), when m is 0 and n is 0, *e is bonded to the central nitrogen atom N, when m is 0 and n is 1, *c is bonded to the central nitrogen atom N, and when m is 1 and n is 0, *e is bonded to one selected from R³¹ to R³⁵,
in the formulas (1-3) to (1-6), when m is 0, *c is bonded to the central nitrogen atom N, and
when R¹² or R¹⁷ is a single bond bonded to *a, and R²² or R²⁷ is a single bond bonded to *b, the formula (1-1) excludes a p-biphenyl group.

2. The compound according to claim 1, wherein one selected from R¹², R¹³, R¹⁴, R¹⁵, R¹⁶, and R¹⁷ is a single bond bonded to *a.

3. The compound according to claim 1 or 2, wherein one selected from R¹² and R¹⁷ is a single bond bonded to *a.

4. The compound according to any one of claims 1 to 3, wherein one selected from R²², R²³, R²⁴, R²⁵, R²⁶, and R²⁷ is a single bond bonded to *b.

5. The compound according to any one of claims 1 to 4, wherein one selected from R²² and R²⁷ is a single bond bonded to *b.

6. The compound according to any one of claims 1 to 5, wherein L¹ and L² are single bonds.

7. The compound according to any one of claims 1 to 6, wherein Ar¹ is a group represented by any one of the formulas (1-1), (1-2), (1-5), and (1-6).

8. The compound according to any one of claims 1 to 7, wherein R³¹ to R³⁵, R⁴¹ to R⁴⁶, R⁵¹ to R⁵⁵, R⁶¹ to R⁶⁸, R⁷¹ to R⁸², R⁹¹ to R¹⁰⁰, R¹⁰¹ to R¹⁰⁸, R¹¹¹ to R¹¹⁶, R¹²¹ to R¹²⁵, and R¹³¹ to R¹³⁵ are each independently a hydrogen atom, a substituted or unsubstituted alkyl group having 1 to 50 carbon atoms, a substituted or unsubstituted aryl group having 6 to 50 ring carbon atoms, or a substituted or unsubstituted heterocyclic group having 5 to 50 ring atoms.

9. The compound according to any one of claims 1 to 8, wherein R³¹ to R³⁵ that are not a single bond bonded to *c are all hydrogen atoms.

10. The compound according to any one of claims 1 to 9, wherein R⁴¹ to R⁴⁶ that are not the single bond bonded to *d and the single bond bonded to *e are all hydrogen atoms.

11. The compound according to any one of claims 1 to 10, wherein R⁵¹ to R⁵⁵ are all hydrogen atoms.

12. The compound according to any one of claims 1 to 11, wherein R⁶¹ to R⁶⁴, and R⁶⁵ to R⁶⁸ that are not the single bond bonded to *f are all hydrogen atoms.

13. The compound according to any one of claims 1 to 12, wherein R⁷¹ to R⁷⁸, and R⁷⁹ to R⁸² that are not the single bond bonded to *g are all hydrogen atoms.

14. The compound according to any one of claims 1 to 13, wherein R⁹² to R⁹⁶, and R⁹¹ and R⁹⁷ to R¹⁰⁰ that are not the single bond bonded to *h are all hydrogen atoms.

15. The compound according to any one of claims 1 to 14, wherein R¹⁰¹ to R¹⁰⁴, and R¹⁰⁵ to R¹⁰⁸ that are not the single bond bonded to *i are all hydrogen atoms.

16. The compound according to any one of claims 1 to 15, wherein R¹¹¹ to R¹¹⁶ that are not the single bond bonding to *j, are not the single bond bonding to *k, and are not the single bond bonding to *l, and R¹²¹ to R¹²⁵, and R¹³¹ to R¹³⁵ are all hydrogen atoms.

17. The compound according to any one of claims 1 to 16, wherein in the formula (1-1), m or n is 0.

18. The compound according to any one of claims 1 to 17, wherein the compound represented by the formula (1) contains at least one deuterium atom.

19. A compound represented by the following formula (2): 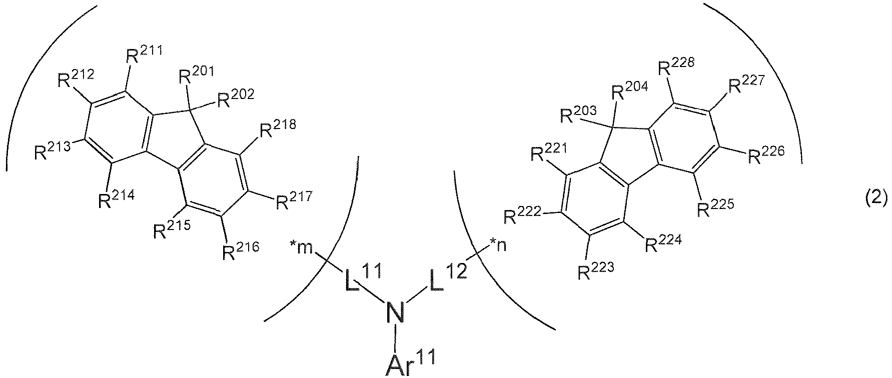 wherein,
one selected from R²⁰¹ to R²⁰⁴ is a methyl group, and the other three selected from R²⁰¹ to R²⁰⁴ are unsubstituted phenyl groups,
R²⁰¹ and R²⁰² are not bonded to each other and therefore do not form a ring structure, and
R²⁰³ and R²⁰⁴ are not bonded to each other and therefore do not form a ring structure.
R²¹¹ to R²¹⁸, and R²²¹ to R²²⁸ are hydrogen atoms.
provided that,
one selected from R²¹¹ to R²¹⁸ is a single bond bonded to *m, and
one selected from R²²¹ to R²²⁸ is a single bond bonded to *n.
L¹¹ and L¹² are each independently a single bond, or a substituted or unsubstituted phenylene group.
Ar¹¹ is a group represented by any one of formulas (2-1) to (2-6): 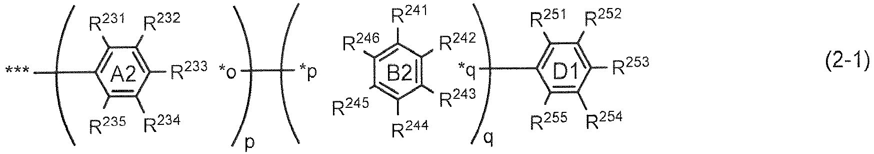 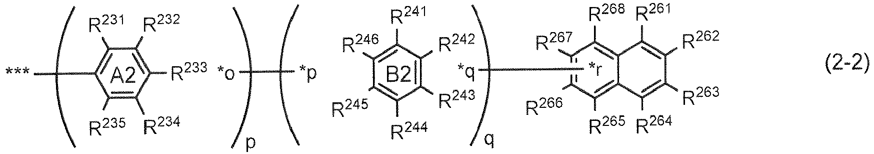 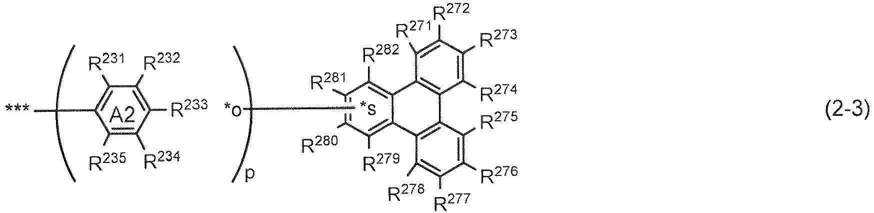 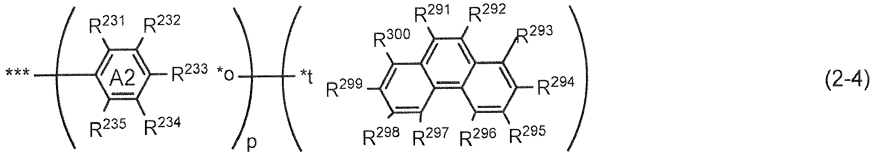 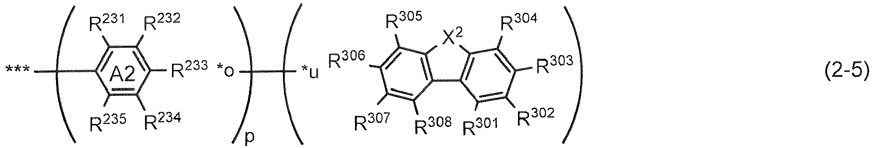 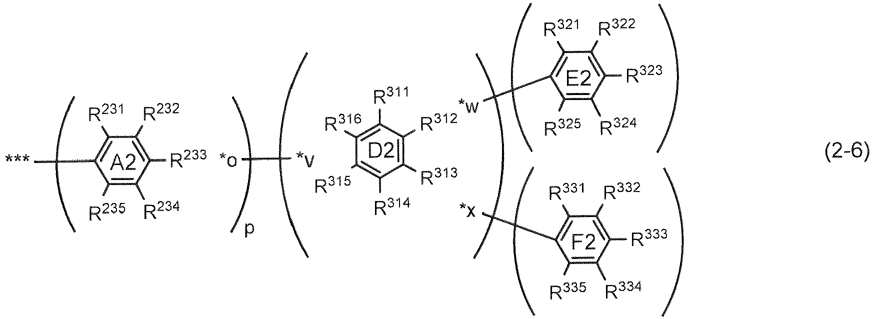
wherein,
R²³¹ to R²³⁵, R²⁴¹ to R²⁴⁶, R²⁵¹ to R²⁵⁵, R²⁶¹ to R²⁶⁸, R²⁷¹ to R²⁸², R²⁹¹ to R³⁰⁰, R³⁰¹ to R³⁰⁸, R³¹¹ to R³¹⁶, R³²¹ to R³²⁵, and R³³¹ to R³³⁵ are each independently a hydrogen atom, a substituted or unsubstituted alkyl group having 1 to 50 carbon atoms, a substituted or unsubstituted cycloalkyl group having 3 to 50 ring carbon atoms, a halogen atom, a cyano group, a nitro group, a substituted or unsubstituted aryl group having 6 to 50 ring carbon atoms, or a substituted or unsubstituted heterocyclic group having 5 to 50 ring atoms.
X² is an oxygen atom, a sulfur atom, or CR^{c}R^{d}; and
R^{c} and R^{d} are each independently a hydrogen atom, or a methyl group, and R^{c} and R^{d} are not bonded to each other and therefore do not form a ring structure.
provided that,
one selected from R²³¹ to R²³⁵ is a single bond bonded to *o,
one selected from R²⁴¹ to R²⁴⁶ is a single bond bonded to *p, and the other one selected from R²⁴¹ to R²⁴⁶ is a single bond bonded to *q,
one selected from R²⁶⁵ to R²⁶⁸ is a single bond bonded to *r,
one selected from R²⁷⁹ to R²⁸² is a single bond bonded to *s,
one selected from R²⁹¹, and R²⁹⁷ to R³⁰⁰ is a single bond bonded to *t,
one selected from R³⁰⁵ to R³⁰⁸ is a single bond bonded to *u,
one selected from R³¹¹ to R³¹⁶ is a single bond bonded to *v, the other one selected from R³¹¹ to R³¹⁶ is a single bond bonded to *w, and the other one selected from R³¹¹ to R³¹⁶ is a single bond bonded to *x,
adjacent two selected from R²³¹ to R²³⁵ that are not the single bond, adjacent two selected from R²⁴¹ to R²⁴⁶ that are not the single bond, adjacent two selected from R²⁵¹ to R²⁵⁵, adjacent two selected from R²⁶¹ to R²⁶⁸ that are not the single bond, adjacent two selected from R²⁷¹ to R²⁸² that are not the single bond, adjacent two selected from R²⁹¹ to R³⁰⁰ that are not the single bond, adjacent two selected from R³⁰¹ to R³⁰⁸ that are not the single bond, adjacent two selected from R³¹¹ to R³¹⁶ that are not the single bond, adjacent two selected from R³²¹ to R³²⁵, and adjacent two selected from R³³¹ to R³³⁵ are not bonded to each other and therefore do not form ring structures,
the benzene ring A2 and the benzene ring B2, the benzene ring A2 and the benzene ring C2, the benzene ring A2 and the benzene ring D2, the benzene ring A2 and the benzene ring E2, the benzene ring A2 and the benzene ring F2, the benzene ring B2 and the benzene ring C2, the benzene ring D2 and the benzene ring E2, and the benzene ring D2 and the benzene ring F2 are not crosslinked,
*** represents a bonding position to the central nitrogen atom N,
p is 0 or 1, and q is 0 or 1,
in the formulas (2-1) and (2-2), when p is 0 and q is 0, *q is bonded to the central nitrogen atom N, when p is 0 and q is 1, *o is bonded to the central nitrogen atom N, and when p is 1 and q is 0, *q is bonded to one selected from R²³¹ to R²³⁵,
in the formulas (2-3) to (2-6), when p is 0, *o is bonded to the central nitrogen atom N,
in the formula (2-5), when X² is CR^{c}R^{d} and p is 0, at least one selected from L¹¹ and L¹² is a substituted or unsubstituted phenylene group,
in the formula (2-5), when X² is an oxygen atom and p is 1, one selected from R³⁰⁶ to R³⁰⁸ is a single bond bonded to *u, and
when R²¹² or R²¹⁷ is a single bond bonded to *m, and R²²² or R²²⁷ is a single bond bonded to *n, the formula (2-1) excludes a p-biphenyl group.

20. The compound according to claim 19, wherein one selected from R²¹², R²¹³, R²¹⁴, R²¹⁵, R²¹⁶, and R²¹⁷ is a single bond bonded to *m.

21. The compound according to claim 19 or 20, wherein one selected from R²¹² and R²¹⁷ is a single bond bonded to *m.

22. The compound according to any one of claims 19 to 21, wherein one selected from R²²², R²²³, R²²⁴, R²²⁵, R²²⁶, and R²²⁷ is a single bond bonded to *n.

23. The compound according to any one of claims 19 to 22, wherein one selected from R²²² and R²²⁷ is a single bond bonded to *n.

24. The compound according to any one of claims 19 to 23, wherein L¹¹ and L¹² are single bonds.

25. The compound according to any one of claims 19 to 24, wherein Ar¹¹ is a group represented by any one of the formulas (2-1), (2-2), (2-5), and (2-6).

26. The compound according to any one of claims 19 to 25, wherein R²³¹ to R²³⁵, R²⁴¹ to R²⁴⁶, R²⁵¹ to R²⁵⁵, R²⁶¹ to R²⁶⁸, R²⁷¹ to R²⁸², R²⁹¹ to R³⁰⁰, R³⁰¹ to R³⁰⁸, R³¹¹ to R³¹⁶, R³²¹ to R³²⁵, and R³³¹ to R³³⁵ are each independently a hydrogen atom, a substituted or unsubstituted alkyl group having 1 to 50 carbon atoms, a substituted or unsubstituted aryl group having 6 to 50 ring carbon atoms, or a substituted or unsubstituted heterocyclic group having 5 to 50 ring atoms.

27. The compound according to any one of claims 19 to 26, wherein R²³¹ to R²³⁵ that are not the single bond bonded to *o are all hydrogen atoms.

28. The compound according to any one of claims 19 to 27, wherein R²⁴¹ to R²⁴⁶ that are not the single bond bonded to *p and are not the single bond bonded to *q are all hydrogen atoms.

29. The compound according to any one of claims 19 to 28, wherein R²⁵¹ to R²⁵⁵ are all hydrogen atoms.

30. The compound according to any one of claims 19 to 29, wherein R²⁶¹ to R²⁶⁴, and R²⁶⁵ to R²⁶⁸ that are not the single bond bonded to *r are all hydrogen atoms.

31. The compound according to any one of claims 19 to 30, wherein R²⁷¹ to R²⁷⁸, and R²⁷⁹ to R²⁸² that are not the single bond bonded to *s are all hydrogen atoms.

32. The compound according to any one of claims 19 to 31, wherein R²⁹² to R²⁹⁶, and R²⁹¹ and R²⁹⁷ to R³⁰⁰ that are not the single bond bonded to *t are all hydrogen atoms.

33. The compound according to any one of claims 19 to 32, wherein R³⁰¹ to R³⁰⁴, and R³⁰⁵ to R³⁰⁸ that are not the single bond bonded to *u are all hydrogen atoms.

34. The compound according to any one of claims 19 to 33, wherein R³¹¹ to R³¹⁶ that are not the single bond bonded to *v, are not the single bond bonded to *w, and are not the single bond bonded to *x, and R³²¹ to R³²⁵, and R³³¹ to R³³⁵ are all hydrogen atoms.

35. The compound according to any one of claims 19 to 34, wherein in the formula (2-1), m or n is 0.

36. The compound according to any one of claims 19 to 35, wherein the compound represented by the formula (2) contains at least one deuterium atom.

37. A material for an organic electroluminescent device, which comprises the compound according to any one of claims 1 to 36.

38. An organic electroluminescent device comprising a cathode, an anode, and organic layers between the cathode and the anode,
wherein in the organic electroluminescent device, the organic layers include a light emitting layer, and at least one layer of the organic layers contains the compound according to any one of claims 1 to 36.

39. The organic electroluminescent device according to claim 38, wherein the organic layers include a hole transporting zone between the anode and the light emitting layer, and the hole transporting zone contains the compound.

40. The organic electroluminescent device according to claim 39, wherein the hole transporting zone includes a first hole transporting layer on the anode side and a second hole transporting layer on the cathode side, and either or both of the first hole transporting layer and the second hole transporting layer contains the compound.

41. The organic electroluminescent device according to claim 40, wherein the first hole transporting layer contains the compound.

42. The organic electroluminescent device according to claim 40 or 41, wherein the second hole transporting layer is adjacent to the light emitting layer.

43. The organic electroluminescent device according to claim 39, wherein the hole transporting zone includes a first hole transporting layer, a second hole transporting layer and a third hole transporting layer in order from the anode side, and only one layer selected from the first to third hole transporting layers, only two layers selected from the first to third hole transporting layers, or all layers of the first to third hole transporting layers contain the compound.

44. The organic electroluminescent device according to claim 43, wherein the light emitting layer and the third hole transporting layer are in direct contact with each other.

45. The organic electroluminescent device according to any one of claims 38 to 44, wherein the light emitting layer contains a fluorescent dopant material.

46. The organic electroluminescent device according to any one of claims 38 to 44, wherein the light emitting layer contains a phosphorescent dopant material.

47. An electronic device comprising the organic electroluminescent device according to any one of claims 38 to 46.
